# EUROPEAN PATENT APPLICATION

(11) **EP 2 662 095 A1**
(43) Date of publication of application: **13.11.2013**
(21) Application number: 13167338.6
(22) Date of filing: 15.04.2011
(51) Int. Cl.: A61K 47/48

(54) **Pyrrolobenzodiazepines and conjugates thereof**

(30) Priority: 15.04.2010 GB 201006341; 06.10.2010 GB 201016802
(62) Divisional of application: 11716146.3
(71) Applicant: Spirogen Sàrl, St-Légier-La Chiésaz (CH)
(72) Inventor: Howard, Philip Wilson, London, Greater London WC1N 1AX (GB); Masterson, Luke, London, Greater London WC1N 1AX (GB); Tiberghien, Arnaud, London, Greater London WC1N 1AX (GB); Flygare, John A, San Francisco, CA California 94080 (US); Gunzner, Janet L, San Francisco, CA California 94080 (US); Polakis, Paul, San Francisco, CA California 94080 (US); Polson, Andrew, San Francisco, CA California 94080 (US); Raab, Helga E, San Francisco, CA California 94080 (US); Spencer, Susan D, San Francisco, CA California 94080 (US)
(74) Representative: Watson, Robert James

(57) **Abstract**

Conjugates and compounds for making conjugates which are PBD molecules linked via the N10 position are disclosed, along with the use of the conjugates for treating proliferative diseases, including cancer.

## Description

The present invention relates to pyrrolobenzodiazepines (PBDs), in particular pyrrolobenzodiazepines having a labile N10 protecting group, in the form of a linker to a cell binding agent.

### Background to the invention

### Pyrrolobenzodiazepines

Some pyrrolobenzodiazepines (PBDs) have the ability to recognise and bond to specific sequences of DNA; the preferred sequence is PuGPu. The first PBD antitumour antibiotic, anthramycin, was discovered in 1965 (Leimgruber, et al., J. Am. Chem. Soc., 87, 5793-5795 (1965); Leimgruber, et al., J. Am. Chem. Soc., 87,5791-5793 (1965)). Since then, a number of naturally occurring PBDs have been reported, and over 10 synthetic routes have been developed to a variety of analogues (Thurston, et al., Chem, Rev. 1994, 433-465 (1994)). Family members include abbeymycin (Hochlowski, et al., J. Antibiotics, 40, 145-148 (1987)), chicamycin (Konishi, et al., J. Antibiotics, 37, 20D-206 (1984)), DC-81 (Japanese Patent 58-180 487; Thurston, et al., Chem. Brit., 26, 767-772 (1990); Bose, et al., Tetrahedron, 48, 751-758 (1992)), mazethramycin (Kuminoto, et al., J. Antibiotics, 33, 665-667 (1980)), neothramycins A and B (Takeuchi, et al., J. Antibiotics, 29, 93-96 (1976)), porothramycin (Tsunakawa, et al., J. Antibiotics, 41, 1366-1373 (1988)), prothracarcin (Shimizu, et al, J. Antibiotics, 29, 492-2503 (1982); Langley and Thurston, J. Org. Chem., 52, 91-97 (1987)), sibanomicin (DC-102)(Hara, et al., J. Antibiotics, 41, 702-704 (1988); Itoh, et al., J. Antibiotics, 41, 1281-1284 (1988)), sibiromycin (Leber, et al., J. Am. Chem. Soc-, 110, 2992-2993 (1988)) and tomamycin (Arima, et al., J. Antibiotics, 25, 437-444 (1972)). PBDs are of the general structure:

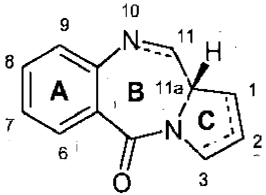

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

The present inventors have previously disclosed in WO 2005/085251, dimeric PBD compounds bearing C2 aryl substituents, such as:

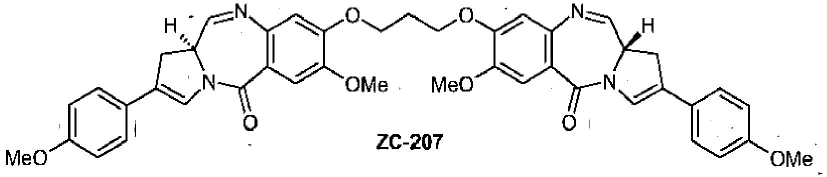

These compounds have been shown to be highly useful cytotoxic agents.

A particularly advantageous pyrrolobenzodiazepine compound is described by Gregson et al. (Chem. Common. 1999, 797-798) as compound **1,** and by Gregson et al. (J. Med. Chem. 2001, 44, 1161-1174) as compound **4a.** This compound, also known as SJG-1 36, is shown below:

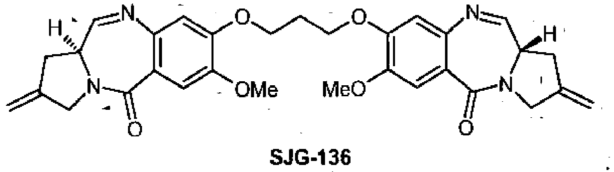

The present inventors have previously disclosed that PBD compounds can be employed as prodrugs by protecting them at the N10 position with a nitrogen protecting group which is removable *in vivo* (WO 00/12507). Many of these protecting groups are carbamates, and are, for example, of the structure: 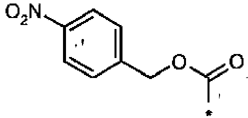 where the asterisk (*) indicates the attachment point to the N10 atom of the PBD.

The present inventors have also described the preparation of PBD compounds having a nitrogen carbamate protecting group at the N10 position (WO 2005/023814). The protecting groups are removable from the N10 position of the PBD moiety to leave an N10-C11 imine bond. A range of protecting groups is described, including groups that can be cleaved by the action of enzymes.

WO 2007/085930 describes the preparation of dimer PBD compounds having linker groups for connection to a cell binding agent, such as an antibody. The linker is present in the bridge linking the monomer PBD units of the dimer.

### Antibody-drug conjugates,

Antibody therapy has been established for the targeted treatment of patients with cancer, immunological and angiogenic disorders (Carter, P. (2006) Nature Reviews Immunology 6:343-357). The use of antibody-drug conjugates (ADC), i.e. immunoconjugates, for the local delivery of cytotoxic or cytostatic agents, i.e. drugs to kill or inhibit tumor cells in the treatment of cancer, targets delivery of the drug moiety to tumors, and intracellular accumulation therein, whereas systemic administration of these unconjugated drug agents may result in unacceptable levels of toxicity to normal cells as well as the tumor cells sought to be eliminated (Xie et al (2006) Expert. Opin. Biol. Ther. 6(3):281-291; Kovtun et al (2006) Cancer Res. 66(6):3214-3121; Law et al (2006) Cancer Res. 66(4):2328-2337; Wu et al (2005) Nature Biotech. 23(9): 1137-1145; Lambert J. (2005) Current Opin. in Pharmacol. 5:543-549; Hamann P. (2005) Expert Opin. Ther. Patents 15(9):1087-1103; Payne, G. (2003) Cancer Cell 3:207-212; Trail et al (2003) Cancer Immunol. Immunother. 52:328-337; Syrigos and Epenetos (1999) Anticancer Research 19:605-614).

Maximal efficacy with minimal toxicity is sought thereby. Efforts to design and refine ADC have focused on the selectivity of monoclonal antibodies (mAbs) as well as drug mechanism of action, drug-linking, drug/antibody ratio (loading), and drug-releasing properties (Junutula, et al., 2008b Nature Biotech., 26(8):925-932; Dornan et al (2009) Blood 114(13):2721-2729; US 7521541; US 7723485; WO2009/052249; McDonagh (2006) Protein Eng. Design & Sel. 19(7): 299-307; Doronina et al (2006) Bioconj. Chem. 17:114-124; Erickson et al (2006) Cancer Res. 66(8):1-8; Sanderson et al (2005) Clin. Cancer Res. 11:843-852; Jeffrey et al (2005) J. Med. Chem. 48:1344-1358; Hamblett et al (2004) Clin. Cancer Res. 10:7063-7070). Drug moieties may impart their cytotoxic and cytostatic effects by mechanisms including tubulin binding, DNA binding, or topoisomerase inhibition, Some cytotoxic drugs tend to be inactive or less active when conjugated to large antibodies or protein receptor ligands.

The present inventors have developed a novel approach to forming PBD conjugates with cell binding agents, and in particular PBD antibody conjugates.

### Summary of the Invention

In a general aspect the present invention provides a conjugate comprising a PBD compound connected through the N10 position via a linker to a cell binding agent. The linker is a labile linker, and may be an enzyme labile linker. The cell binding agent is preferably an antibody.

In one embodiment, the conjugate comprises a cell binding agent connected to a spacer, the spacer connected to a trigger, the trigger connected to a self-immolative linker, and the self-immolative linker connected to the N10 position of the PBD compound.

In a first aspect, the present invention provides novel conjugate compounds of formula (A): 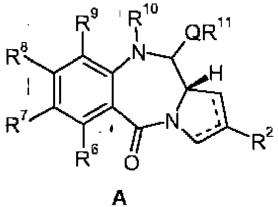 and salts and solvates thereof, wherein:
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
R⁸ is independently selected from H, R, OH, OR, SH, SR, NH₂, NHR, NRR', NO₂, Me₃Sn and halo;
R¹⁰ is a linker connected to a cell binding agent;
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
or any pair of adjacent groups from R⁶ to R⁹ together form a group -O-(CH₂)ₚ-O-, where p is 1 or 2,
or the compound is a dimer with each monomer being of formula (A), or with one monomer being of formula (A) and the other being of formula (B): 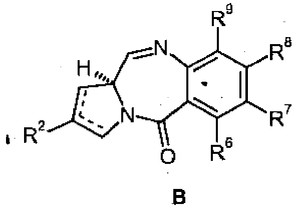
   wherein R², R⁶, R⁹, R⁷, and R⁸ are as defined according to the compounds of formula (A), and the R⁷ groups or R⁸ groups of each monomer form together a dimer bridge having the formula -X-R"-X- linking the monomers;
   wherein R" is a C₃₋₁₂ alkylene group, which chain may be interrupted by one or more heteroatoms. e.g. O, S, N(H), N Me and/or aromatic rings, e.g. benzene or pyridine, which rings are optionally substituted;
   and each X is O, S or N(H);
   or where the compound is a dimer with each monomer being of formula (A), the group R¹⁰ in one of the monomers is either a capping group, R^{C}, or is a linker connected to a cell binding agent. For the avoidance of doubt, the cell binding agent is part of the group R¹⁰.

The present invention also pertains to the use of a conjugate to provide a compound of formula (C) at a target location: ; 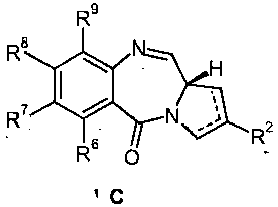 and salts and solvates thereof, wherein:
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
R⁸ is independently selected from H, R, OH, OR, SH, SR, NH₂, NHR, NRR', NO₂, Me₃Sn and halo;
R and R' are independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyrclic ring;
or any pair of adjacent groups from R⁶ to R⁹ together form a group -O-(CH₂)ₚ-O-, where p is 1 or 2
or the compound is a dimer with each monomer being of formula (C), and the R⁷ groups or R⁸ groups of each monomer form together a dimer bridge having the formula -X-R"-X- linking the monomers;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H).

The present invention also pertains to the use of a conjugate to provide a compound of formula (D) at a target location: 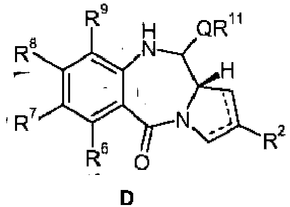 and salts and solvates thereof, wherein:
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
R³ is independently selected from H, R, OH, OR, SH, SR, NH₂, NHR, NRR', NO₂, Me₃Sn and halo;
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
or any pair of adjacent groups from R⁶ to R⁹ together form a group -O-(CH₂)ₚ-O-, where p is 1 or 2;
or the compound is a dimer with each monomer being of formula (D), or with one monomer being of formula (D) and the other being of formula (C);
and the R⁷ groups or R⁸ groups of each monomer form together a dimer bridge having the formula -X-R"-X- linking the monomers;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H);
wherein the monomer unit of formula (C) is as defined above.

The present invention also provides compounds of formula (E) for use in the preparation of the conjugate compounds of the invention: 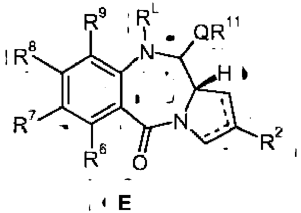 and salts and solvates thereof, wherein:
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
R⁸ is independently selected from H, R, OH, OR, SH, SR, NH₂, NHR, NRR', NO₂, Me₃Sn and halo;
R^{L} is a linker for connection to a cell binding agent;
Q is independently selected from O, S and NH;
R¹¹ is either H, or R or, where Q is O, R¹¹ is SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-mem bered heterocyclic ring;
or any pair of adjacent groups from R⁶ to R⁹ together form a group -O-(CH₂)ₚ-O-, where p is 1 or 2;
or the compound is a dimer with each monomer being of formula (E), or with one monomer being of formula (E) and the other being of formula (B): 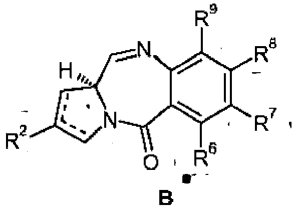
   wherein R², R⁶, R⁹, R⁷, and R⁸ are as defined according to the compounds of formula (A), and the R⁷ groups or R⁸ groups of each monomer form together a dimer bridge having the formula -X-R"-X- linking the monomers;
   wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H);
and where the compound is a dimer with each monomer being of formula (E), the group R^{L} in one of the monomers is either a capping group, R^{C}, or is a linker for connection to a cell binding agent.

Alternatively In one embodiment, R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂.

Alternatively, the novel conjugate compounds may be selected from compounds of formula (A) as described above and (A-I),
where (A-I) is selected from (A-A) and (A-B): 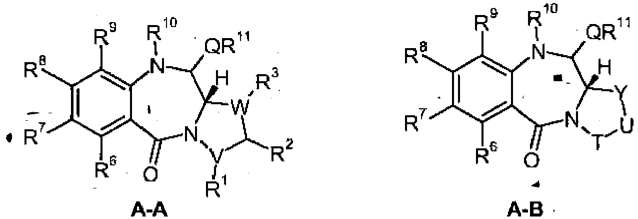 and salts and solvates thereof, wherein:
R⁶, R⁹, R¹⁰, Q, R¹¹, R and R' are as defined according to the compounds of formula (A);
R⁷ is independently selected from H, R, OH, OR, SH, SR, NH₂, NHR, NRR', NO₂, Me₃Sn and halo;
R⁸ is independently selected from H, R, OH, OR, SH, SR, NH₂, NHR, NRR', NO₂, Me₃Sn and halo;
or any pair of adjacent groups from R⁶ to R⁹ together form a group -O-(CH₂)ₚ-O-, where p is 1 or 2,
R² with either of R¹ or R³, together with the carbon atoms of the C ring to which they are attached, form an optionally substituted benzene ring;
V and W are each selected from (CH₂)ₙ, O, S, NR, CHR, and CRR' where n is 1, 2 or 3, except that V is C when R¹ and R², together with the carbon atoms of the C ring to which they are attached, form an optionally substituted benzene ring, and W is C when R³ and R², together with the carbon atoms of the C ring to which they are attached, form an optionally substituted benzene ring;
T is selected from CH₂, NR, CO, BH, SO, and SO₂;
U is selected from CH₂, NR, O and S;
Y is (CH₂)ₙ, where n is 1, 2, 3 or 4;
except that T, U and Y are not all CH₂;
or the compound is a dimer with each monomer being of formula (A), each monomer being of formula (A-I), with one monomer being of formula (A) and the other being of formula (B) as described above or (B-I), or with one monomer being of formula (A-I) and the other being of formula (B) or (B-I),
and (B-I) is selected from (B-A) and (B-B): 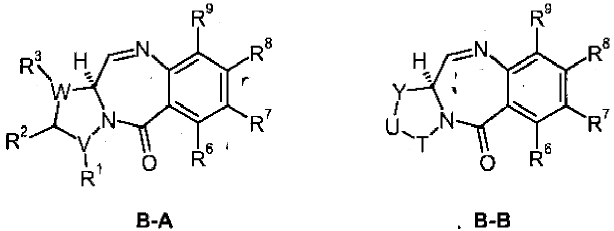
   wherein R¹, R², R³, R⁶, R⁹, R⁷, and R⁸ are as defined according to the compounds of formula (A-I), and the R⁷ groups or R⁸ groups of each monomer form together a dimer bridge having the formula -X-R"-X- linking the monomers;
   wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H);
and where the compound is a dimer with each monomer being of formula (A-I) and/or formula (A), the group R¹⁰ in one of the monomers is either a capping group, R^{C}, or is a linker connected to a cell binding agent.

For convenience, all references to A may be applied to A-I (and A-A and A-B), and all references to B may be applied to B-I (and B-A and B-B). Similar references to C, D and E are also pertinent to (C-I), (D-I) and (E-I), as appropriate.

Alternatively the conjugate may be used to provide a compound at a target location, wherein the compound is a compound of formula (C) as described above or (C-I),
where (C-I) is selected from (C-A) and (C-B): 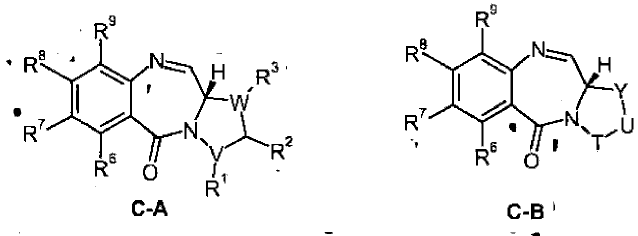 and salts and solvates thereof, wherein:
R⁶, R⁹, R and R' are as defined according to the compounds of formula (C);
R⁷ is independently selected from H, R, OH, OR, SH, SR, NH₂, NHR, NRR', NO₂, Me₃Sn and halo;
R⁸ is independently selected from H, R, OH, OR, SH, SR, NH₂, NHR, NRR', NO₂, Me₃Sn and halo;
or any pair of adjacent groups from R⁶ to R⁹ together form a group -O-(CH₂)ₚ-O-,
R² with either of R¹ or R³, together with carbon atoms of the C ring to which they are attached, form an optionally substituted benzene ring;
V and W are each selected from (CH₂)ₙ, O, S, NR, CHR, and CRR' where n is 1, 2 or 3, except that V is C when R¹ and R², together with carbon atoms of the C ring to which they are attached, form an optionally substituted benzene ring, and W is C when R³ and R², together with carbon atoms of the C ring to which they are attached, form an optionally substituted benzene ring;
T is selected from CH₂, NR, CO, BH, SO, and SO₂;
U is selected from CH₂, NR, O and S;
Y is (CH₂)ₙ, where n is 1, 2, 3 or 4;
except that T, U and Y are not all CH₂;
or the compound is a dimer with each monomer being of formula (C), each monomer being of formula (C-I), or with one monomer being of formula (C) and the other being of formula (C-I), and the R⁷ groups or R⁸ groups of each monomer form together a dimer bridge having the formula -X-R"-X- linking the monomers;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H).

The present invention also pertains to the use of a conjugate to provide a compound at a target location, wherein the compound is a compound of formula (D) as described above or formula (D-I);
where (D-I) is selected from (D-A) and (D-B): 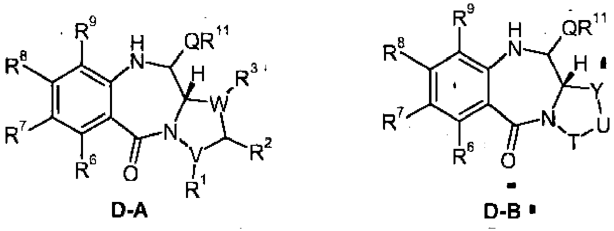 and salts and solvates thereof, wherein:
R⁶, R⁹, R and R' are as defined according to the compounds of formula (D);
R⁷ is independently selected from H, R, OH, OR, SH, SR, NH₂, NHR, NRR', NO₂, Me₃Sn and halo;
R⁸ is independently selected from H, R, OH, OR, SH, SR, NH₂, NHR, NRR', NO₂, Me₃Sn and halo;
or any pair of adjacent groups from R⁶ to R⁹ together form a group -O-(CH₂)ₚ-O-, where p is 1 or 2,
Q is independently selected from O, S and NH;
R¹¹ is either H, or R or, where Q is O, SO₃M, where M is a metal cation;
R² with either of R¹ or R³, together with carbon atoms of the C ring to which they are attached, form an optionally substituted benzene ring;
V and W are each selected from (CH₂)ₙ, O, S, NR, CHR, and GRR' where n is 1, 2 or 3, except that V is C when R¹ and R², together with carbon atoms of the C ring to which they are attached, form an optionally substituted benzene ring, and W is C when R³ and R², together with carbon atoms of the C ring to which they are attached, form an optionally substituted benzene ring;
T is selected from CH₂, NR, CO, BH, SO, and SO₂;
U is selected from CH₂, NR, O and S;
Y is (CH₂)ₙ, where n is 1, 2, 3 or 4;
except that T, U and Y are not all CH2;
or the compound is a dimer with each monomer being of formula (D), each monomer being of formula (D-I), or with one monomer being of formula (D) and the other being of formula (D-I), and the R⁷ groups or R⁸ groups of each monomer form together a dimer bridge having the formula -X-R"-X- linking the monomers;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H).

Alternatively, the present invention also provides compounds of formula (E) as described above and (E-I) for use in the preparation of the conjugate compounds of the invention;
where (E-I) is selected from (E-A) and (E-B): 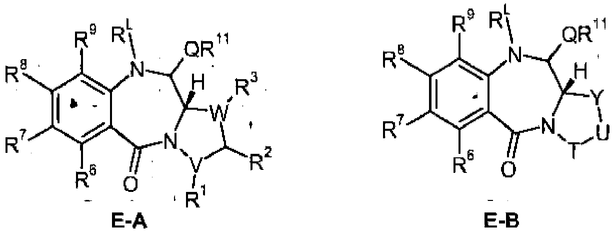 and salts and solvates thereof, wherein:
R⁶, R⁹, R and R' are as defined according to the compounds of formula (D);
R⁷ is independently selected from H, R, OH, OR, SH, SR, NHp, NHR, NRR', NO₂, Me₃Sn and halo;
R⁸ is independently selected from H, R, OH, OR, SH, SR, NH₂, NHR, NRR', NO₂, Me₃Sn and halo;
or any pair of adjacent groups from R⁶ to R⁹ together form a group -O-(CH₂)ₚ-O-, where p is 1 or 2,
R^{L} is a linker for connection to a cell binding agent;
Q is independently selected from O, S and NH;
R¹¹ is either H, or R or, where Q is O, SO₃M, where M is a metal cation;
R² with either of R¹ or R³, together with carbon atoms of the C ring to which they are attached, form an optionally substituted benzene ring;
V and W are each selected from (CH₂)ₙ, O, S, NR, CHR, and GRR' where n is 1, 2' or 3, except that V is C when R¹ and R², together with carbon atoms of the C ring to which they are attached, form an optionally substituted benzene ring, and W is C when R³ and R², together with carbon atoms of the C ring to which they are attached, form an optionally substituted benzene ring;
T is selected from CH₂, NR, CO, BH. SO, and SO₂;
U is selected from CH₂, NR, O and S;
Y is (CH₂)ₙ, where n is 1, 2, 3 or 4;
except that T, U and Y are not all CH₂;
or the compound is a dimer with each monomer being of formula (E), each monomer being of formula (E-I), or with one monomer being of formula (E) or (E-1) and the other being of formula (E), (E-I), (B) or (B-1);
and the R⁷ groups or R⁸ groups of each monomer form together a dimer bridge having the formula -X-R"-X- linking the monomers;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
and each X is O, S or N(H).

### Brief Description of the Drawings

Figure 1 shows particular embodiments of the present invention;
Figures 2 to 6 show the result of biological tests on particular embodiments of the present invention.

### Detailed Description of the Invention

The present invention provides a conjugate comprising a PBD compound connected through the N10 position via a linker to a cell binding agent. In one embodiment, the conjugate comprises a cell binding agent connected to a spacer connecting group, the spacer connected to a trigger, the trigger connected to a self-immolative linker, and the self-immolative linker connected to the N10 position of the PBD compound. Such a conjugate is illustrated below: 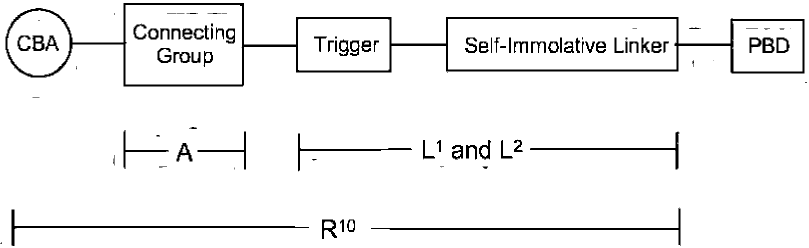 where CBA is a cell binding agent and PBD is a pyrrolobenzodiazepine compound, as described herein. The illustration shows the portions that correspond to R¹⁰, A, L¹ and L² in certain embodiments of the invention.

The present invention is suitable for use in providing a PBD compound to a preferred site in a subject. In the preferred embodiments, the conjugate allows the release of an active PBD compound that does not retain any part of the linker. There is no stub present that could affect the reactivity of the PBD compound.

In certain embodiments, the invention provides conjugates comprising a PBD dimer group having a linker connected to a cell binding agent. The present inventors describe herein methods of synthesis that enable such dimer conjugates to be prepared by the use of novel PBD desymmetrisation techniques.

### Preferences

The following preferences may apply to all aspects of the invention as described above, or may relate to a single aspect. The preferences may be combined together in any combination.

### Double Bond

In one embodiment, there is no double bond present between C1 and C2, and C2 and C3.

In one embodiment, the dotted lines indicate the optional presence of a double bond between C2 and C3, as shown below:

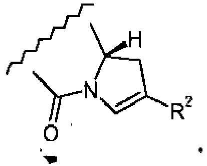

In one embodiment, a double bond is present between C2 and C3 when R² is C₅₋₂₀ aryl or C₁₋₁₂ alkyl.
In one embodiment, the dotted lines indicate the optional presence of a double bond between C1 and C2, as shown below:

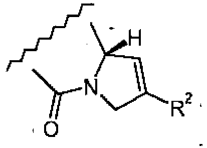

In one embodiment, a double bond is present between C1 and C2 when R² is C₅₋₂₀ aryl or C₁₋₁₂ alkyl.

### R²

In one embodiment, R² is independently selected from H, OH, =O, =CH₂, CN, R, OR, =CH-R^{D}, =C(R^{D})₂, O-SO₂-R, CO₂R and COR, and optionally further selected from halo or dihalo. In one embodiment, R² is independently selected from H, OH, =O, =CH₂, CN, R, OR, =CH-R^{D}, =C(R^{D})₂, O-SO₂-R, CO₂R and COR.
In one embodiment, R² is independently selected from H, =O, =CH₂, R, =CH-R^{D}, and =C(R^{D}))₂.

In one embodiment, R² is independently H.
In one embodiment, R² is independently =O.
In one embodiment, R² is independently =CH₂.
In one embodiment, R² is independently =CH-R^{D}. Within the PBD compound, the group =CH-R^{D} may have either configuration shown below: 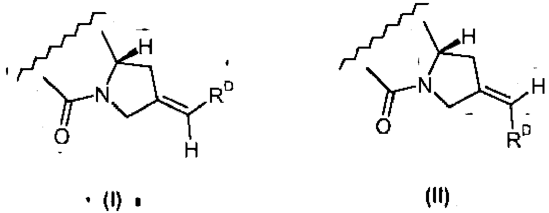 In one embodiment, the configuration is configuration (I).

In one embodiment, R² is independently =C(R^{D})₂.
In one embodiment, R² is independently =CF₂.
In one embodiment, R² is independently R.
In one embodiment, R² is independently optionally substituted C₅₋₂₀ aryl.
In one embodiment, R² is independently optionally substituted C₁₋₁₂ alkyl.

In one embodiment, R² is independently optionally substituted C₅₋₂₀ aryl.
In one embodiment, R² is independently optionally substituted C₅₋₇ aryl.
In one embodiment, R² is independently optionally substituted C₈₋₁₀ aryl.
In one embodiment, R² is independently optionally substituted phenyl.
In one embodiment, R² is independently optionally substituted napthyl.
In one embodiment, R² is independently optionally substituted pyridyl.
In one embodiment, R² is independently optionally substituted quinolinyl or isoquinolinyl.

In one embodiment, R² bears one to three substituent groups, with 1 and 2 being more preferred, and singly substituted groups being most preferred. The substituents may be any position.

Where R² is a C₅₋₇ aryl group, a single substituent is preferably on a ring atom that is not adjacent the bond to the remainder of the compound, i.e. it is preferably β or γ to the bond to the remainder of the compound. Therefore, where the C₅₋₇ aryl group is phenyl, the substituent is preferably in the meta- or para- positions, and more preferably is in the para-position.

In one embodiment, R² is selected from: 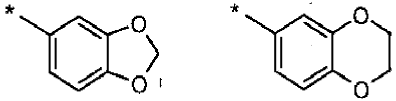 where the asterisk indicates the point of attachment.

Where R² is a C₈₋₁₀ aryl group, for example quinolinyl or isoquinolinyl, it may bear any number of substituents at any position of the quinoline or isoquinoline rings. In some embodiments, it bears one, two or three substituents, and these may be on either the proximal and distal rings or both (if more than one substituent).

In one embodiment, where R² is optionally substituted, the substituents are selected from those substituents given in the substituent section below.
Where R is optionally substituted, the substituents are preferably selected from:
Halo, Hydroxyl, Ether, Formyl, Acyl, Carboxy, Ester, Acyloxy, Amino, Amido, Acylamido, Aminocarbonyloxy, Ureido, Nitro, Cyano and Thioether.

In one embodiment, where R or R² is optionally substituted, the substituents are selected from the group consisting of R, OR, SR, NRR', NO₂, halo, CO₂R, COR, CONH₂, CONHR, and CONRR'.

Where R² is C₁₋₁₂ alkyl, the optional substituent may additionally include C₃₋₂₀ heterocyclyl and C₅₋₂₀ aryl groups.
Where R² is C₃₋₂₀ heterocyclyl, the optional substituent may additionally include C₁₋₁₂ alkyl and C₅₋₂₀ aryl groups.
Where R² is C₅₋₂₀ aryl groups, the optional substituent may additionally include
C₃₋₂₀ heterocyclyl and C₁₋₁₂ alkyl groups.

It is understood that the term "alkyl" encompasses the sub-classes alkenyl and alkynyl as well as cycloalkyl. Thus, where R² is optionally substituted C₁₋₁₂ alkyl, it is understood that the alkyl group optionally contains one or more carbon-carbon double or triple bonds, which may form part of a conjugated system. In one embodiment, the optionally substituted C₁₋₁₂ alkyl group contains at least one carbon-carbon double or triple bond, and this bond is conjugated with a double bond present between C1 and C2, or C2 and C3. In one embodiment, the C₁₋₁₂ alkyl group is a group selected from saturated C₁₋₁₂ alkyl, C₂₋₁₂ alkenyl, C₂₋₁₂ alkynyl and C₃₋₁₂ cycloalkyl.

If a substituent on R² is halo, it is preferably F or Cl, more preferably Cl.

If a substituent on R² is ether, it may in some embodiments be an alkoxy group, for example, a C₁₋₇ alkoxy group (e.g. methoxy, ethoxy) or it may in some embodiments be a C₅₋₇ aryloxy group (e.g phenoxy, pyridyloxy, furanyloxy).

If a substituent on R² is C₁₋₇ alkyl, it may preferably be a C₁₋₄ alkyl group (e.g. methyl, ethyl, propyl, butyl).

If a substituent on R² is C₃₋₇ heterocyclyl, it may in some embodiments be C₆ nitrogen containing heterocyclyl group, e.g. morpholino, thiomorpholino, piperidinyl, piperazinyl. These groups may be bound to the rest of the PBD moiety via the nitrogen atom. These groups may be further substituted, for example, by C₁₋₄ alkyl groups.

If a substituent on R² is bis-oxy-C₁₋₃ alkylene, this is preferably bis-oxy-methylene or bis-oxy-ethylene.

Particularly preferred substituents for R² include methoxy, ethoxy, fluoro, chloro, cyano, bis-oxy-methylene, methyl-piperazinyl, morpholino and methyl-thienyl.

Particularly preferred substituted R² groups include, but are not limited to, 4-methoxy-phenyl, 3-methoxyphenyl, 4-ethoxy-phenyl, 3-ethoxy-phenyl, 4-fluoro-phenyl, 4-chloro-phenyl, 3,4-bisoxymethylene-phenyl, 4-methylthienyl, 4-cyanophenyl, 4-phenoxyphenyl, quinolin-3-yl and quinolin-6-yl, isoquinolin-3-yl and isoquinolin-6-yl, 2-thienyl, 2-furanyl, methoxynaphthyl, and naphthyl.

In one embodiment, R² is halo or dihalo. In one embodiment, R² is -F or -F₂, which substituents are illustrated below as (III) and (IV) respectively:

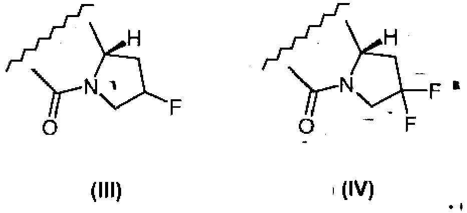

### R^{D}

In one embodiment, R° is independently selected from R, CO₂R, COR, CHO, CO₂H, and halo.
In one embodiment, R^{D} is independently R.
In one embodiment, R^{D} is independently halo.

### R⁶

In one embodiment, R⁶ is independently selected from H, R, OH, OR, SH, SR, NH₂, NHR, NRR', NO₂, Me₃Sn- and Halo.

In one embodiment, R⁶ is independently selected from H, OH, OR, SH, NH₂, NO₂ and Halo.
In one embodiment, R⁶ is independently selected from H and Halo.
In one embodiment, R⁶ is independently H.

In one embodiment, R⁶ and R⁷ together form a group -O-(CH₂)ₚ-O-, where p is 1 or 2.

### R⁷

R⁷ is independently selected from H, R, OH, OR, SH, SR, NH₂, NHR, NRR', NO₂, Me₃Sn and halo.
In one embodiment, R⁷ is independently OR.
In one embodiment, R⁷ is independently OR^{7A}, where R^{7A} is independently optionally substituted C₁₋₆ alkyl.
In one embodiment, R^{7A} is independently optionally substituted saturated C₁₋₆ alkyl.
In one embodiment, R^{7A} is independently optionally substituted C₂₋₄ alkenyl.
In one embodiment, R^{7A} is independently Me.
In one embodiment, R^{7A} is independently CH₂Ph.
In one embodiment, R^{7A} is independently allyl.

In one embodiment, the compound is a dimer where the R⁷ groups of each monomer form together a dimer bridge having the formula X-R"-X linking the monomers.

### R⁸

In one embodiment, the compound is a dimer where the R⁸ groups of each monomer form together a dimer bridge having the formula X-R"-X linking the monomers.

In one embodiment, R⁸ is independently OR^{8A}, where R^{8A} is independently optionally substituted C₁₋₄ alkyl.
In one embodiment, R^{8A} is independently optionally substituted saturated C₁₋₆ alkyl or optionally substituted C₂₋₄ alkenyl.
In one embodiment, R^{8A} is independently Me.
In one embodiment, R^{8A} is independently CH₂Ph.
In one embodiment, R^{8A} is independently allyl.

In one embodiment, R⁸ and R⁷ together form a group -O-(CH₂)ₚ-O-, where p is 1 or 2.
In one embodiment, R⁸ and R⁹ together form a group -O-(CH₂)ₚ-O-, where p is 1 or 2.

### R⁹

In one embodiment, R⁹ is independently selected from H, R, OH, OR, SH, SR, NH₂, NHR, NRR', NO₂, Me₃Sn- and Halo.

In one embodiment, R⁹ is independently H.
In one embodiment, R⁹ is independently R or OR.

### R¹⁰

For the avoidance of doubt, where R¹⁰ is a linker connected to a cell binding agent, the cell binding agent is part of the group R¹⁰.

In certain embodiments of the invention, where the conjugate is a dimer comprising two monomers A, one monomer has a group R¹⁰ that is a linker connected to a cell binding agent, and the other monomer has a group R¹⁰ that is a linker connected to a cell binding agent or a capping group R^{C}. Preferably, the other monomer has a group R¹⁰ that is a capping group R^{C}. Thus, in this preferred embodiment, there is only a single link to the cell binding agent.

In one embodiment, the group R¹⁰ is removable from the N¹⁰ position of the PBD moiety to leave an N10-C11 imine bond, a carbinolamine, a substituted carbinolamine, where QR¹¹ is OSO₃M, a bisulfite adduct, a thiocarbinolamine, a substituted thiocarbinolamine, or a substituted carbinalamine, as illustrated below: 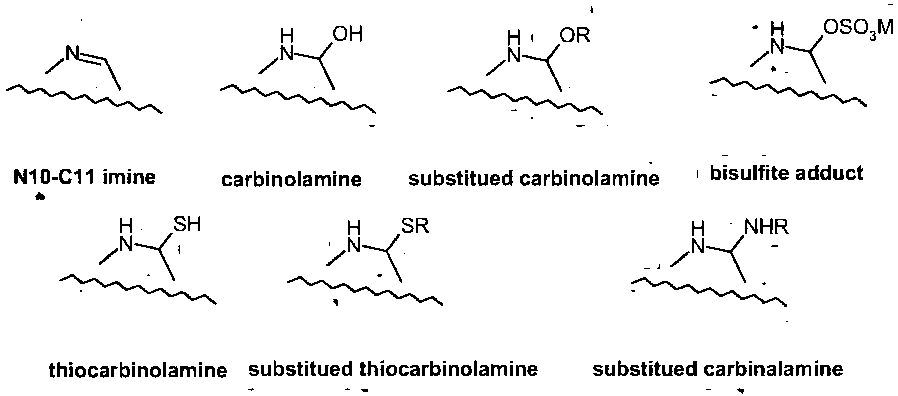 where R and M are as defined for the conjugates of the invention.

In one embodiment, the group R¹⁰ is removable from the N 10 position of the PBD moiety to leave an N 10-C11 imine bond.

In some embodiments, the conjugate of the invention is a dimer compound comprising a monomer of formula (A) and a monomer of formula (B). In this embodiment, the group R¹⁰ need not be removable from the N10 position, as the monomer (B) has suitable functionality at the N 10 and C11 positions for biological activity.
However, it is preferred that the group R¹⁰ is removable thereby to provide a dimer having suitable functionality at the N10 and C11 positions in both monomer units. Such functionality is thought necessary to permit the crosslinking activity of the PBD dimer.

This application is particularly concerned with those R¹⁰ groups which have a carbamate link to the N10 position.

The linker attaches the Cell Binding Agent (CBA), e.g. antibody, to the PBD drug moiety D through covalent bond(s). The linker is a bifunctional or multifunctional moiety which can be used to link one or more drug moiety (D) and an antibody unit (Ab) to form antibody-drug conjugates (ADC). The linker (L) may be stable outside a cell, i.e. extracellular, or it may be cleavable by enzymatic activity, hydrolysis, or other metabolic conditions. Antibody-drug conjugates (ADC) can be conveniently prepared using a linker having reactive functionality for binding to the drug moiety and to the antibody. A cysteine thiol, or an amine, e.g. N-terminus or amino acid side chain such as lysine, of the antibody (Ab) can form a bond with a functional group of a linker or spacer reagent, PBD drug moiety (D) or drug-linker reagent (D-L).

Many functional groups on the linker attached to the N10 position of the PBD moiety may be useful to react with the cell binding agent. For example, ester, thioester, amide, thioamide, carbamate, thiocarbamate, urea, thiourea, ether, thioether, or disulfide linkages may be formed from reaction of the linker-PBD drug intermediates and the cell binding agent. The linkers of the ADC preferably prevent aggregation of ADC molecules and keep the ADC freely soluble in aqueous media and in a monomeric state.
The linkers of the ADC are preferably stable extracellularly. Before transport or delivery into a cell, the antibody-drug conjugate (ADC) is preferably stable and remains intact, i.e. the antibody remains linked to the drug moiety. The linkers are stable outside the target cell and may be cleaved at some efficacious rate inside the cell. An effective linker will: (i) maintain the specific binding properties of the antibody; (ii) allow intracellular delivery of the conjugate or drug moiety; (iii) remain stable and intact, i.e. not cleaved, until the conjugate has been delivered or transported to its targetted site; and (iv) maintain a cytotoxic, cell-killing effect or a cytostatic effect of the PBD drug moiety. Stability of the ADC may be measured by standard analytical techniques such as mass spectroscopy, HPLC, and the separation/analysis technique LC/MS.

Covalent attachment of the antibody and the drug moiety requires the linker to have two reactive functional groups, i.e. bivalency in a reactive sense. Bivalent linker reagents which are useful to attach two or more functional or biologically active moieties, such as peptides, nucleic acids, drugs, toxins, antibodies, haptens, and reporter groups are known, and methods have been described their resulting conjugates (Hermanson, G.T. (1996) Bioconjugate Techniques; Academic Press: New York, p 234-242).

In another embodiment, the linker may be substituted with groups which modulate aggregation, solubility or reactivity. For example, a sulfonate substituent may increase water solubility of the reagent and facilitate the coupling reaction of the linker reagent with the antibody or the drug moiety, or facilitate the coupling reaction of Ab-L with D, or D-L with Ab, depending on the synthetic route employed to prepare the ADC.

In one embodiment, R¹⁰ is a group: 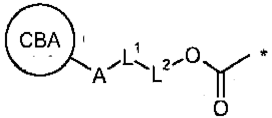 where the asterisk indicates the point of attachment to the N10 position, CBA is a cell hinding agent, L¹ is a linker, A is a connecting group connecting L¹ to the cell binding agent, L² is a covalent bond or together with -OC(=O)- forms a self-immolative linker, and L¹ or L² is a cleavable linker.

L¹ is preferably the cleavable linker, and may be referred to as a trigger for activation of the linker for cleavage.

The nature of L¹ and L², where present, can vary widely. These groups are chosen on the basis of their cleavage characteristics, which may be dictated by the conditions at the site to which the conjugate is delivered. Those linkers that are cleaved by the action of enzymes are preferred, although linkers that are cleavable by changes in pH (e.g. acid or base labile), temperature or upon irradiation (e.g. photolabile) may also be used. Linkers that are cleavable under reducing or oxidising conditions may also find use in the present invention.

L¹ may comprise a contiguous sequence of amino acids. The amino acid sequence may be the target substrate for enzymatic cleavage, thereby allowing release of R¹⁰ from the N10 position.

In one embodiment, L¹ is cleavable by the action of an enzyme. In one embodiment, the enzyme is an esterase or a peptidase.

In one embodiment, L is present and together with -C(=O)O- forms a self-immolative linker. In one embodiment, L² is a substrate for enzymatic activity, thereby allowing release of R¹⁰ from the N10 position.

In one embodiment, where L¹ is cleavable by the action of an enzyme and L² is present, the enzyme cleaves the bond between L¹ and L².

r L¹ and L², where present, may be connected by a bond selected from:
-C(=O)NH-,
-C(=O)O-,
-NHC(=O)-,
-OC(=O)-,
-OC(=O)O-,
-NHC(=O)O-,
-OC(=O)NH-, and
-NHC(=O)NH-.

An amino group of L¹ that connects to L² may be the N-terminus of an amino acid or may be derived from an amino group of an amino acid side chain, for example a lysine amino acid side chain.

A carboxyl group of L¹ that connects to L² may be the C-terminus of an amino acid or may be derived from a carboxyl group of an amino acid side chain, for example a glutamic acid amino acid side chain.

A hydroxyl group of L¹ that connects to L² may be derived from a hydroxyl group of an amino acid side chain, for example a serine amino acid side chain.

The term "amino acid side chain" includes those groups found in: (i) naturally occurring amino acids such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine; (ii) minor amino acids such as ornithine and citrulline; (iii) unnatural amino acids, beta-amino acids, synthetic analogs and derivatives of naturally occurring amino acids; and (iv) all enantiomers, diastereomers, isomerically enriched, isotopically labelled (e.g. ²H, ³H, ¹⁴C, ¹⁵N), protected forms, and racemic mixtures thereof.

In one embodiment, -C(=O)O- and L² together form the group: 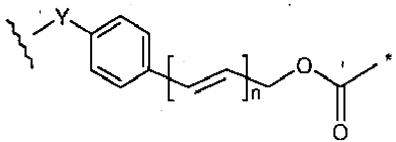 where the asterisk indicates the point of attachment to the N10 position, the wavy line indicates the point of attachment to the linker L¹, Y is -N(H)-, -O-, -C(=O)N(H)- or -C(=O)O-, and n is 0 to 3. The phenylene ring is optionally substituted with one, two or three substituents as described herein. In one embodiment, the phenylene group is optionally substituted with halo, NO₂, R or OR.

In one embodiment, Y is NH.
In one embodiment, n is 0 or 1. Preferably, n is 0.

Where Y is NH and n is 0, the self-immolative linker may be referred to as a p-aminobenzylcarbonyl linker (PABC).

The self-immolative linker will allow for release of the protected compound when a remote site is activated, proceeding along the lines shown below (for n=0): 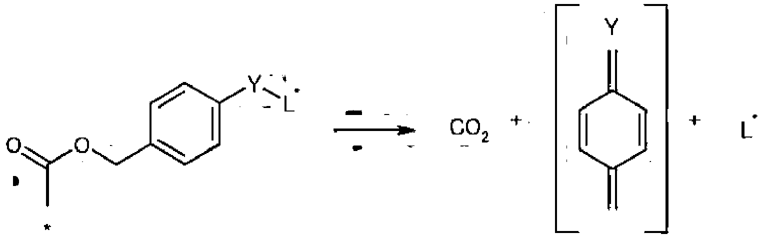 where L is the activated form of the remaining portion of the linker. These groups have the advantage of separating the site of activation from the compound being protected. As described above, the phenylene group may be optionally substituted.

In one embodiment described herein, the group L^{*} is a linker L¹ as described herein, which may include a dipeptide group.

In another embodiment, -C(=O)O- and L² together form a group selected from: 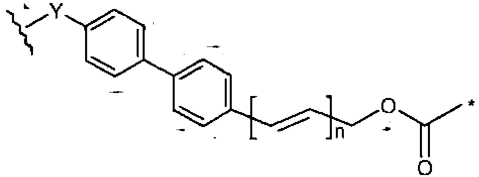 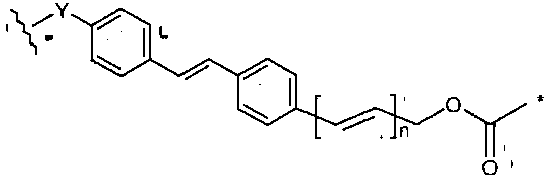 where the asterisk, the wavy line, Y, and n are as defined above. Each phenylene ring is optionally substituted with one, two or three substituents as described herein. In one embodiment, the phenylene ring having the Y substituent is optionally substituted and the phenylene ring not having the Y substituent is unsubstituted. In one embodiment, the phenylene ring having the Y substituent is unsubstituted and the phenylene ring not having the Y substituent is optionally substituted.

In another embodiment, -C(=O)O- and L² together form a group selected from: 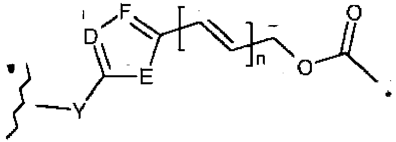 where the asterisk, the wavy line, Y, and n are as defined above, E is O, S or NR, D is N, CH, or CR, and F is N, CH, or CR.

In one embodiment, D is N.
In one embodiment, D is CH.
In one embodiment, E is O or S.
In one embodiment, F is CH.

In a preferred embodiment, the linker is a cathepsin labile linker.

In one embodiment, L¹ comprises a dipeptide The dipeptide may be represented as -NH-X₁-X₂-CO-, where -NH- and -CO- represent the N- and C-terminals of the amino acid groups X₁ and X₂ respectively. The amino acids in the dipeptide may be any combination of natural amino acids. Where the linker is a cathepsin labile linker, the dipeptide may be the site of action for cathepsin-mediated cleavage.

Additionally, for those amino acids groups having carboxyl or amino side chain functionality, for example Glu and Lys respectively, CO and NH may represent that side chain functionality.

In one embodiment, the group X₁-X₂- in dipeptide, -NH-X₁-X₂-CO-, is selected from:
-Phe-Lys-,
-Val-Ala-,
-Val-Lys-,
-Ala-Lys-,
-Val-Cit-,
-Phe-Cit-,
-Leu-Cit-,
-Ile-Cit-,
-Phe-Arg-,
-Trp-Cit-
where Cit is citrulline.

Preferably, the group -X₁-X₂- in dipeptide, -NH-X₁-X₂-CO-, is selected from:
- Phe-Lys-,
- Val-Ala-,
- Val-Lys-,
- Ala-Lys-,
- Val-Cit-.

Most preferably, the group -X₁-X₂- in dipeptide, -NH-X₁-X₂-CO-, is -Phe-Lys- or -Val-Ala-.

Other dipeptide combinations may be used, including those described by Dubowchik et al., Bioconjugate Chemistry, 2002, 13,855-869, which is incorporated herein by reference.

In one embodiment, the amino acid side chain is derivatised, where appropriate. For example, an amino group or carboxy group of an amino acid side chain may be derivatised. In one embodiment, an amino group NH₂ of a side chain amino acid, such as lysine, is a derivatised form selected from the group consisting of NHR and NRR'.
In one embodiment, a carboxy group COOH of a side chain amino acid, such as aspartic acid, is a derivatised form selected from the group consisting of COOR, CONH₂, CONHR and CONRR'.

In one embodiment, the amino acid side chain is chemically protected, where appropriate. The side chain protecting group may be a group as discussed below in relation to the group R^{L}. The present inventors have established that protected amino acid sequences are cleavable by enzymes. For example, it has been established that a dipeptide sequence comprising a Boc side chain-protected Lys residue is cleavable by cathepsin.

Protecting groups for the side chains of amino acids are well known in the art and are described in the Novabiochem Catalog. Additional protecting group strategies are set out in Protective Groups in Organic Synthesis, Greene and Wuts.

Possible side chain protecting groups are shown below for those amino acids having reactive side chain functionality:
Arg: Z, Mtr, Tos;
Asn: Trt, Xan;
Asp: Bzl, t-Bu;
Cys: Acm, Bzl, Bzl-OMe, Bzl-Me, Trt;
Glu: Bzl, t-Bu;
Gin: Trt, Xan;
His: Boc, Dnp, Tos, Trt;
Lys: Boc, Z-Cl, Fmoc, Z, Alloc;
Ser: Bzl, TBDMS, TBDPS;
Thr: Bz;
Trp: Boc;
Tyr: Bzl, Z, Z-Br,

In one embodiment, the side chain protection is selected to be orthogonal to a group provided as, or as part of, a capping group, where present. Thus, the removal of the side chain protecting group does not remove the capping group, or any protecting group functionality that is part of the capping group.

In other embodiments of the invention, the amino acids selected are those having no reactive side chain functionality.' For example, the amino acids may be selected from: Ala, Gly, Ile, Leu, Met, Phe, Pro, and Val.
In one embodiment, the dipeptide is used in combination with a self-immolative linker. The self-immolative linker may be connected to -X₂-.

Where a self-immolative linker is present, -X₂- is connected directly to the self-immolative linker. Preferably the group -X₂-CO- is connected to Y, where Y is NH, thereby forming the group -X₂-CO-NH-.

-NH-X₁- is connected directly to A. A may comprise the functionality -CO- thereby to form an amide link with -X₁-.

In one embodiment, L¹ and L² together with -OC(=O)- comprise the group NH-X₁-X₂-CO-PABC-. The PABC group is connected directly to the N10 position. Preferably, the self-immolative linker and the dipeptide together form the group -NH-Phe-Lys-CO-NH-PABC-, which is illustrated below: 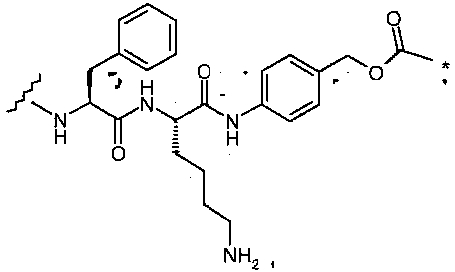 where the asterisk indicates the point of attachment to the N10 position, and the wavy line indicates the point of attachment to the remaining portion of the linker L¹ or the point of attachment to A. Preferably, the wavy line indicates the point of attachment to A. The side chain of the Lys amino acid may be protected, for example, with Boc, Fmoc, or Alloc, as described above.

Alternatively, the self-immolative linker and the dipeptide together form the group -NH-Val-Ala-CO-NH-PABC-, which is illustrated below: 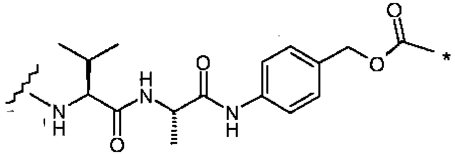 where the asterisk and the wavy line are as defined above.

Alternatively, the self-immolative linker and the dipeptide together form the group -NH-Val-Cit-CO-NH-PABC-, which is illustrated below: 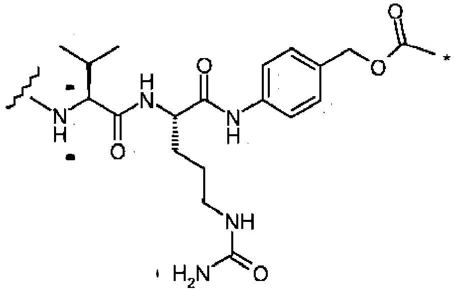 where the asterisk and the wavy line are as defined above.

In some embodiments of the present invention, it may be preferred that if the PBD/drug moiety contains an unprotected imine bond, e.g. if moiety B is present, then the linker does not contain a free amino (H₂N-) group. Thus if the the linker has the structure -A-L¹-L²- then this would preferably not contain a free amino group. This preference is particularly relevant when the linker contains a dipeptide, for example as L¹; in this embodiment, it would be preferred that one of the two amino acids is not selected from lysine.

Without wishing to be bound by theory, the present inventors have found that the combination of an unprotected imine bond in the drug moiety and a free amino group in the linker can cause dimerisation of the drug-linker moiety which may interfere with the conjugation of such a drug-linker moiety to an antibody. The cross-reaction of these groups may be accelerated in the case the free amino group is present as an ammonium ion (H₃N⁺-), such as when a strong acid (e.g. TFA) has been used to deprotect the free amino group.

In one embodiment, A is a covalent bond. Thus, L¹ and the cell binding agent are directly connected. For example, where L¹ comprises a contiguous amino acid sequence, the N-terminus of the sequence may connect directly to the cell binding agent.

Thus, where A is a covalent bond, the connection between the cell binding agent and L¹ may be selected from:
-C(=O)NH-,
-C(=O)O-,
-NHC(=O)-,
-OC(=O)-,
-OC(=O)O-,
-NHC(=O)O-,
-OC(=O)NH-,
-NHC(=O)NH-,
-C(=O)NHC(=O)-,
-S-,
-S-S-,
-CH₂C(=O)-, and
=N-NH-.

An amino group of L¹ that connects to the cell binding agent may be the N-terminus of an amino acid or may be derived from an amino group of an amino acid side chain, for example a lysine amino acid side chain.
An carboxyl group of L¹ that connects to the cell binding agent may be the C-terminus of an amino acid or may be derived from a carboxyl group of an amino acid side chain, for example a glutamic acid amino acid side chain.
A hydroxyl group of L¹ that connects to the cell binding agent may be derived from a hydroxyl group of an amino acid side chain, for example a serine amino acid side chain.
A thiol group of L¹ that connects to the cell binding agent may be derived from a thiol group of an amino acid side chain, for example a serine amino acid side chain.

The comments above in relation to the amino, carboxyl, hydroxyl and thiol groups of L¹ also apply to the cell binding agent.

In one embodiment, L² together with -OC(=O)- represents: 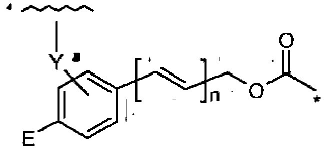 where the asterisk indicates the point of attachment to the N10 position, the wavy line indicates the point of attachment to L¹, n is 0 to 3, Y is a covalent bond or a functional group, and E is an activatable group, for example by enzymatic action or light, thereby to generate a self-immolative unit. The phenylene ring is optionally further substituted with one, two or three substituents as described herein. In one embodiment, the phenylene group is optionally further substituted with halo, NO₂, R or OR. Preferably n is 0 or 1, most preferably 0.

E is selected such that the group is susceptible to activation, e.g. by light or by the action of an enzyme. E may be -NO₂ or glucoronic acid. The former may be susceptible to the action of a nitroreductase, the latter to the action of a β-glucoronidase.

In this embodiment, the self-immolative linker will allow for release of the protected compound when E is activated, proceeding along the lines shown below (for n=0): 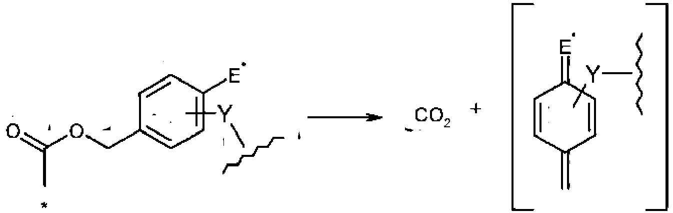 where the asterisk indicates the point of attachment to the N 1 0 position, E^{*} is the activated form of E, and Y is as described above. These groups have the advantage of separating the site of activation from the compound being protected. As described above, the phenylene group may be optionally further substituted.

The group Y may be a covalent bond to L¹.
The group Y may be a functional group selected from:
-C(=O)-
-NH-
-O-
-C(=O)NH-,
-C(=O)O-,
-NHC(=O)-,
-OC(=O)-,
-OC(=O)O-,
-NHC(=O)O-,
-OC(=O)NH-,
-NHC(=O)NH-,
-NHC(=O)NH,
-C(=O)NHC(=O)-, and
-S-.

Where L¹ is a dipeptide, it is preferred that Y is -NH- or -C(=O)-, thereby to form an amide bond between L¹ and Y. In this embodiment, the dipeptide sequence need not be a substrate for an enzymatic activity.

In another embodiment, A is a spacer group. Thus, L¹ and the cell binding agent are indirectly connected.

L¹ and A may be connected by a bond selected from:
-C(=O)NH-,
-C(=O)O-,
-NHC(=O)-,
-OC(=O)-,
-OC(=O)O-,
-NHC(=O)O-,
-OC(=O)NH-, and
-NHC(=O)NH-.

Preferably, the linker contains an electrophilic functional group for reaction with a nucleophilic functional group on the cell binding agent. Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) maleimide groups (ii) activated disulfides, (iii) active esters such as NHS (N-hydroxysuccinimide) esters, HOBt (N-hydroxybenzotriazole) esters, haloformates, and acid halides; (iv) alkyl and benzyl halides such as haloacetamides; and (v) aldehydes, ketones, carboxyl, and, some of which are exemplified as follows:

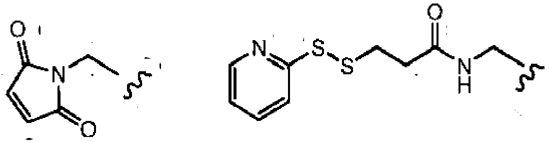

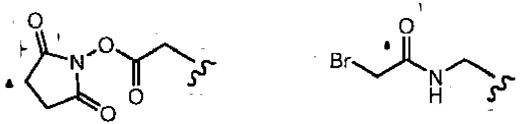

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues). US 7521541 teaches engineering antibodies by introduction of reactive cysteine amino acids.
In some embodiments, a Linker has a reactive nucleophilic group which is reactive with an electrophilic group present on an antibody. Useful electrophilic groups on an antibody include, but are not limited to, aldehyde and ketone carbonyl groups. The heteroatom of a nucleophilic group of a Linker can react with an electrophilic group on an antibody and form a covalent bond to an antibody unit. Useful nucleophilic groups on a Linker include, but are not limited to, hydrazide, oxime, amino, hydroxyl, hydrazine, thiosemicarbazone, hydrazine carboxylate, and arylhydrazide. The electrophilic group on an antibody provides a convenient site for attachment to a Linker.

In one embodiment, the group A is: 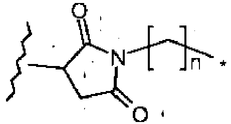 where the asterisk indicates the point of attachment to L¹, the wavy line indicates the point of attachment to the cell binding agent, and n is 0 to 6. In one embodiment, n is 5.

In one embodiment, the group A is: 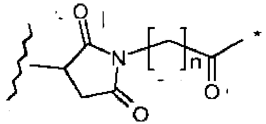 where the asterisk indicates the point of attachment to L¹, the wavy line indicates the point of attachment to the cell binding agent, and n is 0 to 6. In one embodiment, n is 5.

In one embodiment, the group A is: 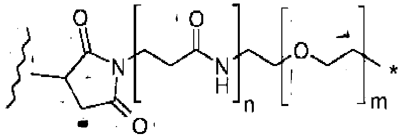 where the asterisk indicates the point of attachment to L¹, the wavy line indicates the point of attachment to the cell binding agent, n is 0 or 1, and m is 0 to 30. In a preferred embodiment, n is and m is 0 to 10, 1 to 8, preferably 4 to 8, and most preferably 4 or 8. In another embodiment, m is 10 to 30, and preferably 20 to 30. Alternatively, m is 0 to 50. In this embodiment, m is preferably 10-40 and n is 1.

In one embodiment, the group A is: 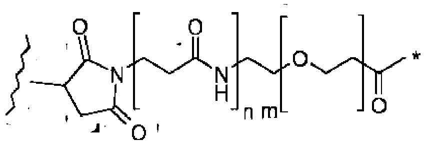 where the asterisk indicates the point of attachment to L¹, the wavy line indicates the point of attachment to the cell binding agent, n is 0 or 1, and m is 0 to 30. In a preferred embodiment, n is 1 and m is 0 to 10, 1 to 8, preferably 4 to 8, and most preferably 4 or 8. In another embodiment, m is 10 to 30, and preferably 20 to 30. Alternatively, m is 0 to 50. In this embodiment, m is preferably 10-40 and n is 1.

In one embodiment, the connection between the cell binding agent and A is through a thiol residue of the cell binding agent and a maleimide group of A.

In one embodiment, the connection between the cell binding agent and A is: 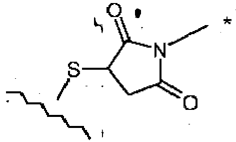 where the asterisk indicates the point of attachment to the remaining portion of A and the wavy line indicates the point of attachment to the remaining portion of the cell binding agent. In this embodiment, the S atom is typically derived from the cell binding agent.

In each of the embodiments above, an alternative functionality may be used in place of the maleimide-derived group shown below: 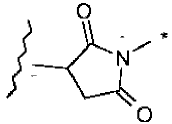 where the wavy line indicates the point of attachment to the cell binding agent as before, and the asterisk indicates the bond to the remaining portion of the A group.

In one embodiment, the maleimide-derived group is replaced with the group: 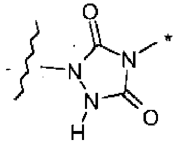 where the wavy line indicates point of attachment to the cell binding agent, and the asterisk indicates the bond to the remaining portion of the A group.

In one embodiment, the maleimide-derived group is replaced with a group, which optionally together with the cell binding agent, is selected from:
-C(=O)NH-,
-C(=O)O-,
-NHC(=O)-,
-OC(=O)-,
-OC(=O)O-,
-NHC(=O)O-,
-OC(=O)NH-,
-NHC(=O)NH-,
-NHC(=O)NH,
-C(=O)NHC(=O)-,
-S-,
-S-S-,
-CH₂C(=O)-
-C(=O)CH₂-,
=N-N H-, and
-NH-N=.

In one embodiment, the maleimide-derived group is replaced with a group, which optionally together with the cell binding agent, is selected from: 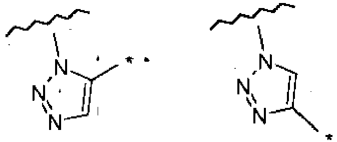 where the wavy line indicates either the poi nt of attachment to the cell binding agent or the bond to the remaining portion of the A group, and the asterisk indicates the other of the point of attachment to the cell binding agent or the bond to the remaining portion of the A group.

Other groups suitable for connecting L¹ to the cell binding agent are described in WO 2005/082023.

The group R¹⁰ is derivable from the group R^{L}. The group R^{L} may be converted to a group R¹⁰ by connection of a cell binding agent to a functional group of R^{L}. Other steps may be taken to convert R^{L} to R¹⁰. These steps may include the removal of protecting groups, where present, or the installation of an appropriate functional group.

### Q

In one embodiment, Q is selected from O, S, or N(H).
Preferably, Q is O.

### R¹¹

In one embodiment, R¹¹ is either H, or R or, where Q is O, SO₃M, where M is a metal cation.
In one embodiment, R¹¹ is H.
In one embodiment, R¹¹ is R.
In one embodiment, where Q is O, R¹¹ is SO₃M, where M is a metal cation. The cation may be Na⁺.

### R^{L}

In one embodiment, R^{L} is a linker for connection to a cell binding agent.

In one embodiment, the linker is provided with a functional group to form a connection to a cell binding agent. This application is particularly concerned with those R^{L} groups which have a carbamate link to the N10 position. The discussion of the linking group in R¹⁰ above is also relevant to their immediate precursors here.

R^{L} is different to R^{C}, which is not suitable for reaction with a cell binding agent. However, in some embodiments, R^{C} may be converted into a group R^{L}, for example by appropriate manipulation of the protecting groups and other functionalities that are, or form part of, R^{C}.

In one embodiment, R^{L} is a group: 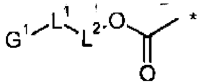 where the asterisk indicates the point of attachment to the N10 position, G¹ is a functional group to form a connection to a cell binding agent, L¹ is a linker, L² is a covalent bond or together with -OC(=O)- forms a self-immolative linker, and L¹ or L² is a cleavable linker.

L¹ and L² are as defined above in relation to R¹⁰. References to connection to A can be construed here as referring to a connection to G¹.

In one embodiment, where L¹ comprises an amino acid, the side chain of that amino acid may be protected. Any suitable protecting group may be used. In one embodiment, the side chain protecting groups are removable with other protecting groups in the compound, where present. In other embodiments, the protecting groups may be orthogonal to other protecting groups in the molecule, where present.

Suitable protecting groups for amino acid side chains include those groups described in the Novabiochem Catalog 2006/2007. Protecting groups for use in a cathepsin labile linker are also discussed in Dubowchik et al.

In certain embodiments of the invention, the group L¹ includes a Lys amino acid residue. The side chain of this amino acid may be protected with a Boc or Alloc protected group. A Boc protecting group is most preferred.

The functional group G¹ forms a connecting group A upon reaction with a cell binding agent.

In one embodiment, the functional group G¹ is or comprises an amino, carboxylic acid, hydroxyl, thiol, or maleimide group for reaction with an appropriate group on the cell binding agent. In a preferred embodiment, G¹ comprises a maleimide group.

In one embodiment, the group G¹ is an alkyl maleimide group. This group is suitable for reaction with thiol groups, particularly cysteine thiol groups, present in the cell binding agent, for example present in an antibody.

In one embodiment, the group G¹ is: 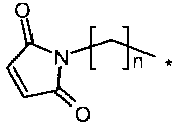 where the asterisk indicates the point of attachment to L¹ and n is 0 to 6. In one embodiment, n is 5.

In one embodiment, the group G¹ is: 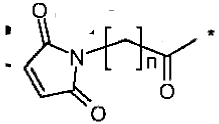 where the asterisk indicates the point of attachment to L¹ and n is 0 to 6. In one embodiment, n is 5.

In one embodiment, the group G¹ is: 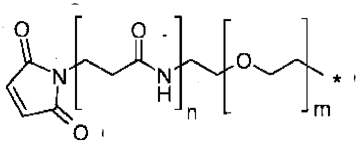 where the asterisk indicates the point of attachment to L¹, n is 0 or 1, and m is 0 to 30. In a preferred embodiment, n is 1 and m is 0 to 10, 1 to 2, preferably 4 to 8, and most preferably 4 or 8. Alternatively, m is 0 to 50. In this embodiment, m is preferably 10-40 and n is 1.

In one embodiment, the group G¹ is: 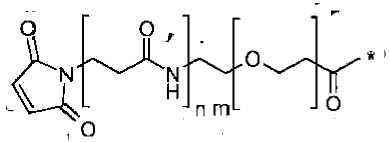 where the asterisk indicates the point of attachment to L¹, n is 0 or 1, and m is 0 to 30. In a preferred embodiment, n is 1 and m is 0 to 10, 1 to 8, preferably 4 to 8, and most preferably 4 or 8. Alternatively, m is 0 to 50. In this embodiment, m is preferably 10-40 and n is 1.

In each of the embodiments above, an alternative functionality may be used in place of the maleimide group shown below: 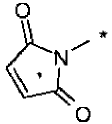 where asterisk indicates the bond to the remaining portion of the G group.

In one embodiment, the maleimide-derived group is replaced with the group: 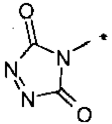 where the asterisk indicates the bond to the remaining portion of the G group.

In one embodiment, the maleimide group is replaced with a group selected from:
-C(=O)OH,
-OH,
-NH₂,
-SH,
-C(=O)CH₂D, where D is Cl, Br or I,
-CHO,
-NHNH₂
-C≡CH, and
-N₃ (azide).

In one embodiment, where L¹ is present, G¹ is -NH₂, -NHMe, -COOH, -OH or -SH.

In one embodiment, where L¹ is present, G¹ is -NH₂ or -NHMe. Either group may be the N-terminal of an L¹ amino acid sequence.

In one embodiment, where L¹ is present, G¹ is -NH₂, and L¹ is an amino acid sequence -X₁-X₂-, as defined above in relation to R¹⁰.

In one embodiment, where L¹ is present, G¹ is COOH. This group may be the C-terminal of an L¹ amino acid sequence.

In one embodiment, where L¹ is present, G¹ is OH.
In one embodiment, where L¹ is present, G¹ is SH.

The group G¹ may be convertable from one functional group to another. In one embodiment, where L¹ is present, G¹ is -NH₂. This group is convertable to another group G¹ comprising a maleimide group. For example, the group -NH₂ may be reacted with an acids or an activated acid (e.g. N-succinimide forms) of those G¹ groups comprising maleimide shown above.

The group G¹ may therefore be converted to a functional group that is more appropriate for reaction with a cell binding agent.

In other embodiments, R^{L} is a group that is a precursor to the linker that is provided with a functional group.

As noted above, in one embodiment, where L¹ is present, G¹ is -NH₂, -NHMe, -COOH, -OH or -SH. In a further embodiment, these groups are provided in a chemically protected form. The chemically protected form is therefore a precursor to the linker that is provided with a functional group.

In one embodiment, G¹ is -NH₂ in a chemically protected form. The group may be protected with a carbamate protecting group. The carbamate protecting group may be selected from the group consisting of:
Alloc, Fmoc, Boc, Troc, Teoc, Cbz and PNZ.
Preferably, where G¹ is -NH₂, it is protected with an Alloc or Fmoc group.

In one embodiment, where G¹ is -NH₂, it is protected with an Fmoc group.

In one embodiment, the protecting group is the same as the carbamate protecting group of the capping group.
In one embodiment, the protecting group is not the same as the carbamate protecting group of the capping group. In this embodiment, it is preferred that the protecting group is removable under conditions that do not remove the carbamate protecting group of the capping group.

The chemical protecting group may be removed to provide a functional group to form a connection to a cell binding agent. Optionally, this functional group may then be converted to another functional group as described above.

In one embodiment, the active group is an amine. This amine is preferably the N-terminal amine of a peptide, and may be the N-terminal amine of the preferred dipeptides of the invention.

The active group may be reacted to yield the functional group that is intended to form a connection to a cell binding agent.

In other embodiments, the linker is a precursor to the linker having an active group. In this embodiment, the linker comprises the active group, which is protected by way of a protecting group. The protecting group may be removed to provide the linker having an active group. Where the active group is an amine, the protecting group may be an amine protecting group, such as those described in Green and Wuts.

The protecting group is preferably orthogonal to other protecting groups, where present, in the group R^{L}.

In one embodiment, the protecting group is orthogonal to the capping group. Thus, the active group protecting group is removable whilst retaining the capping group. In other embodiments, the protecting group and the capping group is removable under the same conditions as those used to remove the capping group.

In one embodiment, R^{L} is: 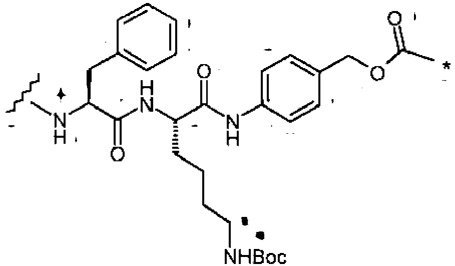 where the asterisk indicates the point of attachment to the N10 position, and the wavy line indicates the point of attachment to the remaining portion of the linker L¹ or the point of attachment to G¹. Preferably, the wavy line indicates the point of attachment to G¹.

In one embodiment, R^{L} is: 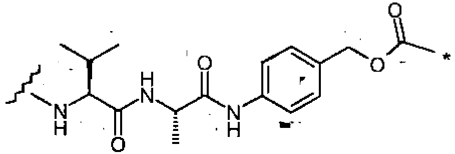 where the asterisk and the wavy line are as defined above.

In one embodiment, R^{L} is:: 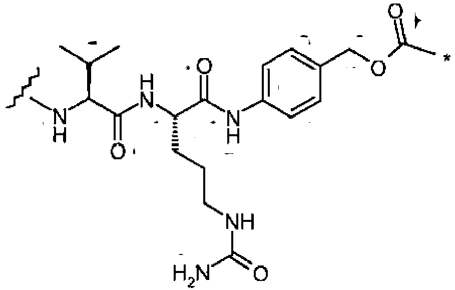 where the asterisk and the wavy line are as defined above.

Other functional groups suitable for use in forming a connection between L¹ and the cell binding agent are described in WO 2005/082023.

Linkers can include protease-cleavable peptidic moieties comprising one or more amino acid units. Peptide linker reagents may be prepared by solid phase or liquid phase synthesis methods (E. Schröder and K. Lübke, The Peptides, volume 1, pp 76-136 (1965) Academic Press) that are well known in the field of peptide chemistry, including t-BOC chemistry (Geiser et al "Automation of solid-phase peptide synthesis" in Macromolecular Sequencing and Synthesis, Alan R. Liss, Inc., 1988, pp. 199-218) and Fmoc/HBTU chemistry (Fields, G. and Noble, R. (1990) "Solid phase peptide synthesis utilizing 9-fluoroenylmethoxycarbonyl amino acids", Int. J. Peptide Protein Res. 35:161-214), on an automated synthesizer such as the Rainin Symphony Peptide Synthesizer (Protein Technologies, Inc., Tucson, AZ), or Model 433 (Applied Biosystems, Foster City, CA).

Exemplary amino acid linkers include a dipeptide, a tripeptide, a tetrapeptide or a pentapeptide. Exemplary dipeptides include: valine-citrulline (vc or val-cit), alanine-phenylalanine (af or ala-phe). Exemplary tripeptides include: glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. Amino acid linker components can be designed and optimized in their selectivity for enzymatic cleavage by a particular enzymes, for example, a tumor-associated protease, cathepsin B, C and D, or a plasmin protease.

Amino acid side chains include those occurring naturally, as well as minor amino acids and non-naturally occurring amino acid analogs, such as citrulline. Amino acid side chains include hydrogen, methyl, isopropyl, isobutyl, sec-butyl, benzyl, p-hydroxybenzyl, -CH₂OH,-CH(OH)CH₃, -CH₂CH₂SCH₃, -CH₂CONH₂, -CH₂COOH, -CH₂CH₂CONH₂, -CH₂CH₂COOH,-(CH₂)₃NHC(=NH)NH₂, -(CH₂)₃NH₂, -(CH₂)₃NHCOCH₃, -(CH₂)₃NHCHO,-(CH₂)₄NHC(=NH)NH₂, -(CH₂)₄NH₂, -(CH₂)₄NHCOCH₃, -(CH₂)₄NHCHO, -(CH₂)₃NHCONH₂,-(CH₂)₄NHCONH₂, -CH₂CH₂CH(OH)CH₂NH₂, 2-pyridylmethyl-, 3-pyridylmethyl-, 4-pyridylmethyl-, phenyl, cyclohexyl, as well as the following structures: 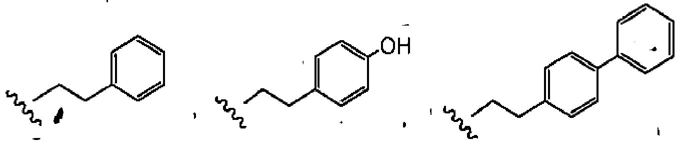 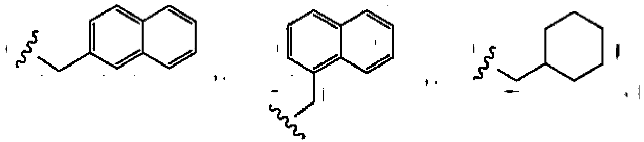 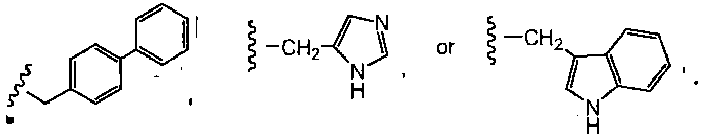 When the amino acid side chains include other than hydrogen (glycine), the carbon atom to which the amino acid side chain is attached is chiral. Each carbon atom to which the amino acid side chain is attached is independently in the (S) or (R) configuration, or a racemic mixture. Drug-linker reagents may thus be enantiomerically pure, racemic, or diastereomeric.

In exemplary embodiments, amino acid side chains are selected from those of natural and non-natural amino acids, including alanine, 2-amino-2-cyclohexylacetic acid, 2-amino-2-phenylacetic acid, arginine, asparagine, aspartic acid, cysteine, glutamine, glutamic acid, glycine, histidine, isoleucine, leucine, lysine, methionine, norleucine, phenylalanine, proline, serine, threonine, tryptophan, tyrosine, valine, γ-aminobutyric acid, α,α-dimethyl γ-aminobutyric acid, β,β-dimethyl γ-aminobutyric acid, ornithine, and citrulline (Cit).

An exemplary valine-citrulline (val-cit-or vc) dipeptide linker reagent useful for constructing a linker-PBD drug moiety intermediate for conjugation to a cell binding agent, e.g. an antibody, having a para-aminobenzylcarbamoyl (PAB) self-immolative spacer has the structure: 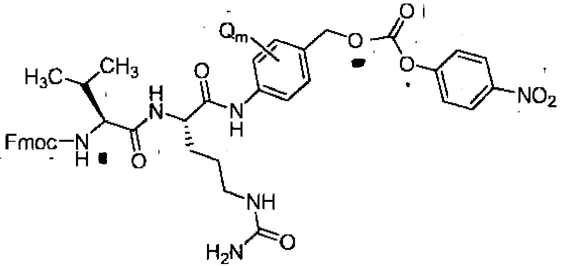 where Q is C₁-C₈ alkyl, -O-(C₁-C₈ alkyl), -halogen, -NO₂ or -CN; and m is an integer ranging from 0-4.

An exemplary phe-lys(Mtr) dipeptide linker reagent having a p-aminobenzyl group can be prepared according to Dubowchik, et al. (1997) Tetrahedron Letters, 38:5257-60, and has the structure: 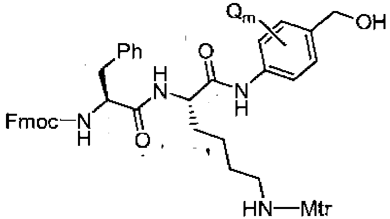 where Mtr is mono-4-methoxytrityl, Q is C₁-C₈ alkyl, -O-(C₁-C₈ alkyl), -halogen, -NO₂ or-CN; and m is an integer ranging from 0-4.

The "self-immolative linker" PAB (para-aminobenzyloxycarbonyl), attaches the drug moiety to the antibody in the antibody drug conjugate (Carl et al (1981) J. Med. Chem. 24:479-480; Chakravarty etal (1983) J. Med. Chem. 26:638-644; US 6214345; US20030130189; US20030096743; US6759509; US20040052793; US6218519; US6835807; US6268488; US20040018194; WO98/13059; US20040052793; US6677435; US5621002; US20040121940; WO2004/032828). Other examples of self-immolative spacers besides PAB include, but are not limited to: (i) aromatic compounds that are electronically similar to the PAB group such as 2-aminoimidazol-5-methanol derivatives (Hay et al. (1999) Bioorg. Med. Chem. Lett. 9:2237), thiazoles (US 7375078), multiple, elongated PAB units (de Groot et al (2001) J. Org. Chem. 66:8815-8830); and ortho or para-aminobenzylacetals; and (ii) homologated styryl PAB analogs (US 7223837). Spacers can be used that undergo cyclization upon amide bond hydrolysis, such as substituted and unsubstituted 4-aminobutyric acid amides (Rodrigues et al (1995) Chemistry Biology 2:223), appropriately substituted bicyclo[2.2.1] and bicyclo[2.2.2] ring systems (Storm et al (1972) J. Amer. Chem. Soc. 94:5815) and 2-aminophenylpropionic acid amides (Amsberry, et al (1990) J. Org. Chem. 55:5867). Elimination of amine-containing drugs that are substituted at glycine (Kingsbury et al (1984) J. Med. Chem. 27:1447) are also examples of self-immolative spacers useful in ADC.

In one embodiment, a valine-citrulline dipeptide PAB analog reagent has a 2,6 dimethyl phenyl group and has the structure:

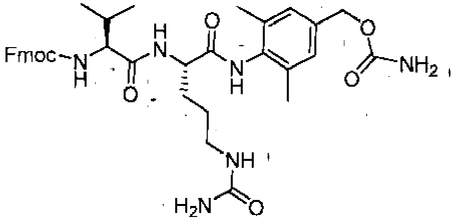

Linker reagents useful for the antibody drug conjugates of the invention include, but are not limited to: BMPEO, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate), and bis-maleimide reagents: DTME, BMB, BMDB, BMH, BMOE, 1,8-bis-maleimidediethyleneglycol (BM(PEO)₂), and 1,11-bis-maleimidotriethyleneglycol (BM(PEO)₃), which are commercially available from Pierce Biotechnology, Inc., ThermoScientific, Rockford, IL, and other reagent suppliers. Bis-maleimide reagents allow the attachment of a free thiol group of a cysteine residue of an antibody to a thiol-containing drug moiety, label, or linker intermediate, in a sequential or concurrent fashion. Other functional groups besides maleimide, which are reactive with a thiol group of an antibody, PBD drug moiety, or linker intermediate include iodoacetamide, bromoacetamide, vinyl pyridine, disulfide, pyridyl disulfide, isocyanate, and isothiocyanate. 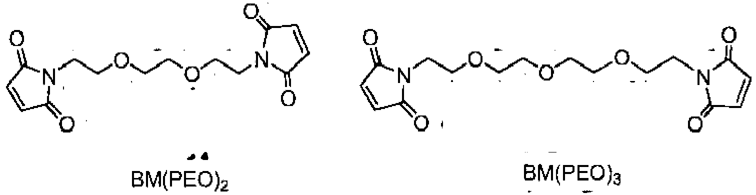 Other embodiments of linker reagents are: N-succinimidyl-4-(2-pyridylthio)pentanoate (SPP), N-succinimidyl-3-(2-pyridyldithio) propionate (SPDP, Carlsson et al (1978) Biochem. J. 173:723-737), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Useful linker reagents can also be obtained via other commercial sources, such as Molecular Biosciences Inc.(Boulder, CO), or synthesized in accordance with procedures described in Toki et al (2002) J. Org. Chem. 67:1866-1872; US 6214345; WO 02/088172; US 2003130189; US2003096743; WO 03/026577; WO 03/043583; and WO 04/032828.

The Linker may be a dendritic type linker for covalent attachment of more than one drug moiety through a branching, multifunctional linker moiety to an antibody (US 2006/116422; US 2005/271615; de Groot et al (2003) Angew. Chem. Int. Ed. 42:4490-4494; Amir et al (2003) Angew. Chem. Int. Ed. 42:4494-4499; Shamis et al (2004) J. Am. Chem. Soc. 126:1726-1731; Sun et al (2002) Bioorganic & Medicinal Chemistry Letters 12:2213-2215; Sun et al (2003) Bioorganic & Medicinal Chemistry 11:1761-1768; King et al (2002) Tetrahedron Letters 43:1987-1990). Dendritic linkers can increase the molar ratio of drug to antibody, i.e. loading, which is related to the potency of the ADC. Thus, where an antibody bears only one reactive cysteine thiol group, a multitude of drug moieties may be attached through a dendritic or branched linker.

One exemplary embodiment of a dendritic type linker has the structure: 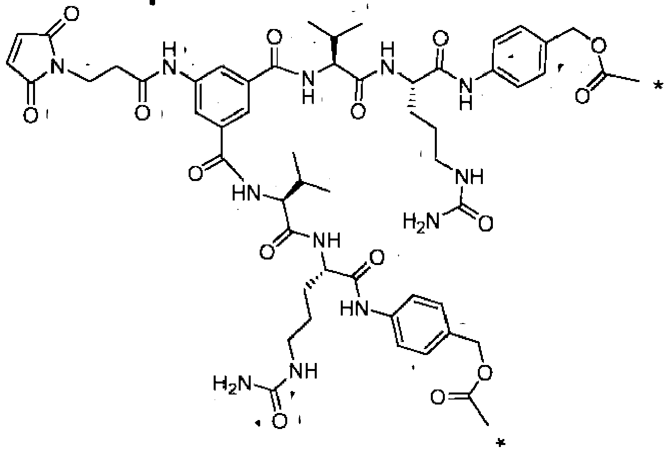 where the asterisk indicate the point of attachment to the N10 position of a PBD moiety.

### Cell Binding Agent

A cell binding agent may be of any kind, and include peptides and non-peptides. These can include antibodies or a fragment of an antibody that contains at least one binding site, lymphokines, hormones, growth factors, nutrient-transport molecules, or any other cell binding molecule or substance.

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, *i.e.,* a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass of immunoglobulin molecule. The immunoglobulins can be derived from any species, including human, murine, or rabbit origin.

"Antibody fragments" comprise a portion of a full length antibody, generally the antigen binding or variable region thereof. Examples of antibody fragments include Fab, Fab', F(ab')₂, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al (1975) Nature 256:495, or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising a VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

Examples of cell binding agents include those agents described for use in WO 2007/085930, which is incorporated herein.

The cell binding agent may be, or comprise, a polypeptide. The polypeptide may be a cyclic polypeptide. The cell binding agent may be antibody. Thus, in one embodiment, the present invention provides an antibody-drug conjugate (ADC).

### Drug loading

The drug loading is the average number of PBD drugs per antibody. Drug loading may range from 1 to 8 drugs (D) per antibody (Ab), i.e. where 1, 2, 3, 4, 5, 6, 7, and 8 drug moieties are covalently attached to the antibody. Compositions of ADC include collections of antibodies conjugated with a range of drugs, from 1 to 8. The average number of drugs per antibody in preparations of ADC from conjugation reactions may be characterized by conventional means such as mass spectroscopy, ELISA assay, electrophoresis, and HPLC. The quantitative distribution of ADC in terms of p may also be determined. By ELISA, the averaged value of p in a particular preparation of ADC may be determined (Hamblett et al (2004) Clin. Cancer Res. 10:7063-7070; Sanderson et al (2005) Clin. Cancer Res. 11:843-852). However, the distribution of p (drug) values is not discernible by the antibody-antigen binding and detection limitation of ELISA. Also, ELISA assay for detection of antibody-drug conjugates does not determine where the drug moieties are attached to the antibody, such as the heavy chain or light chain fragments, or the particular amino acid residues. In some instances, separation, purification, and characterization of homogeneous ADC where p is a certain value from ADC with other drug loadings may be achieved by means such as reverse phase HPLC or electrophoresis.

For some antibody-drug conjugates, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.

Typically, fewer than the theoretical maximum of drug moieties are conjugated to an antibody during a conjugation reaction. An antibody may contain, for example, many lysine residues that do not react with the drug-linker intermediate (D-L) or linker reagent. Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. The loading (drug/antibody ratio) of an ADC may be controlled in several different manners, including: (i) limiting the molar excess of drug-linker intermediate (D-L) or linker reagent relative to antibody, (ii) limiting the conjugation reaction time or temperature, and (iii) partial or limiting reductive conditions for cysteine thiol modification.

Cysteine amino acids may be engineered at reactive sites in an antibody and which do not form intrachain or intermolecular disulfide linkages (Junutula, et al., 2008b Nature Biotech., 26(8):925-932; Dornan et al (2009) Blood 114(13):2721-2729; US 7521541; US 7723485; WO2009/052249). The engineered cysteine thiols may react with linker reagents or the drug-linker reagents of the present invention which have thiol-reactive, electrophilic groups such as maleimide or alpha-halo amides to form ADC with cysteine engineered antibodies and the PBD drug moieties. The location of the drug moiety can thus be designed, controlled, and known. The drug loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody. A drug loading near 2 can be achieved and near homogeneity of the conjugation product ADC.

Where more than one nucleophilic or electrophilic group of the antibody reacts with a drug-linker intermediate, or linker reagent followed by drug moiety reagent, then the resulting product is a mixture of ADC compounds with a distribution of drug moieties attached to an antibody, e.g. 1, 2, 3, etc. Liquid chromatography methods such as polymeric reverse phase (PLRP) and hydrophobic interaction (HIC) may separate compounds in the mixture by drug r loading value. Preparations of ADC with a single drug loading value (p) may be isolated, however, these single loading value ADCs may still be heterogeneous mixtures because the drug moieties may be attached, via the linker, at different sites on the antibody.

Thus the antibody-drug conjugate compositions of the invention include mixtures of antibody-drug conjugate compounds where the antibody has one or more PBD drug moieties and where the drug moieties may be attached to the antibody at various amino acid residues.

In one embodiment, the average number of monomer or dimer pyrrolobenzodiazepine groups per cell binding agent is in the range 1 to 20. In some embodiments the range is selected from 1 to 8, 2 to 8, 2 to 6, 2 to 4, and 4 to 8.
In some embodiments, there is one monomer or dimer pyrrolobenzodiazepine groups per cell binding agent.

### Peptides

In one embodiment, the cell binding agent is a linear or cyclic peptide comprising 4-20, preferably 6-20, contiguous amino acid residues. In this embodiment, it is preferred that one cell binding agent is linked to one monomer or dimer pyrrolobenzodiazepine compound.

In one embodiment the cell binding agent comprises a peptide that binds integrin αᵥβ₆. The peptide may be selective for αᵥβ₆ over XYS.

In one embodiment the cell binding agent comprises the A20FMDV-Cys polypeptide. The A20FMDV-Cys has the sequence: NAVPNLRGDLQVLAQKVARTC. Alternatively, a variant of the A20FMDV-Cys sequence may be used wherein one, two, three, four, five, six, seven, eight, nine or ten amino acid residues is substituted with another amino acid residue.

In one embodiment the antibody is a monoclonal antibody; chimeric antibody; humanized antibody; fully human antibody; or a single chain antibody. One embodiment the antibody is a fragment of one of these antibodies having biological activity. Examples of such fragments include Fab, Fab', F(ab')₂ and Fv fragments.

In these embodiments, each antibody may be linked to one or several monomer or dimer pyrrolobenzodiazepine groups. The preferred ratios of pyrrolobenzodiazepine to cell binding agent are given above.

The antibody may be a domain antibody (DAB).

In one embodiment, the antibody is a monoclonal antibody.

Antibodies for use in the present invention include those antibodies described in WO 2005/082023 which is incorporated herein. Particularly preferred are those antibodies for tumour-associated antigens. Examples of those antigens known in the art include, but are not limited to, those tumour-associated antigens set out in WO 2005/082023. See, for instance, pages 41-55.

The conjugates of the invention are designed to target tumour cells via their cell surface antigens. The antigens are usually normal cell surface antigens which are either over-expressed or expressed at abnormal times. Ideally the target antigen is expressed only on proliferative cells (preferably tumour cells), however this is rarely observed in practice. As a result, target antigens are usually selected on the basis of differential expression between proliferative and healthy tissue.

Antibodies have been raised to target specific tumour related antigens including:
Cripto, CD30, CD19, CD33, Glycoprotein NMB, CanAg, Her2 (ErbB2/Neu), CD56 (NCAM), CD22 (Siglec2), CD33 (Siglec3), CD79, CD138, PSCA, PSMA (prostate specific membrane antigen), BCMA, CD20, CD70, E-selectin, EphB2, Melanotransferin, Muc16 and TMEFF2.

Tumor-associated antigens (TAA) are known in the art, and can prepared for use in generating antibodies using methods and information which are well known in the art. In attempts to discover effective cellular targets for cancer diagnosis and therapy, researchers have sought to identify transmembrane or otherwise tumor-associated polypeptides that are specifically expressed on the surface of one or more particular type(s) of cancer cell as compared to on one or more normal non-cancerous cell(s). Often, such tumor-associated polypeptides are more abundantly expressed on the surface of the cancer cells as compared to on the surface of the non-cancerous cells. The identification of such tumor-associated cell surface antigen polypeptides has given rise to the ability to specifically target cancer cells for destruction via antibody-based therapies.

Examples of TAA include, but are not limited to, TAA (1)-(36) listed below. For convenience, information relating to these antigens, all of which are known in the art, is listed below and includes names, alternative names, Genbank accession numbers and primary reference(s), following nucleic acid and protein sequence identification conventions of the National Center for Biotechnology information (NCBI). Nucleic acid and protein sequences corresponding to TAA (1)-(36) are available in public databases such as GenBank. Tumor-associated antigens targeted by antibodies include all amino acid sequence variants and isoforms possessing at least about 70%, 80%, 85%, 90%, or 95% sequence identity relative to the sequences identified in the cited references, or which exhibit substantially the same biological properties or characteristics as a TAA having a sequence found in the cited references. For example, a TAA having a variant sequence generally is able to bind specifically to an antibody that binds specifically to the TAA with the corresponding sequence listed. The sequences and disclosure in the reference specifically recited herein are expressly incorporated by reference.

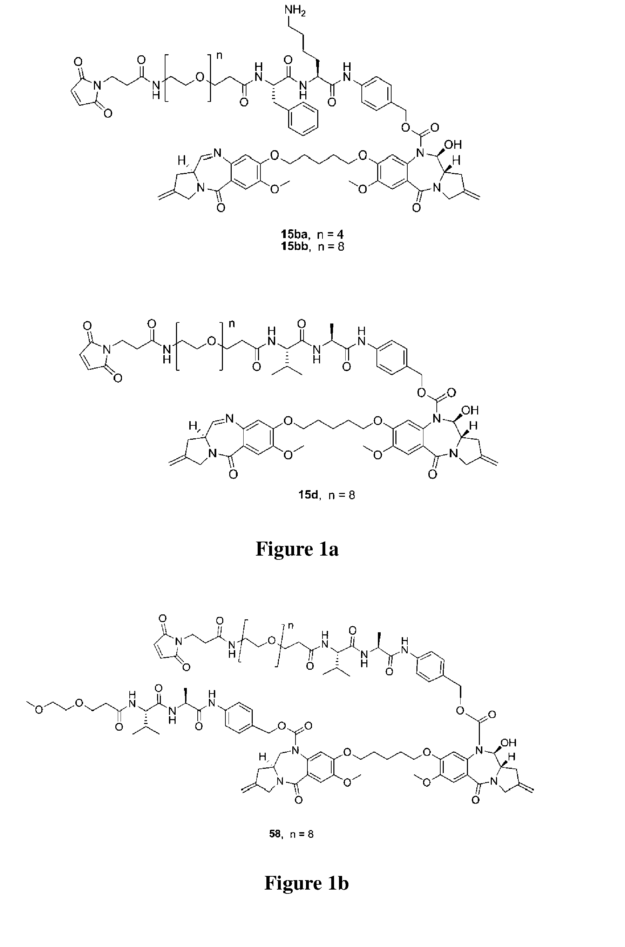

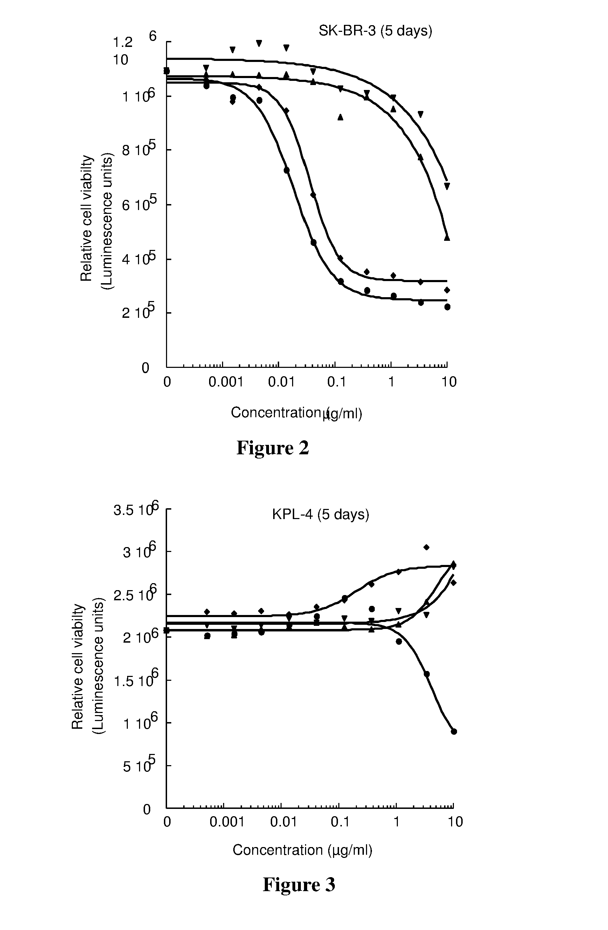

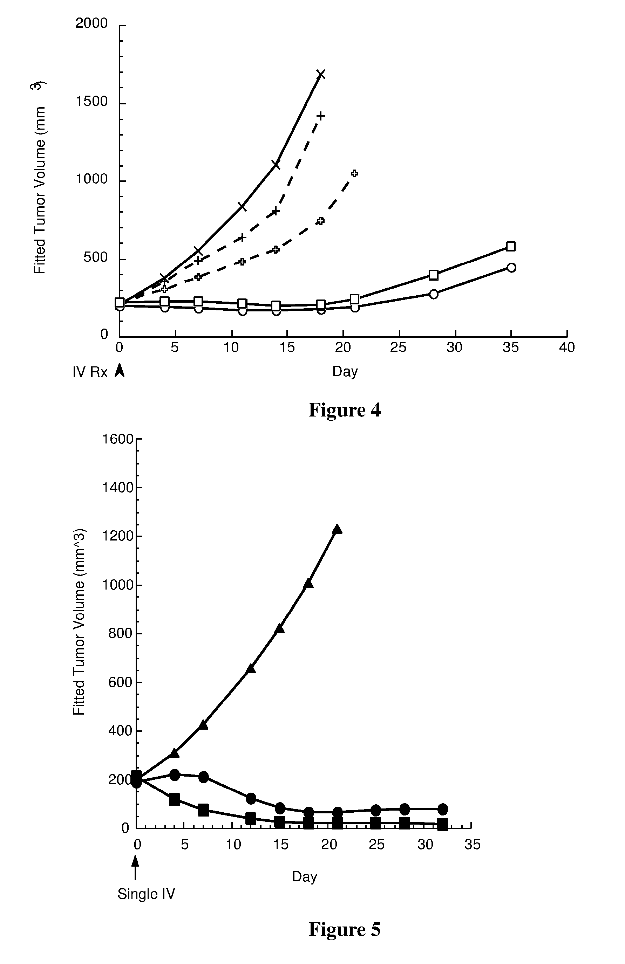

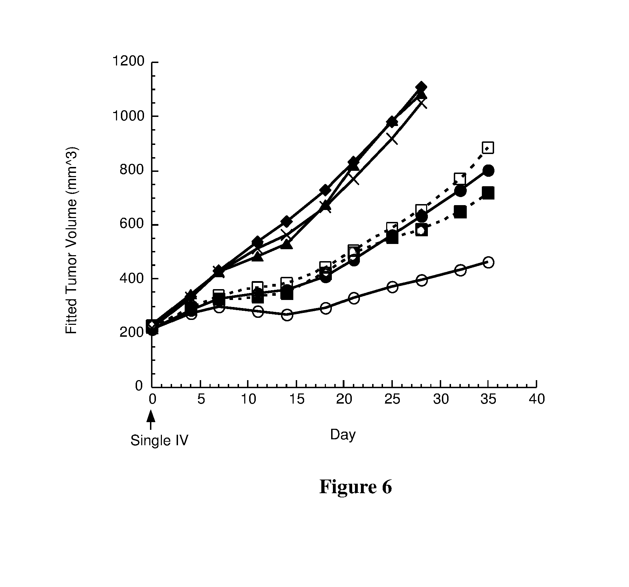

TUMOR-ASSOCIATED ANTIGENS (1)-(36):
(1) BMPR1B (bone morphogenetic protein receptor-type IB, Genbank accession no. NM_001203) ten Dijke,P., et al Science 264 (5155):101-104 (1994), Oncogene 14 (11):1377-1382 (1997)); WO2004/063362 (Claim 2); WO2003/042661 (Claim 12); US2003/134790-A1 (Page 38-39); WO2002/102235 (Claim 13; Page 296); WO2003/055443 (Page 91-92); WO2002/99122 (Example 2; Page 528-530); WO2003/029421 (Claim 6); WO2003/024392 (Claim 2; Fig 112); WO2002/98358 (Claim 1; Page 183); WO2002/54940 (Page 100-101); WO2002/59377 (Page 349-350); WO2002/30268 (Claim 27; Page 376); WO2001/48204 (Example; Fig 4); NP_001194 bone morphogenetic protein receptor, type IB /pid=NP_001194.1. Cross-references: MIM:603248; NP_001194.1; AY065994
(2) E16 (LAT1, SLC7A5, Genbank accession no. NMR_003486) Biochem. Biophys. Res. Commun. 255 (2), 283-288 (1999), Nature 395 (6699):288-291 (1998), Gaugitsch, H.W., et al (1992) J. Biol. Chem. 267 (16):11267-11273); WO2004/048938 (Example 2); WO2004/032842 (Example IV); WO2003/042661 (Claim 12); WO2003/016475 (Claim 1); WO2002/78524 (Example 2); WO2002/99074 (Claim 19; Page 127-129); WO2002/86443 (Claim 27; Pages 222, 393); WO2003/003906 (Claim 10; Page 293); WO2002/64798 (Claim 33; Page 93-95); WO2000/14228 (Claim 5; Page 133-136); US2003/224454 (Fig 3); WO2003/025138 (Claim 12; Page 150); NP_003477 solute carrier family 7 (cationic amino acid transporter, y+system), member 5 /pid=NP_003477.3 - Homo sapiens; Cross-references: MIM:600182; NP_003477.3; NM_015923; NMR_003486_1
(3) STEAP1 (six transmembrane epithelial antigen of prostate, Genbank accession no NM_012449); Cancer Res. 61 (15), 5857-5860 (2001), Hubert, R.S., et al (1999) Proc. Natl. Acad. Sci. U.S.A. 96 (25):14523-14528); W02004/065577 (Claim 6); WO2004/027049 (Fig-1L); EP1394274 (Example 11); WO2004/016225 (Claim 2); WO2003/042661 (Claim 12); US2003/157089 (Example 5); US2003/185830 (Example 5); US2003/064397 (Fig 2); WO2002/89747 (Example 5; Page 618-619); WO2003/022995 (Example 9; Fig 13A, Example 53; Page 173, Example 2; Fig 2A); NP_036581 six transmembrane epithelial antigen of the prostate; Cross-references: MIM:604415; NP_036581.1; NM_012449_1
(4) 0772P (CA125, MUC16, Genbank accession no. AF361486); J. Biol. Chem. 276 (29):27371-27375 (2001)); WO2004/045553 (Claim 14); WO2002/92836 (Claim 6; Fig 12); WO2002/83866 (Claim 15; Page 116-121); US2003/124140 (Example 16); Cross-references: GI:34501467; AAK74120.3; AF361486_1
(5) MPF (MPF, MSLN, SMR, megakaryocyte potentiating factor, mesothelin, Genbank accession no. NM_005823) Yamaguchi, N., et al Biol. Chem. 269 (2), 805-808 (1994), Proc. Natl. Acad. Sci. U.S.A. 96 (20):11531-11536 (1999), Proc. Natl. Acad. Sci. U.S.A. 93 (1):136-140 (1996), J. Biol. Chem. 270 (37):21984-21990 (1995)); WO2003/101283 (Claim 14); (WO2002/102235 (Claim 13; Page 287-288); WO2002/101075 (Claim 4; Page 308-309); WO2002/71928 (Page 320-321); WO94/10312 (Page 52-57); Cross-references: MIM:601051; NP_005814.2; NMR_005823_1
(6) Napi3b (NAPI-3B, NPTlIb, SLC34A2, solute carrier family 34 (sodium phosphate), member 2, type II sodium-dependent phosphate transporter 3b, Genbank accession no. NM_006424) J. Biol. Chem. 277 (22):19665-19672 (2002), Genomics 62 (2):281-284 (1999), Feild, J.A., et al (1999) Biochem. Biophys. Res. Commun. 258 (3):578-582); WO2004/022778 (Claim 2); EP1394274 (Example 11); WO2002/102235 (Claim 13; Page 326); EP0875569 (Claim 1; Page 17-19); WO2001/57188 (Claim 20; Page 329); WO2004/032842 (Example IV); WO2001/75177 (Claim 24; Page 139-140); Cross-references: MIM:604217; NP_006415.1; NMR_006424_1
(7) Sema 5b (FLJ10372, KIAA1445, Mm.42015, SEMA5B, SEMAG, Semaphorin 5b Hlog, , sema domain, seven thrombospondin repeats (type 1 and type 1-like), transmembrane domain (TM) and short cytoplasmic domain, (semaphorin) 5B, Genbank accession no. AB040878); Nagase T., et al (2000) DNA Res. 7 (2):143-150): WO2004/000997 (Claim 1); WO2003/003984 (Claim 1); WO2002/06339 (Claim 1; Page 50); WO2001/88133 (Claim 1; Page 41-43, 48-58); WO2003/054152 (Claim 20); WO2003/101400 (Claim 11); Accession: Q9P283; EMBL; AB040878; BAA95969.1. Genew; HGNC:10737
(8) PSCA hlg (2700050C12Rik, C530008016Rik, RIKEN cDNA2700050C12, RIKEN cDNA 2700050C12 gene, Genbank accession no. AY358628); Ross et al (2002) Cancer Res. 62:2546-2553; US2003/129192 (Claim 2); US2004/044180 (Claim 12); US2004/044179 (Claim 11); US2003/096961 (Claim 11); US2003/232056 (Example 5); WO2003/105758 (Claim 12); US2003/206918 (Example 5); EP1347046 (Claim 1); WO2003/025148 (Claim 20); Cross-references: GI:37182378; AAQ88991.1; AY358628_1
(9) ETBR (Endothelin type B receptor, Genbank accession no. AY275463); Nakamuta M., et al Biochem. Biophys. Res. Commun. 177, 34-39, 1991; Ogawa Y., et al Biochem. Biophys. Res. Commun. 178, 248-255, 1991; Arai H., et al Jpn. Circ. J. 56, 1303-1307, 1992; Arai H., et al J. Biol. Chem. 268, 3463-3470, 1993; Sakamoto A., Yanagisawa M., et al Biochem. Biophys. Res. Commun. 178, 656-663, 1991; Elshourbagy N.A., et al J. Biol. Chem. 268, 3873-3879, 1993; Haendler B., et al J. Cardiovasc. Pharmacol. 20, s1-S4, 1992; Tsutsumi M., et al Gene 228, 43-49, 1999; Strausberg R.L., et al Proc. Natl. Acad. Sci. U. S.A. 99, 16899-16903, 2002; Bourgeois C., et al J. Clin. Endocrinol. Metab. 82, 3116-3123, 1997; Okamoto Y., et al Biol. Chem. 272, 21589-21596, 1997; Verheij J.B., et al Am. J. Med. Genet. 108, 223-225, 2002; Hofstra R.M.W., et al Eur. J. Hum. Genet. 5, 180-185, 997; Puffenberger E.G., et al Cell 79, 1257-1266, 1994; Attie T., et al, Hum. Mol. Genet. 4, 2407-2409, 1995; Auricchio A., et al Hum. Mol. Genet. 5:351-354, 1996; Amiel J., et al Hum. Mol. Genet. 5, 355-357, 1996; Hofstra R.M.W., et al Nat. Genet. 12, 445-447, 1996; Svensson P.J., et al Hum. Genet. 103, 145-148, 1998; Fuchs S., et al Mol. Med. 7, 115-124, 2001; Pingault V., et al (2002) Hum. Genet, 111, 198-206; WO2004/045516 (Claim 1); WO2004/048938 (Example 2); WO2004/040000 (Claim 151); WO2003/087768 (Claim 1); WO2003/016475 (Claim 1); WO2003/016475 (Claim 1); WO2002/61087 (Fig 1); WO2003/016494 (Fig 6); WO2003/025138 (Claim 12; Page 144); WO2001/98351 (Claim 1; Page 124-125); EP0522868 (Claim 8; Fig 2); WO2001/77172 (Claim 1; Page 297-299); US2003/109676; US6518404 (Fig 3); US5773223 (Claim 1a; Col 31-34); WO2004/001004
(10) MSG783 (RNF124, hypothetical protein FLJ20315, Genbank accession no. NM_017763); WO2003/104275 (Claim 1); WO2004/046342 (Example 2); WO2003/042661 (Claim 12); WO2003/083074 (Claim 14; Page 61); WO2003/018621 (Claim 1); WO2003/024392 (Claim 2; Fig 93); WO2001/66689 (Example 6); Cross-references: - LocusID:54894; Nip_060233.2; NM_017763_1
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no.. AF455138); Lab. Invest. 82 (11): 1573-1582 (2002)); WO2003/087306; US2003/064397 (Claim 1; Fig 1); WO2002/72596 (Claim 13; Page 54-55); WO2001/72962 (Claim 1; Fig 4B); WO2003/104270 (Claim 11); WO2003/104270 (Claim 16); US2004/005598 (Claim 22); WO2003/042661 (Claim 12); US2003/060612 (Claim 12; Fig 10); WO2002/26822 (Claim 23; Fig 2); WO2002/16429 (Claim 12; Fig 10); Cross-references: GI:22655488; AAN04080.1; AF455138_1
(12) TrpM4 (BR22450, FLJ20041, TRPM4, TRPM4B, transient receptor potential cation channel, subfamily M, member 4, Genbank accession no. NM_017636); Xu, X.Z., et al Proc. Natl. Acad. Sci. U.S.A. 98 (19):10692-10697 (2001), Cell 109 (3):397-407 (2002), J. Biol. Chem. 278 (33):30813-30820 (2003)); US2003/143557 (Claim 4); WO2000/40614 (Claim 14; Page 100-103); WO2002/10382 (Claim 1; Fig 9A); WO2003/042661 (Claim 12); WO2002/30268 (Claim 27; Page 391); US2003/219806 (Claim 4); WO2001/62794 (Claim 14; Fig 1A-D); Cross-references: MIM:606936; NP_060106.2; NM_017636_1
(13) CRIPTO (CR, CR1, CRGF, CRIPTO, TDGF1, teratocarcinoma-derived growth factor, Genbank accession no. NP_003203 or NMR_003212); Ciccodicola, A., et al EMBO J. 8 (7):1987-1991 (1989), Am. J. Hum. Genet. 49 (3):555-565 (1991)); US2003/224411 (Claim 1); WO2003/083041 (Example 1); WO2003/034984 (Claim 12); WO2002/88170 (Claim 2; Page 52-53); WO2003/024392 (Claim 2; Fig 58); WO2002/16413 (Claim 1; Page 94-95, 105); WO2002/22808 (Claim 2; Fig 1); US5854399 (Example 2; Col 17-18); US5792616 (Fig-2); Cross-references: MIM:187395; NP_003203.1; NM_003212_1
(14) CD21 (CR2 (Complement receptor 2) or C3DR (C3d/Epstein Barr virus receptor) or Hs.73792 Genbank accession no. M26004); Fujisaku et al (1989) J. Biol. Chem. 264 (4):2118-2125); Weis J.J., et al J. Exp. Med. 167, 1047-1066, 1988; Moore M., et al Proc. Natl. Acad. Sci. U.S.A. 84, 9194-9198, 1987; Barel M., et al Mol. Immunol. 35, 1025-1031, 1998; Weis J.J., et al Proc. Natl. Acad. Sci. U.S.A. 83, 5639-5643, 1986; Sinha S.K., et al (1993) J. Immunol. 150, 5311-5320; WO2004/045520 (Example 4); US2004/005538 (Example 1); WO2003/062401 (Claim 9); WO2004/045520 (Example 4); WO91/02536 (Fig 9.1-9.9); WO2004/020595 (Claim 1); Accession: P20023; Q13866; Q14212; EMBL; M26004; AAA35786.1.
(15) CD79b (CD79B, CD79β, IGb (immunoglobulin-associated beta), B29, Genbank accession no. NMR_000626 or 11038674); Proc. Natl. Acad. Sci. U.S.A. (2003) 100 (7):4126-4131, Blood (2002) 100 (9):3068-3076, Muller et al (1992) Eur. J. Immunol. 22 (6):1621-1625); WO2004/016225 (claim 2, Fig 140); WO2003/087768, US2004/101874 (claim 1, page 102); WO2003/062401 (claim 9); WO2002/78524 (Example 2); US2002/150573 (claim 5, page 15); US5644033; WO2003/048202 (claim 1, pages 306 and 309); WO 99/58658, US6534482 (claim 13, Fig 17A/B); WO2000/55351 (claim 11, pages 1145-1146); Cross-references: MIM:147245; NP_000617.1; NM_000626_1
(16) FcRH2 (IFGP4, IRTA4, SPAP1A (SH2 domain containing phosphatase anchor protein 1a), SPAP1B, SPAP1C, Genbank accession no. NMR_030764, AY358130); Genome Res. 13 (10):2265-2270 (2003), Immunogenetics 54 (2):87-95 (2002), Blood 99 (8):2662-2669 (2002), Proc. Natl. Acad. Sci. U.S.A. 98 (17):9772-9777 (2001), Xu, M.J., et al (2001) Biochem. Biophys. Res. Commun. 280 (3):768-775; WO2004/016225 (Claim 2); WO2003/077836; WO2001/38490 (Claim 5; Fig 18D-1-18D-2); WO2003/097803 (Claim 12); WO2003/089624 (Claim 25); Cross-references: MIM:606509; NP_110391.2; NM_030764_1
(17) HER2 (ErbB2, Genbank accession no. M11730); Coussens L., et al Science (1985) 230(4730):1132-1139); Yamamoto T., et al Nature 319, 230-234, 1986; Semba K., et al Proc. Natl. Acad. Sci. U.S.A. 82, 6497-6501, 1985; Swiercz J.M., et al J. Cell Biol. 165, 869-880, 2004; Kuhns J.J., et al J. Biol. Chem. 274, 36422-36427, 1999; Cho H.-S., et al Nature 421, 756-760, 2003; Ehsani A., et al (1993) Genomics 15, 426-429; WO2004/048938 (Example 2); WO2004/027049 (Fig 1I); WO2004/009622; WO2003/081210; WO2003/089904 (Claim 9); WO2003/016475 (Claim 1); US2003/118592; WO2003/008537 (Claim 1): WO2003/055439 (Claim 29; Fig 1A-B); WO2003/025228 (Claim 37; Fig 5C): WO2002/22636 (Example 13; Page 95-107); WO2002/12341 [Claim 68; Fig 7); WO2002/13847 (Page 71-74); WO2002/14503 (Page 114-117); WO2001/53463 (Claim 2; Page 41-46); WO2001/41787 (Page 15); WO2000/44899 (Claim 52; Fig 7); WO2000/20579 (Claim 3; Fig 2); US5869445 (Claim 3; Col 31-38); WO9630514 (Claim 2; Page 56-61); EP1439393 (Claim 7); WO2004/043361 [Claim 7); WO2004/022709; WO2001/00244 (Example 3; Fig 4); Accession: P04626; EMBL; M11767; AAA35808.1. EMBL; M11761; AAA35808.1
(18) NCA (CEACAM6, Genbank accession no. M18728); Barnett T., et al Genomics 3, 59-66, 1988; Tawaragi Y., et al Biochem. Biophys. Res. Commun. 150, 89-96, 1988; Strausberg R.L., et al Proc. Natl. Acad. Sci. U.S.A. 99:16899-16903, 2002; WO2004/063709; EP1439393 (Claim 7); WO2004/044178 (Example 4); WO2004/031238; WO2003/042661 (Claim 12); WO2002/78524 (Example 2); WO2002/86443 (Claim 27; Page 427); WO2002/60317 (Claim 2); Accession: P40199; Q14920; EMBL; M29541; AAA59915.1. EMBL; M18728
(19) MDP (DPEP1, Genbank accession no. BC017023); Proc. Natl. Acad. Sci. U.S.A. 99 (26); 16899-16903 (2002)); WO2003/016475 (Claim 1); WO2002/64798 (Claim 33; Page 85-87); JP05003790 (Fig 6-8); WO99/46284 (Fig 9); Cross-references: MIM: 179780; AAH17023.1; BC017023_1
(20) IL20Rα (IL20Ra, ZCYTOR7, Genbank accession no. AF184971); Clark H.F., et al Genome Res. 13, 2265-2270, 2003; Mungall A.J., et al Nature 425, 805-811, 2003; Blumberg H., et al Cell 104, 9-19, 2001; Dumoutier L., et al J. Immunol. 167, 3545-3549, 2001: Parrish-Novak J., et al J. Biol. Chem. 277, 47517-47523, 2002; Pletnev S., et al (2003) Biochemistry 42:12617-12624; Sheikh F., et al (2004) J. Immunol. 172, 2006-2010; EP1394274 (Example 11); US2004/005320 (Example 5); WO2003/029262 (Page 74-75); WO2003/002717 (Claim 2; Page 63); WO2002/22153 (Page 45-47); US2002/042366 (Page 20-21); WO2001/46261 (Page 57-59); WO2001/46232 (Page 63-65); WO98/37193 (Claim 1; Page 55-59); Accession: Q9UHF4; Q6UWA9; Q96SH8; EMBL; AF184971; AAF01320.1.
(21) Brevican (BCAN, BEHAB, Genbank accession no. AF229053); Gary S.C., et al Gene 256,139-147, 2000; Clark H.F., et al Genome Res. 13, 2265-2270, 2003; Strausberg R.L., et al Proc. Natl. Acad. Sci. U.S.A. 99, 16899-16903, 2002; US2003/186372 (Claim 11); US2003/186373 (Claim 11); US2003/119131 (Claim 1; Fig 52); US2003/119122 (Claim 1; Fig 52); US2003/119126 (Claim 1); US2003/119121 (Claim 1; Fig 52); US2003/119129 (Claim 1); US2003/119130 (Claim 1); US2003/119128 (Claim 1; Fig 52); US2003/119125 (Claim 1); WO2003/016475 (Claim 1); WO2002/02634 (Claim 1)
(22) EphB2R (DRT, ERK, Hek5, EPHT3, Tyro5, Genbank accession no. NM_004442); Ghan,J. and Watt, V.M., Oncogene 6 (6), 1057-1061 (1991) Oncogene 10 (5):897-905 (1995), Annu. Rev. Neurosci. 21:309-345 (1998), Int. Rev. Cytol. 196:177-244 (2000)); WO2003042661 (Claim 12); WO200053216 (Claim 1; Page 41); WO2004065576 (Claim 1); WO2004020583 (Claim 9); WO2003004529 (Page 128-132); WO200053216 (Claim 1; Page 42); Cross-references: MIM:600997; NP_004433.2; NM_004442_1
(23) ASLG659 (B7h, Genbank accession no. AX092328); US2004/0101899 (Claim 2); WO2003104399 (Claim 11); WO2004000221 (Fig 3); US2003/165504 (Claim 1); US2003/124140 (Example 2); US2003/065143 (Fig 60); WO2002/102235 (Claim 13; Page 299); US2003/091580 (Example 2); WO2002/10187 (Claim 6; Fig 10); WO2001/94641 (Claim 12; Fig 7b); WO2002/02624 (Claim 13; Fig 1A-1B); US2002/034749 (Claim 54; Page 45-46); WO2002/06317 (Example 2; Page 320-321, Claim 34; Page 321-322); WO2002/71928 (Page 468-469); WO2002/02587 (Example 1; Fig 1); WO2001/40269 (Example 3; Pages 190-192); WO2000/36107 (Example 2; Page 205-207); WO2004/053079 (Claim 12); WO2003/004989 (Claim 1); WO2002/71928 (Page 233-234, 452-453); WO 01/16318
(24) PSCA (Prostate stem cell antigen precursor, Genbank accession no. AJ297436); Reiter R.E., et al Proc, Natl. Acad. Sci. U.S.A. 95, 1735-1740, 1998; Gu Z., et al Oncogene 19, 1288-1296, 2000; Biochem. Biophys. Res. Commun. (2000) 275(3):783-788; W02004/022709; EP1394274 (Example 11); US2004/018553 (Claim 17); WO2003/008537 (Claim 1); WO2002/81646 (Claim 1; Page 164); W02003/003906 (Claim 10; Page 288); WO2001/40309 (Example 1; Fig 17); US2001/055751 (Example 1; Fig 1b); WO2000/32752 (Claim 18; Fig 1); WO98/51805 (Claim 17; Page 97); WO98/51824 (Claim 10; Page 94); WO98/40403 (Claim 2; Fig 1B); Accession: 043653; EMBL; AF043498; AAC39607.1
(25) GEDA (Genbank accession No. AY260763); AAP14954 lipoma HMGIC fusion-partner-like protein /pid=AAP14954.1 - Homo sapiens (human); WO2003/054152 (Claim 20); WO2003/000842 (Claim 1); WO2003/023013 (Example 3, Claim 20); US2003/194704 (Claim 45); Cross-references: GI:30102449; AAP14954.1; AY260763_1
(26) BAFF-R (B cell -activating factor receptor, BLyS receptor 3, BR3, Genbank accession No. AF116456); BAFF receptor /pid=NP_443177.1 - Homo sapiens: Thompson, J.S., et al Science 293 (5537), 2108-2111 (2001); WO2004/058309; WO2004/011611; WO2003/046422 (Example; Page 32-33); WO2003/014294 (Claim 35; Fig 6B); WO2003/035846 (Claim 70; Page 615-616); WO2002/94852 (Col 136-137); WO2002/38766 (Claim 3; Page 133); WO2002/24909 (Example 3; Fig 3); Cross-references: MIM:606269; NP_443177.1; NM_052945_1; AF132600
(27) CD22 (B-cell receptor CD22-B isoform, BL-CAM, Lyb-8, Lyb8, SIGLEC-2, FLJ22814, Genbank accession No. AK026467); Wilson et al (1991) J. Exp. Med. 173:137-146; WO2003/072036 (Claim 1; Fig 1); Cross-references: MIM:107266; NP_001762.1; NM_001771_1
(28) CD79a (CD79A, CD79α, immunoglobulin-associated alpha, a B cell-specific protein that covalently interacts with Ig beta (CD79B) and forms a complex on the surface with Ig M molecules, transduces a signal involved in B-cell differentiation), pI: 4.84, MW: 25028 TM: 2 [P] Gene Chromosome: 19q13.2, Genbank accession No. NP_001774.10); WO2003/088808, US2003/0228319; WO2003/062401 (claim 9); US2002/150573 (claim 4, pages 13-14); WO99/58658 (claim 13, Fig 16); WO92/07574 (Fig 1); US5644033; Ha et at (1992) J. Immunol, 148(5):1526-1531; Müller et al (1992) Eur. J. Immunol.. 22:1621-1625; Hashimoto et al (1994) Immunogenetics 40(4):287-295; Preud'homme et al (1992) Clin. Exp. Immunol. 90(1):141-146; Yu et al (1992) J. Immunol. 148(2) 633-637; Sakaguchi et at (1988) EMBO J. 7(11):3457-3464
(29) CXCR5 (Burkitt's lymphoma receptor 1, a G protein-coupled receptor that is activated by the CXCL13 chemokine, functions in lymphocyte migration and humoral defense, plays a role in HIV-2 infection and perhaps development of AIDS, lymphoma, myeloma, and leukemia); 372 aa, pI: 8.54 MW: 41959 TM: 7 [P] Gene Chromosome: 11q23.3, Genbank accession No. NP_001707.1); WO2004/040000; WO2004/015426; US2003/105292 (Example 2); US6555339 (Example 2); WO2002/61087 (Fig 1); WO2001/57188 (Claim 20, page 269); WO2001/72830 (pages 12-13); WO2000/22129 (Example 1, pages 152-153, Example 2, pages 254-256); WO99/28468 (claim 1, page 38); US5440021 (Example 2, col 49-52); WO94/28931 (pages 56-58); WO92/17497 (claim 7, Fig 5); Dobner et al (1992) Eur. J. Immunol. 22:2795-2799; Barella et al (1995) Biochem. J. 309:773-779
(30) HLA-DOB (Beta subunit of MHC class II molecule (Ia antigen) that binds peptides and presents them to CD4+ T lymphocytes); 273 aa, pI: 6.56, MW: 30820.TM: 1 [P] Gene Chromosome: 6p21.3, Genbank accession No. NP_002111.1); Tonnelle et al (1985) EMBO J. 4(11):2839-2847; Jonsson et al (1989) Immunogenetics 29(6):411-413; Beck et al (1992) J. Mol. Biol. 228:433-441; Strausberg et al (2002) Proc. Natl. Acad. Sci USA 99:16899-16903; Servenius et al (1987) J. Biol. Chem. 262:8759-8766; Beck et al (1996) J. Mol. Biol. 255:1-13; Naruse et al (2002) Tissue Antigens 59:512-519; WO99/58658 (claim 13, Fig 15); US6153408 (Col 35-38); US5976551 (col 168-170); US6011146 (col 145-146); Kasahara et al (1989) Immunogenetics 30(1):66-68; Larhammar et al (1985) J. Biol. Chem. 260(26):14111-14119
(31) P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability); 422 aa), pI: 7.63, MW: 47206 TM: 1 [P] Gene Chromosome: 17p13.3, Genbank accession No. NP_002552.2); Le et al (1997) FEBS Lett. 418(1-2):195-199: WO2004/047749; WO2003/072035 (claim 10); Touchman et al (2000) Genome Res. 10:165-173; WO2002/22660 (claim 20); WO2003/093444 (claim 1); WO2003/087768 (claim 1); WO2003/029277 (page 82)
(32) CD72 (B-cell differentiation antigen CD72, Lyb-2); 359 aa, pI: 8.66, MW: 40225, TM: 1 [P] Gene Chromosome: 9p13.3, Genbank accession No. NP_001773.1); WO2004042346 (claim 65); WO2003/026493 (pages 51-52, 57-58); WO2000/75655 (pages 105-106); Von Hoegen et al (1990) J. Immunol. 144(12):4870-4877; Strausberg et al (2002) Proc. Natl. Acad. Sci USA 99:16899-16903.
(33) LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis); 661 aa, pI: 6.20, MW: 74147 TM: 1 [P] Gene Chromosome: 5q12, Genbank accession No. ' NP_005573.1); US2002/193567; WO97/07198 (claim 11, pages 39-42); Miura et al (1996) Genomics 38(3):299-304; Miura et al (1998) Blood 92:2815-2822; WO2003/083047; WO97/44452 (claim 8, pages 57-61); WO2000/12130 (pages 24-26)
(34) FcRH1 (Fc receptor-like protein 1, a putative receptor for the immunoglobulin Fc domain that contains C2 type Ig-like and ITAM domains, may have a role in B-lymphocyte differentiation); 429 aa, pI: 5.28, MW: 46925 TM: 1 [P] Gene Chromosome: 1q21-1q22, Genbank accession No. NP_443170.1); WO2003/077836; WO2001/38490 (claim 6, Fig 18E-1-18-E-2); Davis et al (2001) Proc. Natl. Acad. Sci USA 98(17):9772-9777; WO2003/089624 (claim 8); EP1347046 (claim 1); WO2003/089624 (claim 7)
(35) IRTA2 (Immunoglobulin superfamily receptor translocation associated 2, a putative immunoreceptor with possible roles in B cell development and lymphomagenesis; deregulation of the gene by translocation occurs in some B cell malignancies); 977 aa, pI: 6.88, MW: 106468, TM: 1 [P] Gene Chromosome: 1q21, Genbank accession No. Human:AF343662, AF343663, AF343664, AF343665, AF369794, AF397453, AK090423, AK090475, AL834187, AY358085; Mouse:AK089756, AY158090, AY506558; NP_112571.1; WO2003/024392 (claim 2, Fig 97); Nakayama et al (2000) Biochem. Biophys. Res. Commun. 277(1):124-127; WO2003/077836; WO2001/38490 (claim 3, Fig 18B-1-18B-2)
(36) TENB2 (TMEFF2, tomoregulin, TPEF, HPP1, TR, putative transmembrane proteoglycan, related to the EGF/heregulin family of growth factors and follistatin); 374 aa, NCBI Accession: AAD55776, AAF91397, AAG49451, NCBI RefSeq: NP_057276; NCBI Gene: 23671; OMIM: 605734; SwissProt Q9UIK5; Genbank accession No. AF179274; AY358907, CAF85723, CQ782436; WO2004/074320; JP2004113151; WO2003/042661; WO2003/009814; EP1295944 (pages 69-70); WO2002/30268 (page 329); WO2001/90304: US2004/249130; US2004/022727; WO2004/063355; US2004/197325; US20031232350; US2004/005563; US2003/124579; Horie et at (2000) Genomics 67:146-152; Uchida et al (1999) Biochem. Biophys. Res. Commun. 266:593-602; Liang et al (2000) Cancer Res. 60:4907-12; Glynne-Jones et at (2001) Int J Cancer. Oct 15; 94(2):178-84.

The parent antibody may also be a fusion protein comprising an albumin-binding peptide (ABP) sequence (Dennis et al. (2002) "Albumin Binding As A General Strategy For Improving The Pharmacokinetics Of Proteins" J Biol Chem. 277:35035-35043; WO 01/45746). Antibodies of the invention include fusion proteins with ABP sequences taught by: (i) Dennis et al (2002) J Biol Chem. 277:35035-35043 at Tables III and IV, page 35038; (ii) US 2004/0001827 at [0076]; and (iii) WO 01/45746 at pages 12-13, and all of which are incorporated herein by reference.

In one embodiment, the antibody has been raised to target specific the tumour related antigen αᵥβ₆.

The cell binding agent is connected to the linker. In one embodiment, the cell binding agent is connected to A, where present, of the linker.

In one embodiment, the connection between the cell binding agent and the linker is trough a thioether bond.

In one embodiment, the connection between the cell binding agent and the linker is through a disulfide bond.
In one embodiment, the connection between the cell binding agent and the linker is through an amide bond.
In one embodiment, the connection between the cell binding agent and the linker is through an ester bond.

In one embodiment, the connection between the cell binding agent and the linker is formed between a thiol group of a cysteine residue of the cell binding agent and a maleimide group of the linker.

The cysteine residues of the cell binding agent may be available for reaction with the functional group of R^{L} to form a connection. In other embodiments, for example where the cell binding agent is an antibody, the thiol groups of the antibody may participate in interchain disulfide bonds. These interchain bonds may be converted to free thiol groups by e.g. treatment of the antibody with DTT prior to reaction with the functional group of R^{L}.

The cell binding agent may be labelled, for example to aid detection or purification of the agent either prior to incorporation as a conjugate, or as part of the conjugate. The label may be a biotin label. In another embodiment, the cell binding agent may be labelled with a radioisotope.

### R and R'

In one embodiment, R is independently selected from optionally substituted C₁₋₁₂ alkyl, C₃₋₂₀ heterocyclyl and C₅₋₂₀ aryl groups. These groups are each defined in the substituents section below.
In one embodiment, R is independently optionally substituted C₁₋₁₂ alkyl.
In one embodiment, R is independently optionally substituted C₃₋₂₀ heterocyclyl.

In one embodiment, R is independently optionally substituted C₅₋₂₀ aryl.
In one embodiment, R is independently optionally substituted C₁₋₁₂ alkyl.

Described above in relation to R² are various embodiments relating to preferred alkyl and aryl groups and the identity and number of optional substituents. The preferences set out for R² as it applies to R are applicable, where appropriate, to all other groups R, for examples where R⁶, R⁷, R⁸ or R⁹ is R.

The preferences for R apply also to R'.

In some embodiments of the invention there is provided a compound having a substituent group -N RR'. In one embodiment, R and R' together with the nitrogen atom to which they are attached form an optionally substituted 4-, 5-, 6- or 7-membered heterocyclic ring, The ring may contain a further heteroatom, for example N, O or S.
In one embodiment, the heterocyclic ring is itself substituted with a group R. Where a further N heteroatom is present, the substituent may be on the N heteroatom.

### R"

R" is a C₃₋₁₂ alkylene group, which chain may be interrupted by one or more heteroatoms, e.g. O, S, N(H), NMe and/or aromatic rings, e.g. benzene or pyridine, which rings are optionally substituted.
In one embodiment, R" is a C₃₋₁₂ alkylene group, which chain may be interrupted by one or more heteroatoms and/or aromatic rings, e.g. benzene or pyridine.
In one embodiment, the alkylene group is optionally interrupted by one or more heteroatoms selected from O, S, and NMe and/or aromatic rings, which rings are optionally substituted.
In one embodiment, the aromatic ring is a C₅₋₂₀ arylene group, where arylene pertains to a divalent moiety obtained by removing two hydrogen atoms from two aromatic ring atoms of an aromatic compound, which moiety has from 5 to 20 ring atoms.
In one embodiment, R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂.

In one embodiment, R" is a C₃₋₁₂ alkylene group.
In one embodiment, R" is selected from a C₃, C₅, C₇, Cg and a C₁₁ alkylene group.
In one embodiment, R" is selected from a C₃, C₅ and a C₇ alkylene group.
In one embodiment, R" is selected from a C₃ and a C₅ alkylene group.
In one embodiment, R" is a C₃ alkylene group.
In one embodiment, R" is a C₅ alkylene group.

The alkylene groups listed above may be optionally interrupted by one or more heteroatoms and/or aromatic rings, e.g. benzene or pyridine, which rings are optionally substituted. The alkylene groups listed above may be optionally interrupted by one or more heteroatoms and/or aromatic rings, e.g. benzene or pyridine.

The alkylene groups listed above may be unsubstituted linear aliphatic alkylene groups.

### X

In one embodiment, X is selected from O, S, or N(H).
Preferably, X is O.

### A-A, B-A, C-A, D-A and E-A

The compounds of formula A-A, B-A, C-A, D-A and E-A have a group R² which with either of R¹ or R³, together with carbon atoms of the C ring to which they are attached, form an optionally substituted benzene ring.' The optionally substituted benzene ring may be regarded as fused to the C ring of the pyrrolobenzodiazepine. The fused benzene ring may be referred to as the D ring. The structure of the fused ring is illustrated below: 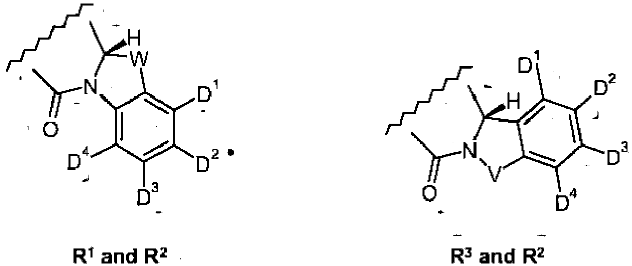 where each of D¹, D², D³ and D⁴ represents H or a substituent.

In one embodiment, the benzene ring is unsubstituted.
In one embodiment, the benzene ring is optionally substituted with one, two, three of four groups selected from OH, CN, R, OR, O-SO₂-R, CO₂R, COR, SH, SR, NH₂, NHR, NRR', NO₂, Me₃Sn and halo.
In one embodiment, the benzene ring is monosubstituted. The monosubstituent may be any one of D¹, D², D³ or D⁴ (the rest being H). In one embodiment the benzene ring is substituted at D², and D¹, D³ and D⁴ are each H. In one embodiment the benzene ring is substituted at D³, and D¹, D² and D⁴ are each H.

In one embodiment, R² with R¹, together with carbon atoms of the C ring to which they are attached, form an optionally substituted benzene ring.

The preferences for V and W are set out below.
*A-B, B-B,* C-B, *D-B* and *E-B*
In one embodiment, U is CH₂ when T is NR, BH, SO, or SO₂.
In one embodiment, T is CH₂ or CO when U is NR, O or S.
In one embodiment, T is selected from CH₂ and CO.
In one embodiment, U is selected from NR, O and S.
In one embodiment, Y is (CH₂)ₙ, where n is 1 or 2.

In one embodiment, the C ring of the compound A-B has a structure selected from those shown below:

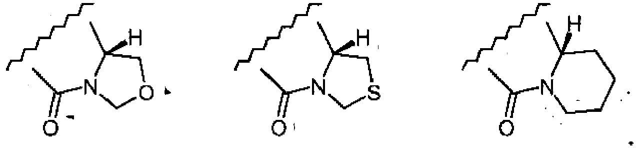

### V and W

V and W are each selected from (CH₂)ₙ, O, S, NR, CHR, and CRR' where n is 2,3 or 4, except that V is C when R¹ and R², together with carbon atoms of the C ring to which they are attached, form an optionally substituted benzene ring, and W is C when R³ and R², together with carbon atoms of the C ring to which they are attached, form an optionally substituted benzene ring.

In one embodiment, when one of V and W is C, the other of V and W is selected from CH₂ and NR.
In one embodiment, when one of V and W is C, the other of V and W is CH₂.

### Dimer Compounds

In one embodiment, the conjugate of the first aspect of the invention, compound C, compound D and compound E are each dimers.

### Conjugates

In one embodiment, the conjugate is a dimer with each monomer being of formula (A).

In one embodiment, the dimer compound is a dimer with each monomer being of formula (A), and the compound having the structure shown below: 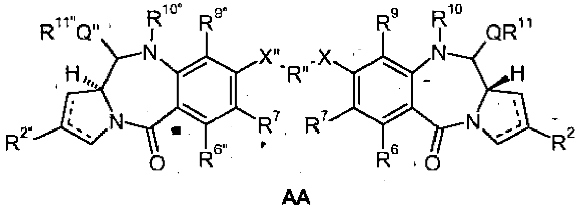 where R^{2"}, R^{6"}, R^{7"}, R^{9"}, R^{10"}, X", Q" and R^{11"} and are as defined according to R², R⁶, R⁷, R⁹, R¹⁰, X, and R¹¹ respectively.

In one embodiment, the conjugate is a dimer with each monomer being of formula (A), and the compound having the structure shown below: 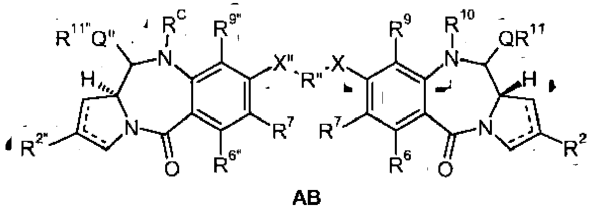 where R^{2"}, R^{6"}, R^{7"}, R^{9"}, X", Q" and R^{11"} and are as defined according to R², R⁶, R⁷, R⁹, X, and R¹¹ respectively, and R^{C} is a capping group. In this embodiment, each group R¹⁰ is a linker connected to a cell binding agent.

In one embodiment, the conjugate is a dimer with one monomer being of formula (A) and the other being of formula (B).

In one embodiment, the conjugate is a dimer with one monomer being of formula (A) and the other being of formula (B), and the compound having the structure shown below: 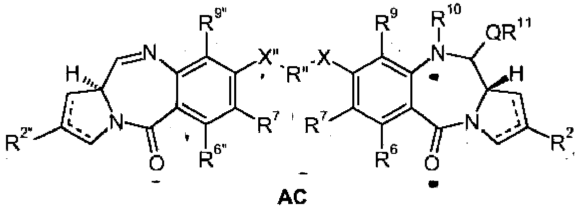 where R^{2"}, R^{6"}, R^{7"}, R^{9"}, X" and R^{11"} and are as defined according to R², R⁶, R⁷, R⁹, X, and R¹¹ respectively,

In one embodiment, the conjugate is a dimer with each monomer being of formula (A), and the groups R², R⁶, R⁹, X, R¹¹ and R⁷ and R⁸ where appropriate, are the same.
In one embodiment, the compound is a dimer with each monomer being of formula (A), the groups R², R⁶, R⁹, X, R¹¹, R¹⁰, and R⁷ and R⁸ where appropriate, are the same. Such a compound may be referred to as a symmetrical dimer.

In one embodiment, the conjugate is a dimer with one monomer being of formula (A) and the other being of formula (B), and the groups R², R⁶, R⁹, and R⁷ and R⁸ where appropriate, of (A) are the same as those groups of compound (B).

For each of the dimer compounds above, a monomer of formula (A) or (B) may be replaced with a monomer of formula (A-I) or (B-I) as described herein.

### Compound C

In one embodiment, compound C is a dimer.
In one embodiment, compound C has the structure shown below: 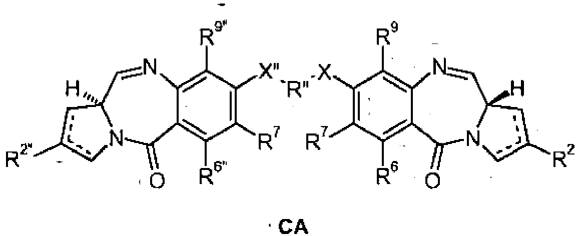 where R^{2"}, R^{6"} , R^{7"}, R^{9"}, and X" are as defined according to R², R⁶, R⁷, R⁹, and X respectively.

### Compound D

In one embodiment, compound D is a dimer.
In one embodiment, compound D has the structure shown below: 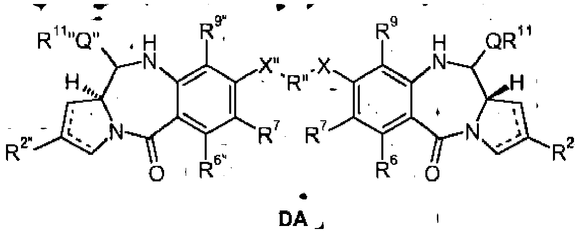 where R^{2"}, R^{6"}, R^{7"}, R^{9"}, Q", R^{11"} and X" are as defined according to R², R⁶, R⁷, R⁹, Q, R¹¹ and X respectively.

### Compound E

In one embodiment, compound E is a dimer.
In one embodiment, compound E has the structure shown below: 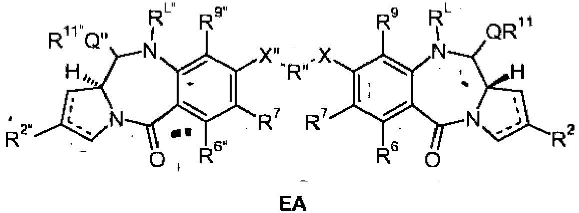 where R^{2"}, R^{6"}, R^{7"}, R^{9"}, R^{11"}, R^{L"}, Q" and X" are as defined according to R², R⁶, R⁷, R⁹, R¹¹, R^{L} and X respectively.

In one embodiment, compound E has the structure shown below: 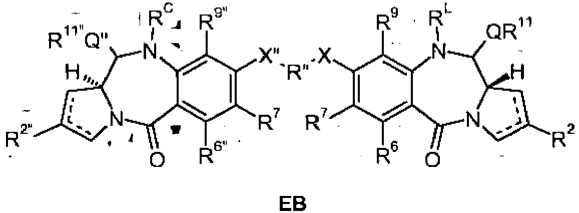 where R^{2"}, R^{6"}, R^{7"}, R^{9"}, R^{11"}, Q" and X" are as defined according to R², R⁶, R⁷, R⁹, R¹¹, and X" are as defined according to R², R⁶, R⁷, and X respectively. R^{C} is a capping group.

In one embodiment, compound E has the structure shown below: 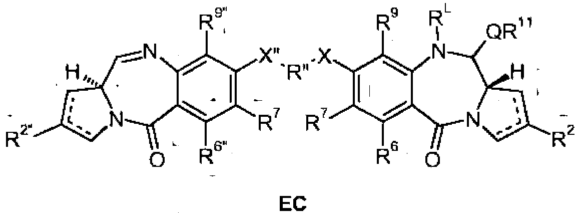 where R^{2"}, R^{6"}, R^{7"}, R^{9"}, and X" are as defined according to R², R⁶, R⁷, R⁹, and X respectively.

### Monomer Compounds

In one embodiment, the conjugate of the first aspect of the invention, compound C, compound D and compound E are monomers.
In one embodiment, a conjugate or compound of formula (C), (D) or (E) may be replaced with a monomer of formula (A-I), (C-I), (D-I) or (E-I) as described herein.

### R^{c}, Capping Group

The conjugate of the first aspect of the invention may have a capping group R^{C} at the N10 position. Compound E may have a capping group R^{C}.

In one embodiment, where the conjugate is a dimer with each monomer being of formula (A), the group R¹⁰ in one of the monomer units is a capping group R^{C} or is a group R¹⁰.

In one embodiment, where the conjugate is a dimer with each monomer being of formula (A), the group R¹⁰ in one of the monomer units is a capping group R^{C}.

In one embodiment, where compound E is a dimer with each monomer being of formula (E), the group R^{L} in one of the monomer units is a capping group R^{C} or is a linker for connection to a cell binding agent.

In one embodiment, where compound E is a dimer with each monomer being of formula (E), the group R^{L} in one of the monomer units is a capping group R^{C}.

The group R^{C} is removable from the N10 position of the PBD moiety to leave an N10-C11 imine bond, a carbinolamine, a substituted carbinolamine, where QR¹¹ is OSO₃M, a bisulfite adduct, a thiocarbinolamine, a substituted thiocarbinolamine, or a substituted carbinalamine.

In one embodiment, R^{C}, may be a protecting group that is removable to leave an N10-C11 imine bond, a carbinolamine, a substituted cabinolamine, or, where QR¹¹ is OSO₃M, a bisulfite adduct. In one embodiment, R^{C} is a protecting group that is removable to leave an N10-C11 imine bond.

The group R^{c} is intended to be removable under the same conditions as those required for the removal of the group R¹⁰, for example to yield an N10-C11 imine bond, a carbinolamine and so on. The capping group acts as a protecting group for the intended functionality at the N10 position. The capping group is intended not to be reactive towards a cell binding agent. For example, R^{C} is not the same as R^{L}.

Compounds having a capping group may be used as intermediates in the synthesis of dimers having an imine monomer. Alternatively, compounds having a capping group may be used as conjugates, where the capping group is removed at the target location to yield an imine, a carbinolamine, a substituted cabinolamine and so on. Thus, in this embodiment, the capping group may be referred to as a therapeutically removable nitrogen protecting group, as defined in the inventors' earlier application WO 00/12507.

In one embodiment, the group R^{C} is removable under the conditions that cleave the linker R^{L} of the group R¹⁰. Thus, in one embodiment, the capping group is cleavable by the action of an enzyme.

In an alternative embodiment, the capping group is removable prior to the connection of the linker R^{L} to the cell binding agent. In this embodiment, the capping group is removable under conditions that do not cleave the linker R^{L}.

Where a compound includes a functional group G¹ to form a connection to the cell binding agent, the capping group is removable prior to the addition or unmasking of G¹.

The capping group may be used as part of a protecting group strategy to ensure that only one of the monomer units in a dimer is connected to a cell binding agent.

The capping group may be used as a mask for a N10-C11 imine bond. The capping group may be removed at such time as the imine functionality is required in the compound. The capping group is also a mask for a carbinolamine, a substituted cabinolamine, and a bisulfite adduct, as described above.

R^{C} may be an N10 protecting group, such as those groups described in the inventors' earlier application, WO 00/12507. In one embodiment, R^{C} is a therapeutically removable nitrogen protecting group, as defined in the inventors' earlier application, WO 00/12507.

In one embodiment, R^{C} is a carbamate protecting group.

In one embodiment, the carbamate protecting group is selected from:
Alloc, Fmoc, Boc, Troc, Teoc, Psec, Cbz and PNZ.
Optionally, the carbamate protecting group is further selected from Moc.

In one embodiment, R^{C} is a linker group R^{L} lacking the functional group for connection to the cell binding agent.

This application is particularly concerned with those R^{C} groups which are carbamates.

In one embodiment, R^{C} is a group: 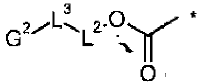 where the asterisk indicates the point of attachment to the N10 position, G² is a terminating group, L³ is a covalent bond or a cleavable linker L¹, L² is a covalent bond or together with OC(=O) forms a self-immolative linker.

Where L³ and L² are both covalent bonds, G² and OC(=O) together form a carbamate protecting group as defined above.

L¹ is as defined above in relation to R¹⁰.
L² is as defined above in relation to R¹⁰.

Various terminating groups are described below, including those based on well known protecting groups.

In one embodiment L³ is a cleavable linker L¹, and L², together with OC(=O), forms a self-immolative linker. In this embodiment, G² is Ac (acetyl) or Moc, or a carbamate protecting group selected from:
Alloc, Fmoc, Boc, Troc, Teoc, Psec, Cbz and PNZ.
Optionally, the carbamate protecting group is further selected from Moc.

In another embodiment, G² is an acyl group -C(=O)G³, where G³ is selected from alkyl (including cycloalkyl, alkenyl and alkynyl), heteroalkyl, heterocyclyl and aryl (including heteroaryl and carboaryl). These groups may be Optionally substituted. The acyl group together with an amino group of L³ or L², where appropriate, may form an amide bond. The acyl group together with a hydroxy group of L³ or L², where appropriate, may form an ester bond.

In one embodiment, G³ is heteroalkyl. The heteroalkyl group may comprise polyethylene glycol. The heteroalkyl group may have a heteroatom, such as O or N, adjacent to the acyl group, thereby forming a carbamate or carbonate group, where appropriate, with a heteroatom present in the group L³ or L², where appropriate.

In one embodiment, G³ is selected from NH₂, NHR and NRR'. Preferably, G³ is NRR.

In one embodiment G² is the group: 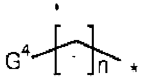 where the asterisk indicates the point of attachment to L³, n is 0 to 6 and G⁴ is selected from OH, OR, SH, SR, COOR, CONH₂, CONHR, CONRR', NH₂, NHR, NRR', NO₂, and halo. The groups OH, SH, NH₂ and NHR are protected. In one embodiment, n is 1 to 6, and preferably n is 5. In one embodiment, G⁴ is OR, SR, COOR, CONH₂, CONHR, CONRR', and NRR'. In one embodiment, G⁴ is OR, SR, and NRR'. Preferably G⁴ is selected from OR and NRR', most preferably G⁴ is OR. Most preferably G⁴ is OMe.

In one embodiment, the group G² is: 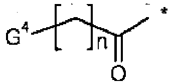 where the asterisk indicates the point of attachment to L³, and n and G⁴ are as defined above.

In one embodiment, the group G² is: 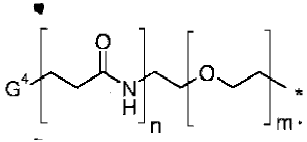 where the asterisk indicates the point of attachment to L³, n is 0 or 1, m is 0 to 50, and G⁴ is selected from OH, OR, SH, SR, COOR, CONH₂, CONHR, CONRR', NH₂, NHR, NRR', NO₂, and halo. In a preferred embodiment, n is 1 and m is 0 to 10, 1 to 2, preferably 4 to 8, and most preferably 4 or 8. In another embodiment, n is 1 and m is 10 to 50, preferably 20 to 40. The groups OH, SH, NH₂ and NHR are protected. In one embodiment, G⁴ is OR, SR, COOR, CONH₂, CONHR, CONRR', and NRR'. In one embodiment, G⁴ is OR, SR, and NRR'. Preferably G⁴ is selected from OR and NRR', most preferably G⁴ is OR. Preferably G⁴ is OMe.

In one embodiment, the group G² is: 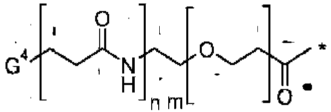 where the asterisk indicates the point of attachment to L³, and n, m and G⁴ are as defined above.

In one embodiment, the group G² is: 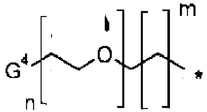 where n is 1-20, m is 0-6, and G⁴ is selected from OH, OR, SH, SR, COOR, CONH₂, CONHR, CONRR', NH₂, NHR, NRR', NO₂, and halo. In one embodiment, n is 1-10. In another embodiment, n is 10 to 50, preferably 20 to 40. In one embodiment, n is 1. In one embodiment, m is 1. The groups OH, SH, NH₂ and NHR are protected. In one embodiment, G⁴ is OR, SR, COOR, CONH₂, CONHR, CONRR', and NRR'. In one embodiment, G⁴ is OR, SR, and NRR'. Preferably G⁴ is selected from OR and NRR', most preferably G⁴ is OR. Preferably G⁴ is OMe.

In one embodiment, the group G² is: 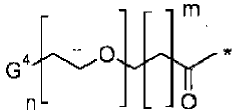 where the asterisk indicates the point of attachment to L³, and n, m and G⁴ are as defined above.

In each of the embodiments above G⁴ may be OH, SH, NH₂ and NHR. These groups are preferably protected.
In one embodiment, OH is protected with Bzl, TBDMS, or TBDPS.
In one embodiment, SH is protected with Acm, Bzl, Bzl-OMe, Bzl-Me, or Trt.
In one embodiment, NH₂ or NHR are protected with Boc, Moc, Z-Cl, Fmoc, Z, or Alloc.

In one embodiment, the group G² is present in combination with a group L³, which group is a dipeptide.

The capping group is not intended for connection to the cell binding agent. Thus, the other monomer present in the dimer serves as the point of connection to the cell binding agent via a linker. Accordingly, it is preferred that the functionality present in the capping group is not available for reaction with a cell binding agent. Thus, reactive functional groups such as OH, SH, NH₂, COOH are preferably avoided. However, such functionality may be present in the capping group if protected, as described above.

In the preparation of the compounds of the invention the capping group may be used to prepare a linker R^{L}.

An exemplary embodiment of an antibody-drug conjugate (ADC) compound comprises an antibody (Ab), and a PBD drug moiety (PBD) wherein the antibody is attached by a linker moiety (L) to PBD; the composition having the formula:

Ab-(L-PBD)ₚ

where p is an integer from 1 to about 8, and represents the drug loading. If Ab is a cysteine engineered antibody, the number of drug moieties which may be conjugated via a thiol reactive linker moiety to an antibody molecule is limited by the number of cysteine residues which are introduced by the methods described herein. Exemplary ADC therefore comprise antibodies which have 1, 2, 3, or 4 engineered cysteine amino acids.

### Preferred Compounds

In one embodiment, the conjugate is a dimer wherein each of the monomers has a C2 methylene group i.e. each R² is =CH₂. It is preferred that the cell binding agent is an antibody.

In another embodiment, the conjugate is a dimer wherein each of the monomers has a C2 aryl group i.e. each R² is optionally substituted C₅₋₂₀ aryl. It is preferred that the cell binding agent is an antibody.

### C2 Alkylene

In one embodiment, the conjugate is a compound: 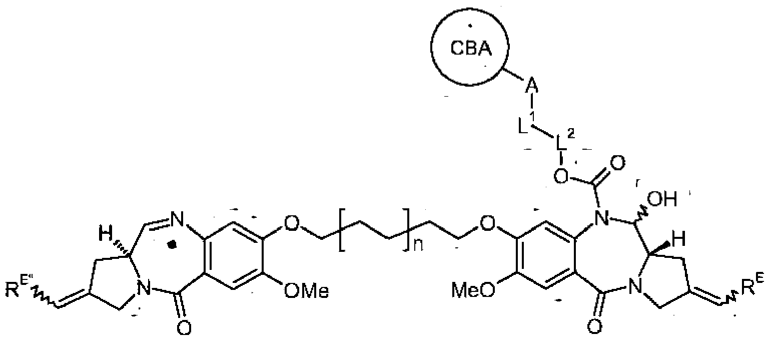 wherein CBA is a cell binding agent such as an antibody or a cyclic or linear peptide, and n is 0 or 1. L¹ and L² are as previously defined, and R^{E} and R^{E}" are each independently selected from H or R^{D}.

In one embodiment, the conjugate is a compound: 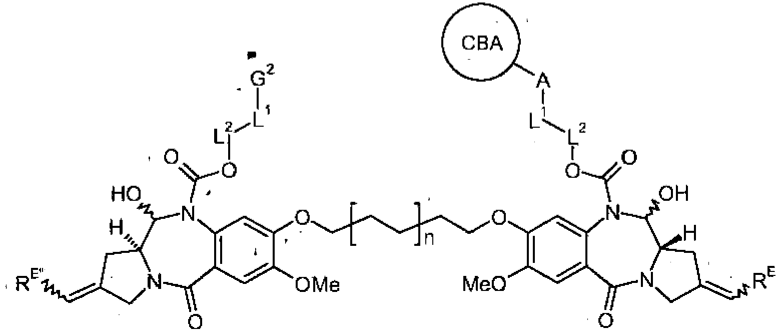 wherein CBA is a cell binding agent such as an antibody or a cyclic or linear peptide, and n is 0 or 1. L¹, L² and G² are as previously defined, and R^{E} and R^{E}" are each independently selected from H or R^{D}.

In one embodiment, the conjugate is a compound: 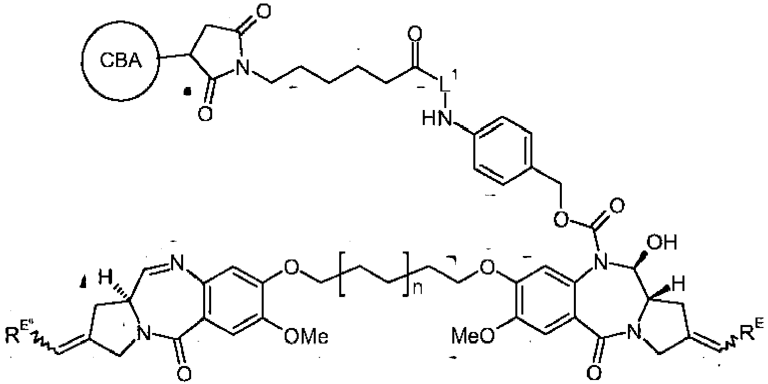 wherein CBA is a cell binding agent such as an antibody or a cyclic or linear peptide, and n is 0 or 1. L¹ is as previously defined, and R^{E} and R^{E}" are each independently selected from H or R^{D}.

In one embodiment, the conjugate is a compound: 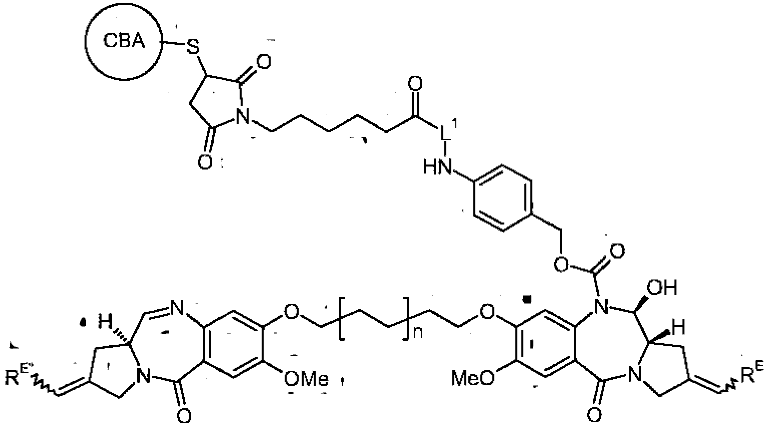 wherein CBA is a cell binding agent such as an antibody or a cyclic or linear peptide, and n is 0 or 1. L¹ is as previously defined, and R^{E} and R^{E}" are each independently selected from H or R^{D}.

In one embodiment, the conjugate is a compound: 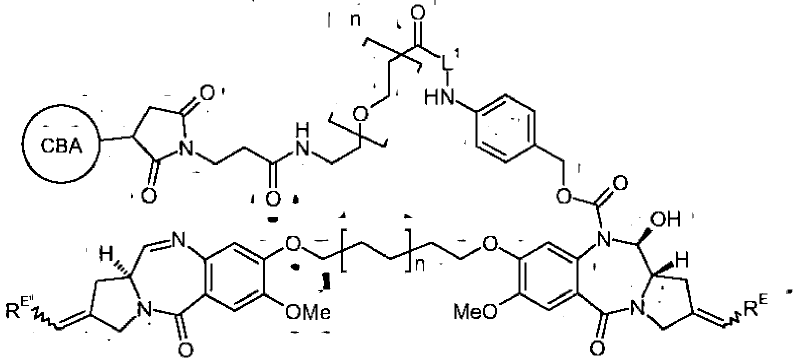 wherein CBA is a cell binding agent such as an antibody or a cyclic or linear peptide, and n is 0 or 1. L¹ is as previously defined, and R^{E} and R^{E}" are each independently selected from H or R^{D}.

In one embodiment, the conjugate is a compound: 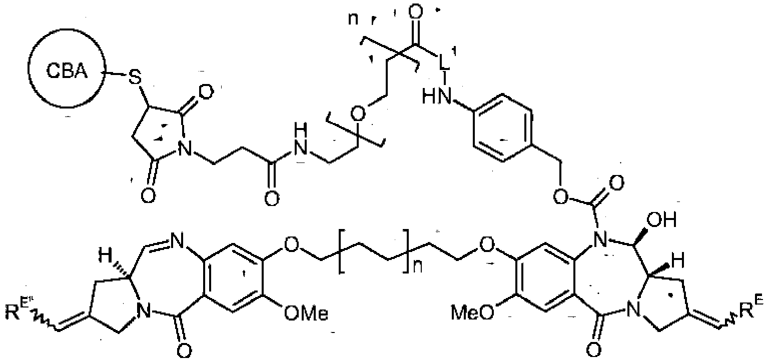 wherein CBA is a cell binding agent such as an antibody or a cyclic or linear peptide, and n is 0 or 1. L¹ is as previously defined, and R^{E} and R^{E}" are each independently selected from H or R^{D}.

For each of the compounds above, the following preferences may apply, where appropriate:
n is 0;
n is 1;
R^{E} is H;
R^{E} is R^{D}, where R^{D} is optionally substituted alkyl;
R^{E} is R^{D}, where R^{D} is methyl;
CBA is an antibody;
CBA is a cyclic peptide;
L¹ is or comprises a dipeptide;
L¹ is (H₂N)-Val-Ala-(CO) or (H₂N)-Phe-Lys-(CO), where (H₂N) and (CO) indicate the respective N and C terminals;
L² is *p*-aminobenzylene;
G² is selected from Alloc, Fmoc, Boc, Troc, Teoc, Psec, Cbz and PNZ.

The following preferences may also apply in addition to the preferences above:
G² is: 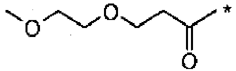 where the asterisk indicates the point of attachment to the N terminal of L¹;
A is: 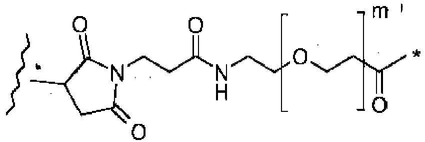 where the asterisk indicates the point of attachment to the N terminal of L¹, the wavy line indicates the point of attachment to the cell binding agent and m is 4 or 8;
A is 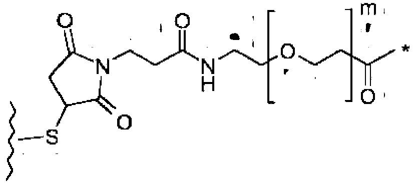 where the asterisk indicates the point of attachment to the N terminal of L¹, the wavy line indicates the point of attachment to the cell binding agent, and m is 4 or 8.

In a particularly preferred embodiment, n is 1; R^{E} is H; CBA is an antibody; L¹ is (H₂N)-Val-Ala-(CO) or (H₂N)-Phe-Lys-(CO), where (H₂N) and (CO) indicate the respective N and C terminals; L² is *p*-aminobenzylene; G² is: 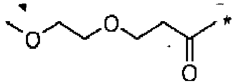 where the asterisk indicates the point of attachment to the N terminal of L¹; and A is 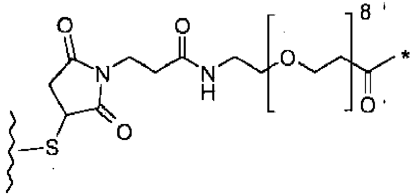 where the asterisk indicates the point of attachment to the N terminal of L¹, and the wavy line indicates the point of attachment to the cell binding agent.

### C2 Aryl

In one embodiment, the conjugate is a compound: 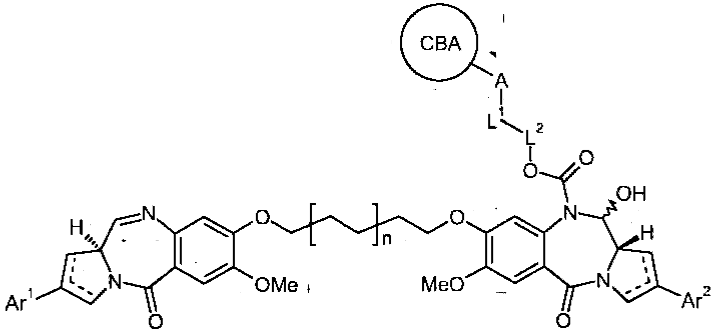 wherein CBA is a cell binding agent such as an antibody or a cyclic or linear peptide, L¹ and L² are as previously defined Ar¹ and Ar² are each independently optionally substituted C₅₋₂₀ aryl, and n is 0 or 1. Ar¹ and Ar² may be the same or different.

In one embodiment, the conjugate is a compound: 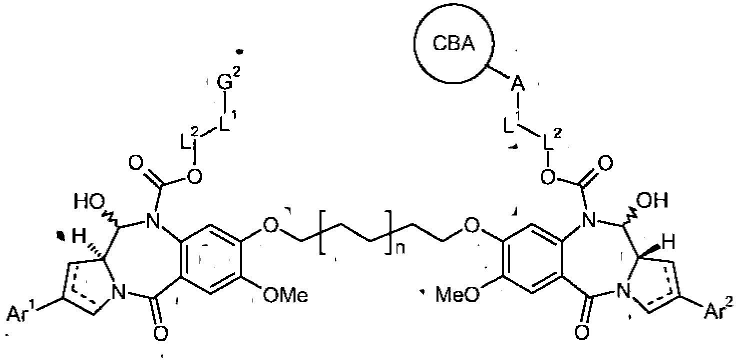 wherein CBA is a cell binding agent such as an antibody or a cyclic or linear peptide, , L¹, L² and G² are as previously defined, Ar¹ and Ar² are each independently optionally substituted C₅₋₂₀ aryl, and n is 0 or 1.

In one embodiment, the conjugate is a compound: 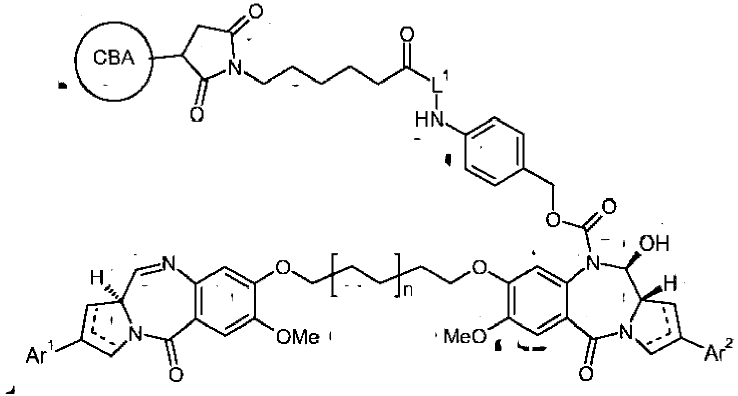 wherein CBA is a cell binding agent such as an antibody or a cyclic or linear peptide, L₁ is as previously defined, Ar¹ and Ar² are each independently optionally substituted C₅₋₂₀ aryl, and n is 0 or 1.

In one embodiment, the conjugate is a compound: 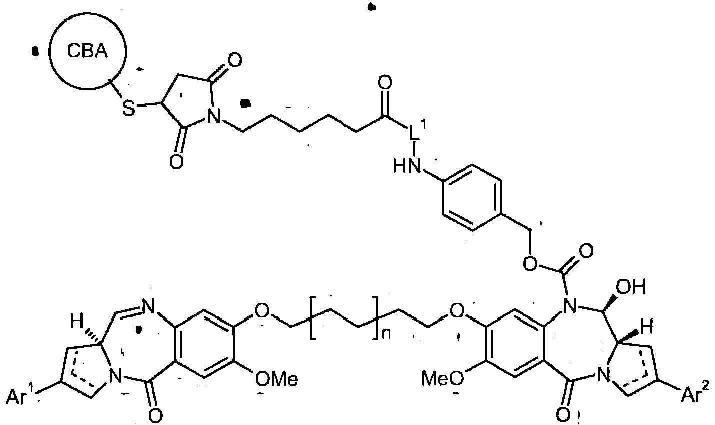 wherein CBA is a cell binding agent such as an antibody or a cyclic or linear peptide, L¹ is as previously defined, Ar¹ and Ar² are each independently optionally substituted C₅₋₂₀ aryl, and n is 0 or 1.

In one embodiment, the conjugate is a compound: 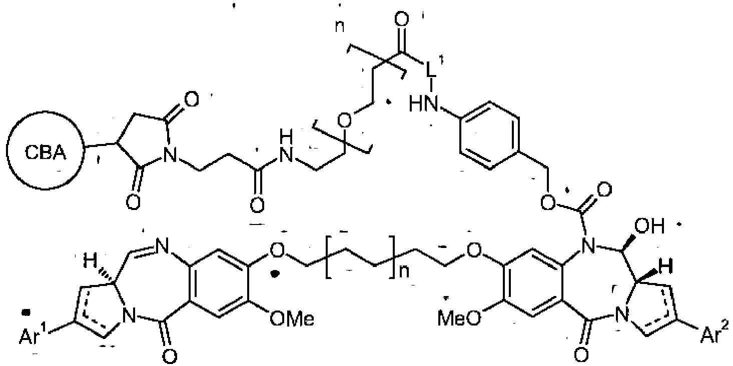 wherein CBA is a cell binding agent such as an antibody or a cyclic or linear peptide, and n is 0 or 1. L¹ is as previously defined, Ar¹ and Ar² are each independently optionally substituted C₅₋₂₀ aryl, and n is 0 or 1.

In one embodiment, the conjugate is a compound: 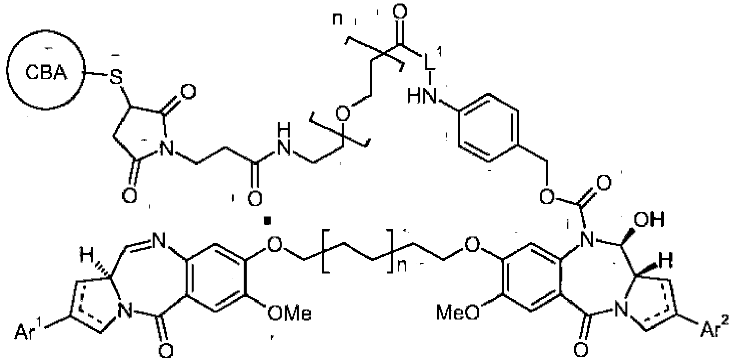 wherein CBA is a cell binding agent such as an antibody or a cyclic or linear peptide, and n is 0 or 1. L¹ is as previously defined, Ar¹ and Ar² are each independently optionally substituted C₅₋₂₀ aryl, and n is 0 or 1.

In one embodiment, Ar¹ and Ar² in each of the embodiments above are each independently selected from optionally substituted phenyl, furanyl, thiophenyl and pyridyl.

In one embodiment, Ar¹ and Ar² in each of the embodiments above is optionally substituted phenyl.

In one embodiment, Ar¹ and Ar² in each of the embodiments above is optionally substituted thiophen-2-yl or thiophen-3-yl.

In one embodiment, Ar¹ and Ar² in each of the embodiments above is optionally substituted' quinolinyl or isoquinolinyl.

The quinolinyl or isoquinolinyl group may be bound to the PBD core through any available ring position. For example, the quinolinyl may be quinolin-2-yl, quinolin-3-yl, quinolin-4yl, quinolin-5-yl, quinolin-6-yl, quinolin-7-yl and quinolin-8-yl. Of these quinolin-3-yl and quinolin-6-yl may be preferred. The isoquinolinyl may be isoquinolin-1-yl, isoquinolin-3-yl, isoquinolin-4yl, isoquinolin-5-yl, isoquinolin-6-yl, isoquinolin-7-yl and isoquinolin-8-yl. Of these isoquinolin-3-yl and isoquinolin-6-yl may be preferred.

### C2 Vinyl

In one embodiment, the conjugate is a compound: 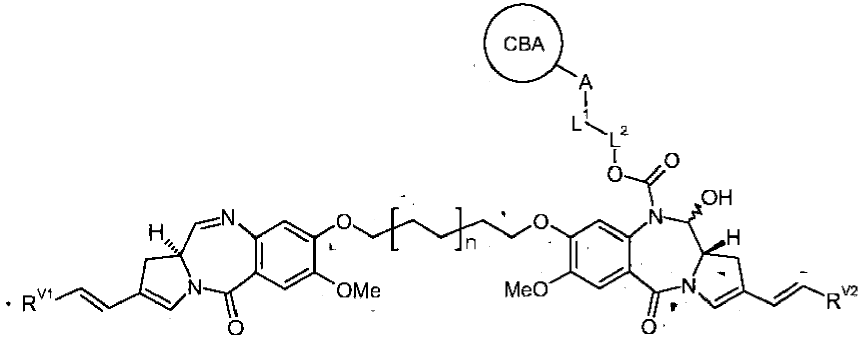 wherein CBA is a cell binding agent such as an antibody or a cyclic or linear peptide, L¹ and L² are as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In one embodiment, the conjugate is a compound: 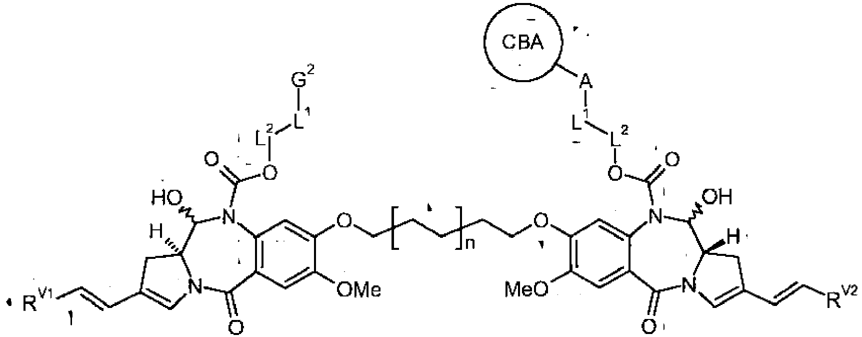 wherein CBA is a cell binding agent such as an antibody or a cyclic or linear peptide, L¹, L² and G² are as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In one embodiment, the conjugate is a compound: 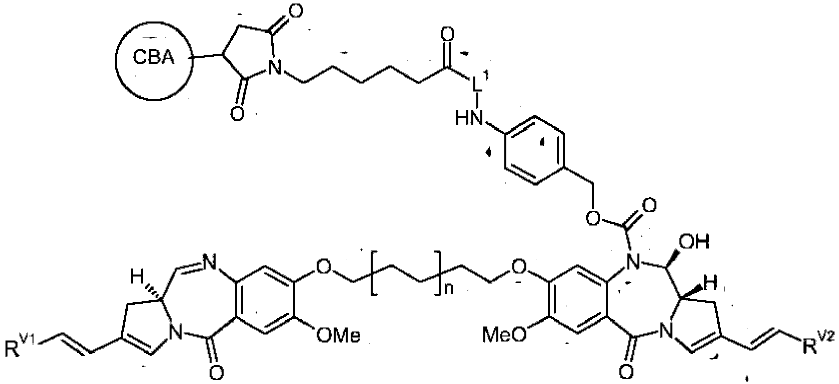 wherein CBA is a cell binding agent such as an antibody or a cyclic or linear peptide, L¹ is as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In one embodiment, the conjugate is a compound: 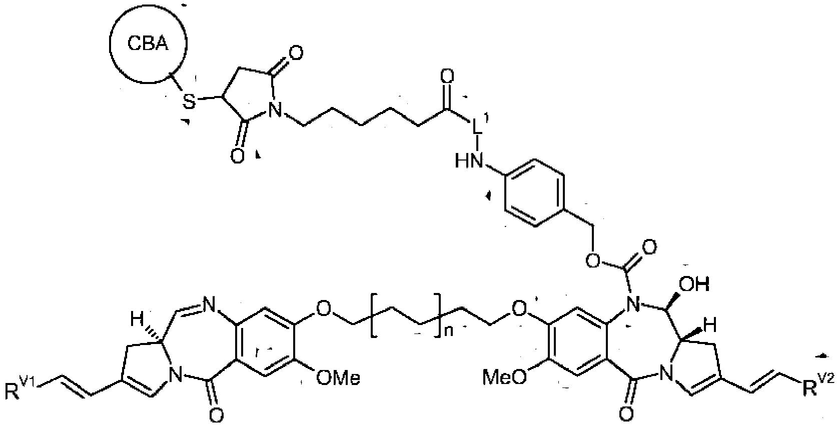 wherein CBA is a cell binding agent such as an antibody or a cyclic or linear peptide, L¹ is as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In one embodiment, the conjugate is a compound: 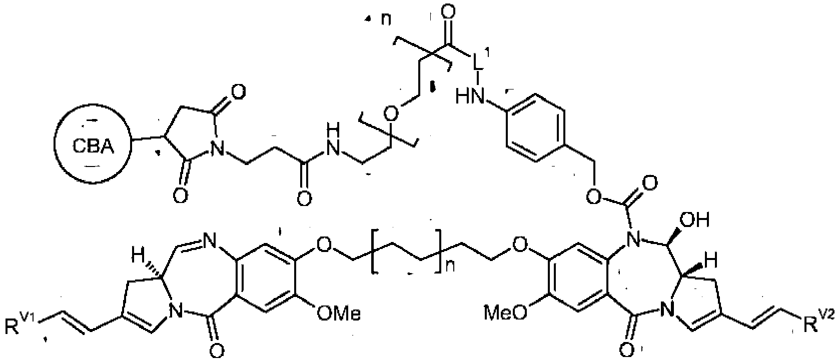 wherein CBA is a cell binding agent such as an antibody or a cyclic or linear peptide, and n is 0 or 1. L¹ is as previously defined, R^{V1} and R^{V2} are indepdently selected from H, methyl, ethyl and phenyl (which phenyl may be optionally substituted with fluoro, particularly ; in the 4 position) and C₅₋₆ heterocyclyl, and n is 0 or 1. R^{V1} and R^{V2} may be the same or different.

In one embodiment, the conjugate is a compound: 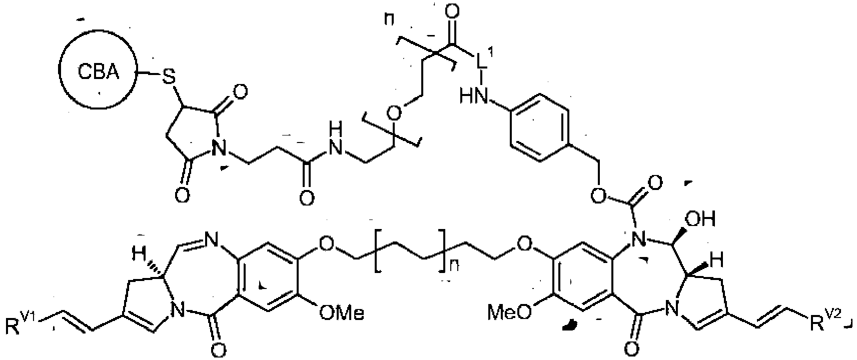 wherein CBA is a cell binding agent such as an antibody or a cyclic or linear peptide, and n is 0 or 1. L¹ is as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In some of the above embodiments, R^{V1} and R^{V2} may be indepdently selected from H, phenyl, and 4-fluorophenyl.

### Preferred Intermediates

The present invention also provides intermediates for use in the preparation of the conjugate compounds described herein.

Preferred intermediates are described below, and correspond closely to the preferred conjugates described above.

In one embodiment, the intermediate is a compound: 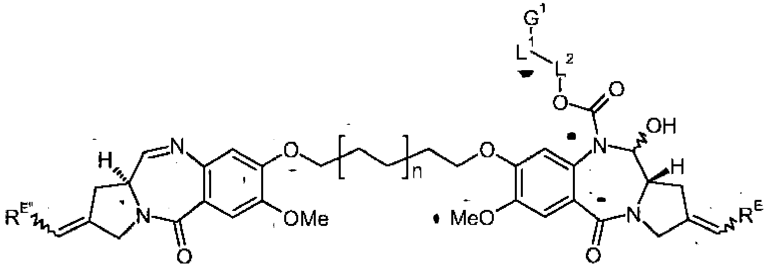 wherein n is 0 or 1, G¹, L¹ and L² are as previously defined, and R^{E} and R^{E}" are each independently selected from H or R^{D}.

In one embodiment, the intermediate is a compound: 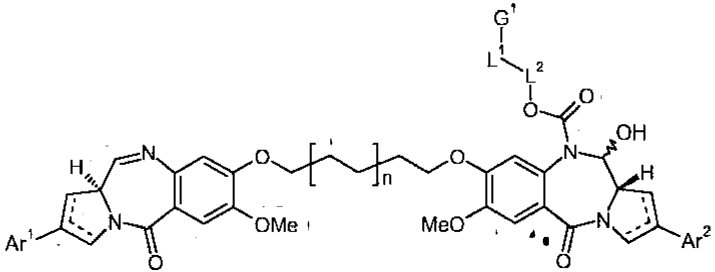 wherein G¹, L¹ and L² are as previously defined Ar¹ and Ar² are each independently optionally substituted C₅₋₂₀ aryl, and n is 0 or 1. Ar¹ and Ar² may be the same or different.

In one embodiment, the intermediate is a compound: 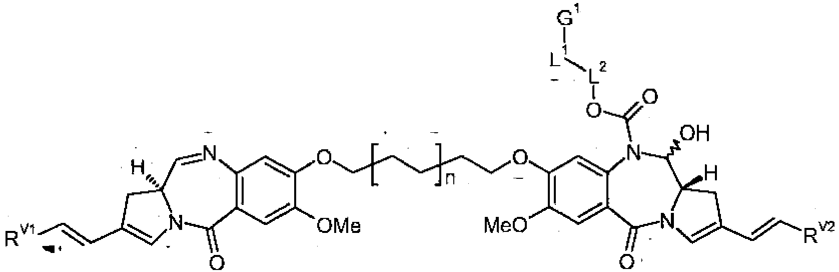 wherein G¹, L¹ and L² are as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In one embodiment, the intermediate is a compound: 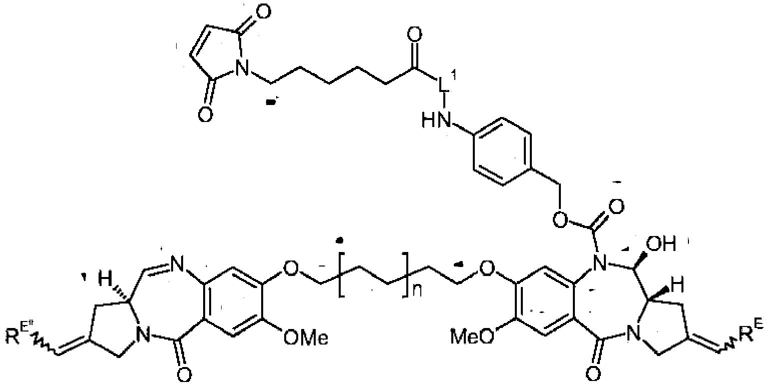 wherein n is 0 or 1, L¹ is as previously defined, and R^{E} and R^{E}" are each independently selected from H or R^{D}.

In one embodiment, the intermediate is a compound: 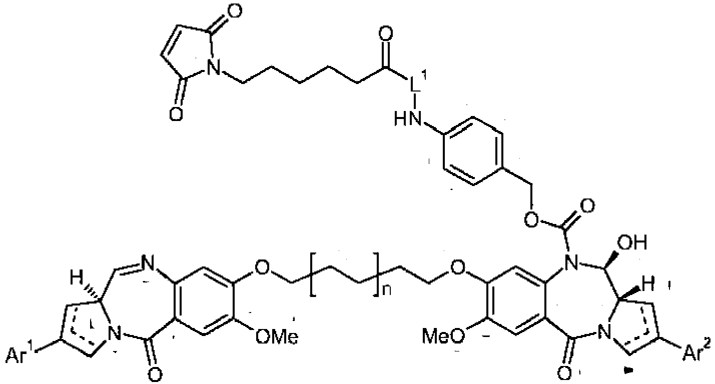 wherein L¹ is as previously defined, Ar¹ and Ar² are each independently optionally substituted C₅₋₂₀ aryl, and n is 0 or 1.

In one embodiment, the intermediate is a compound: 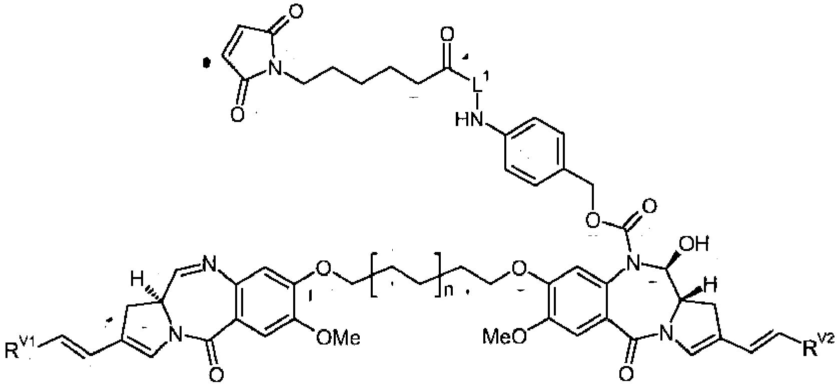 wherein L¹ is as previously defined, and R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In one embodiment, the intermediate is a compound: 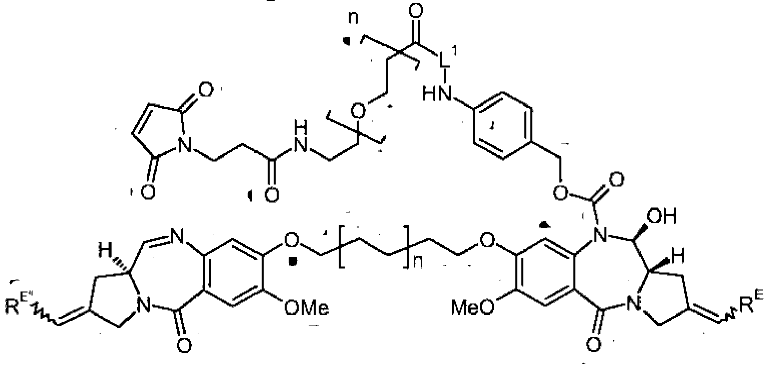 wherein n is 0 or 1, L¹ is as previously defined, and R^{E} and R^{E}" are each independently selected from H or R^{D}.

In one embodiment, the intermediate is a compound: 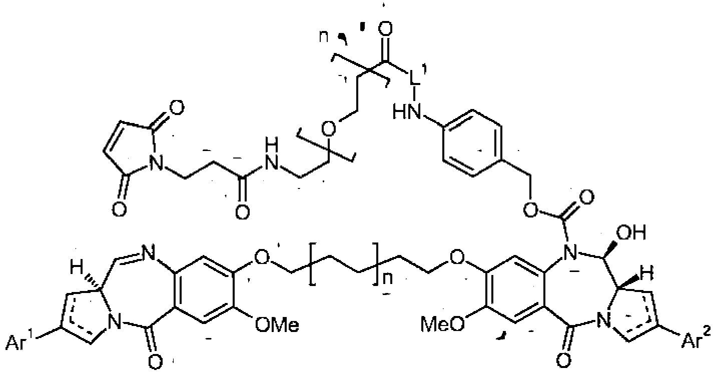 wherein n is 0 or 1, L¹ is as previously defined, Ar¹ and Ar² are each independently optionally substituted C₅₋₂₀ aryl, and n is 0 or 1.

In one embodiment, the intermediate is a compound: 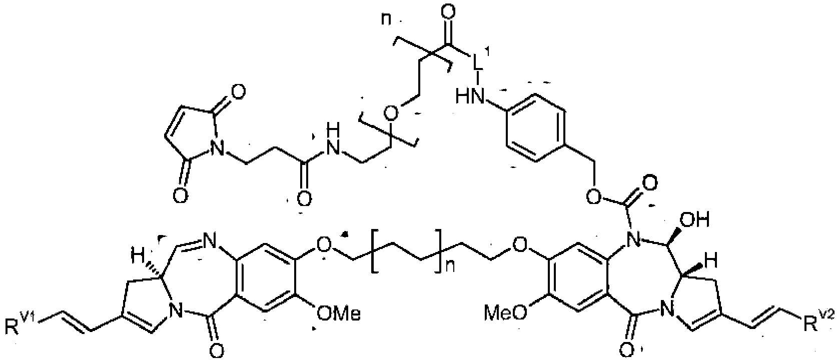 wherein L¹ is as previously defined, R^{V1} and R^{V2} are indepdently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

### Substituents

The phrase "optionally substituted" as used herein, pertains to a parent group which may be unsubstituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

In a preferred embodiment, the substituents described herein (which include optional substituents) are limited to those groups that are not reactive to a cell binding agent. The link to the cell binding agent in the present case is formed from the N10 position of the PBD compound through a linker group (comprising, for example, L¹, L² and A) to the cell binding agent. Reactive functional groups located at other parts of the PBD structure may be capable of forming additional bonds to the cell binding agent (this may be referred to as crosslinking). These additional bonds may alter transport and biological activity of the conjugate. Therefore, in some embodiment, the additional substituents are limited to those lacking reactive functionality.

In one embodiment, the substituents are selected from the group consisting of R, OR, SR, NRR', NO₂, halo, CO₂R, COR, CONH₂, CONHR, and CONRR'.
In one embodiment, the substituents are selected from the group consisting of R, OR, SR, NRR', NO₂, CO₂R, COR, CONH₂, CONHR, and CONRR'.
In one embodiment, the substituents are selected from the group consisting of R, OR, SR, NRR', NO₂, and halo.
In one embodiment, the substituents are selected from the group consisting of R, OR, SR, NRR', and NO₂.
Any one of the embodiment mentioned above may be applied to any one of the substituents described herein. Alternatively, the substituents may be selected from one or more of the groups listed below.

Examples of substituents are described in more detail below.

C₁₋₁₂ alkyl: The term "C₁₋₁₂ alkyl" as used herein, pertains to a monovalent moiety obtained by removing a hydrogen atom from a carbon atom of a hydrocarbon compound having from 1 to 12 carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C₂), n-propyl (C₃), n-butyl (C₄), n-pentyl (amyl) (C₅), n-hexyl (C₆) and n-heptyl (C₇).

Examples saturated branched alkyl groups include iso-propyl (C₃), iso-butyl (C₄), sec-butyl (C₄), tert-butyl (C₄), iso-pentyl (C₅), and neo-pentyl (C₅).

An alkyl group may optionally be interrupted by one or more heteroatoms selected from O, N(H) and S. Such groups may be referred to as "heteroalkyl".

C₂₋₂₀ Heteroalkyl: The term "C₂₋₁₂ heteroalkyl" as used herein, pertains to a monovalent moiety obtained by removing a hydrogen atom from a carbon atom of a hydrocarbon compound having from 2 to 12 carbon atoms, and one or more heteroatoms selected from O, N(H) and S, preferably O and S.

Examples of heteroalkyl groups include, but are not limited to those comprising one or more ethylene glycol units of the type -(OCH₂CH₂)-. The terminal of a heteroalkyl group may be the primary form of a heteroatom, e.g. -OH, -SH or -NH₂. In a preferred embodiment, the terminal is -CH₃.

C₂₋₁₂ Alkenyl: The term "C₂₋₁₂ alkenyl" as used herein, pertains to an alkyl group having one or more carbon-carbon double bonds.

Examples of unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₁₂ alkynyl: The term "C₂₋₁₂ alkynyl" as used herein, pertains to an alkyl group having one or more carbon-carbon triple bonds.

Examples of unsaturated alkynyl groups include, but are not limited to, ethynyl (-C≡CH) and 2-propynyl (propargyl, -CH₂-C≡CH).

C₃₋₁₂ cycloalkyl: The term "C₃₋₁₂ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

C₃₋₂₀ heterocyclyl: The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms..

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅),2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted monocyclic heterocyclyl groups include those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

C₅₋₂₀ aryl: The term "C₅₋₂₀ aryl", as used herein, pertains to a monovalent moiety obtained by removing a hydrogen atom from an aromatic ring atom of an aromatic compound, which moiety has from 3 to 20 ring atoms. Preferably, each ring has from 5 to 7 ring atoms.

In this context, the prefixes (e.g. C₃₋₂₀, C₅₋₇, C₅₋₆, etc.) denote the number of ring atoms, or range of number of ring atoms, whether carbon atoms or heteroatoms. For example, the term "C₅₋₆ aryl" as used herein, pertains to an aryl group having 5 or 6 ring atoms.

The ring atoms may be all carbon atoms, as in "carboaryl groups".
Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic: ring, include, but are not limited to, groups derived from indane (e.g. 2,3-dihydro-1H-indene) (C₉), indene (C₉), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups". Examples of monocyclic heteroaryl groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅):
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅), triazine (C₆); and,
N₄: tetrazole (C₅).

Examples of heteroaryl which comprise fused rings, include, but are not limited to:
C₉ (with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
C₁₁ (with 2 fused rings) derived from benzodiazepine (N₂);
C₁₃ (with 3 fused rings) derived from carbazole (N₁), dibenzofuran (O₁), dibenzothiophene (S₁), carboline (N₂), perimidine (N₂), pyridoindole (N₂); and,
C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

The above groups, whether alone or part of another substituent, may themselves optionally be substituted with one or more groups selected from themselves and the additional substituents listed below.
Halo: -F, -Cl, -Br, and -I.
Hydroxy: -OH.

Ether: -OR, wherein R is an ether substituent, for example, a C₁₋₇ alkyl group (also referred to as a C₁₋₇alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

Alkoxy: -OR, wherein R is an alkyl group, for example, a C₁₋₇ alkyl group. Examples of C₁₋₇ alkoxy groups include, but are not limited to, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

Acetal: -CH(OR¹)(OR²), wherein R¹ and R² are independently acetal substituents, for example, a C₁₋₇alkyl group, a C₃₋₂₀heterocyclyl group, or a C₅₋₂₀ aryl group, preferably a C₁₋₇ alkyl group, or, in the case of a "cyclic" acetal group, R¹ and R², taken together with the two oxygen atoms to which they are attached, and the carbon atoms to which they are attached, form a heterocyclic ring having from 4 to 8 ring atoms. Examples of acetal groups include, but are not limited to, -CH(OMe)₂, -CH(OEt)₂, and -CH(OMe)(OEt).

Hemiacetal: -CH(OH)(OR¹), wherein R¹ is a hemiacetal substituent, for example, a C₁₋₇ alkyl group, a C₃₋₂₀heterocyclyl group, or a C₅₋₂₀ aryl group, preferably a C₁₋₇alkyl group. Examples of hemiacetal groups include, but are not limited to, -CH(OH)(OMe) and - CH(OH)(OEt).

Ketal: -CR(OR¹)(OR²), where R¹ and R² are as defined for acetals, and R is a ketal substituent other than hydrogen, for example, a C₁₋₇alkyl group, a C₃₋₂₀heterocyclyl group, or a C₅₋₂₁aryl group, preferably a C₁₋₇alkyl group. Examples ketal groups include, but are not limited to, -C(Me)(OMe)₂, -C(Me)(OEt)₂, -C(Me)(OMe)(OEt), -C(Et)(OMe)₂, -C(Et)(OEt)₂, and -C(Et)(OMe)(OEt).

Hemiketal: -CR(OH)(OR¹), where R¹ is as defined for hemiacetals, and R is a hemiketal substituent other than hydrogen, for example, a C₁₋₇alkyl group, a C₃₋₂₀heterocyclyl group, or a C₅₋₂₀ aryl group, preferably a C₁₋₇alkyl group. Examples of hemiacetal groups include, but are not limited to, -C(Me)(OH)(OMe), -C(Et)(OH)(OMe), -C(Me)(OH)(OEt), and -C(Et)(OH)(OEt).

Oxo (keto, -one): =O.

Thione (thioketone): =S.

Imino (imine). =NR, wherein R is an imino substituent, for example, hydrogen, C₁₋₇alkyl group, a C₃₋₂₀heterocyclyl group, or a C₅₋₂₀ aryl group, preferably hydrogen or a C₁₋₇alkyl group. Examples of ester groups include, but are not limited to, =NH, =NMe, =NEt, and =NPh.

Formyl (carbaldehyde, carboxaldehyde): -C(=O)H.

Acyl (keto): -C(=O)R, wherein R is an acyl substituent, for example, a C₁₋₇alkyl group (also referred to as C₁₋₇alkylacyl or C₁₋₇alkanoyl), a C₃₋₂₀heterocyclyl group (also referred to as C₃₋₂₀ heterocyclylacyl), or a C₅₋₂₀ aryl group (also referred to as C₅₋₂₀arylacyl), preferably a C₁₋₇alkyl group. Examples of acyl groups include, but are not limited to, -C(=O)CH₃ (acetyl), -C(=O)CH₂CH₃ (propionyl), -C(=O)C(CH₃)₃ (t-butyryl), and -C(=O)Ph (benzoyl, phenone).

Carboxy (carboxylic acid): -C(=O)OH.

Thiocarboxy (thiocarboxylic acid): -C(=S)SH.

Thiolocarboxy (thiolocarboxylic acid): -C(=O)SH.

Thionocarboxy (thionocarboxylic acid): -C(=S)OH.

Imidic acid: -C(=NH)OH.

Hydroxamic acid: -C(=NOH)OH.

Ester (carboxylate, carboxylic acid ester, oxycarbonyl): -C(=O)OR, wherein R is an ester substituent, for example, a C₁₋₇alkyl group, a C₃₋₂₀heterocyclyl group, or a C₅₋₂₀ aryl group, preferably a C₁₋₇ alkyl group. Examples of ester groups include, but are not limited to,' -C(=O)OCH₃, -C(=O)OCH₂CH₃, -C(=O)OC(CH₃)₃, and -C(=O)OPh. r Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Examples of acyloxy groups include, but are not limited to, -OC(=O)CH₃ (acetoxy), -OC(=O)CH₂CH₃, -OC(=O)C(CH₃)₃, -OC(=O)Ph, and -OC(=O)CH₂Ph.

Oxycarboyloxy: -OC(=O)OR, wherein R is an ester substituent, for example, a C₁₋₇alkyl group, a C₃₋₂₀ heterocyclyl group, or a C₅₋₂₀ aryl group, preferably a C₁₋₇ alkyl group. Examples of ester groups include, but are not limited to, -OC(=O)OCH₃, -OC(=O)OCH₂CH₃, -OC(=O)OC(CH₃)₃, and -OC(=O)OPh.

Amino: -NR¹R², wherein R¹ and R² are independently amino substituents, for example, hydrogen, a C₁₋₇alkyl group (also referred to as C₁₋₇ alkylamino or di-C₁₋₇ alkylamino), a C₃₋₂₀ heterocyclyl group, or a C₅₋₂₀aryl group, preferably H or a C₁₋₇alkyl group, or, in the case of a "cyclic" amino group, R¹ and R², taken together with the nitrogen atom to which they are attached, form a heterocyclic ring having from 4 to 8 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to, -NH₂, -NHCH_{3,} -NHC(CH₃)₂, -N(CH₃)₂, -N(CH₂CH₃)₂, and -NHPh. Examples of cyclic amino groups include, but are not limited to, aziridino, azetidino, pyrrolidino, piperidino, piperazino, morpholino, and thiomorpholino.

Amido (carbamoyl, carbamyl, aminocarbonyl, carboxamide): -C(=O)NR¹R², wherein R¹ and R² are independently amino substituents, as defined for amino groups. Examples of amido groups include, but are not limited to, -C(=O)NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Thioamido (thiocarbamyl): -C(=S)NR¹R², wherein R¹ and R² are independently amino substituents, as defined for amino groups. Examples of amido groups include, but are not limited to, -C(=S)NH₂, -C(=S)NHCH₃, -C(=S)N(CH₃)₂, and -C(=S)NHCH₂CH₃.

Acylamido (acylamino): -NR¹C(=O)R², wherein R¹ is an amide substituent, for example, hydrogen, a C₁₋₇alkyl group, a C₃₋₂₀heterocyclyl group, or a C₅₋₂₀aryl group, preferably hydrogen or a C₁₋₇alkyl group, and R² is an acyl substituent, for example, a C₁₋₇alkyl group, a C₃₋₂₀heterocyclyl group, or a C₅₋₂₀aryl group, preferably hydrogen or a C₁₋₇alkyl group. Examples of acylamide groups include, but are not limited to, -NHC(=O)CH₃, -NHC(=O)CH₂CH₃, and -NHC(=O)Ph. R¹ and R² may together form a cyclic structure, as in, for example, succinimidyl, maleimidyl, and phthalimidyl:

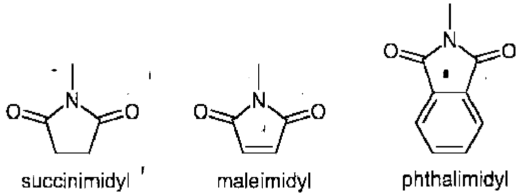

Aminocarbonyloxy: -OC(=O)NR¹R², wherein R¹ and R² are independently amino substituents, as defined for amino groups. Examples of aminocarbonyloxy groups include, but are not limited to, -OC(=O)NH₂, -OC(=O)NHMe, -OC(=O)NMe_{2,} and -OC(=O)NEt₂.

Ureido: -N(R¹)CONR²R³ wherein R² and R³ are independently amino substituents, as defined for amino groups, and R¹ is a ureido substituent, for example, hydrogen, a C₁₋₇alkyl group, a C₃₋₂₀heterocyclyl group, or a C₅₋₂₀aryl group, preferably hydrogen or a C₁₋₇alkyl group. Examples of ureido groups include, but are not limited to, -NHCONH₂, -NHCONHMe, -NHCONHEt, -NHCONMe₂, -NHCONEt₂, -NMeCONH₂, -NMeCONHMe, -NMeCONHEt,-NMeCONMe₂, and -NMeCONEt₂.

Guanidino: -NH-C(=NH)NH₂.

Tetrazolyl: a five membered aromatic ring having four nitrogen atoms and one carbon atom,

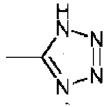

Imino: =NR, wherein R is an imino substituent, for example, for example, hydrogen, a C₁₋₇ alkyl group, a C₃₋₂₀ heterocyclyl group, or a C₅₋₂₀ aryl group, preferably H or a C₁₋₇alkyl group. Examples of imino groups include, but are not limited to, =NH, =NMe, and =NEt.

Amidine (amidino): -C(=NR)NR₂, wherein each R is an amidine substituent, for example, hydrogen, a C₁₋₇alkyl group, a C₃₋₂₀ heterocyclyl group, or a C₅₋₂₀ aryl group, preferably H or a C₁₋₇alkyl group. Examples of amidine groups include, but are not limited to, -C(=NH)NH₂, -C(=NH)NMe₂, and -C(=NMe)NMe₂.
Nitro: -NO₂.
Nitroso: -NO.
Azido: -N₃.
Cyano (nitrile, carbonitrile): -CN.
Isocyano: -NC.
Cyanato: -OCN,
Isocyanato: -NCO.
Thiocyano (thiocyanato): -SCN.
Isothiocyano (isothiocyanato): -NCS.
Sulfhydryl (thiol, mercapto): -SH.

Thioether (sulfide): -SR, wherein R is a thioether substituent, for example, a C₁₋₇alkyl groups (also referred to as a C₁₋₇alkylthio group), a C₃₋₂₀heterocyclyl group, or a C₅₋₂₀ aryl group, preferably a C₁₋₇alkyl group. Examples of C₁₋₇alkylthio groups include, but are not limited to, -SCH₃ and -SCH₂CH₃.

Disulfide: -SS-R, wherein R is a disulfide substituent, for example, a C₁₋₇ alkyl group, a C₃₋₂₀ heterocyclyl group, or a C₅₋₂₀aryl group, preferably a C₁₋₇alkyl group (also referred to herein as C₁₋₇alkyl disulfide). Examples of C₁₋₇alkyl disulfide groups include, but are not limited to, -SSCH₃ and -SSCH₂CH₃.

Sulfine (sulfinyl, sulfoxide): -S(=O)R, wherein R is a sulfine substituent, for example, a C₁₋₇ alkyl group, a C₃₋₂₀ heterocyclyl group, or a C₅₋₂₀ aryl group, preferably a C₁₋₇ alkyl group. Examples of sulfine groups include, but are not limited to, -S(=O)CH₃ and -S(=O)CH₂CH₃. Sulfone (sulfonyl): -S(=O)₂R, wherein R is a sulfone substituent, for example, a C₁₋₇alkyl group, a C₃₋₂₀heterocyclyl group, or a C₅₋₂₀ aryl group, preferably a C₁₋₇ alkyl group, including, for example, a fluorinated or perfluorinated C₁₋₇alkyl group. Examples of sulfone groups include, but are not limited to, -S(=O)₂CH₃ (methanesulfonyl, mesyl), -S(=O)₂CF₃ (triflyl), -S(=O)₂CH₂CH₃ (esyl), -S(=O)₂C₄F₉ (nonaflyl), -S(=O)₂CH₂CF₃ (tresyl), -S(=O)₂CH₂CH₂NH₂ (tauryl), -S(=O)₂Ph (phenylsulfonyl, besyl), 4-methylphenylsulfonyl (tosyl), 4-chlorophenylsulfonyl (closyl), 4-bromophenylsulfonyl (brosyl), 4-nitrophenyl (nosyl), 2-naphthalenesulfonate (napsyl), and 5-dimethylamino-naphthalen-1-ylsulfonate (dansyl).

Sulfinic acid (sulfino): -S(=O)OH, -SO₂H.

Sulfonic acid (sulfo): -S(=O)₂OH, -SO₃H.

Sulfinate (sulfinic acid ester): -S(=O)OR; wherein R is a sulfinate substituent, for example, a C₁₋₇alkyl group, a C₃₋₂₀heterocyclyl group, or a C₅₋₂₀ aryl group, preferably a C₁₋₇alkyl group. Examples of sulfinate groups include, but are not limited to, -S(=O)OCH₃ (methoxysulfinyl; methyl sulfinate) and -S(=O)OCH₂CH₃ (ethoxysulfinyl; ethyl sulfinate).

Sulfonate (sulfonic acid ester): -S(=O)₂OR, wherein R is a sulfonate substituent, for example, a C₁₋₇alkyl group, a C₃₋₂₀ heterocyclyl group, or a C₅₋₂₀ aryl group, preferably a C₁₋₇alkyl group. Examples of sulfonate groups include, but are not limited to, -S(=O)₂OCH₃ (methoxysulfonyl; methyl sulfonate) and -S(=O)₂OCH₂CH₃ (ethoxysulfonyl; ethyl sulfonate).

Sulfinyloxy: -OS(=O)R, wherein R is a sulfinyloxy substituent, for example, a C₁₋₇alkyl group, a C₃₋₂₀heterocyclyl group, or a C₅₋₂₀aryl group, preferably a C₁₋₇alkyl group. Examples of sulfinyloxy groups include, but are not limited to, -OS(=O)CH₃ and -OS(=O)CH₂CH₃.

Sulfonyloxy: -OS(=O)₂R, wherein R is a sulfonyloxy substituent, for example, a C₁₋₇alkyl group, a C₃₋₂₀heterocyclyl group, or a C₅₋₂₀aryl group, preferably a C₁₋₇alkyl group. Examples of sulfonyloxy groups include, but are not limited to, -OS(=O)₂CH₃ (mesylate) and -OS(=O)₂CH₂CH₃ (esylate).

Sulfate: -OS(=O)₂OR; wherein R is a sulfate substituent, for example, a C₁₋₇ alkyl group, a C₃₋₂₀ heterocyclyl group, or a C₅₋₂₀aryl group, preferably a C₁₋₇alkyl group. Examples of sulfate groups include, but are not limited to, -OS(=O)₂OCH₃ and -SO(=O)₂OCH₂CH₃. Sulfamyl (sulfamoyl; sulfinic acid amide; sulfinamide): -S(=O)NR¹R², wherein R¹ and R² are independently amino substituents, as defined for amino groups. Examples of sulfamyl groups include, but are not limited to, -S(=O)NH₂, -S(=O)NH(CH₃), -S(=O)N(CH₃)₂, -S(=O)NH(CH₂CH₃), -S(=O)N(CH₂CH₃)₂, and -S(=O)NHPh.

Sulfonamido (sulfinamoyl; sulfonic acid amide; sulfonamide): -S(=O)₂NR¹R², wherein R¹ and R² are independently amino substituents, as defined for amino groups. Examples of sulfonamido groups include, but are not limited to, -S(=O)₂NH₂, -S(=O)₂NH(CH₃), -S(=O)₂N(CH₃)₂, -S(=O)₂NH(CH₂CH₃), -S(=O)₂N(CH₂CH₃)₂, and -S(=O)₂NHPh.

Sulfamino: -NR¹S(=O)₂OH, wherein R¹ is an amino substituent, as defined for amino groups. Examples of sulfamino groups include, but are not limited to, -NHS(=O)₂OH and -N(CH₃)S(=O)₂OH.

Sulfonamino, -NR¹S(=O)₂R, wherein R¹ is an amino substituent, as defined for amino groups, and R is a sulfonamino substituent, for example, a C₁₋₇alkyl group, a C₃₋₂₀ heterocyclyl group, or a C₅₋₂₀aryl group, preferably a C₁₋₇alkyl group. Examples of sulfonamino groups include, but are not limited to, -NHS(=O)₂CH₃ and -N(CH₃)S(=O)₂C₆H₅.

Sulfinamino: -NR¹S(=O)R, wherein R¹ is an amino substituent, as defined for amino groups, and R is a sulfinamino substituent, for example, a C₁₋₇alkyl group, a C₃₋₂₀ heterocyclyl group, or a C₅₋₂₀aryl group, preferably a C₁₋₇alkyl group. Examples of sulfinamino groups include, but are not limited to, -NHS(=O)CH₃ and -N(CH₃)S(=O)C₆H₅.

Phosphino (phosphine): -PR₂, wherein R is a phosphino substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphino groups include, but are not limited to, -PH₂, -P(CH₃)₂, -P(CH₂CH₃)₂, -P(t-Bu)₂, and -P(Ph)₂.

Phospho: -P(=O)₂.

Phosphinyl (phosphine oxide): -P(=O)R₂, wherein R is a phosphinyl substituent, for example, a C₁₋₇ alkyl group, a C₃₋₂₀ heterocyclyl group, or a C₅₋₂₀ aryl group, preferably a C₁₋₇ alkyl group or a C₅₋₂₀ aryl group. Examples of phosphinyl groups include, but are not limited to, -P(=O)(CH₃)₂, -P(=O)(CH₂CH₃)₂, -P(=O)(t-Bu)₂, and -P(=O)(Ph)₂.

Phosphonic acid (phosphono): -P(=O)(OH)₂.

Phosphonate (phosphono ester): -P(=O)(OR)₂, where R is a phosphonate substituent, for example, -H, a C₁₋₇ alkyl group, a C₃₋₂₀ heterocyclyl group, or a C₅₋₂₀ aryl group, preferably -H, a C₁₋₇alkyl group, or a C₅₋₂₀aryl group. Examples of phosphonate groups include, but are not limited to, -P(=O)(OCH₃)₂, -P(=O)(OCH₂CH₃)₂, -P(=O)(O-t-Bu)₂, and -P(=O)(OPh)₂.

Phosphoric acid (phosphonooxy): -OP(=O)(OH)₂.

Phosphate (phosphonooxy ester): -OP(=O)(OR)₂, where R is a phosphate substituent, for example, -H, a C₁₋₇ alkyl group, a C₃₋₂₀ heterocyclyl group, or a C₅₋₂₀ aryl group, preferably -H, a C₁₋₇alkyl group, or a C₅₋₂₀aryl group. Examples of phosphate groups include, but are not limited to, -OP(=O)(OCH₃)₂, -OP(=O)(OCH₂CH₃)₂, -OP(=O)(O-t-Bu)₂, and -OP(=O)(OPh)₂.

Phosphorous acid: -OP(OH)₂.

Phosphite: -OP(OR)₂, where R is a phosphite substituent, for example, -H, a C₁₋₇alkyl group, a C₃₋₂₀ heterocyclyl group, or a C₅₋₂₀ aryl group, preferably -H, a C₁₋₇ alkyl group, or a C₅₋₂₀ aryl group. Examples of phosphite groups include, but are not limited to, -OP(OCH₃)₂, -OP(OCH₂CH₃)₂, -OP(O-t-Bu)₂, and -OP(OPh)₂,

Phosphoramidite: -OP(OR¹)-NR²₂, where R¹ and R² are phosphoramidite substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidite groups include, but are not limited to, -OP(OCH₂CH₃)-N(CH₃)₂, -OP(OCH₂CH₃)-N(i-Pr)₂, and -OP(OCH₂CH₂CN)-N(i-Pr)₂.

Phosphoramidate: -OP(=O)(OR¹)-NR²₂, where R¹ and R² are phosphoramidate substituents, for example, -H, a (optionally substituted) C₁₋₇alkyl group, a C₃₋₂₀ heterocyclyl group, or a C₅₋₂₀aryl group, preferably -H, a C₁₋₇alkyl group, or a C₅₋₂₀ aryl group. Examples of phosphoramidate groups include, but are not limited to, -OP(=O)(OCH₂CH₃)-N(CH₃)₂. -OP(=O)(OCH₂CH₃)-N(i-Pr)₂, and -OP(=O)(OCH₂CH₂CN)-N(i-Pr)₂.

Alkylene

C₃₋₁₂ alkylene: The term "C₃₋₁₂ alkylene", as used herein, pertains to a bidentate moiety obtained by removing two hydrogen atoms, either both from the same carbon atom, or one from each of two different carbon atoms, of a hydrocarbon compound having from 3 to 12 carbon atoms (unless otherwise specified), which may be aliphatic or alicyclic, and which may be saturated, partially unsaturated, or fully unsaturated. Thus, the term "alkylene" includes the sub-classes alkenylene, alkynylene, cycloalkylene, etc., discussed below.

Examples of linear saturated C₃₋₁₂ alkylene g rou ps include, but are not limited to, -(CH₂)ₙ-where n is an integer from 3 to 12, for example, -CH₂CH₂CH₂- (propylene), -CH₂CH₂CH₂CH₂- (butylene), -CH₂CH₂CH₂CH₂CH₂- (pentylene) and -CH₂CH₂CH₂CH-₂CH₂CH₂CH₂- (heptylene).

Examples of branched saturated C₃₋₁₂alkylene groups include, but are not limited to, -CH(CH₃)CH₂-, -CH(CH₃)CH₂CH₂-, -CH(CH₃)CH₂CH₂CH₂-, -CH₂CH(CH₃)CH₂-, -CH₂CH(CH₃)CH₂CH₂-, -CH(CH₂CH₃)-, -CH(CH₂CH₃)CH₂-, and -CH₂CH(CH₂CH₃)CH₂-.

Examples of linear partially unsaturated C₃₋₁₂ alkylene groups (C₃₋₁₂ alkenylene, and alkynylene groups) include, but are not limited to, -CH=CH-CH₂-, -CH₂-CH=CH₂-, -CH=CH-CH₂-CH₂-, -CH=CH-CH₂-CH₂-CH₂-, -CH=CH-CH=CH-, -CH=CH-CH=CH-CH₂-, - CH=CH-CH=CH-CH₂-CH₂-, -CH=CH-CH₂-CH=CH-, -CH=CH-CH₂-CH₂-CH=CH-, and -CH₂-C=C-CH₂-.

Examples of branched partially unsaturated C₃₋₁₂alkylene groups (C₃₋₁₂alkenylene and alkynylene groups) include, but are not limited to, -C(CH₃)=CH-, -C(CH₃)=CH-CH₂-, -CH=CH-CH(CH₃)- and -C≡C-CH(CH₃)-.

Examples of alicyclic saturated C₃₋₁₂ alkylene groups (C₃₋₁₂ cycloalkylenes) include, but are not limited to, cyclopentylene (e.g. cyclopent-1,3-ylene), and cyclohexylene (e.g. cyclohex-1,4-ylene).

Examples of alicyclic partially unsaturated C₃₋₁₂alkylene groups (C₃₋₁₂ cycloalkylenes) include, but are not limited to, cyclopentenylene (e.g. 4-cyclopenten-1,3-ylene), cyclohexenylene (e.g. 2-cyclohexen-1,4-ylene; 3-cyclohexen-1,2-ylene; 2,5-cyclohexadien-1,4-ylene).

### Includes Other Forms

Unless otherwise specified, included in the above are the well known ionic, salt, solvate, and protected forms of these substituents. For example, a reference to carboxylic acid (-COOH) also includes the anionic (carboxylate) form (-COO⁻), a salt or solvate thereof, as well as conventional protected forms. Similarly, a reference to an amino group includes the protonated form (-N⁺HR¹R²), a salt or solvate of the amino group, for example, a hydrochloride salt, as well as conventional protected forms of an amino group. Similarly, a: reference to a hydroxyl group also includes the anionic form (-O⁻), a salt or solvate thereof, as well as conventional protected forms.

### Salts

It may be convenient or desirable to prepare, purify, and/or handle a corresponding salt of the active compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al⁺³. Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g. -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, trifluoroacetic acid and valeric. Examples of suitable polymeric organic anions include, but are not limited to, those derived from the following polymeric acids: tannic acid, carboxymethyl cellulose.

### Solvates

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a tri-hydrate, etc.

The invention includes compounds where a solvent adds across the imine bond of the PBD moiety, which is illustrated below where the solvent is water or an alcohol (R^{A}OH, where R^{A} is C₁₋₄ alkyl): 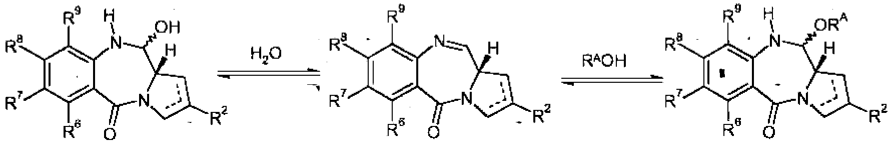 These forms can be called the carbinolamine and carbinolamine ether forms of the PBD (as described in the section relating to R¹⁰ above). The balance of these equilibria depend on the conditions in which the compounds are found, as well as the nature of the moiety itself.

These particular compounds may be isolated in solid form, for example, by lyophilisation.

### Isomers

Certain compounds of the invention may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and l-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or *R* and *S,* are used to denote the absolute configuration of the molecule about its chiral center(s). The prefixes d and l or (+) and (-) are employed to designate the sign of rotation of plane-polarized light by the compound, with (-) or l meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g. C₁₋₇ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hyroxyazo, and nitro/aci-nitro.

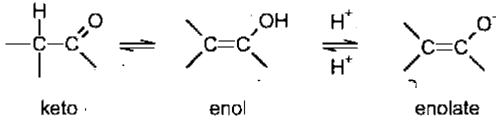

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Note that specifically included in the term "isomer" are compounds with one or more isotopic substitutions. For example, H may be in any isotopic form, including ¹H*,* ²H (D), and ³H (T); C may be in any isotopic form, including ¹²C, ¹³C, and ¹⁴C; O may be in any isotopic form, including ¹⁶O and ¹⁸O; and the like.

Examples of isotopes that can be incorporated into compounds of the invention include isotopes of hydrogen, carbon, nitrogen, oxygen, phosphorous, fluorine, and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl, and ¹²⁵I. Various isotopically labeled compounds of the present invention, for example those into which radioactive isotopes such as 3H, 13C, and 14C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labelled or substituted therapeutic compounds of the invention may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased in vivo half-life or reduced dosage requirements. An 18F labeled compound may be useful for PET or SPECT studies. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., 2H or D) may afford certain therapeutic advantages resulting from greater metabolic stability, for example increased in vivo half-life or reduced dosage requirements or an improvement in therapeutic index. It is understood that deuterium in this context is regarded as a substituent. The concentration of such a heavier isotope, specifically deuterium, may be defined by an isotopic enrichment factor. In the compounds of this invention any atom not specifically designated as a particular isotope is meant to represent any stable isotope of that atom.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

### Biological Activity

### In vitro cell proliferation assays

Generally, the cytotoxic or cytostatic activity of an antibody-drug conjugate (ADC) is measured by: exposing mammalian cells having receptor proteins, e.g. HER2, to the antibody of the ADC in a cell culture medium; culturing the cells for a period from about 6 hours to about 5 days; and measuring cell viability. Cell-based *in vitro* assays are used to measure viability (proliferation), cytotoxicity, and induction of apoptosis (caspase activation) of an ADC of the invention.

The *in vitro* potency of antibody-drug conjugates can be measured by a cell proliferation assay. The CellTiter-Glo^{®} Luminescent Cell Viability Assay is a commercially available (Promega Corp., Madison, WI), homogeneous assay method based on the recombinant expression of *Coleoptera* luciferase (US Patent Nos. 5583024; 5674713 and 5700670). This cell proliferation assay determines the number of viable cells in culture based on quantitation of the ATP present, an indicator of metabolically active cells (Crouch et al (1993) J. Immunol. Meth. 160:81-88; US 6602677). The GellTiter-Glo^{®} Assay is conducted in 96 well format, making it amenable to automated high-throughput screening (HTS) (Cree et al (1995) AntiCancer Drugs 6:398-404). The homogeneous assay procedure involves adding the single reagent (CellTiter-Glo^{®} Reagent) directly to cells cultured in serum-supplemented medium. Cell washing, removal of medium and multiple pipetting steps are not required. The system detects as few as 15 cells/well in a 384-well format in 10 minutes after adding reagent and mixing. The cells may be treated continuously with ADC, or they may be treated and separated from ADC. Generally, cells treated briefly, i.e. 3 hours, showed the same potency effects as continuously treated cells.

The homogeneous "add-mix-measure" format results in cell lysis and generation of a luminescent signal proportional to the amount of ATP present. The amount of ATP is directly proportional to the number of cells present in culture. The Cell Titer-Glo^{®} Assay generates a "glow-type" luminescent signal, produced by the luciferase reaction, which has a half-life generally greater than five hours, depending on cell type and medium used. Viable cells are reflected in relative luminescence units (RLU). The substrate, Beetle Luciferin, is oxidatively decarboxylated by recombinant firefly luciferase with concomitant conversion of ATP to AMP and generation of photons.

### In vivo efficacy

The *in vivo* efficacy of antibody-drug conjugates (ADC) of the invention can be measured by, tumor xenograft studies in mice. For example, the in vivo efficacy of an anti-HER2 ADC of the invention can be measured by a high expressing HER2 transgenic explant mouse model. An allograft is propagated from the Fo5 mmtv transgenic mouse which does not respond to, or responds poorly to, HERCEPTIN® therapy. Subjects were treated once with ADC at certain dose levels (mg/kg) and PBD drug exposure (µg/m²); and placebo buffer control (Vehicle) and monitored over two weeks or more to measure the time to tumor doubling, log, cell kill, and tumor shrinkage.

### Use

The conjugates of the invention may be used to provide a PBD compound at a target location.

The target location is preferably a proliferative cell population. The antibody is an antibody for an antigen present in a proliferative cell population.

In one embodiment the antigen is absent or present at a reduced level in a non-proliferative cell population compared to the amount of antigen present in the proliferative cell population, for example a tumour cell population.

At the target location the linker may be cleaved so as to release a compound of formula (D). Thus, the conjugate may be used to selectively provide a compound of formula (D) to the target location.

The linker may be cleaved by an enzyme present at the target location.

The target location may be *in vitro, in vivo* or *ex vivo.*

The antibody-drug conjugate (ADC) compounds of the invention include those with utility for anticancer activity. In particular, the compounds include an antibody conjugated, i.e. covalently attached by a linker, to a PBD drug moiety, i.e. toxin. When the drug is not conjugated to an antibody, the PBD drug has a cytotoxic effect. The biological activity of the PBD drug moiety is thus modulated by conjugation to an antibody. The antibody-drug conjugates (ADC) of the invention selectively deliver an effective dose of a cytotoxic agent to tumor tissue whereby greater selectivity, i.e. a lower efficacious dose, may be achieved.

Thus, in one aspect, the present invention provides a conjugate compound as described herein for use in therapy.

In a further aspect there is also provides a conjugate compound as described herein for use in the treatment of a proliferative disease. A second aspect of the present invention provides the use of a conjugate compound in the manufacture of a medicament for treating a proliferative disease.

One of ordinary skill in the art is readily able to determine whether or not a candidate conjugate treats a proliferative condition for any particular cell type. For example, assays which may conveniently be used to assess the activity offered by a particular compound are described in the examples below.

The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g. histocytoma, glioma, astrocyoma, osteoma), cancers (e.g. lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), leukemias, psoriasis, bone diseases, fibroproliferative disorders (e.g. of connective tissues), and atherosclerosis. Cancers of particular interest include, but are not limited to, leukemias and ovarian cancers.

Any type of cell may be treated, including but not limited to, lung, gastrointestinal (including, e.g. bowel, colon), breast (mammary), ovarian, prostate, liver (hepatic), kidney (renal), bladder, pancreas, brain, and skin.

In one embodiment, the treatment is of a pancreatic cancer.
In one embodiment, the treatment is of a tumour having αᵥβ₆ integrin on the surface of the cell.

It is contemplated that the antibody-drug conjugates (ADC) of the present invention may be used to treat various diseases or disorders, e.g. characterized by the overexpression of a tumor antigen. Exemplary conditions or hyperproliferative disorders include benign or malignant tumors; leukemia, haematological, and lymphoid malignancies. Others include neuronal, glial, astrocytal, hypothalamic, glandular, macrophagal, epithelial, stromal, blastocoelic, inflammatory, angiogenic and immunologic, including autoimmune, disorders. Generally, the disease or disorder to be treated is a hyperproliferative disease such as cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

autoimmune diseases for which the ADC compounds may be used in treatment include rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), osteoarthritis, autoimmune gastrointestinal and liver disorders (such as, for example, inflammatory bowel diseases (e.g. ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, , for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g. Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

### Methods of Treatment

The conjugates of the present invention may be used in a method of therapy. Also provided is a method of treatment, comprising administering to a subject in need of treatment a therapeutically-effective amount of a conjugate compound of the invention. The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound of the invention may be administered alone or in combination with other treatments, either simultaneously or sequentially dependent upon the condition to be treated. Examples of treatments and therapies include, but are not limited to, chemotherapy (the administration of active agents, including, e.g. drugs, such as chemotherapeutics); surgery; and radiation therapy.

A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Chemotherapeutic agents include compounds used in "targeted therapy" and conventional chemotherapy..

Examples of chemotherapeutic agents include: erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL®, Bristol-Myers Squibb Oncology, Princeton, N.J.), trastuzumab (HERCEPTIN®, Genentech), temozolomide (4-methyl-5-oxo- 2,3,4,6,8-pentazabicyclo [4.3.0] nona-2,7,9-triene-9-carboxamide, CAS No. 85622-93-1, TEMODAR®, TEMODAL®, Schering Plough), tamoxifen ((Z)-2-[4-(1,2-diphenylbut-1-enyl)phenoxy]-*N,N*-dimethylethanamine, NOLVADEX®, ISTUBAL®, VALODEX®), and doxorubicin (ADRIAMYCIN®), Akti-1/2, HPPD, and rapamycin.
More examples of chemotherapeutic agents include: oxaliplatin (ELOXATIN®, Sanofi), bortezomib (VELCADE®, Millennium Pharm.), sutent (SUNITINIB®, SU11248, Pfizer), letrazole (FEMARA®, Novartis), imatinib mesylate (GLEEVEC®, Novartis), XL-518 (Mek inhibitor, Exelixis, WO 2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX®, AstraZeneca), leucovorin (folinic acid), rapamycin (sirolimus, RAPAMUNE®, Wyeth), lapatinib (TYKERB®, GSK572016, Glaxo Smith Kline), Ionafarnib (SARASAR™, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA™, Johnson & Johnson), ABRAXANE™ (Cremophor-free), albumin-engineered nanoparticle formulations of paclitaxel (American Pharmaceutical Partners, Schaumberg, II), vandetanib (rINN, ZD6474, ZACTIMA®, AstraZeneca), chloranmbucil, AG1478, AG1571 (SU 5271; Sugen), temsirolimus (TORISEL®, Wyeth), pazopanib (GlaxoSmithKline), canfosfamide (TELCYTA®, Telik), thiotepa and cyclosphosphamide (CYTOXAN®, NEOSAR®); alkyl sulfonates such as busulfan, improsulfan and piposulfan; aziridines such as benzodopa, carboquone, meturedopa, and uredopa; ethylenimines and; methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g. calicheamicin, calicheamicin gamma1I, calicheamicin omegal1 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, nemorubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE®); novantrone; teniposide; edatrexate; daunomycin; aminopterin; capecitabine (XELODA®, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate): (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE®, Genta Inc.); (vii) ribozymes such as VEGF expression inhibitors (e.g., ANGIOZYME®) and H ER2 expression inhibitors; (viii) vaccines such as gene therapy vaccines, for example, ALLOVECTIIN®, LEUVECTIN®, and VAXID®; PROLEUKIN® rIL-2; topoisomerase 1 inhibitors such as LURTOTECAN®; ABARELIX® rmRH; (ix) anti-angiogenic agents such as bevacizumab (AVASTIN®, Genentech); and pharmaceutically acceptable salts, acids and derivatives of any of the above.
Also included in the definition of "chemotherapeutic agent" are therapeutic antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG™, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth).

Humanized monoclonal antibodies with therapeutic potential as chemotherapeutic agents in combination with the conjugates of the invention include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

Pharmaceutical compositions according to the present invention, and for use in accordance with the present invention, may comprise, in addition to the active ingredient, i.e. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

### Formulations

While it is possible for the conjugate compound to be used (e.g., administered) alone, it is often preferable to present it as a composition or formulation.

In one embodiment, the composition is a pharmaceutical composition (e.g., formulation, preparation, medicament) comprising a conjugate compound, as described herein, and a pharmaceutically acceptable carrier, diluent, or excipient.

In one embodiment, the composition is a pharmaceutical composition comprising at least one conjugate compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In one embodiment, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

Another aspect of the present invention pertains to methods of making a pharmaceutical composition comprising admixing at least one [¹¹C]-radiolabelled conjugate or conjugate-like compound, as defined herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulation may be prepared to provide for rapid or slow release; immediate, delayed, timed, or sustained release; or a combination thereof.

Formulations suitable for parenteral administration (e.g., by injection), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the active ingredient in the liquid is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets,

### Dosage

It will be appreciated by one of skill in the art that appropriate dosages of the conjugate compound, and compositions comprising the conjugate compound, can vary from patient two patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the active compound is in the range of about 100 ng to about 25 mg (more typically about 1 µg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

In one embodiment, the active compound is administered to a human patient according to the following dosage regime: about 100 mg, 3 times daily.

In one embodiment, the active compound is administered to a human patient according to the following dosage regime: about 150 mg, 2 times daily.

In one embodiment, the active compound is administered to a human patient according to the following dosage regime: about 200 mg, 2 times daily.

However in one embodiment, the conjugate compound is administered to a human patient according to the following dosage regime: about 50 or about 75 mg, 3 or 4 times daily.

In one embodiment, the conjugate compound is administered to a human patient according to the following dosage regime: about 100 or about 125 mg, 2 times daily.

The dosage amounts described above may apply to the conjugate (including the PBD moiety and the linker to the antibody) or to the effective amount of PBD compound provided, for example the amount of compound that is releasable after cleavage of the linker.

For the prevention or treatment of disease, the appropriate dosage of an ADC of the invention will depend on the type of disease to be treated, as defined above, the severity and course of the disease, whether the molecule is administered for preventive or therapeutic purposes, previous therapy, the patient's clinical history and response to the antibody, and the discretion of the attending physician. The molecule is suitably administered to the patient at one time or over a series of treatments. Depending on the type and severity of the disease, about 1 µg/kg to 15 mg/kg (e.g. 0.1-20 mg/kg) of molecule is an initial candidate dosage for administration to the patient, whether, for example, by one or more separate administrations, or by continuous infusion. A typical daily dosage might range from about 1 µg/kg to 100 mg/kg or more, depending on the factors mentioned above. An exemplary dosage of ADC to be administered to a patient is in the range of about 0.1 to about 10 mg/kg of patient weight. For repeated administrations over several days or longer, depending on the condition, the treatment is sustained until a desired suppression of disease symptoms occurs. An exemplary dosing regimen comprises a course of administering an initial loading dose of about 4 mg/kg, followed by additional doses every week, two weeks, or three weeks of an ADC. Other dosage regimens may be useful. The progress of this therapy is easily monitored by conventional techniques and assays.

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount," as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

### Preparation of Antibody drug conjugates

Antibody drug conjugates may be prepared by several routes, employing organic Chemistry reactions, conditions, and reagents known to those skilled in the art, including: (1) reaction of a nucleophilic group or an electrophilic group of an antibody with a bivalent linker reagent, to form antibody-linker intermediate Ab-L, via a covalent bond, followed by reaction with an activated drug moiety reagent ; and (2) reaction of a drug moiety reagent with a linker reagent, to form drug-linker reagent D-L, via a covalent bond, followed by reaction with the nucleophilic group or an electrophilic group of an antibody. Conjugation methods (1) and (2) may be employed with a variety of antibodies, and linkers to prepare the antibody-drug conjugates of the invention.
Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Each cysteine disulfide bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

Antibody-drug conjugates may also be produced by modification of the antibody to introduce electrophilic moieties, which can react with nucleophilic substituents on the linker reagent. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In I one embodiment, reaction of the carbohydrate portion of a glycosylated antibody with either galactose oxidase or sodium meta-periodate may yield carbonyl (aldehyde and ketone) groups in the protein that can react with appropriate groups on the drug (Hermanson, G.T. (1996) Bioconjugate Techniques; Academic Press: New York, p 234-242). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Reactive nucleophilic groups may be introduced on the anthracycline derivative compounds by standard functional group interconversions. For example, hydroxyl groups may be converted to thiol groups by Mitsunobu-type reactions, to form thiol-modified drug compounds.

### The Subject/Patient

The subject/patient may be an animal, mammal, a placental mammal, a marsupial (e.g., kangaroo, wombat), a monotreme (e.g., duckbilled platypus), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the subject/patient may be any of its forms of development, for example, a foetus. In one preferred embodiment, the subject/patient is a human.

In one embodiment, the patient is a population where each patient has a tumour having αᵥβ₆ integrin on the surface of the cell.

### Synthesis

In one embodiment, a dimer conjugate of formula VIII may be prepared from compounds I and II as shown in Scheme 1.

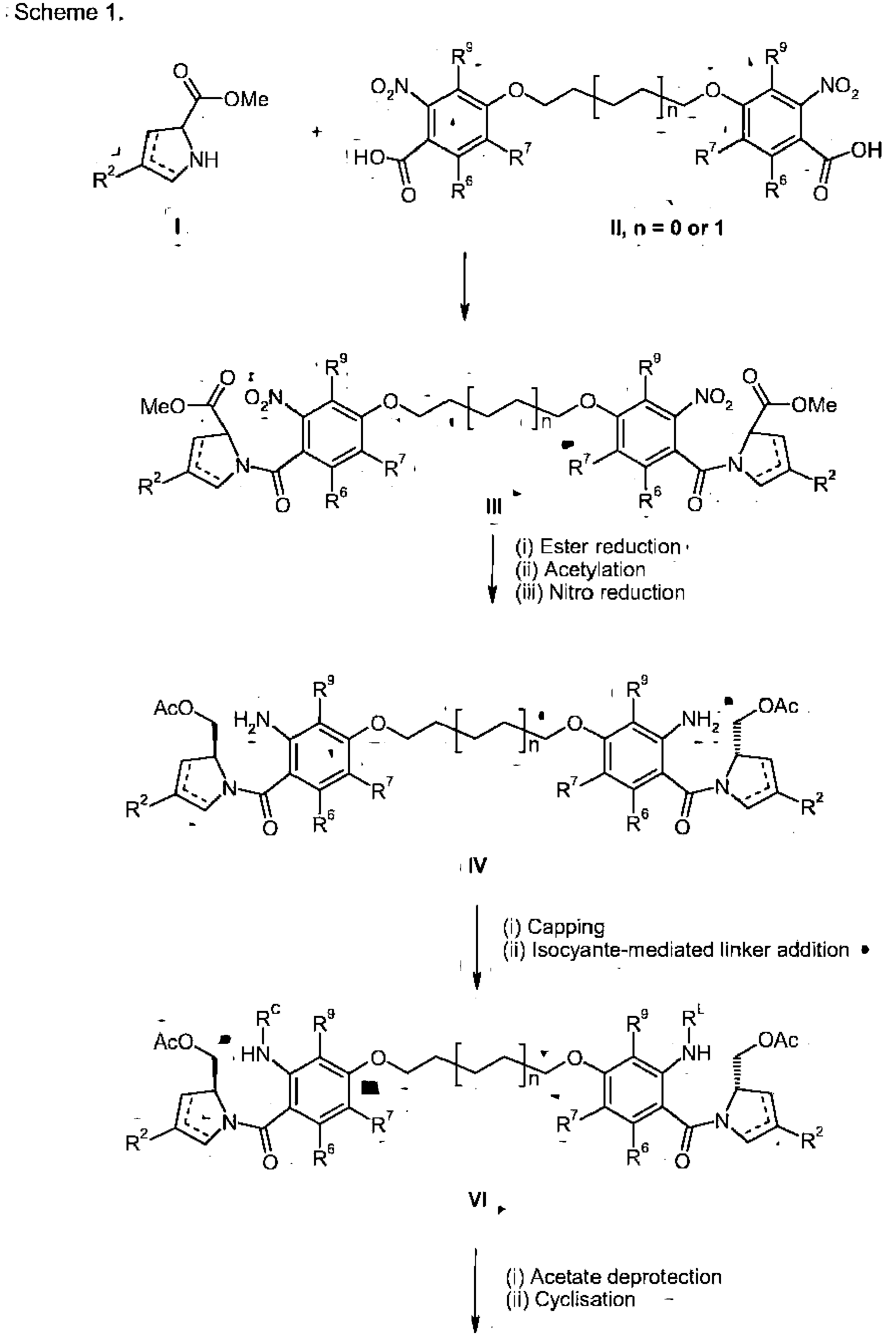

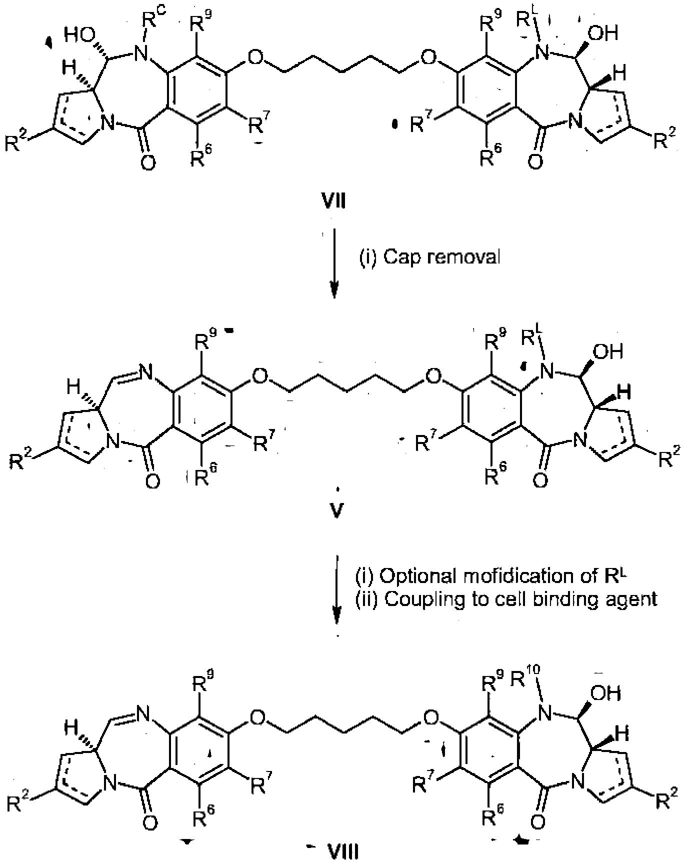

In general, unsymmetrical dimers may be prepared by treating bis-amino compounds of formula **IV** with one equivalent of a commercially available (or readily prepared) chloroformate reagent in order to break the symmetry of the molecules. The remaining free amine can then be functionalised independently to introduce the required therapeutically labile progroup (R^{L}). Further functional group manipulation to close the PBD B-ring, remove protecting and capping groups and introduce the antibody-linking functional group, e.g. G¹, affords the target molecule.

Compounds of formula **IV** are typically prepared by coupling a suitably functionalised C-ring fragment (I) to an A-ring containing dimer core of formula **II.** C-ring fragments may be prepared from known carbamate protected methyl 4-oxoprolinate building blocks. Olefination under Wittig or Horner-Emmons conditions can be employed to furnish endo- or exo-unsaturated alkenes. Alternatively, tandem triflation and Suzuki coupling reactions can be used to obtain 4-aryl substituted 3,4 or 4,5-unsaturated C-ring fragments. C-ring and A-ring fragments can be coupled under standard conditions in the presence of triethylamine, using acid chloride derivatives of the A-ring fragments to give molecules of formula III. Compounds of type **III** can be reduced, without affecting *endo* or *exo* C-ring unsaturation, with zinc in acetic acid to afford molecules of formula **IV.**

Unsymmetrical carbamates of type VI can be prepared by treating bis-amines of type IV with a single equivalent of a commercially available (or readily prepared) chloroformates in the presence of pyridine or triethylamine. Chloroformates may be selected to afford carbamate capping units (R^{C}) which are either orthogonal or identical to those used in the progroup (R^{L}). Identical carbamates allow simultaneous removal of both protecting groups saving synthetic steps. However, removal of the capping carbamates (R^{C}) requires addition of antibody-linking functionality to take place in the presence of a sensitive N10-C11 imine or carbinolamine moiety. If necessary this situation can be avoided by the use of orthogonal carbamate protecting groups which allow addition of antibody-linking moieties whilst keeping the N10-C11 carbinolamine moiety protected. In this strategy the N10-C11 moiety must be unmasked in the presence of the antibody-linking moiety and the reagents used must be compatible with this moiety. For example, if an N10-C11 imine is to be unmasked in the presence of a maleimide group, Troc and Teoc would be suitable R^{C} groups as the deprotecting agents, Cd/Pb couple and TBAF, should not affect the maleimide group. On the other hand the Alloc group should be avoided as π-allyl scavengers such as pyrrolidine may add in 1,4-fashion to the maleimide group. The R^{L} carbamate may be introduced by converting the remaining amino group to an isocyanate and quenching it with the R^{L} alcohol. Alternatively the R^{L} alcohol can be converted to a chloroformate or functional equivalent (fluoroformate, *p*-nitrocarbonate, pentafluorocarbonate or hydroxybenzotriazole carbonate). Finally, the remaining amino group can be converted to a reactive p-nitrocarbamate, pentafluorocarbamate or hydroxybenzotriazole carbamate which can be displaced with the R^{L} alcohol to afford molecules of formula **VI.**

Molecules of formula **VII** can be prepared from molecules of formula **VI** by removing the acetate protecting groups, with potassium carbonate in aqueous methanol, or in the presence of an Fmoc group in R^{L} with lithium triethylborohydride. Oxidation with Dess-Martin periodinane (or alternatively TPAP/NMO, PDC or under Swern conditions) affords the ring closed product.

Conjugates of formula **V** may be prepared from molecules of formula **VII** by removal of the capping group R^{C}, elaboration of R^{L} to include an antibody-linking moiety (e.g. a maleimidocaproyl group) which can be conjugated to a cell binding agent, such as an antibody, under standard conditions (see Dubowchik et al. Bioconjugate Chemistry, 2002, 13,855-869). The elaboration of R^{L} may include the step of extending the group to include a spacer element, such as a group G¹, which may then be used to connect to a cell binding agent (thereby forming the group A).

Monomer compounds and symmetrical dimers may be prepared in a similar manner to the unsymmetrical dimer as described above.

In another embodiment, a conjugate of formula **XVIII** may be prepared from compound **IX** as shown in Scheme 2.

### Compound II

The synthesis of compounds of formula (II) is described in the applicant's earlier application, WO 2006/111759 and is also described by Gregson et al. (J. Med. Chem. 2001, 44, 1161-1174). The preparation of compound (II) as described therein is specifically incorporated by reference herein. Compound (IIa) has a three carbon linker. Compound (IIb) has a five carbon linker.

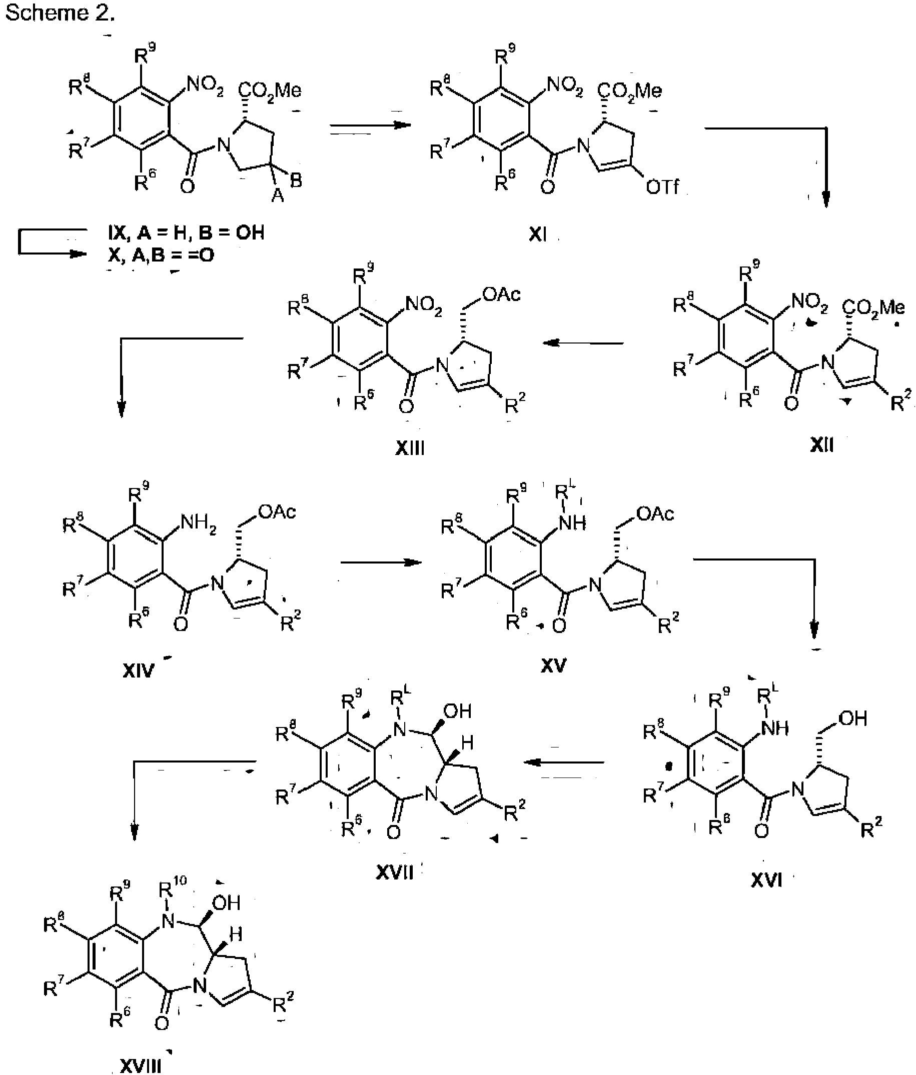

In this scheme the group R² is a C₅₋₂₀ aryl group. Compounds of formula **IX** are described in WO 2004/043963.

The compounds of formula **X** can be synthesised from compounds of formula **IX** by oxidation for example using: TCCA and TEMPO; BAIB and TEMPO; TPAP; Dess-Martin conditions; or Swem conditions.

Compounds of formula **IX** may be synthesised by coupling appropriate compounds of formulae B and C, or activated derivatives thereof:

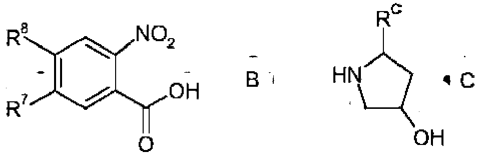

Compound of formulae B and C are generally commercially available or readily synthesisable. If compound B is a dimer, then this may be synthesised as described in WO 00/12508.

Compounds of formula **XI** may be prepared from a compound of formula **X** in a method comprising treating **X** with the appropriate anhydride and anhydrous 2,6-lutidine or anhydrous 2,6-*t*Bu-pyridine at a temperature of -35°C or lower in a dry organic solvent under a inert atmosphere. **XI** is substantially free of the compound having a C1-C2 double bond.

Note, the preparation of compounds having a C1-C2 double bond is described by Kang et al., Chem. Commun., 2003, 1680-1689

Compounds of formula **XI** can be converted into compounds of formula **XII.** The conversion (a Suzuki coupling) is carried out by palladium catalysed cross coupling of **XI** with the appropriate aryl boron derivative. The palladium catalyst may be any suitable catalyst, for example Pd(PPh₃)₄, Pd(OCOCH₃)₂, PdCl₂, Pd(dba)₃.

Compounds of formula **XII** can be converted into compounds of formula **XIV** via compound **XIII.** The conversion is achieved by first reducing of the ester and reprotection as an acetate (or silyl ether in an alternative approach). The reduction can be achieved by standard means, for example with LiAlH₄ or NaBH₄. Reprotection as an acetate can be achieved, for example, by reaction with acetyl chloride (reprotection as a silyl ether can be achieved, for example, by reaction with the appropriate silyl chloride). The reduction of the nitro group is then carried out using, for example, zinc in acetic acid.

Compounds of formula **XIV** can be converted into compounds of formula **XV**. This conversion is usually achieved by reaction of **XIV** with triphosgene to obtain the isocyanate followed by reaction with R^{L}-OH. This approach is described in WO 2005/023814. Alternatively, simple nitrogen protecting groups can also be introduced as a chloroformate, fluoroformate or azidoformate. The more complex nitrogen protecting groups, as well as the simple nitrogen protecting groups, can be introduced as D-succinamide carbonates, D-pentaflurophenyl carbonates and D-nitrophenyl carbonates.

The conversion of **XV** to **XVII** may be achieved by initial removal of the acetate protecting group, with potassium carbonate in aqueous methanol, or with lithium triethylborohydride. Oxidation with Dess-Martin periodinane (or alternatively TPAP/NMO, TFAA/DMSO, SO₃.Pyridine complex/DMSO, PDC, PCC, BAIB/TEMPO or under Swern conditions) affords the ring closed product. If a silyl ether is used instead of an acetate, the conversion of **XV** to **XVII** may be achieved by initial removal of the silyl ether protecting group, for example using TBAF in THF, acetic acid in aqueous THF, CsF in DMF or HF in pyridine, followed by oxidation as described above.

The compound **XVIII** is then attached to a cell binding agent. The sequence of step or steps from **XVII** to **XVIII** depends on the nature of R^{L}. This group may be modified, and then attached to a cell binding agent to form a conjugate of the invention. For example, a protecting group cap may be removed to provide a functionality suitable for reaction with a cell binding agent. In other steps, this same functionality may be used to connect to a further spacer element, such as a group G¹, and that spacer element may then in turn be connected to the cell binding agent (thereby forming the group A).

In some embodiments of the invention there are provided compounds of formula A-I, including compounds of formula A-A and A-B. Compounds of this type may be prepared using methods similar to those described in WO 2010/091150. The intermediate compounds described in WO 2010/091150 may also be employed in the methods described above.

For example, the dimer compound (15) shown in paragraph [164] may be used as compound **(III)** in Scheme I above. Monomer compounds of the type shown as compounds (3), (6) and (9) This, and further adaptations, would be apparent to one of skill in the art.

### Preferred Syntheses

In one embodiment, the conjugate is compound **14,** and is prepared as shown in Scheme 3. The dipeptides 7a,b and 8 are prepared as described in the experimental section below. In that scheme, the linker portions L¹ and L² have the structures:

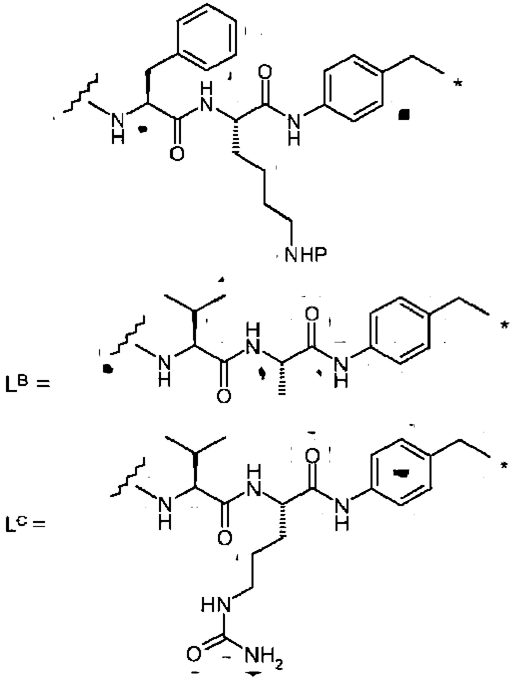

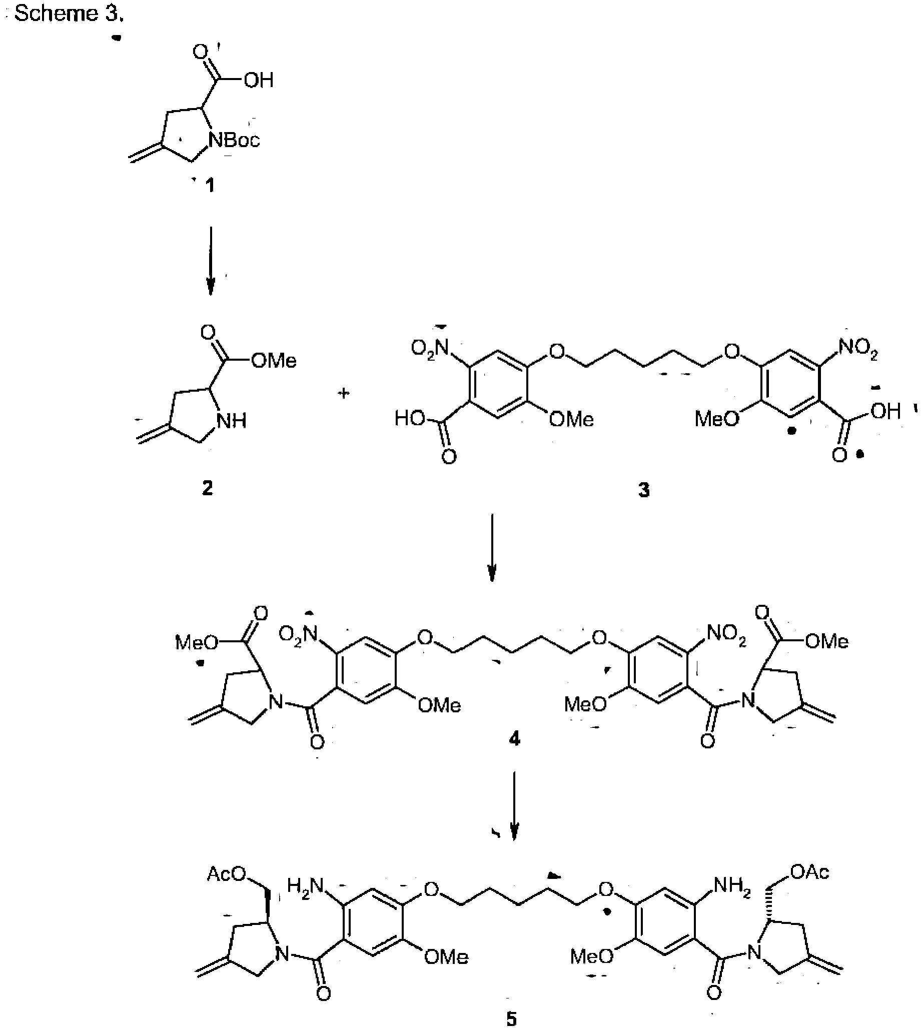

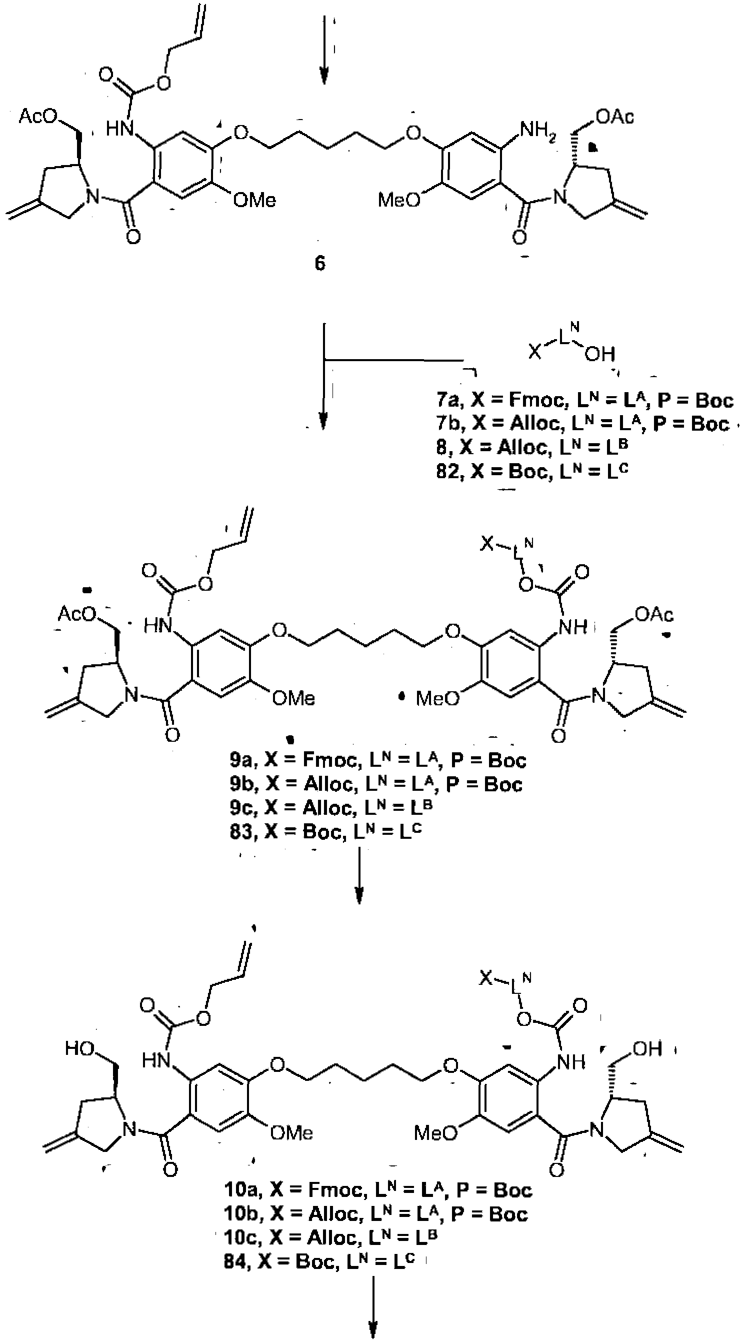

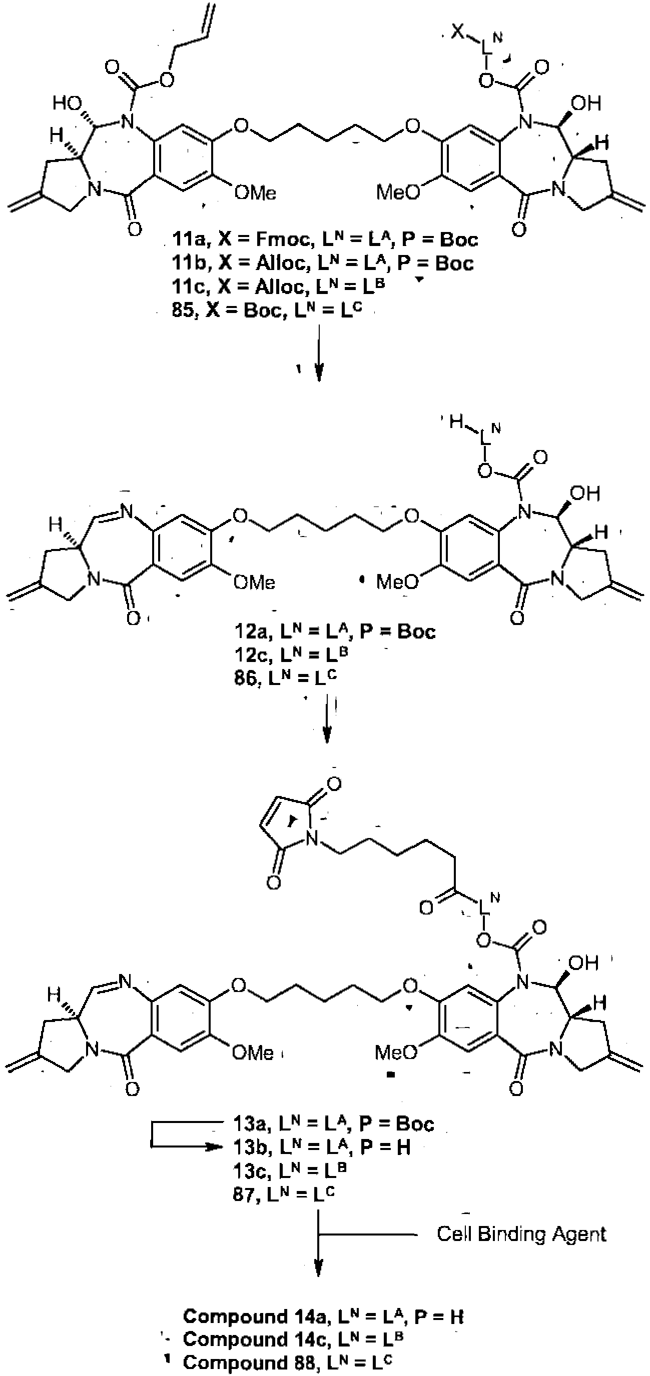

The compound 13c, where the dipeptide corresponds to L², may be prepared from 12c by an analogous method.

In one embodiment, the conjugate is compound **16a or 16b,** and the compound is prepared as shown in Scheme 4 below, where compound **12a** may be prepared as described above:

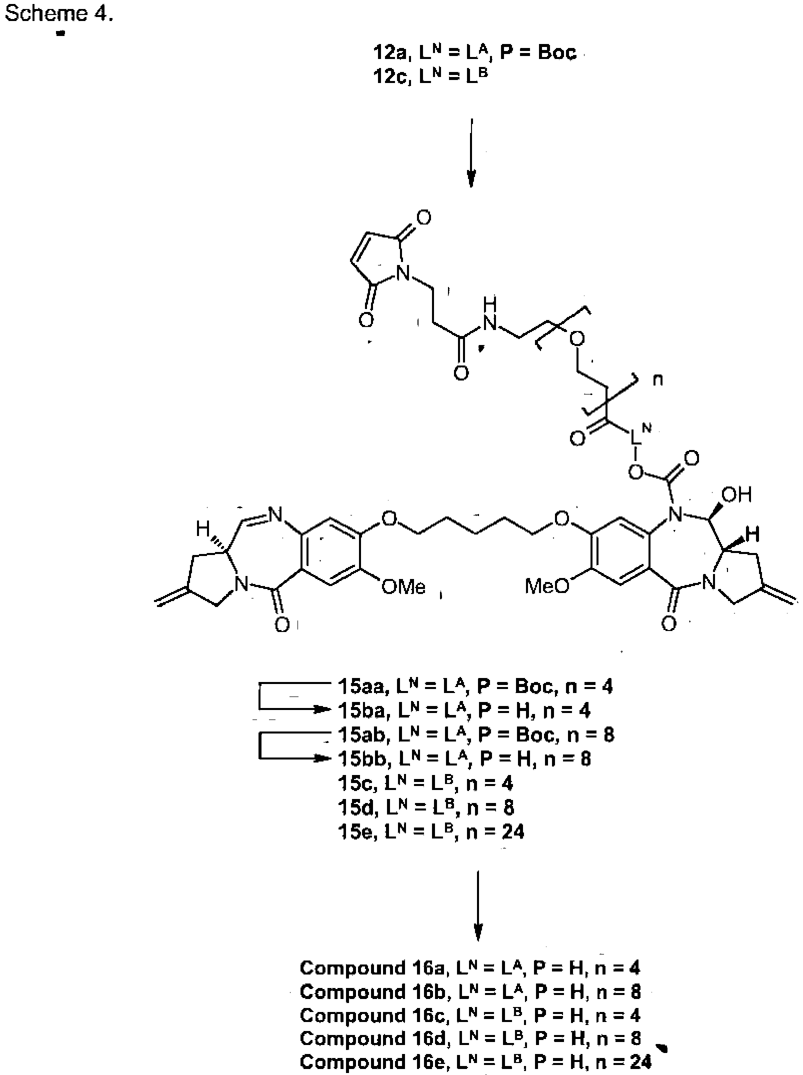

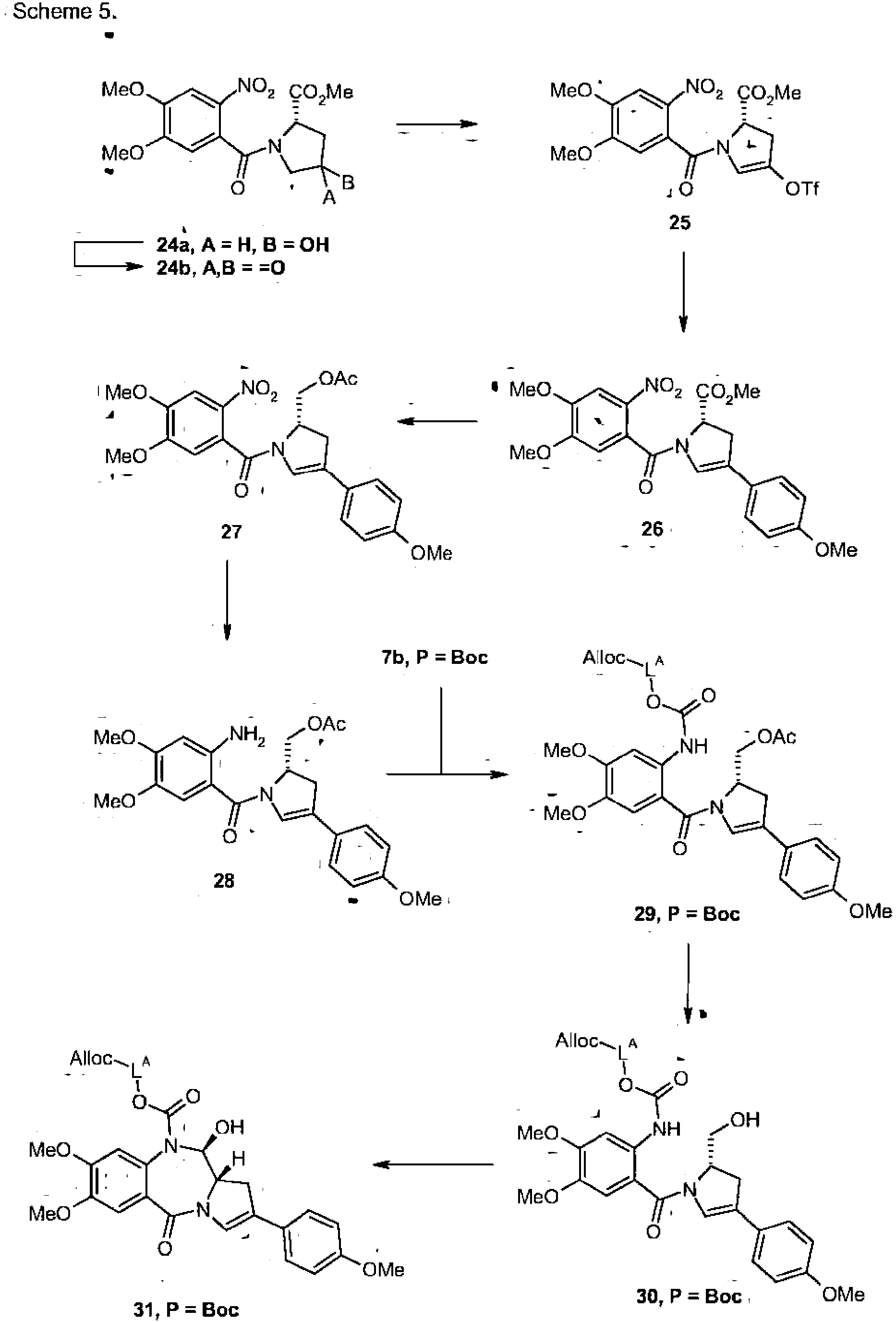

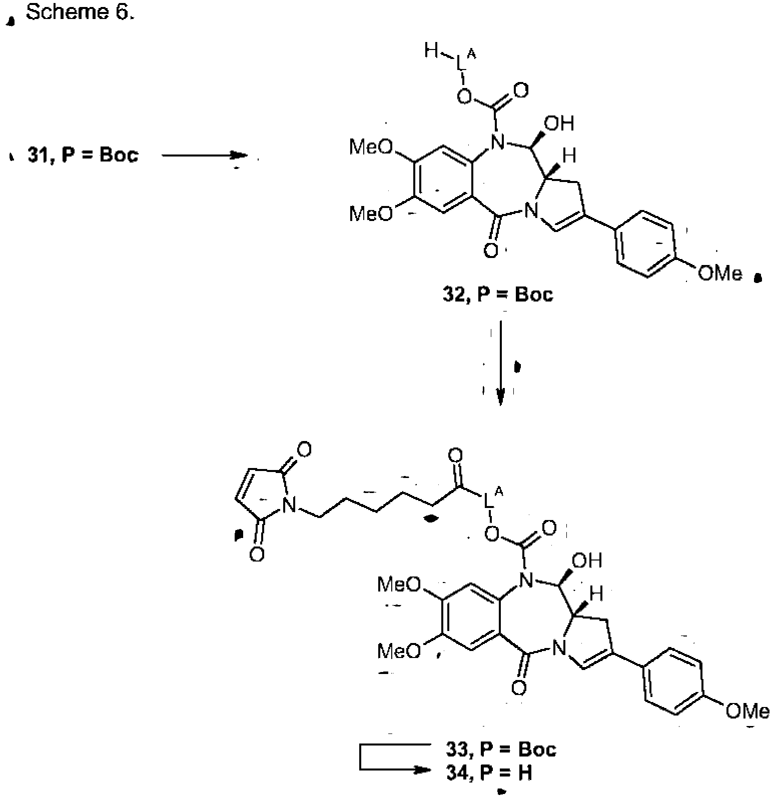

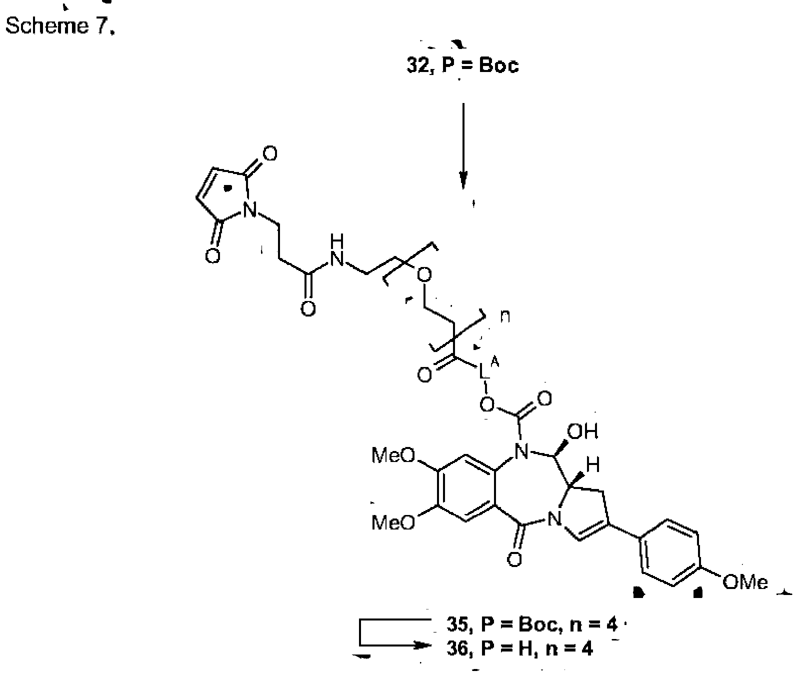

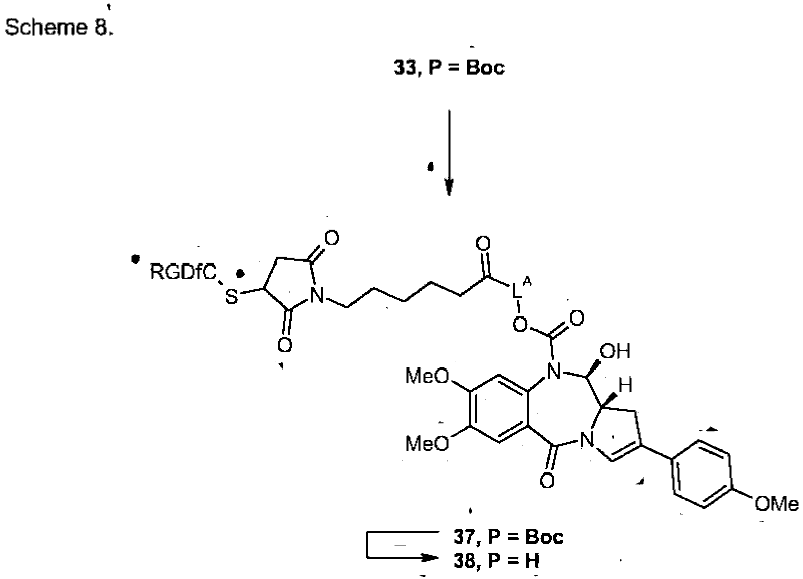

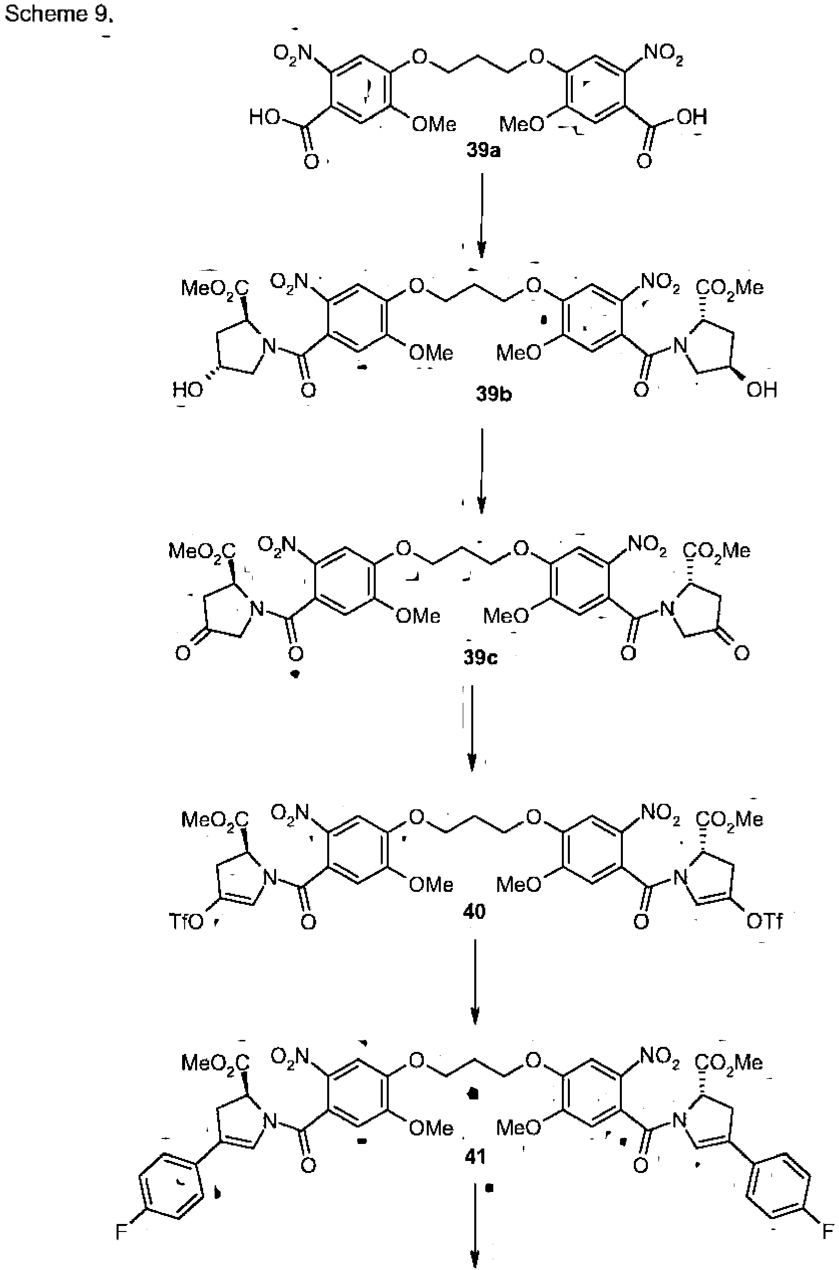

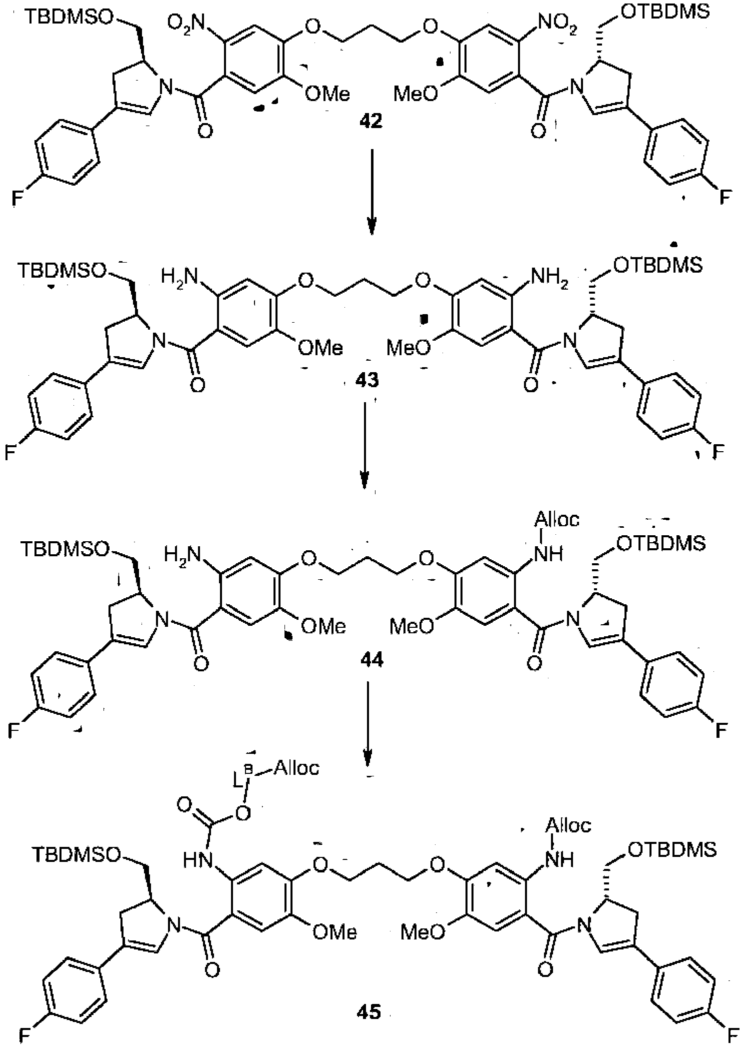

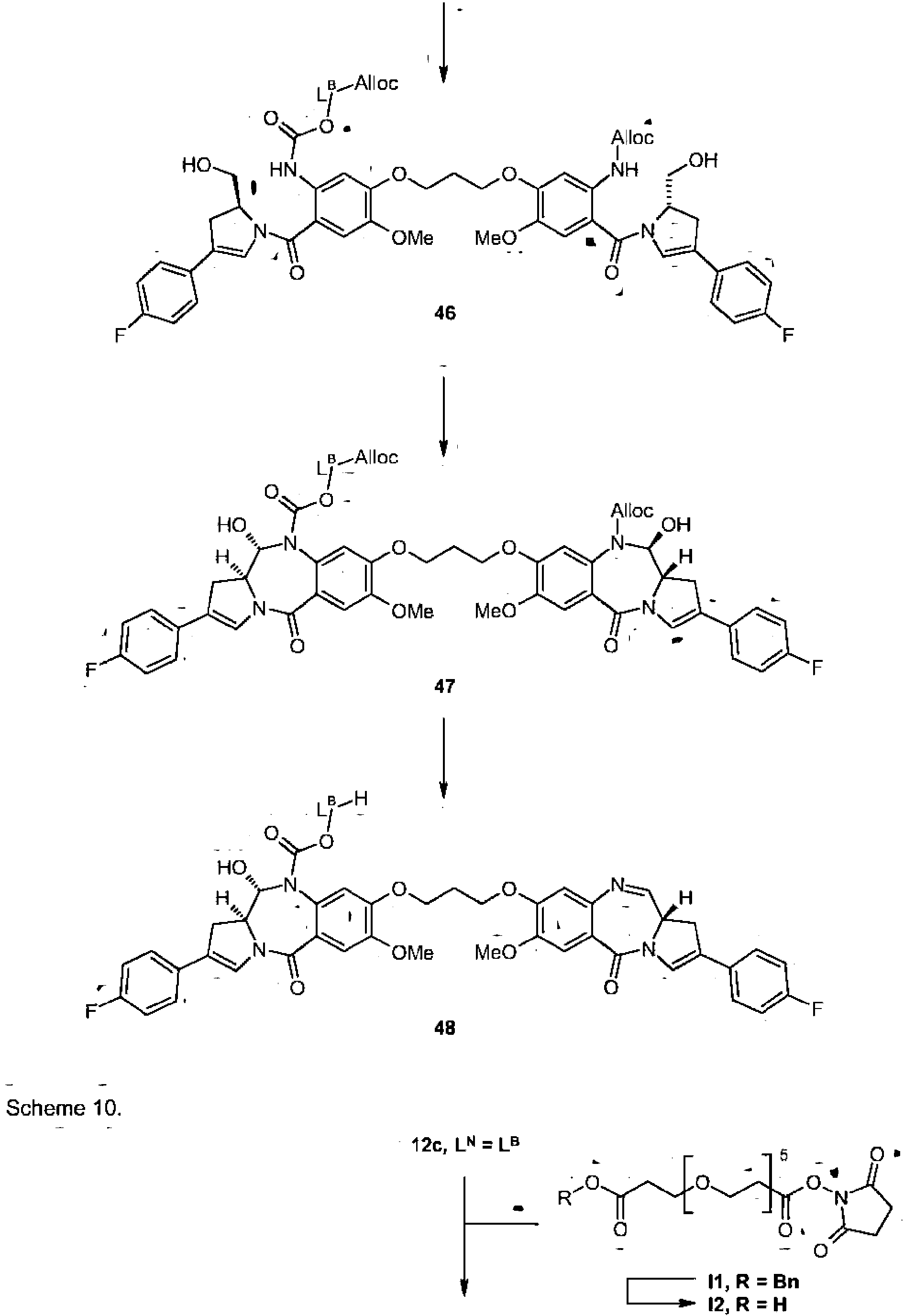

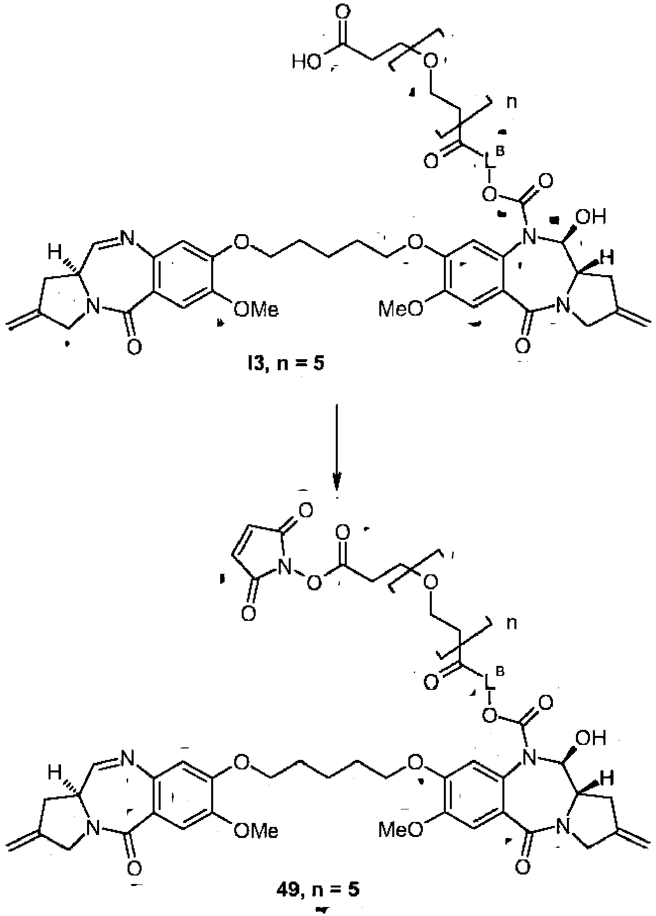

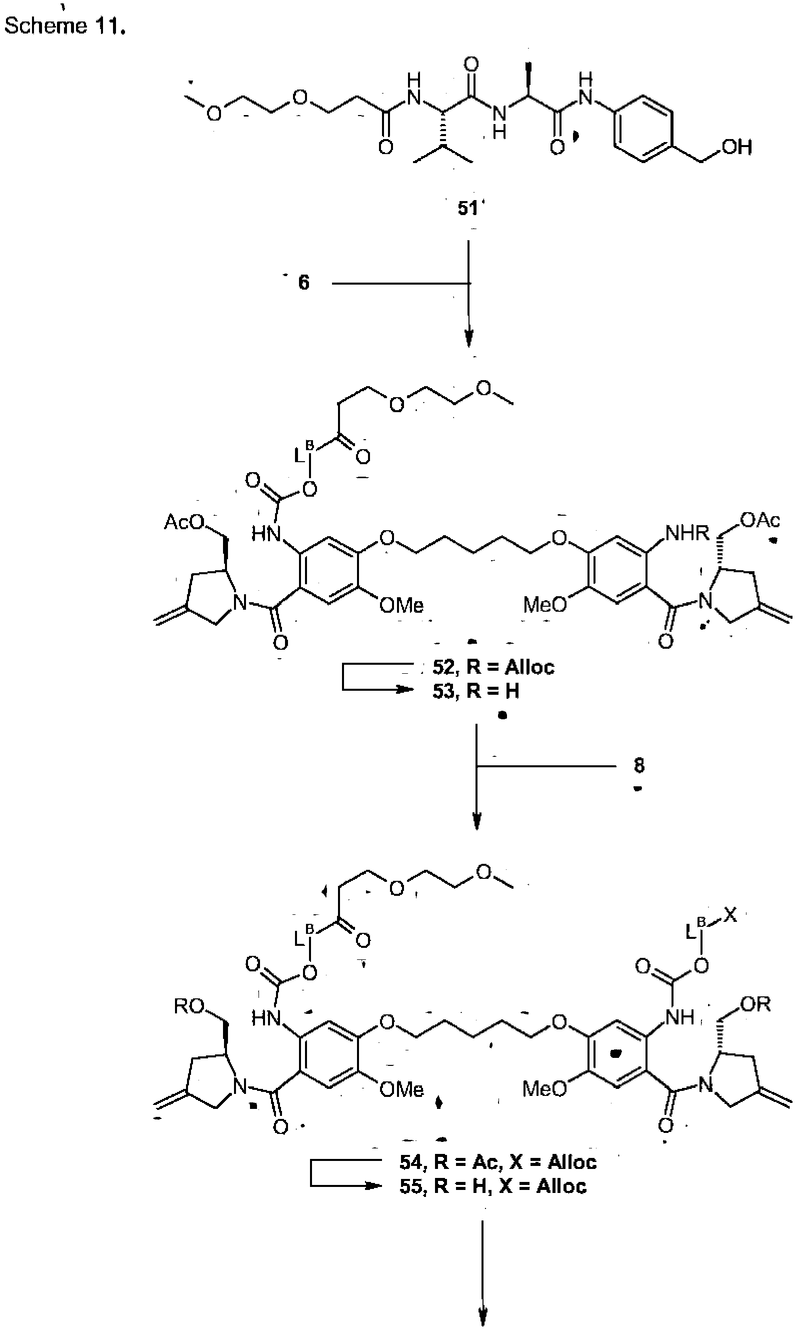

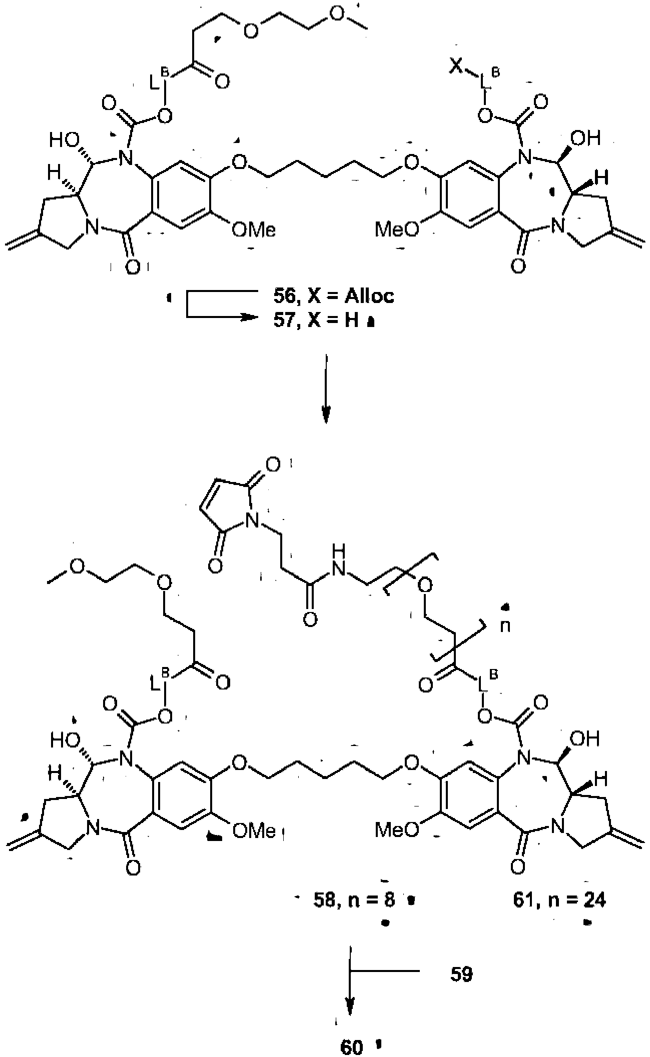

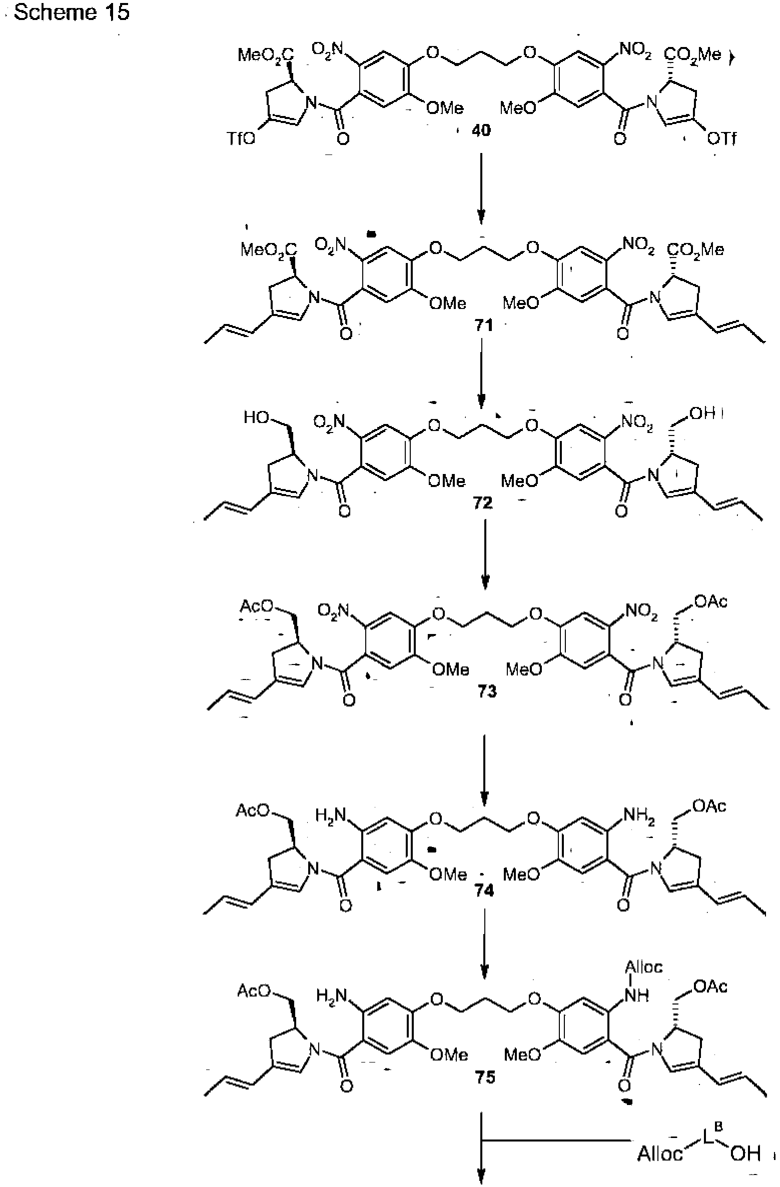

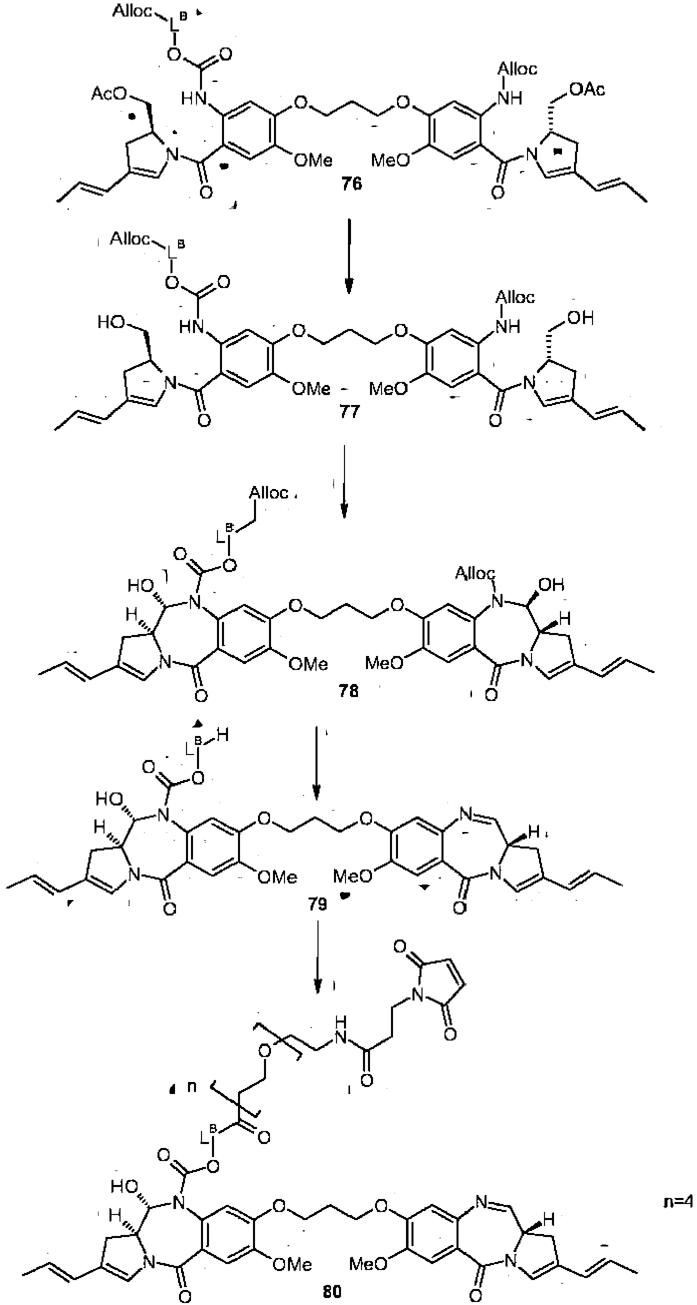

The invention will now be further described with reference to the following non-limiting Examples. Other embodiments of the invention will occur to those skilled in the art in the light of these.

The disclosure of all references cited herein, inasmuch as it may be used by those skilled in the art to carry out the invention, is hereby specifically incorporated herein by cross-preference.

### Experimental

### General Information

Reaction progress was monitored by thin-layer chromatography (TLC) using Merck Kieselgel 60 F254 silica gel, with fluorescent indicator on aluminium plates. Visualisation of TLC was achieved with UV light or iodine vapour unless otherwise stated. Flash chromatography was performed using Merck Kieselgel 60 F254 silica gel. Extraction and chromatography solvents were bought and used without further purification from Fisher Scientific, U.K. All chemicals were purchased from Aldrich, Lancaster or BDH.

The LC/MS conditions were as follows: The HPLC (Waters Alliance 2695) was run using a mobile phase of water (A) (formic acid 0.1%) and acetonitrile (B) (formic acid 0.1%). Gradient: initial composition 5% B over 1.0 min then 5% B to 95% B within 3 min. The composition was held for 0.5 min at 95% B, and then returned to 5% B in 0.3 minutes. Total gradient run time equals 5 min. Flow rate 3.0 mL/min, 400µL was split via a zero dead volume tee piece which passes into the mass spectrometer. Wavelength detection range: 220 to 400 nm. Function type: diode array (535 scans). Column: Phenomene^{®} Onyx Monolithic C18 50 x 4.60 mm.

The following semi-preparative HPLC method was used: Reverse-phase high-performance liquid chromatography (HPLC) was carried out on Zorbax Eclipse XDB C-18 columns of the following dimensions: 150 x 4.6 mm for analysis, and 250 x 9.4 mm for preparative work. All HPLC experiments were performed with gradient conditions: initial fixed composition 5% B to 50% B over 20 min, held for 5 min at 50% B, then 50% B to 100% B within 2 min, held for 3 min at 100% B, returned to 5% B in 2 min and held for 3 min. Total duration of gradient run was 35 min. Eluents used were solvent A (H₂O with 0.02% TFA) and solvent B (CH₃CN with 0.02% TFA). Flow rates used were 1.20 ml/min for analytical, and 5.00 ml/min for preparative HPLC.

### Compound 2 - (S)-2-(methoxycarbonyl)-4-methylenepyrrolidinium chloride

Compound 2 is also described for use in WO 2007/085930 in the preparation of PBD compounds.

Compound 2 may be prepared from *trans*-4-hydroxy-proline as described in WO 2007/085930, which is hereby incorporated by reference. In particular Example 13, describing the preparation of the TFA salt of compound **2,** is particularly relevant.

Alternatively, compound **2** may be prepared from compound **1** as described below.

### (S)-1-tert-Butyl-2-methyl 4-methylenepyrrolidine-1,2-dicarboxylate

Potassium carbonate (19.92 g, 14 mmol, 3 eq.) was added to a stirred solution of compound **1** (10.92 g, 48 mmol, 1 eq.) in DMF (270 mL). The resulting white suspension was stirred at room temperature for 30 mins, at which point iodomethane (21.48 g/ 9.5 mL, 151 mmol, 3.15 eq.) was added. The reaction mixture was allowed to stir at room temperature for 3 days. DMF was removed by rotary evaporation under reduced pressure to afford a yellow residue which was partitioned between ethylacetate and water. The organic layer was separated and the aqueous phase was extracted with ethylacetate. The combined organic layers were washed with water, brine and dried over magnesium sulphate. The ethylacetate was removed by rotary evaporation under reduced pressure to give the crude product as a yellow oil. The crude product was purified by flash chromatography [85% n-hexane/15% ethylacetatel to afford the product as a colourless oil (see also F Manfré et al., J. Org. Chem. 1992, 57, 2060-2065).

### (S)-2-(Methoxycarbony)-4-methylenepyrrolidiniumchloride

A solution of hydrochloric acid in dioxane (4M, 63 mL, 254.4 mmol, 4.5 eq.) was added to (**S**)-1-*tert*-butyl 2-methyl 4-methylenepyrrolidine-1,2-dicarboxylate (13.67 g, 56.6 mmol, 1 eq.) at room temperature. Effervescence was observed indicating liberation of CO₂ and removal of the Boc group. The product precipitated as a white solid and additional dioxane was added to facilitate stirring, and the reaction mixture was allowed to stir for an hour and then diluted with ether. The precipitated product was collected by vacuum filtration and washed with additional ether. Air drying afforded the desired product **2** as a white powder (9.42 g, 94%) (see also P Herdwijn et al., Canadian Journal of Chemistry. 1982, 60, 2903-7).

### Compound 3

Compound **3** may be prepared as described in WO 2006/111759 and Gregson et al.

### Compound 4

Compound **4** may be prepared from compound **3** and compound **2.**

A catalytic amount of anhydrous DMF (0.5 mL) was added to a stirred suspension of oxalyl chloride (9.1 g, 6.25 mL, 71.7 mmol, 3 eq.) and compound **3** (11.82 g, 23.9 mmol, **1** eq.) in anhydrous DCM (180 mL) at room temperature. Vigorous effervescence was observed after the addition of DMF and the reaction mixture was allowed to stir for 18 h in a round bottom flask fitted with a calcium chloride drying tube. The resulting clear solution was evaporated under reduced pressure and the solid triturated with ether. The solid product was collected by vacuum filtration, washed with additional ether and dried *in vacuo* at 40°C for 1.5 hours. This solid was then added portion wise to a suspension of the compound **2** (9.35 g, 52.6 mmol, 2.2 eq.) in TEA (12.08 g, 119.6 mmol, 5 eq.) and dry DCM (110 mL), maintaining the temperature between -40 and -50°C with the aid of a dry ice/acetonitrile bath. The reaction mixture was allowed to stir at -40°C for 1 hour and then allowed to warm to room temperature at which point LCMS indicated the complete consumption of the starting material. The reaction mixture was diluted with additional DCM and washed sequentially with aqueous hydrochloric acid (1M, 2 x 200 mL), saturated aqueous sodium bicarbonate (2 x 250 mL), water (250 mL), brine (250 mL), dried over magnesium sulphate. DCM was removed by rotary evaporation under reduced pressure to afford the product as a yellow foam (13.94 g, 79 %). Analytical Data: RT 3.95 min; MS (ES⁺) *m*/*z* (relative intensity) 741 ([*M* + 1]^{+.}, 100).

### Compound 5

Compound **5** may be prepared from compound **4** in three steps via the bis-alcohol and the bis-acetate.

### bis-Alcohol

Solid lithium borohydride (0.093 g, 4.3 mmol, 3 eq.) was added in one portion to a solution of ester **4** (1.05 g, 142 mmol, 1 eq.) in dry THF (10 mL) under a nitrogen atmosphere at 0°C (ice bath). The reaction mixture was allowed to stir at 0°C for 30 mins and then allowed to warm to room temperature at which point precipitation of an orange gum was observed. The reaction mixture was allowed to stir at room temperature for a futher 2 hours and then cooled in an ice bath and treated with water (20 mL) to give a yellow suspension. Hydrochloric acid (1M) was carefully added (vigorous effervescence!) until effervescence ceased. The reaction mixture was extracted with ethylacetate (4 x 50 mL) and the combined organic layers were washed with water (100 mL), brine (100 mL) and dried over magnesium sulphate. Ethylacetate was removed by rotary evaporation under reduced pressure to yield the bis-alcohol product as a yellow foam (0.96 g, 99 %). The reaction was repeated on a 12.4 g scale to yield 11.06 g of product (96%). Analytical Data: RT 3.37 min; MS (ES⁺) *m*/*z* (relative intensity) 685 ([*M* + 1]^{+.}, 100).

### bis-Acetate

A solution of acetyl chloride (3.4 g/3.1 mL, 43.5 mmol, 2.6 eq.) in dry DCM (100 mL) was added dropwise to a stirred solution of the *bis*-alcohol (11.46 g, 16.73 mmol, 1 eq.) and triethylamine (5.07 g, 6.98 mL, 50.2 mmol, 3 eq.) in dry DCM (200 mL) at 0°C under a nitrogen atmosphere. The reaction mixture was allowed to warm to room temperature and stirring was continued for one hour. TLC and LCMS revealed that the reaction was complete. The reaction mixture was washed with brine (200 mL) and dried over magnesium sulphate. Removal of DCM by rotary evaporation under reduced pressure gave the crude product. Flash chromatography [gradient elution 20% n-hexane/80% ethylacetate to 10% n-hexane/90% ethylacetate] furnished pure bis-acetate as a yellow foam (10.8 g, 84%). Analytical Data: RT 3.35 min; MS (ES⁺) *m*/*z* (relative intensity) 769 ([*M* + 1]^{+.}, 100).

### Compound 5

Zinc powder (14.2 g, 2.17 mmol, 30 eq.) was added to a solution of the bis-acetate (5.56 g, 7.24 mmol, 1 eq.) in ethanol (250 mL) and acetic acid (65 mL). The stirred reaction mixture was heated at reflux, with the yellow solution becoming colourless (zinc aggregation was also observed making it difficult to stir the reaction). The reaction was allowed to continue for one hour at which point LCMS indicated that the reaction was complete. The reaction mixture was allowed to cool, filtered through celite and the filter pad washed with DCM. The filtrate was washed with water (3 x 500 mL), saturated aqueous sodium bicarbonate (2 x 250 mL), brine (500 mL) and dried over magnesium sulphate. Rotary evaporation under reduced pressure yielded the product **5** as an off-white foam (4.71 g, 92%). Analytical Data: RT 3.33 min; MS (ES⁺) *m*/*z* (relative intensity) 709 ([*M* + 1]^{+.}, 100).

### Compound 6

Compound **6** may be prepared from compound **5** in three steps.

### Mono-Alloc Product

A solution of allyl chloroformate (0.634 g/0.56 mL, 5.6 mmol, 0.9 eq.) in dry DCM (150 mL) was added drop wise to a solution of compound **5** (4.145 g, 5.8 mmol, 1 eq.) and pyridine (0.106 g/0.11 mL, 11.1 mmol, 1.9 eq.) in dry DCM (500 mL) at -78°C (dry ice/acetone bath). The reaction mixture was stirred at -78 °C for 1 hour and then allowed to reach room temperature. The reaction mixture was washed with saturated aqueous copper sulphate solution (2 x 300 mL), water (400 mL), brine (400 mL) and dried over magnesium sulphate. Rotary evaporation under reduced pressure afforded the crude product as a dark foam. Purification by flash chromatography [40% n-hexane160% ethyl acetate to 5% methanol/95% ethyl acetate] gave the *bis*-alloc product (0.84 g), the desired mono-alloc product (1.94 g, 44%) and recovered *bis*-aniline (0.81 g).

Analytical Data: RT 3.32 min; MS (ES⁺) *m*/*z* (relative intensity) 793 ([*M* + 1]^{+.}, 100); MS (ES⁻) *m*/*z* (relative intensity) 791 ([M - -1])^{-.}, 100).

### Isocyanate

Triethylamine (0.018 g/25 µL, 0.18 mmol, 1.35 eq.) was added to a stirred solution of the mono-alloc product (0.106 g, 0.134 mmol, 1 eq.) and triphosgene (0.015 g, 4.8 x 10⁻² mmol, 0.36 eq.) in dry toluene (5 mL) under a nitrogen atmosphere at -10°C. After 1 hour IR spectroscopy revealed an isocyanate stretch at 2268 cm⁻¹ and the reaction mixture was allowed to reach room temperature.

### Compound 6

A solution of alcohol **6a** (0.106g, 0.15 mmol, 1.1 eq.) and triethylamine (0.018 g, 25 µL, 0.18 mmol, 1.35 eq.) in dry THF (5 mL) was added drop wise to the freshly prepared isocyanate. The reaction mixture was heated at reflux for 4 hours at which time TLC revealed the formation of a new product. The reaction mixture was evaporated to dryness and partitioned between DCM and water. The aqueous layer was separated and the organic phase was washed with brine (100 mL) and dried over magnesium sulphate. Rotary evaporation under reduced pressure afforded the crude product as a yellow oil which was purified by flash chromatography [gradient elution 40% n-hexane/60% ethylacetate to 20% n-hexane/80% ethylacetate, with 5% increments in ethylacetate] to afford the desired product as a white foam (0.092 g, 45% yield).

Analytical Data: RT 4.05 min; MS (ES⁺) *m*/*z* (relative intensity) 1540 ([*M* + 2]^{+.}, 30); 1557 ([*M* + 18]^{+.} , 50); MS (ES⁻) *m*/*z* (relative intensity) 1585 ([M - +2Na])^{-.}, 50).

### Compound 7

Compound **7** may be prepared from compound **6** in two steps.

### bis Deacetylated Product

A solution of superhydride™ in THF (1M, 0.35 mL, 0.35 mmol, 4 eq.) was added drop wise via syringe to a stirred solution of acetate **6** (0.135 g, 8.8 x 10⁻² mmol, 1 eq.) in dry THF (7 mL) at -78°C (dry ice/acetone). The reaction mixture was allowed to stir at -78°C for one hour at which time LCMS revealed the absence of starting material and the formation of two new compounds corresponding to the mono and *bis* deacetylated product. A further aliquot of superhydride™ (1M, 0.35 mL, 0.35 mmol, 4 eq.) was added to the reaction mixture and stirring continued for a further hour. LCMS at this point revealed complete conversion to the *bis* deacetylated product. Citric acid (1M, 1 mL) was added to the reaction mixture (vigorous effervescence!) which was then allowed to reach room temperature at which point a further aliquot of citric acid (1M, 1 mL) was added. Solvent was removed by rotary evaporation under reduced pressure and the resulting residue was partitioned between ethylacetate (25 mL) and water (25 mL). The aqueous phase was separated and the ethylacetate layer washed with water (25 mL), brine (25 mL) and dried over magnesium sulphate. Removal of the solvent by rotary evaporation under reduced pressure afforded the crude product as a yellow oil which was subjected to flash chromatography [gradient elution ethylacetate→1% methanol/99% ethylacetate to 2% methanol 98% ethylacetate] to afford the pure product as a colourless glass (0.056 g, 44%). Analytical Data: RT 3.78 min; MS (ES⁺) *m*/*z* (relative intensity) 1456 ([*M* + 1]^{+.}, 75).

### Compound 7

Dess-Martin periodinane (0.026g, 6.1 x 10⁻⁵ mol, 2.1 eq) was added in one portion to a solution of the *bis* deacetylated product (0.042 g, 2.9 x 10⁻⁵ mol, 1 eq) in dry DCM (5 mL) under a nitrogen atmosphere. The solution was stirred at room temperature for 4h at which time LCMS indicated that reaction was complete. The cloudy suspension was filtered washing with DCM (20 mL). The filtrate was washed with saturated aqueous sodium bicarbonate solution (25 mL), water (25 mL), brine (25 mL) and dried over magnesium sulphate. The solvent was removed by rotary evaporation under reduced pressure to give product **7** as an off-white foam (0.035 g, 84%). Analytical Data: RT 3.70 min; MS (ES⁺) *m*/*z* (relative intensity) 1451 ([*M* + 1]^{+.}, 30).

### Compound 14

Compound **14a or 14b** may be prepared from compound 7 via compound **8.** The Alloc protecting group in compound **7** may be removed under appropriate conditions, for example tetrakis(triphenylphosphine)palladium(0) in the presence of pyrollidine. Under these conditions the Fmoc protecting group is also removed. Alternatively, the Fmoc group may be removed in a separate step, either before or after the removal of the Alloc group, using piperidine in DMF. The product of the deprotection step is an imine-carbinolamine product having imine functionality at the N10-C11 position of one PBD monomer, and a carbinolamine functionality at N10-C11 position of the other monomer, wherein the carbinolamine has a linker -C(=O)-L¹-NH₂ at the N10 position.

The imine-carbinolamine may be reacted with MC-OSu in the presence of base to generate compound **8.** See, for example, Dubowchik et al., Bioconjugate Chem. 2002, 13, 855-869.

The amino acid side chain protecting group may then be removed from compound **8,** for example under acidic conditions. The resulting product may then be conjugated to an appropriate antibody bearing thiol functionality to give compound **9.**

In one example, an antibody may be treated with DTT to reduce interchain disulfide bonds. The resulting antibody, bearing free thiol groups, may then be reacted with maleimide-containing compound derived from compound **8** to generate compound **9.** Compound **9** may be purified, for example by diafiltration. See, for example, Dubowchik et al., Bioconjugate Chem. 2002, 13, 855-869.

### Compound 18

Dicyclohexylcarbodiimide (2.46 g, 11.92 mmol, 1.05 eq.) was added to a suspension of N-hydroxysuccinimide (1.44 g, 12.5 mmol, 1.1 eq.) and N-alloc phenylalanine (17) (2.83 g, 11.35 mmol, 1 eq.) in dry DCM (120 mL) at 0°C. The mixture was stirred at 0°C for 30min then at room temperature for 16h. The reaction mixture was filtered and the filtrate evaporated under reduced pressure. The residue was re-dissolved in DCM (50 mL), allowed to stand for 1h and filtered to remove precipitated dicyclohexylurea. Evaporation under reduced pressure gave the product as a white solid (3.91 g, 99%). Analytical Data: RT 2.93 min; MS (ES⁺) *m*/*z* (relative intensity) 369 ([*M* + Na]^{+.}, 50).

### Compound 20

A solution of succinimide **(18)** (3.91 g, 11.29 mmol, 1 eq.) in THF (50 mL) was added to a solution of H-Lys(Boc)-OH **(19)** (2.92 g, 11.85 mmoL, 1.05 eq.) and NaHCO₃ (1.04 g, 12.42 mmoL, 1.1 eq.) in THF (50 mL) and H₂O (100 mL). The mixture was stirred at room temperature for 72 h and the THF was evaporated under reduced pressure. The pH was adjusted to pH 3-4 with citric acid to precipitate a white gum. This was extracted with ethylacetate (2 x 250 mL) and the combined extracts were washed with H₂O (200 mL), brine (200 mL), dried (MgSO₄) and evaporated under reduced pressure to give the product as a white foam (4.89 g, 91%). Analytical Data: RT 3.03 min; MS (ES⁺) *m*/*z* (relative intensity) 478 ([M + 1])^{+.}, 80).

### Compound 7b

EEDQ (2.66 g, 10.75 mmol, 1.05 eq.) was added to a solution of *p*-aminobenzyl alcohol **(21)** (1.32 g, 10.75 mmoL, 1.05 eq.) and Alloc-Phe-Lys(Boc)-OH (4.89 g, 10.24 mmol, 1.0 eq.) in dry THF (75 mL). The mixture was stirred at room temperature for 18h. The solvent was evaporated under reduced pressure to give a pale brown solid. The solid was triturated with diethyl ether and filtered washing with an excess of diethyl ether. This afforded the product as a white solid (4.54 g, 76%). Analytical Data: RT 3.08 min; MS (ES⁺) *m*/*z* (relative intensity) 583.8 ([*M* + 1]^{+.}, 100).

The synthesis of 7b is described below in Scheme 12.

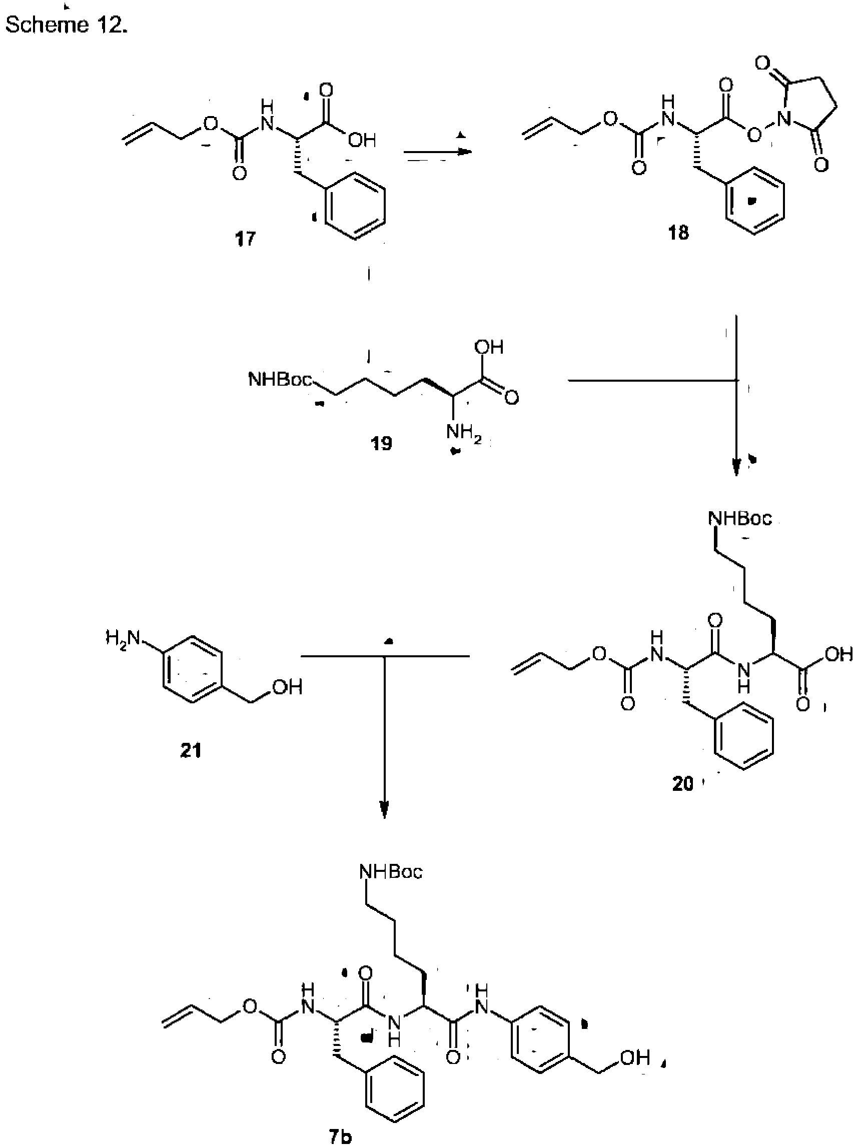

### Compound 9b

Triethylamine (0.18 g, 0.25 mL, 1.79 mmol, 2.3 eq.) was added to a stirred solution of the mono-alloc protected bis-aniline **(6)** (0.608 g, 0.77 mmol, 1.06 eq.) and triphosgene (0.088 g, 0.3 mmol, 0.39 eq.) in dry THF (5 mL) under a nitrogen atmosphere at -10°C. The reaction mixture was allowed to reach room temperature, a sample was treated with methanol, and analysed by LCMS as the methyl carbamate.

A solution of the benzyl-alcohol **(7b)** (0.422 g, 0.72 mmol, 1.0 eq.) and triethylamine (0.18 g, 0.25 mL, 1.79 mmol, 2.3 eq.) in dry THF (20 mL) was added drop-wise to the freshly prepared isocyanate. The reaction mixture was heated at 60-65°C for 4 hours then allowed to stir for 18 hours at room temperature at which time LCMS revealed the formation of a new product. The reaction mixture was evaporated to dryness to afford the crude product as a yellow oil which was purified by flash chromatography [gradient elution 50% *n*-hexane/50% ethylacetate to 10% *n*-hexane/90% ethylacetate in 10% increments] to give the desired product as a white foam (0.385 g, 38%). Analytical Data: RT 3.78 min; MS (ES⁺) *m*/*z* (relative intensity) 1402.8 ([*M* + H]⁺, 15).

### Compound 10b

A solution of K₂CO₃ (0.158 g, 1.15 mmoL, 5 eq.) in H₂O (1 mL) was added to a solution of the acetate **(9b)** (0.32 g, 0.23 mmol, 1 eq.) in methanol (6 mL). The reaction mixture was stirred at room temperature for 30 min. The methanol was evaporated under reduced pressure, the residue was diluted with H₂O (50 mL) and extracted with ethylacetate (3 x 75 mL). The combined ethylacetate extracts were washed with H₂O (100 mL), brine (100 mL), dried (MgSO₄) and evaporated under reduced pressure to give the product as a white foam (0.292 g, 97%). Analytical Data: RT 3.52 min; MS (ES⁺) *m*/*z* (relative intensity) 1318.6 ([*M* + 1]^{+.}, 15).

### Compound 11b

Dess-Martin periodinane (0.197 g, 0.465 mmoL, 2.1 eq.) was added in one portion to a solution of the bis deacetylated product **(10b)** (0.292 g, 0.22 mmoL, 1 eq.) in dry DCM (15 mL) under a nitrogen atmosphere. The solution was stirred at room temperature for 3.5 h at which time LCMS indicated that reaction was complete. The reaction mixture was diluted with DCM (50 mL) and washed with saturated aqueous sodium bicarbonate solution (3 x 100 mL), water (100 mL), brine (100 mL) and dried over magnesium sulphate. The solvent was removed by rotary evaporation under reduced pressure to give the crude product. Purification by flash column chromatography [gradient elution 80% ethylacetate/20% *n*-hexane to 100% ethylacetate in 5% increments] gave the product **11b** as a yellow foam (0.235 g, 81%). Analytical Data: RT 3.42 min; MS (ES⁺) *m*/*z* (relative intensity) 1314.8 ([*M* + 1]^{+.}, 8).

### Compound 12a

Pd(PPh₃)₄ (4 mg, 3.5 x 10⁻⁶ moL 0.02 eq.) was added to a solution of the *bis*-alloc compound (**11b**) (0.230 g, 0.175 mmol, 1 eq.) and pyrrolidine (31 mg, 36 µL, 0.44 mmol, 2.5 eq.) in dry DCM (10 mL) under a nitrogen atmosphere. The solution was stirred at room temperature for 3 hours at which time LCMS indicated that unreacted (**11b**) remained. Further equivalents of pyrrolidine (31 mg, 36 µL, 0.44 mmoL, 2.5 eq.) and Pd(PPh₃)₄ (4 mg, 3.5 x 10⁻⁶ mol, 0.02 eq) were added and the reaction was stirred at room temperature for a further 18h. LCMS indicated that the reaction was complete. The reaction mixture was diluted with DCM (40 mL) and washed with saturated aqueous ammonium chloride solution (100 mL), water (100 mL), brine (100 mL), dried (MgSO₄) and evaporated under reduced pressure to give a yellow foam. This was triturated with diethyl ether to give the product (0.187 g, 95%) which was used without further purification. Analytical Data: RT 2.80 min; MS (ES⁺) *m*/*z* (relative intensity) 1128.5 ([*M* + 1]^{+.}, 20).

### Compound 23

A solution of AllocVal-OSu **(22)** ((RT 2.67 min; MS (ES⁺) *m*/*z* (relative intensity) 321.4 ([*M* + Na]^{+.}, 57) prepared according to the method for the preparation of compound (**16**)) (11.67 g, 39.0 mmoL, 1 eq.) in THF (50 mL) was added to a solution of H-Ala-OH (3.66 g, 41.08 mmoL, 1.05 eq.) and NaHCO₃ (3.61 g, 43.03 mmol, 1.1 eq.) in THF (100 mL) and H₂O (100 mL). The mixture was stirred at room temperature for 72 hours and the THF was evaporated under reduced pressure. The pH was adjusted to pH 3-4 with citric acid to precipitate a white gum. This was extracted with ethylacetate (6 x 150 mL) and the combined extracts were washed with H₂O (200 mL), brine (200 mL), dried (MgSO₄) and evaporated under reduced pressure to give a white solid. Trituration with diethyl ether (excess) afforded the pure product **23** as a white powder (7.93 g, 74%).

Analytical Data: RT 2.17 min; MS (ES⁺) *m*/*z* (relative intensity) 295 *([M* + Na]^{+.}, 63), 273 ([*M* + 1]^{+.}, 60).

### Compound 8

EEDQ (4.79 g, 19.3 mmol, 1.05 eq.) was added to a solution of *p*-aminobenzyl alcohol **(21)** (2.38 g, 19.3 mmoL, 1.05 eq.) and Alloc-Val-Ala-OH (5.02 g, 18.4 mmol, 1.0eq) in dry THF (100 mL). The mixture was stirred at room temperature for 72 hours. The solvent was evaporated under reduced pressure to give a pale brown solid. The solid was triturated with diethyl ether and filtered, washing with an excess of diethyl ether. This afforded the product as a white solid (6.2 g, 89%). Analytical Data: RT 2.50 min; MS (ES⁺) *m*/*z* (relative intensity) 400.6 ([*M* + Na]^{+.}, 50), 378.6 ([M + 1]^{+.}, 60).

The synthesis of 8 is shown below in Scheme 13.

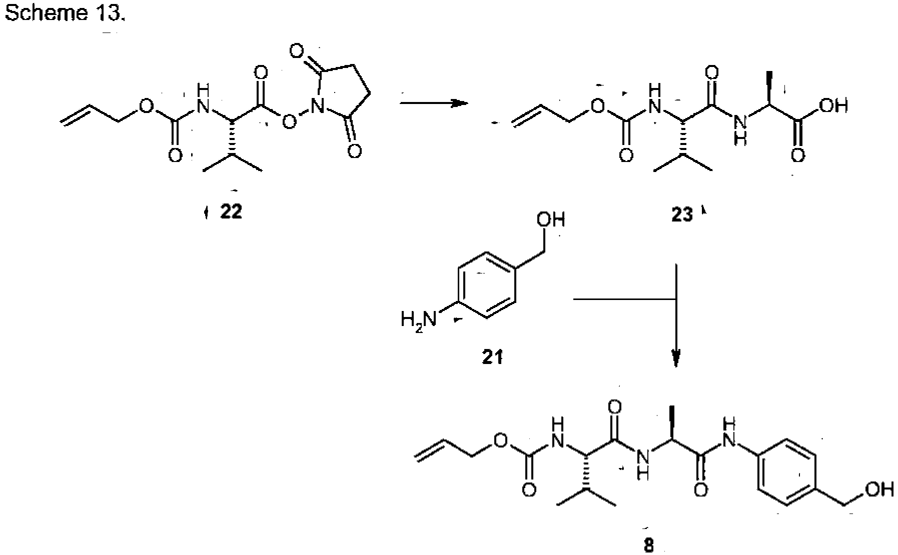

### Compound 9c

Triethylamine (0.16 g, 0.22 mL 1.59 mmol, 2.2 eq.) was added to a stirred solution of the mono-alloc protected bis-aniline **(6)** (0.572 g, 0.72 mmol, 1 eq.) and triphosgene (0.077 g, 0.26 mmol, 0.36 eq.) in dry THF (20 mL) under a nitrogen atmosphere at room temperature. The reaction mixture was heated to 40°C, a sample was treated with methanol and analysed by LCMS as the methyl carbamate.

A solution of the benzyl-alcohol **(8)** (0.4 g, 1.06 mmol, 1.5 eq.) and triethylamine (0.109 g, 0.15 mL, 1.08 mmol, 1.5 eq.) in dry THF (20 mL) was added drop-wise to the freshly prepared isocyanate. The reaction mixture was monitored by LCMS at 30 min intervals. After 3 h LCMS showed conversion to product, the presence of methyl carbamate and mono-alloc protected *bis*-aniline **(6).** A further portion of triphosgene (0.038 g, 0.128 mmol, 0.18 eq) was added and the reaction continued at 40°C for a further 18 h. The reaction mixture was evaporated to dryness to afford the crude product as a yellow oil which was purified by flash chromatography [gradient elution 100% chloroform to 97% chloroform/Methanol 3% in 0.5% increments] to give the desired product as a white foam (0.59 g, 69%). Analytical Data: RT 3.58 min; MS (ES⁺) *m*/*z* (relative intensity) 1197 ([M + 1]^{+.}, 60).

### Compound 10c

A solution of K₂CO₃ (0.195 g, 1.41 mmoL, 5 eq.) in H₂O (1.4 mL) was added to a solution of the acetate **(9c)** (0.338 g, 0.282 mmol, 1 eq.) in methanol (8.5 mL). The reaction mixture was stirred at room temperature for 30 min. The methanol was evaporated under reduced pressure, the residue was diluted with H₂O (50 mL) and extracted with ethylacetate (3 x 75 mL). The combined ethylacetate extracts were washed with H₂O (100 mL), brine (100 mL), dried (MgSO₄) and evaporated under reduced pressure to give the product as a white foam (0.298 g, 95%). Analytical Data: RT 3.28 min; MS (ES⁺) *m*/*z* (relative intensity) 1113 ([*M*+ 1]^{+.}, 40).

### Compound 11c

Dess-Martin periodinane (0.312 g, 0.74 mmol, 2.1 eq.) was added in one portion to a solution of the *bis* deacetylated product **(10c)** (0.39 g, 0.35 mmol 1 eq.) in dry DCM (20 mL) under a nitrogen atmosphere. The solution was stirred at room temperature for 3.5h at which time LCMS indicated that reaction was complete. The reaction mixture was diluted with DCM (50 mL) and washed with saturated aqueous sodium bicarbonate solution (3 x 100 mL), water (100 mL), brine (100 mL) and dried (MgSO₄). The solvent was removed by rotary evaporation under reduced pressure to give the crude product. Purification by flash column chromatography [gradient elution 100% chloroform to 97% chloroform/3% methanol in 1% increments] gave the product as a white solid (0.201 g, 52%). Analytical Data: RT 3.15 min; MS (ES⁺) *m*/*z* (relative intensity) 1109 ([M + 1]^{+.}, 30), MS (ES⁻) *m*/*z* (relative intensity) 1107 ([*M* - 1]^{-.}, 100).

### Compound 12c

Pd(PPh₃)₄ (8 mg, 7 x 10⁻⁶ moL 0.04 eq.) was added to a solution of the *bis*-alloc compound **(11c)** (0.190 g, 0.17 mmoL, 1.0 eq.) and pyrrolidine (61 mg, 71 µL, 0.86 mmoL, 5.0 eq.) in dry DCM (5 mL) under a nitrogen atmosphere. The solution was stirred at room temperature for 3h to give a cloudy suspension. The solvent was evaporated under reduced pressure and the residue was triturated with ethylacetate to give an off white solid which was collected by filtration to give the product (0.13 g, 82%) which was used without further purification. Analytical Data: RT 2.55 min; MS (ES⁺) *m*/*z* (relative intensity) 922 ([M + 1]^{+.}, 52).

### Compound 13a

EEDQ (18.4 mg, 7.45 x 10⁻⁵ mol, 2.2 eq.) was added to a solution of amine dipeptide (**12a**) (40 mg 3.5 x 10⁻⁵ mol, 1.0 eq.) and maleimide caproic acid (8.2 mg, 3.9 x 10⁻⁵ mol 1.1 eq.) in DCM (2 mL) and methanol (1 mL). The solution was stirred at 40°C for 72 h. The solvent was evaporated under reduced pressure. The residue was dissolved in DCM (50 mL) and washed with saturated aqueous NaHCO₃ solution (2 x 50 mL), H₂O (50 mL), brine (50 mL), dried (MgSO₄) and evaporated under reduced pressure to give a white foam. This was triturated with diethyl ether and filtered washing with an excess of diethyl ether to afford the product as a white solid (36 mg, 78%). Analytical Data: RT 3.27 min; MS (ES⁺) *m*/*z* (relative intensity) 1320 ([*M*+ 1]^{+.}, 75).

### Compound 13b

Cold trifluoroacetic acid (13 mL) was added to maleimide derivative **(1 3a)** (65 mg, 4.9 x 10⁻⁵ mol) at 0°C. The solution was stirred at this temperature for 30 min and the trifluoroacetic acid was evaporated under reduced pressure. The residue was redissolved in anhydrous DCM (5 mL). The solvent was evaporated and the residue triturated with diethyl ether and the resultant yellow solid collected by filtration and dried under vacuum (64 mg, 97%). Analytical Data: RT 2.83 min; MS (ES⁺) *m*/*z* (relative intensity) 1222 ([*M* + 2]^{+.}, 5).

Coupling of a maleimide-PEG-succinimide reagent with **12a** or **12b** provides the PBD drug-linkers **15.** Figure 1a shows the structures of PBD drug-linkers MP-PEG4-Phe-Lys-PAB-PBD **15ba,** MP-PEG8-Phe-Lys-PAB-PBD **15bb** and MP-PEGB-Val-Ala-PAB-PBD **15d,** where PEG is ethyleneoxy, and PAB is *para*-aminobenzyloxycarbonyl.

### Compound 15aa

The amine dipeptide **(1 2a)** (83 mg, 7.4 x 10⁻⁵ mol, 1 eq.) was dissolved in a mixture of dry 10% DMF/DCM (2 mL) and maleimide-4Peg-succinimide (353 µL of a 250 mmol solution in dry DCM) was added followed by N,N-diisopropylethylamine (8.2 mg, 11 µL, 8.1 x 10⁻⁵ mol, 1.1 eq.). The solution was stirred at room temperature for 72 h under a nitrogen atmosphere. The solvent was evaporated under reduced pressure. Purification by flash column chromatography [gradient elution 100% chloroform to 92% chloroform/8% methanol in 1% increments] afforded the product as a yellow foam (85 mg, 76%). Analytical Data: RT 3.13 min; MS (ES⁺) *m*/*z* (relative intensity) 1526 ([*M* + 1]^{+.}, 5).

### Compound 15ab

The amine dipeptide (**12a**) (70 mg, 76.2 x 10⁻⁵ mol, 1 eq.) was d issolved in a mixture of dry 10% DMF/DCM (2 mL) and maleimide-8Peg-succinimide (263 µL of a 250 mmol solution in dry DCM) was added followed by NN-diisopropylethylamine (6.9 mg, 9.5 µL, 6.6 x 10⁻⁵ mol, 1.06 eq.). The solution was stirred at room temperature for 72 h under a nitrogen atmosphere. The solvent was evaporated under reduced pressure. Purification by flash column chromatography [gradient elution 100% chloroform to 92% chloroform/8% methanol in 1% increments] afforded the product as a brown foam (44 mg, 41.5%). Analytical Data: RT 3.20 min; MS (ES⁺) *m*/*z* (relative intensity) 1703 ([*M* + 2]^{+.}, 5).

*Compound **15ba*** (*MP*-*PEG4*-*Phe-Lys-PAB-PBD;* (*11S,11aS)-4-((2S,5S)-2-(4-aminobutyl)-5-benzyl-25-(2,5-dioxo-2,5-dihydro-1IH-pyrrol-1-yl)-4,1,23-trioxo-10,13,16,19-tetraoxa-3,6,22-triazapentacosanamido)benzyl 11-hydroxy-7-methoxy-8-(5-((S)-7-methoxy-2-methylene-5-oxo-2,3,5,11a-tetrahydro-pyrrolo [2,1-c][1,4]benzodiazepin-8-yloxy)pentyloxy)-2-methylene-5-oxo-2,3,11,11a-tetrahydro-pyrrolo[2,1-c][1,4]benzodiazepine-10(5H)-carboxylate)* Cold trifluoroacetic acid (17 mL) was added to maleimide derivative (**15aa**) (85 mg, 5.6 x 10⁻⁵ mol) at 0°C. The solution was stirred at this temperature for 30 min and the trifluoroacetic acid was evaporated under reduced pressure. The residue was redissolved in anhydrous DCM (5 mL). The solvent was evaporated and the residue triturated with diethyl ether and the resultant yellow solid collected by filtration and dried under vacuum (70 mg, 81 %). Analytical Data: RT 2.78 min; MS (ES⁺) *m*/*z* (relative intensity) 1444 ([*M* + 2]^{+.}, 1).

*Compound **15bb** (MP-PEG8-Phe-Lys-PAB-PBD; (11S,11aS)-4-((2S,5S)-2-(5-aminobutyl)-5-benzyl-37-(2,5-dioxo-2,5-dihydro-1H-pyrrol-1-yl)-4,7,35-trioxo-10,13,16,19,22,25,28,31-octaoxa-3,6,34-triazaheptatriacontanamido)benzyl 11-hydroxy-7-methoxy-8-(5-((S)-7-methoxy-2-methylene-5-oxo-2,*3,5,*11a-tetrahydro-pyrrolo[2,1-c][1,4]benzodiazepin-8-yloxy)pentoxy)-2-methylene-5-oxo-2,3,11,11a-tetrahydro-pyrrolo[2,1-c][1,4]benzodiazepine-10(5H)-carboxylate)*
Cold trifluoroacetic acid (9 mL) was added to maleimide derivative (**15ab**) (44 mg, 2.6 x 10⁻⁵ mol) at 0°C. The solution was stirred at this temperature for 30 min and the trifluoroacetic acid was evaporated under reduced pressure. The residue was redissolved in anhydrous DCM (5 mL). The solvent was evaporated and the residue triturated with diethyl ether and the resultant yellow solid collected by filtration and dried under vacuum (40 mg, 91%).

Analytical Data: RT 2.80 min; MS (ES⁺) *m*/*z* (relative intensity) 1603 ([M + 2]^{+.}, 1).

*Compound **15d** (MP-PEG8-Val-Ala-PAB-PBD; (11S,11aS)-4-((2S,5S)-37-(2,5-dioxo-2,5-dihydro-1H-pyrrol-1-yl)-5-isopropyl-2-methyl-4,7,35-trioxo-10,13,16,19,22,25,28,31-octaoxa-3,6,34-triazaheptatriacontanamido)benzyl 11-hydroxy-7-methoxy-8-(5-((S)-7-methoxy-2-methylene-5-oxo-2,3,5,11a-tetrahydro-pyrrolo[2,1-c][1,4]benzodiazepin-8-yloxy)pentyloxy)-2-methylene-5-oxo-2,3,11,11a-tetrahydro-pyrrolo[2,1-c][1,4]benzodiazepine-10(5H)-carboxylate)*
EEDQ (12 mg, 4.8 x 10⁻⁵ mol, 1,1 eq.) was added to a suspension of amine dipeptide (**12c**) (40.3 mg 4.4 x 10⁻⁵ mol, 1.0 eq.) and maleimide-8 Peg-acid (28 mg, 4.8 x 10⁻⁵ mol, 1.1 eq.) in dry DCM (5 mL). Dry dimethylacetamide (0.05 mL) was added to give a pale yellow solution which was stirred at room temperature for 18h. The solvent was evaporated under reduced pressure and the residue was triturated with diethyl ether. The resultant solid product was purified by flash column chromatography. Analytical Data: RT 2.90 min; MS (ES⁺) *m*/*z* (relative intensity) 1496 ([M + H]^{+.}, 40).

### Compound 15e

*N,N*-Diisopropyldiethylamine (10.8 µL, 8 mg, 7.6 x 10⁻⁵ mol, 2.2 eq) was added to a solution of amine dipeptide **(12c)** (32 mg, 3.5 x 10⁻⁵ mol, 1.0 eq) and maleimide-dPeg®24-NHS ester ' (58 mg, 4.16 x 10⁻⁵ mol, 1.2 eq) in dry DCM (5 mL). The solution was stirred at room temperature for 96 h. The reaction mixture was diluted with DCM (15 mL) and washed with saturated NaHCO₃ (25 mL), brine (25 mL), dried (MgSO₄) and evaporated under reduced pressure to give a pale yellow glass. Purification by flash column chromatography [gradient elution 100% chloroform to 91% chloroform/9% methanol in 1% increments] gave the product as a viscous yellow gum (17 mg, 22%).

### Compound 16d

Peptide biotin-A20FMDV-Cys **(59)** that is highly selective for the integrin αᵥβ₆, which is significantly up-regulated by many cancers, was selected for conjugation of the PBD-linker derivatives.

A solution of the peptide **(59)** (11.3 mg, 4.35 µmol, 0.98 eq.) in 1/1 acetonitrile/water (2 mL) was added to a solution of (**15d**) (6.91 mg, 4.62 µmol, 1.0 eq.) in 1/1 acetonitrile /water (3 mL). The solution was stirred at room temperature for 96 h. The acetonitrile was evaporated under reduced pressure and the water was removed by lyophilisation to give a white foam. Purification by semi-preparative HPLC followed by lyophilisation gave the product as a white foam (3.8 mg, 21%). Analytical Data: MS (MaldiTOF) *m*/*z* (relative intensity) 3991.1 ([M + H]^{+.}, 100).

### Compound 24a

Compound **24a** is disclosed as Compound 3 of WO 2004/043963.

### Compound 24b

Solid TCCA (18 g, 77.4 mmol, 1.1 eq.) was added portionwise to a solution of TEMPO (730 mg, 4.67 mmol, 0.07 eq.) and alcohol **24a** (25g, 70.5 mmol, 1eq.), in DCM (500 mL) at 0°C. A slight exotherm was observed. The reaction was deemed complete by TLC (ethyl acetate) and LC /MS (2.38 min (ES+) *m*/*z* (relative intensity) 353.34 ([M + H]^{+.}, 100)) after 30 minutes. The suspension was filtered through celite and washed with DCM. The filtrate was washed with aqueous sodium bisulfite, followed by saturated NaHCO₃ (caution, vigorous effervescence), brine (100 mL) and dried (MgSO₄). Filtration and evaporation of the solvent *in vacuo* afforded the crude product which was purified by flash column chromatography (elution: 20:80 v/v *n*-hexane/EtOAc) to afford the ketone **24b** as a white solid (20 g, 80%). Analytical Data: [α]²⁶_{D} = 15° (c = 0.2, CHCl₃); MS (ES⁺) *m*/*z* (relative intensity) 353.34 ([*M* + H]^{+.}, 100); ); IR (ATR, CHCl₃) 1748, 1642, 1518, 1425, 1335, 1222, 1176, 1063, 1032, 986, 857, 785, 756 cm⁻¹.

### Compound 25

Anhydrous 2,6-lutidine (0.395 mL, 365 mg, 3.40 mmol) was injected in one portion to a vigorously stirred solution of ketone **24b** (200 mg, 0.57 mmol) in dry DCM (10 mL) at -45°C (dry ice/acetonitrile cooling bath) under a nitrogen atmosphere. Anhydrous triflic anhydride, taken from a freshly opened ampoule (477 µL, 800 mg, 2.83 mmol), was injected rapidly dropwise, while maintaining the temperature at -40°C or below. The reaction mixture was allowed to stir at -45°C for 1 hour at which point TLC (50/50 v/v *n*-hexane/EtOAc) revealed the complete consumption of starting material. The cold reaction mixture was immediately diluted with DCM (20 mL) and, with vigorous shaking, washed with water (1 x 50 mL), 5%' citric acid solution (1 x 50 mL) saturated NaHCO₃ (50 mL), brine (30 mL) and dried (MgSO₄). Filtration and evaporation of the solvent *in vacuo* afforded the crude product which was purified by flash column chromatography (gradient elution: 60:40 v/v *n*-hexane/EtOAc to 50:50 v/v *n*-hexane/EtOAc) to afford the triflate **25** as a yellow foam (151 mg, 55%).

None of the corresponding 1,2 unsaturated compound was visible by NMR. Analytical Data: [α]²⁸_{D} = -55° (c = 0.2, CHCl₃); ¹H NMR (400 MHz, CDCl₃) δ 7.75 (s, 1 H), 6.92 (s, 1 H), 6.25 (t, 1 H, *J* = 1.84 Hz), 5.19 (dd, 1 H, *J* = 5.05, 11.93 Hz), 4.03 (s, 6H), 3.90 (s, 3H), 3.50 (ddd, 1H, *J* = 2.29, 11.96, 16.59 Hz), 3.02 (ddd, 1 H, *J* = 1.60, 5.05, 16.58 Hz); IR (ATR, CHCl₃) 1748, 1653, 1577, 1522, 1415, 1335, 1276, 1205, 1130, 1061, 1024, 933, 908, 820, 783, 757, 663, cm⁻¹; MS (ES⁺) *m*/*z* (relative intensity) 485.45 ([*M* + H]^{+.}, 100).

### Compound 26

Pd(PPh₃)₄ (860 mg, 744 µmol, 0.04 eq) was added to a stirred mixture of enol triflate **25** (9.029 g, 18.6 mmol, 1 eq), 4-methoxyphenylboronic acid (3.67 g, 24.1 mmol, 1.3 eq), Na₂CO₃ (5.13 g, 48.3 mmol, 2.6 eq), EtOH (45 mL), toluene (90 mL) and water (45 mL). The reaction mixture was allowed to stir under a nitrogen atmosphere overnight after which time the complete consumption of starting material was observed by TLC (60/40 EtOAc/hexane) and LC/MS (3.10 min (ES+) *m*/*z* (relative intensity) 443.38 ([*M* + H]^{+.}, 100)). The reaction mixture was diluted with EtOAc (400 mL) and washed with H₂O (2 x 300 mL), brine (200 mL), dried (MgSO₄), filtered and evaporated under reduced pressure to provide the crude product. Purification by flash chromatography (gradient elution: 60:40 v/v hexane/EtOAc to 40:60 v/v hexane/EtOAc) afforded C2-aryl compound **26** as an orange solid (7.0 g, 85%). Analytical Data: [α]²⁵_{D} = -122° (c = 0.2, CHCl₃); ¹H NMR (400 MHz, CDCl₃) δ 7.78 (s, 1H), 7.30 (d, 2H, *J =* 8.81 Hz), 6.95 (s, 1H), 6.87 (s, 1H), 6.83 (d, 2H, *J* = 8.88 Hz), 5.03 (dd, 1H, *J =* 11.71, 5.28 Hz), 3.95 (s, 3H), 3.93 (s, 3H), 3.76 (s, 3H), 3.73 (s, 3H), 3.48-3.43 (m, 1H), 2.99-2.93 (m, 1H), ¹³C NMR (100 MHz, CDCl₃) δ 170.7, 162.5, 158.4, 153.9, 149.1, 137.9, 126.3, 125.6, 125.3. 122.9. 122.3, 113.8, 110.03, 107.6, 59.7, 57.9, 56.5, 56.2, 55.1, 54.9, 52.2, 33.9, 20.7, 14..0; IR (ATR, CHCl₃) 1736, 1624, 1575, 1516, 1424, 1326, 1253, 1178, 1069, 1031, 863, 820, 803, 786, 757, 653, 617 cm⁻¹; MS (ES⁺) *m*/*z* (relative intensity) 443.38 ([*M* + H]^{+.}, 100.

### Compound 27

LiBH₄ (464 mg, 21.3 mmol, 1.5 eq) was added portionwise to a stirred solution of the ester **26** (6.28 g, 14.2 mmol, 1 eq) in anhydrous THF (100 mL) and EtOH (120 mL). An exotherm accompanied by vigorous foaming was observed and the temperature was maintained between 15°C and 25°C with the aid of a cooling bath (ice/water). The reaction mixture was allowed to stir for 1 hour after which time the complete conversion of starting material was observed by TLC (ethyl acetate). The reaction mixture was carefully diluted with ethyl acetate (500 mL) and excess borohydride destroyed with cold aqueous citric acid. The organic layer was washed with 1N aqueous HCL (100mL) followed by saturated aqueous NaHCO3 (100 mL), brine (100 mL), dried over MgSO₄, filtered and evaporated under reduced pressure at 35°C to provide the intermediate alcohol (4.50 g, 10.8 mmol, 76% intermediate yield) which was immediately redissolved in anhydrous DCM (200 mL). The solution was cooled to 0°C and TEA (2.26 mL, 0.162 mmol, 1.5 eq) was added, followed by a solution of acetyl chloride (1 mL, 14.0 mmol, 1.3 eq.) in anhydrous DCM (30 mL). The reaction mixture was allowed to warm up to room temperature and react for 1 hour. Complete reaction was observed by TLC (EtOAc). The solution was washed with 2N aqueous citric acid (50 mL), saturated aqueous NaHCO₃ (50 mL), brine (50 mL), dried over MgSO₄, filtered and evaporated under reduced pressure. The residue was purified by flash chromatography (gradient from 50/50 up to 60/40 EtOAc/hexane) to yield 2.65 g (41% over two steps) of pure product as an orange solid. Analytical Data: [α]²⁴_{D} = -130° (c = 0.28, CHCl₃); ¹H NMR (400 MHz, CDCl₃) δ 7.74 (s, 1 H), 7.12 (d, 2H, *J* = 8.84 Hz), 6.91 (br s, 1H), 6.80 (d, 2H, *J =* 8.88 Hz), 6.15 (s, 1H), 5.04-5.00 (m, 1H), 4.61-4.42 (m, 2H), 4.01 (s, 6H), 3.78 (s, 3H), 3.35-3.25 (m, 1H), 2.85-2.79 (m, 1H), 2.06 (s, 3H). ¹³C NMR (100 MHz, CDCl₃) δ 171.1, 159.1, 149.5, 126.1, 126.0. 114.1, 107.2, 56.8, 56.6, 55.3, 33.5, 20.9; IR (ATR, CHCl₃) 1731, 1643, 1623, 1577, 1517, 1421, 1333, 1278, 1248, 1222, 1183, 1076, 1059, 1032, 864, 821, 802, 787, 756, 644, 619 cm⁻¹; MS (ES⁺) *m*/*z* (relative intensity) 456.81 ([*M* + H]^{+.}, 100.

### Compound 28

Zinc dust (365 mg, 5.58 mmol, 15 eq.) was added to a solution of compound **27** (170 mg, 0.372 mmol, 1eq) in ethanol (7.6 mL) and acetic acid (1.97 mL). The mixture was vigorously stirred and heated to reflux. TLC monitoring (ethyl acetate) and LC/MS (2.97 min (ES+) *m*/*z* (relative intensity) 427.57 ([*M* + H]^{+.}, 100)) revealed that the reaction was complete after 5 min. The reaction was allowed to cool, filtered through celite and washed with DCM (50 mL). The filtrate was washed with water (3 x 30 mL), saturated NaHCO₃ (2 x 30 mL), brine (30 mL), dried over MgSO₄, filtered and evaporated under reduced pressure. The residue was purified by flash chromatography (gradient from 60/40 up to 80/20 EtOAc/Hexane) to yield 140 mg (88 %) of pure product as a white foam. Analytical Data: [α]²⁴_{D} = -108° (c = 0.20, CHCl₃); ¹H NMR (400 MHz, CDCl₃) δ 7.22 (d, 2H, *J* = 8.80 Hz), 6.89 (br s, 1H), 6.86 (d, 2H, *J* = 8.82 Hz), 6.80 (s, 1H), 6.29 (s, 1 H), 5.02-4.96 (m, 1H), 4.50-4.40 (m, 4H), 3.89 (s, 3H), 3.82 (s, 3H), 3.81 (s, 1 H), 3.30-3.25 (m, 1 H), 2.85-2.79 (m, 1 H), 2.06 (s, 3H). ¹³C NMR (100 MHz, CDCl₃) δ194.6, 171.1, 171.0, 170.4, 164.0, 160.8, 155.1, 149.5, 146.2, 143.6, 130.5, 129.2, 125.8, 115.5, 114.1, 107.6, 105.4, 100.9, 63.5, 60.4, 56.9, 56.3, 37.4, 21.0, 20.6, 14.2; IR (ATR, CHCl₃) 1733, 1589, 1512, 1396, 1209, 1176, 1113, 1031, 823, 791, 762 cm⁻¹; MS (ES⁺) *m*/*z* (relative intensity) 427.57 ([*M* + H]^{+.}, 100).

### Compound 29

A solution of amine **28** (400 mg, 0.93 mmol, 1 eq) and TEA (350 µL, 2.5 mmol, 2.6 eq.) in dry THF was added dropwise to a freshly prepared solution of triphosgene (125 mg, 0.42 mmol, 0.45 eq] in dry THF (4 mL) at 0°C. The white suspension was allowed to stir at 0°C for 10min. A solution of alcohol **7b** (Alloc-Phe-Lys(Boc)-PABOH, 546 mg, 0.93 mmol, 1 eq) and TEA (350 µL, 2.5 mmol, 2.6 eq) in dry THF (40 mL) was added rapidly. The white suspension was allowed to stir at room temperature for 15 minutes, then heated at 65°C for 2 hours, then allowed to stir at room temperature overnight. The white TEA salts were removed by filtration through cotton wool. The filtrate was concentrated and purified by flash chromatography (gradient, 1% MeOH in chloroform up to 3% MeOH in chloroform) to yield 700mg of desired carbamate (72%). Analytical Data: [α]²⁴_{D} = -30.2° (c = 0.18, CHCl₃); ¹H NMR (400 MHz, CDCl₃) δ 8.54 (s, 1H), 8.43 (s, 1 H), 7.74 (s, 1 H), 7.45 (d, 2H, *J* = 8.43 Hz), 7.23 (d, 2H, *J* = 8.52 Hz), 7.16-7.06 (m, 7H), 6.78-6.72 (m, 4H), 6.46 (d, 1 H, *J* = 7.84 Hz), 5.82-5.73 (m, 1 H), 5.30 (s, 1H), 5.19-5.06 (m, 2H), 5.03 (d, 1H, *J* = 1.29 Hz), 4.93-4.87 (m, 1H), 4.63 (m, 1H), 4.47-4.28 (m, 6H), 3.87 (s, 3H), 3.76 (s, 3H), 3.72 (s, 1H), 3.16-3.09 (m, 1H), 3.07-2.95 (m, 4H), 2.72-2.67 (m, 1H), 1.95-1.83 (m, 1H), 1.60-1.51 (m, 1H), 1.43-1.39 (m, 2H), 1.35 (s, 9H), 1.28-1.19 (m, 2H). ¹³C NMR (100 MHz, CDCl₃) δ 171.5, 171.1, 169.2, 165.8, 159.1, 156.2, 153.8, 151.6, 144.1, 137,8, 135.8, 132.2, 131.3, 129.1, 129.0, 128.9, 127.3, 126.2, 125.9, 123.5, 123.4, 119.9, 118.2, 114.2, 111.3, 66.5, 66.2, 64.0, 56.5, 56.1, 55.3, 53.8, 33.1, 30.9, 29.4, 28.4, 22.6, 20.8; IR (ATR, CHCl₃) 1697, 1652, 1604, 1516, 1456, 1418, 1245, 1225, 1177, 1115, 1033, 824, 750 cm⁻¹; MS (ES⁺) *m*/*z* (relative intensity) 1036.25 ([*M* + H]^{+.}, 100).

### Compound 30

An aqueous solution (3.3 mL) of potassium carbonate (600 mg, 4.34 mmol, 5 eq.) was added to a solution of acetate ester **29** (920 mg, 0.89 mmol, 1 eq.) in methanol (20 mL). The reaction mixture was allowed to stir at room temperature for 50 min at which point TLC (chloroform/methanol, 90/10) showed completion. The mixture was partitioned between water (150 mL) and dichloromethane (200 mL). The organic phase was washed with 1N citric acid (50 mL), followed by brine (50 mL) dried over MgSO₄, filtered and evaporated under reduced pressure to yield the desired alcohol **30** (700 mg, 79 %). Analytical Data: [α]²⁴_{D} = -61° (*c* = 0.18, CHCl₃); ¹H NMR (400 MHz, CDCl₃) δ 8.60 (s, 1 H), 8.51 (s, 1H), 7.64 (s, 1H), 7.42 (d, 2H, *J =* 8.38 Hz), 7.24-7.18 (m, 2H), 7.1-7.05 (m, 7H), 6.83-6.66 (m, 5H), 5.81-5.71 (m, 1H), 5.43 (s, 1H), 5.18-5.08 (m, 2H), 4.99 (s, 2H), 4.75-4.69 (m, 2H), 4.48-4.25 (m, 5H), 3.86 (s, 3H), 3.82-3.76 (m, 2H), 3.74 (s, 3H), 3.72 (s, 3H), 3.19-3.12 (m, 1H), 3.05-2.92 (m, 4H), 2.62-2.57 (m, 1 H), 1.85-1.75 (m, 2H), 1.59-1.51 (m, 1H), 1.38-1.34 (m, 11H), 1.28-1.18 (m, 2H). ¹³C NMR (100 MHz, CDCl₃) δ 171.7, 169.5, 167.0, 159.2, 156.3, 153.9, 151.6, 144.4, 137.8, 135.9, 132.3, 131.9, 129.2, 128.8, 127.2, 126.0, 124.5, 123.3, 120.1, 118.1, 114.2, 111.3, 66.6, 66.1, 61.6, 56.5, 56.1, 55.3, 53.8, 39.9, 33.6, 31.1, 29.4, 28.4, 22.6; MS (ES⁺) *m*/*z* (relative intensity) 994.7 ([*M* + H]⁺, 100).

### Compound 31

Alcohol 30 (500 mg, 0.503 mmol, 1eq.) was dissolved in anhydrous DCM (50 mL) at room temperature. Solid Dess-Martin periodinane (300 mg, 0.707 mmol, 1.4 eq.) was added to the mixture, followed by a further 75 mg at 1 h, followed by a further 57 mg at 2 h, and 31 mg at 5 h taking the total mass of Dess-Martin Periodinane to 463 mg (1.09 mmol, 2.17 eq.). The reaction was continuously monitored by TLC [chloroform / methanol, 95/5, two elutions). After 6.5 hours, the reaction was worked up by partitioning the reaction mixture between DCM and saturated aqueous NaHSO₃. The organic layer was then washed with saturated aqueous NaHCO₃, followed by brine, dried over MgSO₄, filtered and evaporated under reduced pressure. The residue was purified by flash chromatography (gradient from 0/100 up to 2/98 methanol/chloroform) to yield 259 mg (52%) of pure product **31.** Analytical Data: [α]²⁴_{D} = +106° (c = 0.16, CHCl₃); ¹H NMR (400 MHz, CDCl₃) δ 8.68 (s, 1H), 7.54-7.46 (m, 2H), 7.36 (s, 1 H), 7.31-7.16 (m, 8H), 6.89 (d, 2H, *J* = 8.70 Hz), 6.75 (bs, 1 H), 6.61 (s, 1 H), 5.89-5.84 (m, 2H), 5.45 (d, 1H, J = 4.80 Hz), 5.28-5.08 (m, 3H), 4.84-4.76 (m, 2H), 4.58-4.47 (m, 4H), 4.28 (bs, 1H), 4.02-3.95 (m, 1H), 3.92 (s, 3H), 3.83 (s, 3H), 3.74 (s, 2H), 3.41-3.32 (m, 1H), 3.16-3.02 (m, 5H), 2.03-1.83 (m, 1H), 1.68-1.61 (m, 1H), 1.55-1.39 (m, 11H), 1.36-1.28 (m, 2H). ¹³C NMR (100 MHz, CDCl₃) δ 171.7, 169.5, 163.2, 159.1, 156.3, 151.1, 148.6, 138.0, 135.9, 132.3, 129.2, 128.8, 127.2, 126.3, 126.2, 121.7, 120.0, 118.2, 114.2, 112.7, 110.7, 86.2, 79.3, 67.6, 66.2, 59.5, 56.4, 56.15, 56.1, 55.3, 53.8, 39.9, 38.1, 35.1, 31.0, 29.4, 28.4, 22.7 ; IR (ATR, CHCl₃) 3313, 2935, 2356, 1691, 1603, 1512, 1431, 1253, 1177, 1119, 1033, 824, 750, 698 cm⁻¹; MS (ES⁺) *m*/*z* (relative intensity) 992.41 ([*M* + H]⁺, 100).

### Compound 32

Solid Pd(PPh₃)₄ (8 mg, 6-9 µmol, 0.02 eq.) was added to a freshly prepared solution of starting material **31** (346 mg, 0.349 mmol, 1 eq.) and pyrrolidine (43.3 µL, 0.523 mmol, 1.5 eq.) in dry DCM (10 mL) under inert atmosphere at room temperature. The reaction was complete after 45 min as indicated by TLC (90/10 v/v chloroform/methanol) and LC/MS (2.93 min (ES+) *m*/*z* (relative intensity) 908.09 ([M + H]^{+.}, 100)). The volatiles were removed by evaporation under reduced pressure. The residue was purified by flash chromatography (gradient from 2 / 98 up to 5/95 methanol/chloroform) to yield 298 mg (94 %) of pure product **32.** Analytical Data: ¹H NMR (400 MHz, CDCl₃) δ 8.87 (s, 1 H), 7.86 (d, 1H, *J* = 8.06 Hz), 7.51 (d, 2H, *J* = 8.42 Hz), 7.36-7,11 (m, 9H), 6.89 (d, 2H, *J* = 8.73 Hz), 6.58 (bs, 1 H), 5.85 (d, 1 H, *J* = 9.47 Hz), 5.32 (m, 1H), 4.83 (d, 1H, *J* = 11.68 Hz), 4.65 (m, 1H), 4.47 (q, 1H, *J* = 6.14 Hz), 4.03-3.97 (m, 1H), 3.94 (s, 3H), 3.84 (s, 3H), 3.74-3.69 (m, 4H), 3.39-3.33 (m, 1H), 3.26 (dd, 1H, *J* = 13.73 Hz, *J* = 4.00 Hz), 3.16-3.03 (m, 3H), 3.26 (dd, 1 H, *J* = 8.91 Hz, *J* = 13.74 Hz), 2.05-1.96 (m, 1H), 1.78-1.49 (m, 3H), 1.48-1.42 (m, 9H), 1.42-1.24 (m, 2H), ¹³C NMR (100 MHz, CDCl₃) δ 175.45, 163.2, 159.1, 156.2, 148.6, 137.3, 129.3, 128.8, 127.0, 126.2, 126.2, 121.7, 119.8, 114,2, 112.6, 56.2, 55.3, 40.7, 35.1, 30.5, 28.4, 22.8; MS (ES⁺) m/z (relative intensity) 908.09 ([*M* + H]⁺, 100).

### Compound 33

Solid EEDQ (108 mg, 0.436 mmol, 2eq.) was added to a solution of amine **32** (199 mg, 0.219 mmol, 1 eq.) and maleimido hexanoic acid (57 mg, 0.269, 1.23 eq.) in a mixture of DCM (6 mL) and methanol (3 mL). The solution was allowed to stir at room temperature for 24 hours. The reaction was found to be complete by LC/MS (3.45 min (ES+) *m*/*z* (relative intensity) 1101.78 ([*M* + H]^{+.}, 100)). The volatiles were removed by evaporation under reduced pressure. The residue was partitioned between DCM and saturated aqueous NaHCO₃, washed with brine, dried over MgSO₄, filtered and evaporated under reduced pressure. The residue was purified by flash chromatography (gradient from 1/99 up to 2.5/97.5 methanol/chloroform) to yield 165 mg (68 %) of pure product **33.** Analytical Data: [α]²⁴_{D} = +94° (c = 0.09, CHCl₃); ¹H NMR (400 MHz, CDCl₃) δ 8.62 (s, 1H), 7.46-7.39 (m, 2H), 7.27 (s, 1 H), 7.22-7.06 (m, 9H), 6.79 (d, 2H, *J* = 8.65 Hz), 6.75 (bs, 1 H), 6.57 (s, 2H), 6.52 (s, 1 H), 6.28 (s, 1 H), 5.77 (bs, 1 H), 5.21 (s, 1H), 4.75-4.60 (m, 2H), 4.40 (q, 1 H, *J* = 5.54 Hz), 3.93-3.86 (m, 1H), 3.83 (s, 3H), 3.73 (s, 3H), 3.65 (s, 2H), 3.36 (t, 2H, *J* = 7.16 Hz), 3.30-3.23 (m, 1H), 3.08-2.90 (m, 5H), 2.09 (t, 2H, *J* = 7.14 Hz), 1.93-1.75 (m, 1H), 1.62-1.31 (m, 17H), 1.30-1.04 (m, 6H). ¹³C NMR (100 MHz, CDCl₃) δ 173.5, 170.9, 170.3, 169.6, 163.2, 159.1, 156.3, 151.1, 148.6, 136.1. 134.0. 129.1, 128.7, 127.2, 126.3, 126.2, 124.9, 123.3, 121.7, 120.0, 114.2, 112.7, 110.7, 86.1, 79.3, 67.6, 59.5, 56.2, 56.1, 55.3, 54.6, 53.9, 53.4, 37.8, 37.5, 36.7, 35.2, 31.0, 29.4, 28.5, 28.2, 26.2, 24.8, 22.7; MS (ES⁺) *m*/*z* (relative intensity) 1101.78 ([*M* + H]^{+.}, 100).

### Compound 34

A chilled solution of 10 % TFA in DCM (12 mL) was added to a chilled (-20°C) sample of **33** (75 mg, 0.068 mmol, 1 eq.). The reaction was monitored by LC/MS (2.87 min (ES+) *m*/*z* (relative intensity) 1001.13 ([*M* + H]^{+.}, 100)). Temperature reached highs of -10°C without side-reactions. The reaction reached completion after 4 hours. The reaction mixture was poured into deionised water (50 mL) and freeze-dried overnight (liquid nitrogen bath, allowed to evaporate without refill) to yield the pure TFA salt **34** (75 mg, 99%). Analytical Data: 1H : NMR (400 MHz, CDCl₃) δ 9.05 (s, 1H), 7.87-7.62 (m, 4H), 7.35 (m, 2H), 7.24 (s, 1H), 7.16-7.11 (m, 2H), 7.10-6.96 (m, 8H), 6.92 (s, 1H), 6.82-6.66 (m, 3H), 6.63-6.42 (m, 3H), 5.75 (d, 1 H, *J* = 9.54 Hz), 5.15-5.03 (m, 1H), 4.77-4.74 (m, 1H), 4.68-4.56 (m, 1H), 4.39 (s, 1 H), 3.97-3.84 (m, 1H), 3.80 (s, 3H), 3.72 (s, 3H), 3.65 (s, 2H), 3.33-3.21 (m, 3H), 3.04-2.69 (m, 5H), 2.04 (m, 2H), 1.979-1.64 (m, 1 H), 1.63-1.45 (m, 3H), 1.44-1.12 (m, 6H), 1.0 7-0.95 (m, 2H). MS (ES⁺) *m*/*z* (relative intensity) 1001.13 *([M* + H]^{+.}, 100).

### Compounds 35

Amine **32** (99 mg, 0.109 mmol, 1 eq) was added to a solution of NHS-PEG₄-Maleimide (Thermo Scientific, 61.6 mg, 0.120 mmol, 1.1 eq) and TEA (18.2 µL, 0.130 mmol, 1.2 eq.) in a mixture of anhydrous DCM (5 mL) and DMF (1 mL). The reaction was allowed to stir at room temperature overnight at which point it was found almost to be complete by LC/MS (3.27 min (ES+) *m*/*z* (relative intensity) 1307.55 ([*M* + H]^{+.}, 100)). The volatiles were removed by evaporation under reduced pressure. The residue was purified by flash chromatography (gradient from 3/97 up to 5/95 methanol/chloroform) to yield 71 mg (50%) of pure product **35.** Analytical Data: ¹H NMR (400 MHz, CDCl₃) δ 8.58 (s, 1H), 7.50 (d, 2H, *J* = 8.47 Hz), 7.28 (s, 1H), 7.25-7.20 (m, 2H), 7.18-7.01 (m, 9H), 6.89 (d, 1H, *J* = 7.58 Hz), 6.79 (d, 2H, *J* = 8.68 Hz), 6.59 (s, 2H), 6.51 (s, 1 H), 5.77 (d, 1 H, *J* = 6.42 Hz), 5.25 (d, 1 H, *J* = 11.43 Hz), 4.83-4.64 (m, 2H), 4.63-4.49 (m, 1 H), 4.43-4.38 (m, 1H), 4.18 (s, 1H), 3.96-3.85 (m, 1H), 3.84 (s, 3H), 3.76-3.56 (m, 9H), 3.57-3.34 (m, 15H), 3.34-3.20 (m, 3H), 3.15 (dd, 1 H, *J* = 14.22 Hz, *J* = 5.60 Hz), 3.07-2.89 (m, 4H), 2.48-2.29 (m, 4H), 1.97-1.90 (m, 1H), 1.61-1.39 (m, 3H), 1.35 (s, 9H), 1.29-1.12 (m, 4H). MS (ES⁺) *m*/*z* (relative intensity) 1307.55 ([*M* + H]^{+.}, 100).

### Compound 36

A chilled solution of 10 % TFA in DCM (10 mL) was added to a chilled (-20°C) sample of **35** (70 mg, 0.054 mmol, 1 eq.). The reaction was monitored by LC/MS (2.77 min (ES+) *m*/*z* (relative intensity) 1206.94 ([*M* + H]^{+.}, 100)). The reaction reached completion after 18 hours at -25°C. The reaction mixture was poured into deionised water (50 mL) and freeze-dried overnight (liquid nitrogen bath, allowed to evaporate without refill) to yield the pure TFA salt **36** (75 mg, 99%).

### Compound 37

A solution of **33** (9.5 mg, 8.6 µmol, 1 eq) in methanol (1.5 mL) was added to a solution of the cyclic thiopeptide c(RGDfC) (5 mg, 8.6 µmol, 1 eq., from pepnet.com) in a mixture of methanol (2 mL) and water (1 mL). The reaction mixture was allowed to stir for 1 hour. The resulting precipitate was collected by filtration and rinsed with a mixture of methanol and water (0.6 mL / 0.4 mL) and dried by vacuum suction to give 8 mg (55%) of pure product as shown by LC/MS (3.03 min (ES+) m/z (relative intensity) 1681.19 (*[*M + H]^{+.}, 10), 790.85 (100)).

### Compound 38

Compound **37** (7 mg) was treated with 10% TFA in DCM (1 mL) at 0°C (ice bath) for 2 hours. The reaction was found to be complete as shown by LC/MS (2.70 min (ES+) *m*/*z* (relative intensity) 791.44 ([(M + 2H)/2] ^{+.}, 100)). The reaction mixture was poured into deionised water (10 mL) and freeze-dried overnight (liquid nitrogen bath, allowed to evaporate without refill) to yield the pure TFA salt **38** (7 mg, quantitative Yield).

### Compound 39a

Compound **39a** and its synthesis is disclosed in WO 00/012508 and WO 2006/111759.

### Compound 39b

Method I: A catalytic amount of DMF (2 drops) was added (effervescence!) to a stirred solution of the nitro-acid **39a** (1.0 g, 2.15 mmol) and oxalyl chloride (0.95 mL, 1.36 g, 10.7 mmol) in dry THF (20 mL). The reaction mixture was allowed to stir for 16 hours at room temperature and the solvent was removed by evaporation in *vacuo.* The resulting residue was re-dissolved in dry THF (20 mL) and the acid chloride solution was added dropwise to a stirred mixture of (2*S*,4*R*)-methyl-4-hydroxypyrrolidine-2-carboxylate hydrochloride (859 mg, 4.73 mmol) and TEA (6.6 mL, 4.79 g, 47.3 mmol) in THF (10 mL) at -30°C (dry ice/ethylene glycol) under a nitrogen atmosphere. The reaction mixture was allowed to warm to room temperature and stirred for a further 3 hours after which time TLC (95:5 v/v CHCl₃/MeOH) and LC/MS (2.45 min (ES+) m/z (relative intensity) 721 ([*M* + H]^{+.}, 20)) revealed formation of product. Excess THF was removed by rotary evaporation and the resulting residue was dissolved in DCM (50 mL). The organic layer was washed with 1 N HCl (2 x 15 mL), saturated NaHCO₃ (2 x 15 mL), H₂O (20 mL), brine (30 mL) and dried (MgSO₄). Filtration and evaporation of the solvent gave the crude product as a dark coloured oil. Purification by flash chromatography (gradient elution: 100% CHCl₃ to 96:4 v/v CHCl₃/MeOH) isolated the pure amide **39b** as an orange coloured glass (840 mg, 54%).

Method II: Oxalyl chloride (9.75 mL, 14.2 g, 111 mmol) was added to a stirred suspension of the nitro-acid **39a** (17.3 g, 37.1 mmol) and DMF (2 mL) in anhydrous DCM (200 mL), Following initial effervescence the reaction suspension became a solution and the mixture was allowed to stir at room temperature for 16 hours. Conversion to the acid chloride was confirmed by treating a sample of the reaction mixture with MeOH and the resulting bis-methyl ester was observed by LC/MS. The majority of solvent was removed by evaporation *in vacuo,* the resulting concentrated solution was re-dissolved in a minimum amount of dry DCM and triturated with diethyl ether. The resulting yellow precipitate was collected by filtration, washed with cold diethyl ether and dried for 1 hour in a vacuum oven at 40°C. The solid acid chloride was added portionwise over a period of 25 minutes to a stirred suspension of (2*S*,4*R*)-methyl-4-hydroxypyrrolidine-2-carboxylate hydrochloride (15.2 g, 84.0 mmol) and TEA (25.7 mL, 18.7 g, 185 mmol) in DCM (150 mL) at -40°C (dry ice/CH₃CN). Immediately, the reaction was complete as judged by LC/MS (2.47 min (ES+) *m*/*z* (relative intensity) 721 ([*M* + H]^{+.}, 100)). The mixture was diluted with DCM (150 mL) and washed with 1N HCl (300 mL), saturated NaHCO₃ (300 mL), brine (300 mL), filtered (through a phase separator) and the solvent evaporated *in vacuo* to give the pure product **39b** as an orange solid (21.8 g, 82%). Analytical Data: [α]²²_{D} = -46.1° (c = 0.47, CHCl₃); ¹H NMR (400 MHz, CDCl₃) (rotamers) δ 7.63 (s, 2H), 6.82 (s, 2H), 4.79-4.72 (m, 2H), 4.49-4.28 (m, 6H), 3.96 (s, 6H), 3.79 (s, 6H), 3.46-3.38 (m, 2H), 3.02 (d, 2H, *J* = 11.1 Hz), 2.48-2.30 (m, 4H), 2.29-2.04 (m, 4H); ¹³C NMR (100 MHz, CDCl₃) (rotamers) δ 172.4,166.7, 154.6, 148.4, 137.2, 127.0, 109.7, 108.2, 69.7, 65.1, 57.4, 57.0, 56.7, 52.4, 37.8, 29.0; IR (ATR, CHCl₃) 3410 (br), 3010, 2953, 1741, 1622, 1577, 1519, 1455, 1429, 1334, 1274, 1211, 1177, 1072, 1050, 1008, 871 cm⁻¹; MS (ES⁺) *m*/*z* (relative intensity) 721 ([*M* + H]^{+.}, 47), 388 (80); HRMS [M + H]^{+.} theoretical C₃₁H₃₆N₄O₁₆ *m*/*z* 721.2199, found (ES⁺) *m*/*z* 721.2227.

### Compound 39c

Solid TCCA (32 g, 137 mmol, 2.2 eq.) was added portionwise to a solution of TEMPO (1g, 6.4 mmol, 0. 1 eq) and bis-alcohol **18** (45 g, 62.5 mmol, 1 eq.), in normal DCM (500 mL) at 0°C. A slight exotherm was observed. The reaction was deemed complete by TLC (Ethyl Acetate) and LC /MS (2.95 min (ES+) m/z (relative intensity) 718.10 *([M* + H]^{+.}, 100)) after 30 minutes. The suspension was filtered through celite and washed with DCM. The filtrate was washed with aqueous sodium bisulfite, followed by saturated NaHCO₃ (caution, vigorous effervescence), brine (200 mL) and dried (MgSO₄). Filtration and evaporation of the solvent *in vacuo* afforded the crude product which was purified by flash column chromatography (elution: 20:80 v/v *n*-hexane/EtOAc) to afford the ketone **39c** as a white solid (28.23 g, 63%). Analytical Data: [α]²¹_{D} = +18° (c = 0.2, CHCl₃); MS (ES⁺) *m*/*z* (relative intensity) 718.10 ([*M* + H]^{+.}, 100); ¹H NMR (400 MHz, CDCl₃) mixture of rotamers δ 7.70 (m, 2H), 6.79 (m, 2H), 5.27 (m, 1H), 4.44 (m, 1H), 4.30 (m, 4H), 3.93 (m, 6H), 3.81 (s, 3H),3.75 (m, 1 H), 3.63 (s, 2H), 3.58 (m, 1 H), 3.09-2.89 (m, 2H), 2.74-2.53 (m, 2H), 2.40 (p, 2H, *J* = 5.73 Hz); ¹³C NMR (100 MHz, CDCl₃) mixture of rotamers δ 206.5, 206.4, 206.0, 205.9, 171.2, 171.1, 170.6, 167.0, 166.7, 155.0, 154.5, 148.8, 137.7, 137.3, 126.4, 125.4, 109.8, 109.1, 108.6, 108.4, 108.4, 65.7, 65.6, 65.5, 60.4, 57.9, 56.7, 56.7, 55.1, 53.6, 52.9, 52.9, 51.6, 41.2, 40.1, 28.7, 28.6, 21.0, 14.1; IR (ATR, CHCl₃) 1764, 1650, 1578, 1518, 1415, 1333, 1274, 1217, 1060, 870, 824 759 cm⁻¹

### Compound 40

Anhydrous 2,6-lutidine (4.26 mL, 3.92 g, 36.6 mmol) was injected in one portion to a vigorously stirred solution of bis-ketone **39c** (4.23 g, 5.90 mmol) in dry DCM (100 mL) at -45°C (dry ice/acetonitrile cooling bath) under a nitrogen atmosphere. Anhydrous triflic anhydride, taken from a freshly opened ampoule (5.96 mL, 10 g, 35.4 mmol), was injected rapidly dropwise, while maintaining the temperature at -40°C or below. The reaction mixture was allowed to stir at -45°C for 1 hour at which point TLC (50/50 v/v *n*-hexane/EtOAc) revealed the complete consumption of starting material. The cold reaction mixture was immediately diluted with DCM (200 mL) and, with vigorous shaking, washed with water (1 x 300 mL), 5% citric acid solution (1 x 200 mL) saturated NaHCO₃ (200 mL), brine (150 mL) and dried (MgSO₄). Filtration and evaporation of the solvent in *vacuo* afforded the crude product which was purified by flash column chromatography (gradient elution: 70:30 v/v *n*-hexane/EtOAc to 40:60 v/v *n*-hexane/EtOAc) to afford the bis-triflate **40** as a yellow foam (1.32 g, 23%). Analytical Data: [α]²⁵_{D} = -68° (c = 0.2, CHCl₃); ¹H NMR (400 MHz, CDCl₃) δ 7.73 (s, 2H), 6.85 (s, 2H), 6.20 (t, 2H, *J* = 1.81 Hz), 5.13 (dd, 2H, *J*= 5.05, 11.93 Hz), 4.33 (t, 4H, *J* = 5.91 Hz), 3.95 (s, 6H), 3.84 (s, 6H), 3.43 (ddd, 2H, *J* = 2.28, 11.92, 16.59 Hz), 2.96 (ddd, 2H, *J* = 1.60, 5.05, 16.58 Hz), 2.44 (p, 2H, *J* = 5.79 Hz); ¹³C NMR (100 MHz, CDCl₃) δ 169.4, 164.1, 154.7, 149.2, 138.0, 135.2, 124.4, 121.1, 120.0, 116.8, 110.0, 108.4, 65.7, 65.6, 57.0, 56.8, 53.1, 33.3, 28.6; IR (ATR, CHCl₃) 1748, 1654, 1576, 1522, 1418, 1337, 1277, 1207, 1131, 1061, 1023, 910, 821, 757 cm⁻¹; MS (ES⁺) *m*/*z* (relative intensity) 981.86 ([*M* + H]^{+.}, 100).

### Compound 41

Pd(PPh₃)₄ (660 mg, 571 µmol, 0.08 eq) was added to a stirred mixture of bis enol triflate 40 (7 g, 7.13 mmol, 1 eq), 4-fluorophenylboronic acid (2.6 g, 18.5 mmol, 2.6 eq), Na₂CO₃ (3.93 g, 37.0 mmol, 5.2 eq), EtOH (25 mL), toluene (50 mL) and water (25 mL). The reaction mixture was allowed to stir under a nitrogen atmosphere overnight after which time the complete consumption of starting material was observed by TLC (60/40 EtOAc/Hexane) and LC/MS (3.68 min (ES+) *m*/*z* (relative intensity) 873.90 ([M + H]^{+.}, 100)). The reaction mixture was diluted with EtOAc (300 mL) and washed with H₂O (2 x 200 mL), brine (100 mL), dried (MgSO₄), filtered and evaporated under reduced pressure to provide the crude product. Purification by flash chromatography (gradient elution: 50:50 v/v Hexane/EtOAc to 80:20 v/v Hexane/EtOAc) afforded bis C2-aryl compound **41** as an orange solid (5.46 g, 88%). Analytical Data: [α]²²_{D} = -107° (*c* = 0.2, CHCl₃); ¹H NMR (400 MHz, CDCl₃) δ 7.76 (s, 2H), 7.14-7.04 (m, 4H), 6.97-6.87 (m, 6H), 6.31 (s, 2H), 5.18 (dd, 2H, *J* = 11.68, 5.03 Hz), 4.36 (t, 4H, *J* = 5.87 Hz), 3.97 (s, 6H), 3.84 (s, 6H), 3.53-3.39 (m, 2H), 3.00 (ddd, 2H, *J* = 1.22, 5.01, 16.28 Hz), 2.46 (p, 2H, *J* = 5.98 Hz); ¹³C NMR (100 MHz, CDCl₃) δ171.0, 163.3, 148.9, 138.0, 128.1, 126.3, 126.2, 125.8, 123.1, 122.6, 115.7, 115.5, 110.3, 108.5, 65.7, 58.3, 56.8, 34.7, 28.7; IR (ATR, CHCl₃) 1738, 1650, 1578, 1512, 1416, 1333, 1275, 1212, 1064, 1023, 869, 808, 758, 654, 613 cm⁻¹; MS (ES⁺) *m*/*z* (relative intensity) 873.90 ([*M* + H]^{+.}, 100)).

### Compound 42

LiBH₄ (132 mg, 21.3 mmol, 3 eq.) was added in one portion to a stirred solution of the ester **41** (5.30 g, 6.07 mmol, 1 eq.) in anhydrous THF (100 mL) at 0°C. The reaction mixture was allowed to warm up to room temperature and to stir for 1 hour after which time the complete conversion of starting material directly was observed by LC/MS (3.42 min (ES+) *mlz* (relative intensity) 818.35 ([*M* + H]^{+.}, 100)). The reaction mixture was carefully diluted with ethyl acetate (500 mL) and excess borohydride destroyed with cold aqueous citric acid. The organic layer was washed with 1N aqueous HCL (100mL) followed by saturated aqueous NaHCO₃ (100 mL), brine (100 mL), dried over MgSO₄, filtered and evaporated under reduced pressure at 35°C to provide the intermediate alcohol which was immediately re-dissolved in anhydrous DCM (200 mL). The solution was cooled to 0°C and imidazole (3.97 g, 58.0 mmol, 9.6 eq.) was added, followed by TBDMS-Cl (4.390 g, 29.1 mmol, 4.8 eq.). The reaction mixture was allowed to warm up to RT and react for 2 hours. . Complete reaction was observed by TLC (EtOAc/hexane, 50/50) and LC/MS (4.23 min (ES+) *m*/*z* (relative intensity) 1045.99 ([*M* + H]^{+.}, 100)). The solution was washed with 2N aqueous citric acid (50 mL), saturated aqueous NaHCO₃ (50 mL), brine (50 mL), dried over MgSO₄, filtered and evaporated under reduced pressure. The residue was purified by flash chromatography (gradient from 80/20 up to 60/40 hexane/EtOAc) to yield 2.45 g (38.6% over two steps) of pure product as an orange solid. Analytical Data: [α]²²_{D}= -123° (*c* = 0.18, CHCl₃); ¹H NMR (400 MHz, CDCl₃) δ 7.76 (s, 2H), 7.17-7.06 (m, 4H), 6.96-6.87 (m, 4H), 6.81 (s, 2H), 6.17 (s, 2H), 4.84-4.72 (m, 2H), 4.35 (t, 4H, *J* = 5.87 Hz), 3.93 (s, 6H), 3.25-3.07 (m, 2H), 3.03-2.91 (m, 2H) 2.45 (p, 2H, *J =* 5.92 Hz), 0.84 (s, 18H), 0.07 (s, 12H); ¹³C NMR (100 MHz, CDCl₃) δ 163.2, 160.7, 154.5, 148.6, 137.9, 130.0, 126.7, 126.3, 126.2, 124.3, 123.0, 115.6, 115.4, 110.0, 108.5, 65.7, 60.4, 59.2, 56.7, 33.2, 28.7, 25.8, 25.7, 21.0, 18.2, 14.2, -5.3; IR (ATR, CHCl₃) 2953, 1742, 1650, 1576, 1512, 1417, 1334, 1274, 1214, 1063, 1023, 869, 808, 759, 654, 612 cm⁻¹; MS (ES⁺) *m*/*z* (relative intensity) 1045.99 ([*M* + H]^{+.}, 100).

### Compound 43

A solution of formic acid in ethanol (5% v/v, 100mL) was added to a suspension of bis-nitro compound **42** (2.35 g, 2.25 mmol, 1 eq.) and zinc dust (8.82 g, 0.135 mmol, 60 eq.) in ethanol (35 mL). The reaction mixture was allowed to stir at room temperature for 25 min at which point TLC (methanol/chloroform, 2/98) and LC/MS (4.23 min (ES+) *m*/*z* (relative intensity) 986.3 ([*M*+ H]^{+.}, 10), 493.9 ([(*M* + 2H)/2]^{+.}, 100)) revealed complete reaction. The suspension was filtered and the filtrate was partitioned between ethyl acetate (400 mL) and saturated aqueous NaHCO₃ (200 mL). The organics were washed with brine (100 mL), dried over MgSO₄, filtered and evaporated under reduced pressure to yield pure bis-amine (2.20g, 98 %) which taken through directly to the next step.

### Compound 44

A solution of allyl chloroformate (0.209 mL, 1.97 mmol, 0.9 eq.) in dry DCM (50 mL) was added dropwise to a solution of bis-anilino compound **43** (2.15 g, 2.18 mmol, 1 eq.) and pyridine (0.335 mL, 4.14 mmol, 1.9 eq.) in dry DCM (250 mL) at -78°C. The reaction mixture was allowed to stir at -78°C for 2 hours, and allowed to warm up to room temperature. The solution was washed with saturated aqueous copper sulphate (2 x 50 mL), water (250 mL), with brine (100 mL), dried over MgSO₄, filtered and evaporated under reduced pressure. The residue was purified by flash chromatography (gradient from 70/30 up to 30/70 hexane/EtOAc) to yield 668 mg (26.5%) of bis-Alloc protected compound as indicated by LC/MS [4.45 min (ES+) *m*/*z* (relative intensity) 1154.32 ([*M* + H]^{+.}, 100)) and 800 mg of desired mono-alloc protected compound slightly contaminated (4.32 min (ES+) *m*/*z* (relative intensity) 1070.58 ([*M* + H]^{+.}, 100)). This compound was purified further by flash chromatography (gradient from 40/60 up to 20/80 hexane/diethyl ether) to give 700 mg (30 %) of desired pure mono-alloc compound. Analytical Data: [α]²²_{D} = -41° (*c* = 0.16, CHCl₃); ¹H NMR (400 MHz, CDCl₃) δ 8.72 (bs, 1H), 7.88 (s, 2H), 7.25-7.18 (m, 4H), 7.02-6.93 (m, 4H), 6.93-6.83 (m, 3H), 6.80 (s, 1H), 6.36 (s, 1H), 6.00-5.84 (m, 1H), 5.32 (dd, 1H, *J* = 1.37, *J =* 17.21 Hz), 5.21 (dd, 1H, *J* = 0.90, *J* = 10.40 Hz), 4.85-4.71 (m, 2H), 4.60 (dd, 2H, *J* = 1.02, *J* = 5.62 Hz), 4.46 (s, 2H), 4.31 (t, 2H, *J* = 5.96 Hz), 4.25 (t, 2H, *J* = 6.31 Hz), 3.98 (m, 2H), 3.86 (m, 2H), 3.81 (s, 3H), 3.76 (s, 3H), 3.19-3.05 (m, 2H), 3.05-2.93 (m, 2H), 2.41 (p, 2H, *J =* 6.16 Hz), 0.84 (m, 18H), 0.05 (m, 12H); IR (ATR, CHCl₃) 2952, 2359, 1732, 1652, 1601, 1507, 1472, 1406, 1225, 1119, 836, 777, 668 cm⁻¹; MS (ES⁺) *m*/*z* (relative intensity) 1070.58 ([*M* + H]^{+.}, 100).

### Compound 45

A solution of amine **44** (650 mg, 0.607 mmol, 1 eq.) and TEA (220 µL, 1.58 mmol, 2.6 eq) in dry THF was added dropwise to a freshly prepared solution of triphosgene (81 mg, 0.273 mmol, 0.45 eq.) in dry THF (4 mL) at 0°C. The white suspension was allowed to stir at 0°C for 10 min. A solution of alcohol (Alloc-Val-Ala-PABOH, 229 mg, 0.607 mmol, 1 eq.) and TEA (220 µL, 1.58 mmol, 2.6 eq) in dry THF (30 mL) was added rapidly. The white suspension was allowed to stir at room temperature for 15 minutes, then heated at 65°C for 2 hours and then allowed to stir at room temperature overnight. The white TEA salts were removed by filtration through cotton wool. The filtrate was concentrated and purified by flash chromatography (Gradient, 0% MeOH in chloroform up to 3% MeOH in chloroform) to yield 220 mg of desired carbamate (25%). Analytical Data: [α]²⁴_{D} = -46.1° (*c* = 0.13, CHCl₃); ¹H NMR (400 MHz, CDCl₃) δ 8.70 (bs, 2H), 8.46 (s, 1H), 7.83 (s, 2H), 7.50 (m, 2H), 7.31 (d, 2H, *J* = 8.50 Hz) 7.25-7.15 (m, 4H), 7.03-6.93 (m, 4H), 6.92-6.77 (m, 4H), 6.51 (d, 1H, *J* = 7.48Hz), 5.99-5.81 (m, 1H), 5.38-5.15 (m, 5H), 5.13-5.03 (m, 2H), 4.77 (bs, 2H), 4.66-4.53 (m, 5H), 4.38-4.22 (m, 4H), 4.08-3.94 (m, 3H), 3.93-3.81 (m, 2H), 3.79 (s, 6H), 3.20-3.05 (m, 2H), 3.05-2.94 (m, 2H), 2.41 (p, 2H, *J* = 5.95 Hz), 2.22-2.08 (m, 1 H), 1.45 (d, 3H, *J* = 7.03 Hz), 0.94 (dd, 6H, *J* = 6.81, 14.78 Hz), 0.84 (m, 18H), 0.14-0.02 (m, 12H); ¹³C NMR (100 MHz, CDCl₃) δ 171.7, 169.8, 165.9, 163.1, 153.6, 151.0, 144.3, 137.8, 132.4, 132.3, 132.0, 130.2, 129.2, 126.2, 126.1, 125.3, 123.3, 123.2, 119.8, 118.2, 118.0, 115.7, 115.5, 112.0, 106.0, 66.5, 66.2, 65.8, 65.4, 62.5, 60.4, 59.5, 56.6, 49.6, 30.8. 28.9, 25.7, 19.2, 18.2, 18.1, 17.7, 17.3, 14.2, -5.4; IR (ATR, CHCl₃) 2950, 2356, 1725, 1691, 1602, 1512, 1408, 1201, 1109, 1023, 832, 774, 668 cm⁻¹; MS (ES⁺) *m*/*z* (relative intensity) 1473.43 ([M + H]^{+.}, 100).

### Compound I2

1-Benzyl 19-(2,5-dioxopyrrolidin-1-yl) 4,7,10,13,16-pentaoxanonadecane-1,19-dioate (**I1**) (100 mg, 0.19 mmol, 1 eq.) in dry ethyl acetate (15 mL) was hydrogenated at 30 psi over 10% Palladium on carbon (10 mg, 10 wt %) for 45 minutes. The reaction mixture was filtered through celite washing with dry ethyl acetate. Evaporation under reduced pressure gave the product **I2** as a colourless oil (74 mg, 89%). Analytical Data: R_{T} (not visible on LC) MS (ES⁺) *m*/*z* (relative intensity) 458 ([M + Na]⁺ , 55), 436 ([M + H]⁺, 12).

### Compound I3

*N,N*-Diisopropyldiethylamine (8.4 µL, 6 mg, 5.97 x 10⁻⁵ mol, 1.1 eq) was added to a solution of amine dipeptide (**12c**) (50 mg, 5.42 x 10⁻⁵ mol, 1.0 eq.) and acid-dPeg®5-NHS ester (**I2**) (28 mg, 6.5 x 10⁻⁵ mol, 1.2 eq.) in dry DCM (5 mL). The solution was stirred at room temperature for 24 hours. The reaction mixture was evaporated under reduced pressure to give a pale yellow oil. Purification by flash column chromatography [gradient elution 96% chloroform/4% methanol to 92% chloroform/8% methanol in 0.5% increments] gave the product **I3** as a yellow glass (42 mg, 64%). Analytical Data: R_{T} 2.78 min; MS (ES⁺)*m*/*z* (relative intensity) 1242 ([M + H]^{+.},40).

### Compound 49

1-Ethy)-3-(3-dimethylaminopropyl)carbodiimide (9.1 mg, 4.76 x 10⁻⁵ mol, 1.6 eq.) was added to a solution of N-hydroxysuccinimide (5.8 mg, 5.06 x 10⁻⁵ mol, 1.7 eq.) and acid (**I3**) in dry DCM (6 mL) under a nitrogen atmosphere. The reaction mixture was stirred at room temperature for 48 hours. The solution was filtered washing with DCM (10 mL). The DCM solution was washed with water (20 mL), brine (20 mL), dried (MgSO₄) and evaporated under reduced pressure. The product **49** (32 mg, 80%) was used without further purification. Analytical Data: R_{T} 2.88 min; MS (ES⁺) *m*/*z* (relative intensity)1339 ([M + H]^{+.}, 100).

### Compound 50

Pd(PPh₃)₄ (61 mg, 0.053 mmol, 0.01 eq.) was added to a solution of the alloc compound **(8)** (2.0 g, 5.3 mmol, 1.0 eq.) and pyrrolidine (0.47 g, 0.55 mL, 6.63 mmol, 1.25 eq.) in dry DCM under a nitrogen atmosphere. The solution was stirred at room temperature for 16 hours. LCMS indicated the presence of unreacted alloc compound. Further portions of pyrrolidine (0.38 g, 0.44 mL, 5.3 mmol, 1.0 eq.) and Pd(PPh₃)₄ (61 mg, 0.053 mmol, 0.01 eq.) were added and the reaction was continued for a further 30 minutes. The solvent was evaporated under reduced pressure. Purification by flash column chromatography [gradient elution 100% chloroform then 98% chloroform/2% methanol to 90% chloroform/10% methanol in 1% increments] gave the product **50** as a white powder (1.37 g, 88%). Analytical Data: R_{T} 0.33 min; MS (ES⁺) *m*/*z* (relative intensity) 294 ([M + H]^{+.},60 ), MS (ES⁻) *m*/*z* (relative intensity) 292 ([*M*-H]^{-.}, 100).

### Compound 51

EEDQ (1.22 g, 4.93 mmol, 1.05 eq.) was added to a solution of amine dipeptide **(50)** (1.37 g, 4.69 mmol, 1 eq.) and m-dPeg®2 acid (0.73 g, 4.93 mmol, 1.05 eq.) in dry THF (60 mL). The solution was stirred at room temperature for 5 days. The solvent was evaporated under reduced pressure. The residue was purified by flash column chromatography [100% chloroform to 95% chloroform/5% methanol in 1 % increments] to give the product **51** as a white solid (1.46 g, 74%). Analytical Data: R_{T} 2.22 min; MS (ES⁺) *m*/*z* (relative intensity) 446 ([M + Na]^{+.},80 ), 424 ([M + H]^{+.},70), MS (ES⁻) *m*/*z* (relative intensity) 422 ([*M*- H]^{-.}*,* 100).

The synthesis of **51** is shown below in Scheme 14.

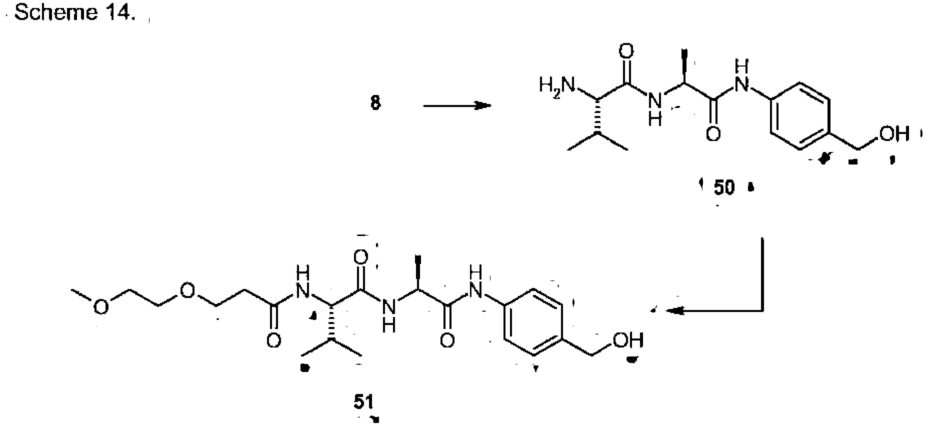

### Compound 52

Triethylamine (0.47 g, 0.65 mL 4.66 mmol, 2.2 eq.) was added to a stirred solution of the mono-alloc protected bis-aniline (6) (1.68 g, 2.12 mmol, 1 eq.) and triphosgene (0.226 g, 0.76 mmol, 0.36 eq.) in dry THF (40 mL) under a nitrogen atmosphere at room temperature. The reaction mixture was heated to 40°C, a sample was treated with methanol and analysed by LCMS as the methyl carbamate.

A solution of the dPeg®2-benzyl-alcohol (**51**) (1.35 g, 3.18 mmol, 1.5 eq.) and Triethylamine (0.32 g, 0.44 mL, 3.18 mmol, 1.5 eq.) in dry THF (60 mL) was added drop-wise to the freshly prepared isocyanate. The reaction mixture was monitored by LCMS at 30 minute intervals. , After 3 hours LC-MS showed conversion to product, the presence of methyl carbamate and mono-alloc protected *bis*-aniline **(9).** A further portion of triphosgene (0.056 g, 0.19 mmol,' 0.09 eq.) was added and the reaction continued at 40°C for a further 3 hours., The reaction mixture was evaporated to dryness to afford the crude product as a yellow oil which was purified by flash chromatography [gradient elution 100% chloroform to 95% chloroform)/5% methanol in 1% increments] to give the desired product **52** as a yellow foam (1.91 g, 73%). Analytical Data: R_{T} min 3.42 min MS (ES⁺) *m*/*z* (relative intensity) 1243 ([M + H]^{+.}, 50), MS (ES⁻) *m*/*z* (relative intensity) 1241 ([M - H])^{-.}, 100).

### Compound 53

Pd(PPh₃)₄ (35 mg, 3.0 x 10⁻⁵ mol 0.02 eq.) was added to a solution of the alloc compound **(52)** (1.87 g, 1.5 mmol, 1.0 eq.) and pyrrolidine (0.27 mg, 310 µL, 3.8 mmol, 2.5 eq.) in dry DCM (30 mL) under a nitrogen atmosphere. The solution was stirred at room temperature for 4 hours. The solvent was evaporated under reduced pressure and the product was purified by flash column chromatography [gradient elution 100% chloroform to 95% chloroform/5% methanol in 1 % increments] to give the product **53** yellow foam (1.57 g, 90%). Analytical Data: R_{T} min 3.17 min MS (ES⁺) *m*/*z* (relative intensity) 1159 ([M + H]^{+.}. 65).

### Compound 54

Triethylamine (0.26 g, 0.36 mL 2.6 mmol, 2.2 eq.) was added to a stirred solution of the mono-protected *bis*-aniline **(53)** (1.37 g, 1.18 mmol, 1 eq.) and triphosgene (0.126 g, 0.43 mmol, 0.36 eq.) in dry THF (40 mL) under a nitrogen atmosphere at room temperature. The reaction mixture was heated to 40°C, a sample was treated with methanol and analysed by LC-MS as the methyl carbamates indicating complete isocynate formation.

A solution of the benzyl alcohol **(8)** (0.67 g, 1.8 mmol, 1.5 eq.) and triethylamine (0.18 g, 0.25 mL, 1.8 mmol, 1.5 eq.) in dry THF (50 mL) was added drop-wise to the freshly prepared isocyanate. The reaction mixture was monitored by LCMS and was complete after 18 hours at 40°C. The reaction mixture was evaporated to dryness to afford a yellow oil which was purified by flash chromatography [gradient elution 95% ethylacetate/5% methanol to 93% ethylacetate/7% methanol in 1 % increments] to give the desired product **54** as a white foam (1.21 g, 66%). Analytical Data: R_{T} min 3.42 min MS (ES⁺) m/z (relative intensity) 1562 ([M + H]⁺, 15).

### Compound 55

A solution of K₂CO₃ (0.522 g, 3.78 mmol, 5 eq.) in H₂O (5.0 mL) was added to a solution of the acetate **(54)** (1.18 g, 0.756 mmol, 1 eq.) in methanol (29 mL). The reaction mixture was stirred at room temperature for 2 hours. The methanol was evaporated under reduced pressure, the residue was diluted with H₂O (50 mL) and extracted with ethyl acetate (3 x 100 mL). The combined ethyl acetate extracts were washed with H₂O (200 mL), brine (200 mL), dried (MgSO₄) and evaporated under reduced pressure to give the product **55** as a white foam (1.052 g, 94%). Analytical Data: R_{T} min 3.15 min MS (ES⁺) *m*/*z* (relative intensity) 1478 ([M + H]^{+.}, 5), MS (ES⁻) *m*/*z* (relative intensity) 1477 ([M - H])^{-.}, 100).

### Compound 56

Dess-Martin periodinane (0.152 g, 0.36 mmol, 2.1 eq.) was added in one portion to a solution of the bis deacetylated product **(55)** (0.252 g, 0.17 mmol 1 eq.) in dry DCM (5 mL) under a nitrogen atmosphere. The solution was stirred at room temperature for 2 hours at which time LCMS indicated that reaction was complete. The reaction mixture was diluted with DCM (50 mL) and washed with saturated aqueous sodium bicarbonate solution (3 x 100 mL), water (100 mL), brine (100 mL) and dried (MgSO₄). The solvent was removed by rotary evaporation under reduced pressure to give the crude product. Purification by flash column chromatography [gradient elution 100% chloroform to 92% chloroform/8% methanol in 1% increments] gave the product **56** as a white foam (0.17 g, 68%). Analytical Data: R_{T} min 6.17 min MS (ES⁺) *m*/*z* (relative intensity) 1474 ([M + H]^{+.}, 5), MS (ES⁻) *m*/*z* (relative intensity) 1472 ([M - H])^{-.}, 100).

### Compound 57

Pd(PPh₃)₄ (8 mg, 6.9 µmol, 6.0 eq.) was added to a solution of the alloc compound **(56)** (160 mg, 0.108 mmol, 1.0 eq.) and 0.5 M pyrrolidine solution in DCM (0.27 mL, 0.135 mmol, 1.25 eq.) in dry DCM (18 mL) under a nitrogen atmosphere. The solution was stirred at room temperature for 30 min. The solvent was evaporated under reduced pressure. Purification by flash column chromatography [gradient elution 100% chloroform to 91% chloroform/9% methanol in 1% increments] gave the product **57** as a white powder (0.114 g, 74%). Analytical Data: R_{T} min 2.60 min MS (ES⁺) *m*/*z* (relative intensity) 1390 ([M + H]^{+.}, 5).

Coupling of a maleimide-PEG-succinimide reagent with **57** provides the PBD drug-linker 58. Figure 1b shows the structures of PBD drug-linker MP-PEGB-Val-Ala-PAB-(imp)PBD **58** where MP is maleimidopropanamide, PEG is ethyleneoxy, and PAB is para-aminobenzyloxycarbonyl, and imp is the *N*-10 imine protecting group: 3-(2-methoxyethoxy)propanoate-Val-Ala-PAB.

*Compound **58** (MP-PEG8-Val-Ala-PAB-(imp)PBD; (11S,11aS)-4-((2S,5S)-37-(2,5-dioxo-2,5-dihydro-1H-pyrrol-1-yl)-5-isopropyl-2-methyl-4,7,35-trioxo-10,13,16,19,22,25,28,31-octaoxa-3,6,34-triazaheptatriacontanamido)benzyl 11-hydroxy-8-(5-((11S*,*11aS)11-hydroxy-10-((4-((10S*, *13S)*- *10-isopropyl-13-methyl-8,11-dioxo-2,5-dioxa-9,12-diazatetradecanamido)benzyloxy)carbonyl)-7-methoxy-2-methylene-5-oxo-2,3,5,10*,*11*,*11a-hexahydro-*pyrrolobenzo[2,1-c]*[1,4]diazepin-8-yloxy)pen*t*yloxy)*-*7-methoxy-2-methylene-5-oxo-2,3,11,11a*-*tetrahydro*-pyrrolobenzo[2,1-c]*[1,4]diazepine-10(5H)-carboxylate)* A 0.5 M solution of *N,N*-diisopropyldiethylamine in dry DCM (176 µL, 8.8 x 10⁻⁵ mol, 2.2 eq.) was added to a solution of amine dipeptide **(57)** (55 mg, 3.96 x 10⁻⁵ mol, 1.0 eq.) and maleimide-dPeg®8-NHS ester (33 mg, 4.75 x 10⁻⁵mol, 1.2 eq.) in dry DCM (6 mL). The solution was stirred at room temperature for 24 hours. The reaction mixture was evaporated under reduced pressure and the residue redissolved in DCM (50 mL). The DCM solution was extracted with saturated sodium hydrogen carbonate (2 x 100 mL), water (100 mL), brine (100 mL), dried (MgSO₄) and evaporated under reduced pressure to give a yellow gum. Purification by flash column chromatography [gradient elution chloroform to 91 % chloroform/9% methanol in 1% increments] gave the product **58** as a white foam (41 mg, 52%). Analytical Data: RT min 5.8 min. MS (MaldiTOF) *m*/*z* (relative intensity) 1987.9 ([M + Na]^{+.}, 100).

### Conjugate 60

Peptide biotin-A20FMDV-Cys **(59)** that is highly selective for the integrin αᵥβ₆, which is significantly up-regulated by many cancers, was selected for conjugation of the PBD-linker derivatives. A solution of the peptide **(59)** (7.7 mg, 3.08 µmol, 1.2 eq) in 1/1 acetonitrile/water (1 mL) was added to a solution of **(58)** (5.05 mg, 2.57 µmol, 1.0 eq) in 1/1 acetonitrile /water (2 mL). The solution was stirred at room temperature for 24 hours. The acetonitrile was evaporated under reduced pressure and the water was removed by lyophilisation to give the product **60** a white foam. Purification by semi-preparative HPLC followed by lyophilisation gave the product as a white foam (3.4 mg, 29%). Analytical Data: MS (MaldiTOF) *m*/*z* (relative intensity) 4458.3 ([M + H]^{+.}, 100).

*Compound **61** (MP-PEG24-Val-Ala-PAB-(imp) PBD; (11**S**,11a**S**)-4-((2**S**,*5***S**)*-*16*-*(2,5*-*dioxo-2,5-dihydro-1H-pyrrol-1-yl)-5-isopropyl-2-methyl-4,7,14-trioxo-10-oxa-3,6,13-triazahexadecanamido)benzyl 11-hydroxy-8-((5-(((11**S**,11a**S)**-11-hydroxy-10-(((4-((10**S**,13**S**)-10-isopropyl-13-methyl-8,11-dioxo-2,5-dioxa-9,12-diazatetradecanamido)benzyl)oxy)carbonyl)-7-methoxy-2-methylene-5-oxo-2,3,5,10*, *11*,*11a-hexahydro-1H-pyrrolo[2,1-c][1,4]benzodiazepin-8-yl)oxy)pentyl)oxy)-7-methoxy-2-methylene-5-oxo-2,3,11,11a*-*tetrahydro*-*1H*-*pyrrolo[2*,*1-c][1*,*4]benzodiazepine*-*10(5H)-carboxylate)*
NN-diisopropyldiethylamine (6 µL, 4.3 x 10⁻⁵ mol, 2.2 eq) was added to a solution of amine dipeptide (57) (27 mg, 1.94 x 10⁻⁵ mol, 1 eq) and maleimide-dPeg®24-NHS ester (30 mg, 2.13 x 10⁻⁵ mol, 1.1 eq) in dry DCM (5 mL). The solution was stirred at room temperature for 24 hours. The reaction mixture was evaporated under reduced pressure and the residue redissolved in DCM (25 mL). The DCM solution was extracted with saturated sodium hydrogen carbonate (2 x 50 mL), water (50 mL), brine (50 mL), dried (MgSO₄) and evaporated under reduced pressure to give a yellow gum. Purification by flash column chromatography [gradient elution chloroform to 91 % chloroform/9% methanol in 1% increments] gave the product as a colourless gum (22 mg, 42%). Analytical Data: MS (MaldiTOF) *m*/*z* (relative intensity) 2691.8 ([M + H]^{+.}, 100).

### (2S,2'S,E)-dimethyl 1,1'-(4,4'-(propane-1,3-diylbis(oxy)bis(5-methoxy-2-nitrobenzoyl))bis(4-((E)-prop-1-en-1-yl)-2,3-dihydro-1H-pyrrole-2-carboxylate)(71)

Tetrakis(triphenylphosphine)palladium(0) (21.6mg) was added to triflate **40** (230 mg), *trans-*propenylboronic acid (52.3 mg) and sodium carbonate (129 mg) in a mixture of toluene (5 mL), ethanol (2.5 mL) and water (2.5 mL). The reaction mixture was allowed to stir for 3 hours under an argon atmosphere at 32°C. The reaction mixture was diluted with ethyl acetate and washed with water, brine and dried over magnesium sulphate. After filtration excess ethyl acetate was removed by rotary evaporation under reduced pressure. The crude coupling product was purified by flash column chromatography (silica gel; gradient 50%/50% ethyl acetate/hexane to 80%/20% ethyl acetate/hexane). Pure fractions were combined and removal of excess eluent afforded the pure product **71** as an orange solid (110 mg, 61.4 % yield, LC/MS 3.52 mins, *m*/*z* ES⁺764.92). The reaction was repeated on a larger scale to afford 7.21 g of the Suzuki coupling product. ¹H NMR (400 MHz, CD₃OD). δ 7.78 (s, 2H) 6.92 (s, 2H), 5.98 (d, 2H), 5.89 (s, 2H), 5.46-5.55 (m, 2H), 5.10 (dd, 2H), 4.37 (t, 4H), 3.93-4.00 (m, 6H), 3.86 (s, 6H), 3.19-3.26 (m,2H), 2.80 (dd, 2H), 2.45-2.51 (m, 2H), 1.77 (d, 6H OCH₃).

### (S,E)-((propane-1,3-diylbis(oxy))bis(5-methoxy-2-nitro-4,1-phenylene))bis(((S)-2-(hydroxymethyl)-4-((E)-prop-1-en-1-yl)-2,3-dihydro-1H-pyrrol-1-yl)methanone) (72)

The *bis*-ester **71** (7.21 g) was added in one portion as a solid to a solution of lithium borohydride (622 mg) in dry tetrahydrofuran (300 mL), at 0°C (ice bath). The ice bath was removed and the reaction mixture allowed to reach room temperature. After **1** hour, TLC (following mini work up with ethyl acetate water) revealed that the reaction was not complete and so additional lithium borohydride (0.75 equivalents) was added. The reaction mixture was allowed to stir for a further 2.5 hours at which time TLC (following mini work up) revealed the reaction to be complete. Remaining lithium borohydride was quenched with a large excess of ethyl acetate (ice bath) and the reaction mixture was allowed to stir for 50 mins. The organic phase was washed with water, brine and dried over magnesium sulphate. Magnesium sulphate was removed by vacuum filtration and the ethyl acetate removed by rotary evaporation under reduced pressure to afford the diol **72** (5.46 g, 82 % yield) which was used in the next reaction without further purification (LC/MS 3.17 mins, *m*/*z* ES⁺ 708.84). ¹H NMR (400 MHz, CD₃OD). δ 7.78 (s, 2H) 6.85 (s, 2H), 5.97 (d, 2H), 5.77 (s, 2H), 5.61-5.53 (m, 2H), 4.75-4.82 (m, 2H), 4.38 (t, 4H), 3.89-4.00 (m, 12H), 3.01-3.08 (m, 2H) 2.46-2.51 (m, 4H), 1.77 (d, 6H OCH₃).

### ((2S,2'S,E)-1,1-(4,4'-(propane-1,3-diylbis(oxy))bis(5-methoxy-2-nitrobenzoyl))bis(4-((E)-prop-1-en-1-yl)-2,3-dihydro-1H-pyrrole-2,1-diyl)bis(methy/ene) diacetate (73)

A solution of acetyl chloride (1.64 mL) in dry dichloromethane (40 mL) was added dropwise to a solution of the bis alcohol **72** (6.2 g) in dichloromethane (200 mL) in the presence of triethylamine (3.68 mL) at 0°C. The reaction mixture was allowed to warm to room temperature and the reaction was monitored by TLC and LC/MS. Once reaction was complete the organic phase was washed sequentially with water, citric acid (0.5 N), saturated sodium bicarbonate and brine. The organic layer was dried over magnesium sulphate, filtered and excess dichloromethane removed by rotary evaporation under reduced pressure. The residue was subjected to column chromatography (silica gel; gradient, 60% ethyl acetate/ 40% hexane to 70% ethyl acetate/30 % hexane). Pure fractions were combined and removal of excess eluent afforded the *bis*-acetate **73** (2.50 g, 36% yield, LC/MS 3.60 mins, m/z ES⁺ 792.63). ¹H NMR (400 MHz, CDCl₃) δ 7.77 (d, *J* = 3.4 Hz, 2H), 6.89 (s, 2H), 5.99 (d, *J* = 15.2 Hz, 2H), 5.78 (s, 2H), 5.65 - 5.45 (m, *J* = 15.4, 6.8 Hz, 2H), 5.02 - 4.86 (m, *J* = 9.7, 5.5 Hz, 2H), 4.57 (s, 2H), 4.37 (t, *J* = 5.9 Hz, 4H), 4.00 (s, 6H), 3.10 -2.92 (m, *J =* 10.7 Hz, 2H), 2.60 (dd, *J* = 16.3, 3.1 Hz, 2H), 2.52 - 2.43 (m, 2H), 2.10 (s, 6H), 1.78 (d, *J* = 6.7 Hz, 4H).

### ((2S,2'S,E)-1,1'-(4,4'-(propane-1,3-diy/bis(oxy))bis(2-amino-5-methoxybenzoyl))bis(4-((E)-prop-1-en-1-yl)-2,3-dihydro-1H-pyrro/e-2,1-diyl))bis(methylene) diacetate (74)

Zinc powder (10 g) was added to a solution of bis-nitro compound **73** (2.5 g) in ethanol (20 mL) and ethyl acetate (20 mL), followed by a solution of formic acid in ethanol (5% v/v; 100 mL). The reaction was exothermic with the temperature rising to 33°C, the temperature was brought down to 15°C with an ice bath and the reaction mixture was allowed to stir whilst being closely monitored by TLC and LC/MS. After 30 mins, the reaction was deemed complete as no trace of starting material, or intermediates were detected. The mixture was decanted and filtered through cotton wool. The filtrate was partitioned between ethyl acetate (300 mL) and saturated aqueous NaHCO₃ (300 mL). The organic layer was further washed with brine (200 mL) and dried over magnesium sulphate. Excess solvents were removed by rotary evaporation under reduced pressure to afford the product **74** (2.09 g; 90 % yield, LC/MS 3.35 mins, m/z ES⁺ 732.06). ¹H NMR (400 MHz, CDCl₃) δ 6.76 (s, 2H), 6.45 (s, 2H), 6.33 (s, 2H), 6.12 (d, *J* = 15.3 Hz, 2H), 5.54 (dq, *J* = 13.2, 6.6 Hz, 2H), 4.90 (td, *J* = 9.6, 4.5 Hz, 2H), 4.48 (s, 4H), 4.42 - 4.33 (m, 4H), 4.23 (t, *J* = 6.1 Hz, 4H), 3.79 (s, 6H). 2.95 (dd, *J* = 16.0, 10.4 Hz, 2H), 2.55 (dd, *J* = 16.2. 3.5 Hz, 2H), 2.42 - 2.32 (m, 2H), 2.07 (s, 6H), 1.81 (d, *J* = 6.6 Hz, 6H).

### ((S)-1-(4-(3-(4-((S)-2-(acetoxymethyl)-4-((E)-prop-1-en-1-yl)-2,3-dihydro-1H-pyrrole-1-carbonyl)-5-(((allyloxy)carbonyl)amino)-2-methoxyphenoxy)propoxy)-2-amino-5-methoxybenzoyl)-4-((E)-prop-1-en-1-yl)-2,3-dihydro-1H-pyrrol-2-yl)methyl (75)

A solution of allyl chloroformate in dry dichloromethane was added drop-wise to a solution of the bis-aniline **74** and pyridine in dry dichloromethane at -78°C. The reaction mixture was allowed to stir at -78°C for 2 hours and then allowed to return to room temperature. The reaction mixture was washed sequentially with aqueous copper II sulphate, water, saturated sodium bicarbonate and brine. The organic layer was dried over magnesium sulphate, filtered under vacuum and excess dichloromethane was removed by rotary evaporation under reduced pressure. TLC and LC/MS revealed the presence of both the desired mono Alloc product **75** and the *bis-*Alloc product. The product mixture was subjected to column chromatography (silica gel; gradient 40% ethyl acetate/60% hexane to 70% ethyl acetate/ 40% hexane). Pure fractions containing the desired mono Alloc product **75** were collected and combined, excess eluent was removed by rotary evaporation under reduced pressure to afford the product (580 mL, 25 % yield). LC/MS 3.58 mins, ES⁺ 817.02 ¹H NMR (400 MHz, CDCl₃) δ 8.60 (s, 1H), 7.85 (s, 1H), 6.80 (s, 1H), 6.72 (s, 1H), 6.42 (s, 1H), 6.37 (s, 1H), 6.33 (s, 1 H), 6.08 (dd, **J** = 15.4, 6.1 Hz, 2H), 6.00 - 5.87 (m, 1 H), 5.62 - 5.44 (m, 2H), 5.34 (dd, **J** = 17.2, 1.4 Hz, 1H), 5.23 (dd, **J =** 10.4, 1.2 Hz, 1H), 4.88 (qd, **J =** 9.5, 4.5 Hz, 2H), 4.67-4.57 (m, 2H), 4.50 - 4.25 (m, 8H), 4.22 (t, **J** = 6.3 Hz, 2H), 3.82 (s, 3H), 3.76 (s, 3H), 3.00-2.85 (m, 2H), 2.58 - 2.47 (m, 2H), 2.37 (p, **J** = 6.1 Hz, 2H), 2.06 (s, 6H), 1.81 - 1.73 (m, 6H).

### ((2S)-1-(4-(3-(4-((S)-2-(acetoxymethyl)-4-((E)-prop-1-en-1-yl)-2,3-dihydro-1H-pyrrole-1-carbonyl)-5-((((4-(2-(2-(((allyloxy)carbonyl)amino)-3-methylbutanamido)propanamido)benzyl)oxy)carbonyl)amino)-2-methoxyphenoxy)propoxy)-2-(((allyloxy)carbonyl)amino)-5-methoxybenzoyl)-4-((E)-prop-1-en-1-yl)-2,3-dihydro-1H-pyrrol-2-yl)methyl acetate (76)

Dry triethylamine (0.206 mL) was added to a stirred solution of the mono-alloc protected *bis-*aniline **75** (560 mg) and triphosgene (72 mg) in dry tetrahydrofuran (20 mL) under an inert atmosphere. The reaction mixture was heated at 40°C and a sample was removed and treated with methanol. LC/MS revealed complete conversion to the methyl carbamate indicating that the free amine group had been successfully converted to the reactive isocyanate intermediate. A solution of the alloc-val-ala-PABOH (381 mg) and triethylamine (0.14 mL) in dry tetrahydrofuran (20 mL) was rapidly injected into the reaction vessel at 40°C. The reaction mixture was allowed to stir at room temperature over night after which time a sample was removed and treated with methanol. LC/MS revealed no trace of methyl carbamate indicating that all the isocyanate had been consumed. The reaction mixture was evaporated to dryness to afford the crude product which was purified by column chromatography (silica gel; gradient chloroform to 2% methanol/ 98% chloroform). Pure fractions were collected and combined and removal of excess eluent by rotary evaporation under reduced pressure afforded the pure product **76** (691 mg, 84 % yield). LC/MS 3.73 mins, ES⁺1220.21.

### Allyl 4-(2-(2-(((allyloxy)carbonyl)amino)-3-methylbutanamido)propanamido)benzyl ((S,E)-(propane-1,3-diylbis(oxy))bis(2-((S)-2-(hydroxymethyl)-4-((E)-prop-1-en-1-yl)-2,3-dihydro-1H-pyrrole-1-carbonyl)-4-methoxy-5,1-phenylene))dicarbamate (77)

An aqueous solution of potassium carbonate (770 mg in 4.8 mL water) was added to a solution of the bis-acetate **76** (680 mg) in methanol (29 mL) at room temperature. The deacetylation was complete within 30 mins as monitored by LC/MS. The reaction mixture was diluted with dichloromethane (200 mL) and the organic phase washed sequentially with citric acid (0.5 N, 100 mL), water (200 mL) and brine (100 mL). The organic phase was dried over magnesium sulphate, the suspension was filtered (vacuum filtration) and excess solvent removed by rotary evaporation under reduced pressure. The residue was subjected to column chromatography (silica gel; gradient 1.5% methanol / 98.5% chloroform to 3.5% methanol 96.5% chloroform). Pure fractions were combined and removal of excess eluent by rotary evaporation under reduced pressure afforded the diol **77** (530 mg, 84% yield). LC/MS 3.40 mins, ES⁺ 1136.49.

### (11S, 11aS)-allyl8-(3-(((11S, 11aS)-10-(((4-((S)-2-((S)-2-(((allyloxy)carbonyl)amino)-3-methylbutanamido)propanamido)benzyl)oxy)carbonyl)-11-hydroxy-7-methoxy-5-oxo-2-((E)-prop-1-en-1-yl)-5, 10, 11,11a-tetrahydro-pyrrolo[2,1-c][1,4]benzodiazepin-8-yl)oxy)propoxy)-11-hydroxy-7-methoxy-5-oxo-2-((E)-prop-1-en-1-yl)-11, 11a-dihydro-pyrrolo[2,1-c][1,4]benzodiazepine-10(5H)-carboxylate (78)

Dess-Martin periodinane (373 mg, 4 eq.) was added in one portion to a solution of 77 (250 mg) and pyridine (0.36 mL, 20 eq.) in dry dichloromethane (10 mL) at room temperature. Close monitoring by TLC (5 % methanol/ chloroform) revealed the disappearance of starting material after 30 minutes. The reaction was worked up with a solution of sodium metabisulphite and sodium hydrogen carbonate, followed by brine. The dichloromethane layer was dried over magnesium sulphate and vacuum filtered. The dichloromethane solution was then treated with a catalytic amount of DMAP (c. 10 mg), causing the main product spot to coalesce into one as observed by TLC/LC/MS. The solution was filtered and the dichloromethane removed by rotary evaporation under reduced pressure. The resulting residue was subjected to column chromatography (silica gel; gradient 1.5% methanol/ 98.5% chloroform to 3% methanol/ 97% chloroform). Pure fractions were collected and removal of eluent by rotary evaporation under reduced pressure afforded the desired cyclised product **78** (62 mg, 25 % yield). LC/MS 3.35 mins, ES⁺1132.19, ES⁻1130.25.

### (11S,11aS)-4-((S)-2-((S)-2-amino-3-methylbutanamido)propanamido)benzyl 11-hydroxy-7-methoxy-8-(3-(((S)-7-methoxy-5-oxo-2-((E)-prop-1-en-1-yl)-5,11a-dihydro-pyrrolo[2, 1-c-][1,4]d benzoiazepin-8-yl)oxy)propoxy)-5-oxo-2-((E)-prop-1-en-1-yl)-11,11a-dihydro-pyrrolo[2,1-c][1,4]benzodiazepine-10(5H)-carboxylate (79)

Pd(PPh₃)₄ (1.9 mg) was added to a solution of the alloc compound (**78**) (62 mg) and pyrrolidine (22.6 µL) in dry DCM (3 mL) under an argon atmosphere. The solution was stirred at room temperature for 1.5 hours. The solvent was evaporated under reduced pressure. Purification by flash column chromatography [gradient elution 3% methanol /97% chloroform to /90% chloroform 10% methanol] gave the product as a white powder (26 mg, 50%). LC/MS: RT 2.70 min MS (ES⁺) 946.17.

### (11S, 11aS)-4-((2S,5S)-25-(2,5-dioxo-2,5-dihydro-1H-pyrrol-1-yl)-5-isopropyl-2-methyl-4,7,23-trioxo-10,13,16,19-tetraoxa-3,6,22-triazapentacosanamido)benzyl 11-hydroxy-7-methoxy-8-(3-(((S)-7-methoxy-5-oxo-2-((E)-prop-1-en-1-yl)-5,11a-dihydro-pyrrolo[2,1-c][1,4]benzodiazepin-8-yl)oxy)propoxy)-5-oxo-2-((E)-prop-1-on-1-yl)-11,11a-dihydro-pyrrolobenzo[2,1-c][1,4]diazepine-10(5H)-carboxylate (80)

A solution of *N,N*-diisopropyldiethylamine i(2.6 µL) was added to a solution of amine dipeptide **79** (13 mg) and maleimide-dPeg®4-NHS ester (8.5mg), in dry DCM (4 mL) The solution was stirred at room temperature for 24 h. The reaction mixture was evaporated under reduced pressure and the residue subjected to semi-preparative TLC (10% methanol/90% chloroform) to afford a pure sample of the desired maleimide 14. LC-MS retention time 2.87 min ES⁺ 1344.29.

### Boc-Val-Cit-PABOH (82)

A solution of Boc-Vai-OSu (10.0 g, 31.8 mmol, 1 eq.) in THF (50 mL) was added to a solution of H-Cit-OH (5.85 g. 33.4 mmol, 1.05 eq.) and NaHCO₃ (2.94 g, 34.9 mmoL, 1.1 eq.) in THF (50 mL) and H₂O (100 m L). The mixture was stirred at room temperature for 72 hours and the THF was evaporated under reduced pressure. The pH was adjusted to 3 with citric acid to precipitate a white gum. This was extracted with 10% IPA/ethylacetate (8 x 150 mL), the combined extracts were washed with brine (300 mL) and dried (MgSO₄). Evaporation under reduced pressure gave a white foam which was dried under reduced pressure for 18 hours. The foam was suspended in ether with sonication followed by filtration to give the product as a fine white powder (10.6 g, 89%). A portion of this material (7.2 g, 19.2 mmol, 1 eq), *p*-aminobenzyl alcohol (2.6 g, 21.15 mmol, 1.1 eq.) and EEDQ (9.5 g, 38.5 mmol, 2.0 eq.) in DCM/MeOH (100 mL/50 mL) were stirred at room temperature for 24 hours. The solvent was evaporated under reduced pressure and the residual gum was triturated with ether with sonication, the resulting product was collected by filtration and dried under reduced pressure to give the product **82** as a white solid (6.6 g, 71%). Analytical Data: RT 2.42 min; MS (ES⁺) *m*/*z* (relative intensity) 479.8 ([*M* + 1]⁺, 60), MS (ES⁻) *m*/*z* (relative intensity) 477.6 ([M - H])⁻. 90).

The synthesis of compound **82** is shown in scheme 16 below.

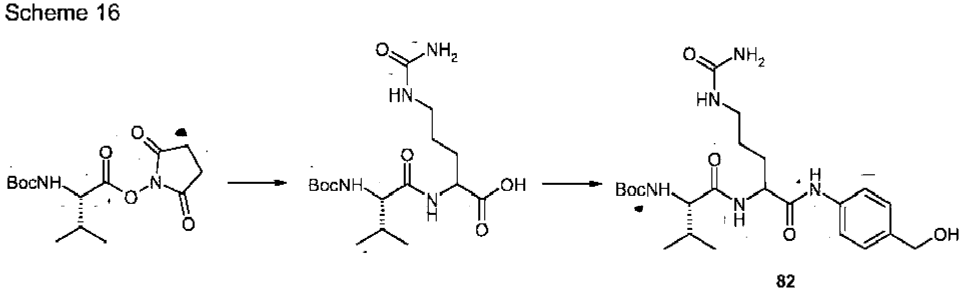

### ((S)-1-(4-((5-(4-((S)-2-(acetoxymethyl)-4-methylenepyrrolidine-1-carbonyl)-5-((((4-((S)-2-((S)-2-((tert-butoxycarbonyl)amino)-3-methylbutanamido)-5-ureidopentanamido)benzyl)oxy)carbonyl)amino)-2-methoxyphenoxy)pentyl)oxy)-2 (((allyloxy)carbonyl)amino)-5-methoxybenzoyl)-4-methylenepyrrolidin-2-yl)methyl acetate (83)

Triethylamine (0.14 g, 0.19 mL 1.4 mmol, 2.2 eq.) was added to a stirred solution of the mono-alloc protected bis-aniline **(6)** (0.505 g, 0.64 mmol, 1 eq.) and triphosgene (0.068 g, 0.23 mmol, 0.36 eq.) in dry THF (10 mL) under an argon atmosphere at room temperature. The reaction mixture was heated to 40°C, a sample was treated with methanol and analysed by LCMS as the methyl carbamate.

A solution of the benzyl alcohol **(82)** (0.46 g, 0.96 mmol, 1.5 eq.) and triethylamine (0.096 g, 0.13 mL, 0.96 mmol, 1.5 eq.) in dry THF/DMF (20 mL/1 mL) was added drop-wise to the freshly prepared isocyanate. The reaction mixture was monitored by LC-MS and was complete after 2 hours at 40°C. The reaction mixture was evaporated to dryness and the residue partitioned between 10% IPA/DCM and water. The organic portion was separated and washed with water (100 mL), brine (100 mL), dried (MgSO₄) and evaporated under reduced pressure to give a brown foam. Purification by flash column chromatography [gradient elution chloroform to 93% chloroform/7% methanol in 1% increments] gave the product as a white solid (0.5 g, 60%). Analytical Data: RT 3.42 min; MS (ES⁺) *m*/*z* (relative intensity) 1298 ([*M*+H]⁺, 100).

### Allyl 4-((S)-2-((S)-2-((tert-butoxycarbonyl)amino)-3-methylbutanamido)-5-ureidopentanamido)benzyl ((S)-(pentane-1,5-diylbis(oxy))bis(2-((S)-2-(hydroxymethyl)-4-methylenepyrrolidine-1-carbonyl)-4-methoxy-5,1-phenylene))dicarbamate (84)

A solution of K₂CO₃ (0.28 g, 2.0 mmol, 5.4 eq.) in H₂O (2 mL) was added to a solution of the acetate **(83)** (0.49 g, 0.4 mmol, 1 eq.) in methanol (10 mL). The reaction mixture was stirred at room temperature for 2 hours. The methanol was evaporated under reduced pressure, the residue was diluted with H₂O (10 mL) and acidified to pH3 with 1M citric acid. The mixture was extracted with DCM (4 x 50 m L) and the combined extracts were washed with brine (100 mL), dried (MgSO₄) and evaporated under reduced pressure to give the product as a white foam (0.43 g, 94%). Analytical Data: RT 3.12 min; MS (ES⁺) *m*/*z* (relative intensity) 1214 ([*M* + H]⁺, 100), MS (ES⁻) *m*/*z* (relative intensity) 1212 ([M - H])^{-.}, 100).

### (11S, 11aS)-allyl 8-((5-(((11S, 11aS)-10-(3-(4-((S)-2-((S)-2-((tert-butoxycarbonyl)amino)-3-methylbutanamido)-5-ureidopentanamido)phenyl)propanoyl)-11-hydroxy-7-methoxy-2-methylene-5-oxo-2,3,5,10,11,11a-hexahydro-1H-pyrrolo[2,1-c][1,4]benzodiazepin-8-yl)oxy)pentyl)oxy)-11-hydroxy-7-methoxy-2-methylene-5-oxo-2,3,11,11a-tetrahydro-1H-pyrrolo[2,1-c][1,4]benzodiazepine-10(5H)-carboxylate (85)

Stabilised 45 wt% 2-iodoxybenzoic acid (IBX) (0.18 g, 0.29 mmol, 2.4 eq.) was added in one portion to a solution of the bis deacetylated product **(55)** (0.147 g, 0.12 mmol, 1 eq.) in dry DMSO (4 mL) under a nitrogen atmosphere. The solution was stirred at room temperature for 26 hours. A further portion of IBX (15 mg, 2.4 x 10⁻⁵, 0.2 eq) was added and the reaction was continued for a further 18 hours. The reaction mixture was diluted with H₂O (10 mL), extracted with 10%MeOH/DCM (4 x 25 mL) and the combined extracts were washed with saturated aqueous sodium bicarbonate solution (2 x 100 mL), water (100 mL), brine (100 mL) and dried (MgSO₄). The solvent was removed by rotary evaporation under reduced pressure to give the crude product. Purification by flash column chromatography [gradient elution 100% dichloromethane to 94% dichloromethane/6% methanol in 1% increments] gave the product **85** as a white solid (77 mg, 53%). Analytical Data: RT 2.98 min; MS (ES⁺) *m*/*z* (relative intensity) 1210 ([*M* + H]⁺, 100), MS (ES⁻) *m*/*z* (relative intensity) 1208 ([M - H])⁻, 100).

### (11S, 11aS)-allyl 8-((5-(((11S, 11aS)-10-(((4-((S)-2-((S)-2-amino-3-methylbutanamido)-5-ureidopentanamido)benzyl)oxy)carbonyl)-11-hydroxy-7-mathoxy-2-methylene-5-oxo-2,3,5, 10,11,11a-hexahydro-1H-pyrrolo[2,1-c][1,4]benzodiazepin-8-yl)oxy)pentyl)oxy)-11-hydroxy-7-methoxy-2-methylene-5-oxo-2,3,11,11a-tetrahydro-1H-pyrrolo[2,1-c][1,4]benzodiazepine-10(5H)-carboxylate (86)

Cold trifluoroacetic acid (3 mL) was added to the Boc protected compound **(85)** (72 mg, 6.0 x 10⁻⁵ mol) at 0°C. The solution was stirred at this temperature for 15 minutes. The reaction mixture was poured onto ice and the pH was adjusted to pH 8 with saturated NaHCO₃ solution. The solution was extracted with DCM (4 x 25 mL) and the combined extracts were washed with saturated brine (100 mL), dried (MgSO₄) and evaporated under reduced pressure to give the product as a white solid (55 mg, 83%). Analytical Data: RT 2.53 min; MS (ES⁺) *m*/*z* (relative intensity) 1110 ([*M* + H]⁺, 100), MS (ES⁻) *m*/*z* (relative intensity) 1108 ([M-H])⁻,100).

### (11S,11aS)-4-((S)-2-((S)-2-amino-3-methylbutanamido)-5-ureidopentanamido)benzyl 11-hydroxy-7-methoxy-8-((5-(((S)-7-methoxy-2-methylene-5-oxo-2,3,5,11a-tetrahydro-1H-pyrrolo[2,1-c][1,4]benzodiazepin-8-yl)oxy)pentyl)oxy)-2-methylene-5-oxo-2,3,11,11a-tetrahydro-1H-pyrrolo[2,1-c][1,4]benzodiazepine-10(5H)-carboxylate (86)

Pd(PPh₃)₄ (2.7 mg, 2.3 µmol, 0.03 eq.) was added to a solution of the alloc compound **(85)** (80 mg, 72 µmol, 1.0 eq.) and pyrrolidine (30 µL, 26 mg, 0.36 mmol, 5 eq.) in dry DCM (3 mL) under a nitrogen atmosphere. The solution was stirred at room temperature for 2 hours. The solvent was evaporated under reduced pressure. Purification by flash column chromatography [gradient elution 90% chloroform/10% methanol to 76% chloroform/24% methanol] gave the product as a white powder (62.5 g, 86%), Analytical Data: RT 2.45 min ' MS (ES⁺) m/z (relative intensity) 1008 ([M + H]⁺, 80).

### (11S, 11aS)-4-((S)-2-((S)-2-(6-(2,5-dioxo-2,5-dihydro-1H-pyrrol-1-yl)hexanamido)-3-methylbutanamido)-5-ureidopentanamido)benzyl 11-hydroxy-7-methoxy-8-((5-(((S)-7-methoxy-2-methylene-5-oxo-2,3,5,11a-tetrahydro-1H-pyrrolo[2,1-c][1,4]benzodiazepin-8-yl)oxy)pentyl)oxy)-2-methylene-5-oxo-2,3,11,11a-tetrahydro-1H-pyrrolo[2,1-c][1,4]benzodiazepine-10(5H)-carboxylate (87)

*N,N-*diisopropyldiethylamine (12 µL, 7.1 x 10⁻⁵ mol, 5.0 eq) was added to a solution of amine dipeptide **(86)** (14.2 mg, 1.4 x 10⁻⁵ mol, 1 eq) and 6-maleimide-hexanoic acid-NHS ester (4.8 mg, 1.55 x 10⁻⁵ mol, 1.1 eq) in dry DCM/DMA (2 mL/0.2 mL) under an argon atmosphere The solution was stirred at room temperature for 72 hours. The reaction mixture was evaporated under reduced pressure and the residue purified by flash column chromatography [gradient elution chloroform to 93% chloroform/7% methanol in 1% increments] to give the product as an off-white foam (5 mg, 29%). Analytical Data: RT 2.83 min MS (ES⁺) *m*/*z* (relative intensity) 1201 ([M + H]⁺, 100).

### Reduction/Oxidation of ThioMabs for Conjugation

Full length, cysteine engineered monoclonal antibodies (ThioMabs) expressed in CHO cells were reduced with about a 20-40 fold excess of TCEP (tris(2-carboxyethyl)phosphine hydrochloride or DTT (dithiothreitol) in 50 mM Tris pH 7.5 with 2 mM EDTA for 3 hrs at 37 °C or overnight at room temperature.(Getz et al (1999) Anal. Biochem. Vol 273:73-80; Soltec Ventures, Beverly, MA). The reduced ThioMab was diluted and loaded onto a HiTrap S column in 10 mM sodium acetate, pH 5, and eluted with PBS containing 0.3M sodium chloride. Alternatively, the antibody was acidified by addition of 1/20^{th} volume of 10% acetic acid, diluted with 10 mM succinate pH 5, loaded onto the column and then washed with 10 column volumes of succinate buffer. The column was eluted with 50 mM Tris pH7.5, 2 mM EDTA.

The eluted reduced ThioMab was treated with 200 nM aqueous copper sulfate (CuSO₄) or 15 fold molar excess of DHAA (dehydroascorbic acid) Oxidation of the interchain disulfide bonds was complete in about three hours or more. Ambient air oxidation was also effective. The re-oxidized antibody was dialyzed into 20 mM sodium succinate pH 5,150 mM NaCl, 2 mM EDTA and stored frozen at -20 °C.

### Conjugation of ThioMabs with drug-linker compounds to prepare antibody-drug conjugates

The reoxidized ThioMabs as described above, were combined with a 2.5 to 10 fold excess of drug-linker intermediate **(15ba, 15bb, 15d, 58)** mixed, and let stand for about an hour at room temperature to effect conjugation and form the ThioMab antibody-drug conjugates **101-115** in Table 1. The conjugation mixture was purified by gel filtration, cation exchange chromatography, or dialysis to remove excess drug-linker intermediate and other impurities.

**Table 1**

| ADC | ADC (Ab-drug/linker) | Drug-linker Compound | DAR (drug to antibody ratio) | Figures |
|---|---|---|---|---|
| **101** | Tr-MP-PEG8-Phe-Lys-PAB-PBD | **15bb** | 1.3 | 2,3,4 |
| **102** | antiCD22-MP-PEG8-Phe-Lys-PAB-PBD | **15bb** | 1.2 | 2,3 |
| **103** | Tr-MP-PEG4-Phe-Lys-PAB-PBD | **15ba** | 1.37 | 2,3,4 |
| **104** | antiCD22-MP-PEG4-Phe-Lys-PAB-PBD | **15ba** | 1.2 | 2,3 |
| **105** | trastuzumab-MP-pEG8-Phe-Lys-PAB-PBD | **15bb** | 1 | 6 |
| **106** | trastuzumab-MP-PEG4-Phe-Lys-PAB-PBD | **15ba** | 1.1 | 6 |
| **107** | antiSteap1-MP-PEG8-Phe-Lys-PAB-PBD | **15bb** | 0.5 | 4,6 |
| **108** | antiSteap1-MP-PEG4-Phe-Lys-PAB-PBD | **15ba** | 1.5 | 4,6 |
| **109** | antiSteap1-MP-PEG8-Val-Ala-PAB-(imp)PBD | **58** | 1.75 | 5 |
| **110** | antiCD22-MP-PEG8-Val-Ala-PAB-(imp)PBD | **58** | 1.8 | 5 |
| **111** | antiSteap1-MP-PEG8-Val-Ala-PAB-PBD | **15d** | 1.8 | |
| **112** | gD5B60-MP-PEG8-Val-Ala-PAB-PBD | **15d** | 1.85 | |
| **113** | gD5B60-MP-PEG8-Val-Ala-PAB-(imp)PBD | **58** | 1.9 | |
| **114** | trastuzumab-MP-PEG8-Val-Ala-PAB-PBD | **15d** | 1.7 | |
| **115** | trastuzumab-MP-PEG8-Val-Ala-PAB-(imp)PBD | **58** | 1.8 | |

| | | | | |
|---|---|---|---|---|
| Tr = thio trastuzumab, anti HER2, 4D5 HC A118C (Sequential numbering), A114C (Kabat numbering) imp = *N*-10 imine protected: 3-(2-methoxyethoxy)propanoate-Val-Ala-PAB- | | | | |

In particular, drug-linker intermediate **15d** (MW 1496.65) was solubilized in DMA (dimethylacetamide) to a concentration of 20 mM. Re-oxidized, cysteine engineered H118C trastuzumab antibody (Tr) was thawed and a 3 fold molar excess of **15d** was added. The reaction was carried out at pH 5 after experiments showed increased antibody aggregation at higher pH. The extent of drug conjugation was monitored by LC-MS analysis. An additional 1-fold equivalent of **15d** was added after 3 hrs and the reaction was allowed to proceed overnight at 4°C to give crude ADC **114.**

The antibody-drug conjugate, trastuzumab-MP-PEG8-Val-Ala-PAB-PBD **114,** was then applied to a cation exchange column after dilution with succinate, washed with at least 10 column volumes of succinate and eluted with PBS. The antibody-drug conjugate **114** was formulated into 20 mM His/acetate pH 5, 240 mM sucrose using gel filtration columns. The-antibody-drug conjugate **114** was characterized by UV spectroscopy to determine protein concentration, analytical SEC (size-exclusion chromatography) for aggregation analysis and LC-MS before and after reduction to determine drug loading.

Size exclusion chromatography was performed using a Shodex KW802.5 column in 0.2M potassium phosphate pH 6.2 with 0.25 mM potassium chloride and 15% IPA at a flow rate of 0.75 ml/min. Aggregation state of the conjugate was determined by integration of eluted peak area absorbance at 280 nm. SEC analysis showed 4.1% by integrated area of aggregated ADC at 8.08 min and 95.9% monomeric ADC **114** at 8.99 min.

LC-MS analysis was performed using an Agilent QTOF 6520 ESI instrument. As an example, **114** trastuzumab-MP-PEG8-Val-Ala-PAB-(imp)PBD was reduced with DTT (dithiothreitol) and loaded onto a 1000 A, 8µm PLRP-S column (Varian) heated to 80°C and eluted with a gradient of 30% B to 40% B in 5 minutes. Mobile phase A was H₂O with 0.05% TFA, mobile phase B was acetonitrile with 0.04% TFA. The flow rate was 0.5 ml/min. Protein elution was monitored by UV absorbance detection at A 280nm prior to electrospray ionization and TOF analysis. Baseline chromatographic resolution of naked light chain, residual naked heavy chain and drugged heavy chain was achieved. The obtained m/z spectra were deconvoluted using Agilent Mass Hunter(TM) software to calculate the mass of the reduced antibody fragments.

Molecular weight (MW) of MP-PEG8-Val-Ala-PAB-(imp)PBD **58** (Figure 1) = 1964 daltons Observed deconvoluted masses:
23440 daltons corresponds to MW of naked LC
50627 daltons corresponds to MW of naked HC
52591 daltons corresponds to MW of drugged HC

Thus, the observed peak at 52591 daltons corresponds to the expected heavy chain (HC) fragment (50627 daltons) bearing one drug moiety, drug-linker intermediate 58 (1964 daltons).

When the antibody for conjugation to a PBD drug-linker intermediate is not a cysteine-engineered antibody, the inter-chain disulfide bonds are partially reduced by the addition of about a 2.2 molar excess of TCEP in phosphate pH 7.5 for 2 hourrs at 37°C. Each equivalent of TCEP theoretically results in 2 reactive cysteines. Typically for a target drug/antibody ratio (DAR) of about 3.5, a 1.8-2 molar excess of TCEP is added. No purification step is typically needed following the reduction. A slight excess (1.2-1.5 X) of drug-linker intermediate to reactive cysteines, about 8 molar equivalents of drug-linker intermediate to antibody, is added and the reaction is carried out for about 1 hour at room temperature. Purification may be conducted by diafiltration, ion exchange or gel filtration. The DAR may be determined by hydrophobic-interaction chromatography (HIC), or LC-MS of the reduced conjugate, using UV A280 area integration.

### In vitro cell proliferation assay

Efficacy of ADC were measured by a cell proliferation assay employing the following protocol (CellTiter Glo Luminescent Cell Viability Assay, Promega Corp. Technical Bulletin TB288; Mendoza et al (2002) Cancer Res. 62:5485-5488):
1. An aliquot of 100 µl of cell culture containing about 10⁴ cells (for example, KPL-4, a human breast cancer cell line, Kurebayashi et al (1999) Brit. Jour. Cancer 79(5-6):707-717), SKBR-3, BT474, MCF7 or MDA-MB-468) in medium was deposited in each well of a 96-well, opaque-walled plate.
2. Control wells were prepared containing medium and without cells.
3. ADC was added to the experimental wells and incubated for 3-5 days.
4. The plates were equilibrated to room temperature for approximately 30 minutes.
5. A volume of CellTiter-Glo Reagent equal to the volume of cell culture medium present in each well was added.
6. The contents were mixed for 2 minutes on an orbital shaker to induce cell lysis.
7. The plate was incubated at room temperature for 10 minutes to stabilize the luminescence signal.
8. Luminescence was recorded and reported in graphs as RLU = relative luminescence units.
Certain cells are seeded at 1000-2000/well or 2000-3000/wellin a 96-well plate, 50 uL/well. After one or two days, ADC are added in 50 µL volumes to final concentration of 9000, 3000, 1000, 333, 111, 37, 12.4, 4.1, or 1.4 ng/mL, with "no ADC" control wells receiving medium alone. Conditions are in duplicate or triplicate After 3-5 days, 100 µL/well Cell TiterGlo II is added (luciferase-based assay; proliferation measured by ATP levels) and cell counts are determined using a luminometer. Data are plotted as the mean of luminescence for each set of replicates, with standard deviation error bars. The protocol is a modification of the CellTiter Glo Luminescent Cell Viability Assay (Promega):
1. Plate 1000 cells/ well in 50 µL/well of FBS/glutamine media. Allow cells to attach overnight.
2. ADC is serially diluted 1:3 in media beginning at at working concentration 18 µg/ml (this results in a final concentration of 9 µg/ml). 50 µL of diluted ADC is added to the 50 µL of cells and media already in the well.
3. Incubate 72-96 hrs (the standard is 72 hours, but watch the 0 ug/mL concentration to stop assay when the cells are 85-95% confluent).
4. Add 100 µL/well of Promega Cell Titer Glo reagent, shake 3 min. and read on luminometer

### Results

Figure 2 shows a plot of SK-BR-3 in vitro cell viability at 5 days versus concentrations of: Tr-MP-PEG8-Phe-Lys-PAB-PBD **101** (●), antiCD22-MP-PEG8-Phe-Lys-PAB-PBD **102** (▲), Tr-MP-PEG4-Phe-Lys-PAB-PBD **103** (◆), and antiCD22-MP-PEG4-Phe-Lys-PAB-PBD **104,** where Tr is anti HER2 thio trastuzumab 4D5 HC A118G, Heavy chain cysteine engineered antibody mutants are numbered by the Sequential numbering scheme.

Proliferation of the HER2 expressing SK-BR-3 cells is inhibited selectively by the antiHER2 antibody-drug conjugates **101** and **103,** but not by the antiCD22 antibody drug conjugates **102** and **104.** These results confirm the target-dependent, selective killing effect in vitro of the PBD antibody-drug conjugates.

Figure 3 shows a plot of KPL-4 in vitro cell viability at 5 days versus concentrations of: Tr-MP-PEG8-Phe-Lys-PAB-PBD **101** (●), antiCD22-MP-PEG8-Phe-Lys-PAB-PBD **102** (▲), Tr-MP-PEG4-Phe-Lys-PAB-PBD **103** (◆), and antiCD22-MP-PEG4-Phe-Lys-PAB-PBD **104** (▼), where Tr is anti HER2 thio trastuzumab 4D5 HC A118C, Heavy chain cysteine engineered antibody mutants are numbered by the Sequential numbering scheme.

Antibody-drug conjugates, trastuzumab-MP-PEG8-Val-Ala-PAB-PBD **114** and trastuzumab-MP-PEGB-Val-Ala-PAB-(imp)PBD **115** were tested against SK-BR-3, KPL-4, and MPF-7 (Levenson et al (1997) Cancer Res. 57(15):3071-3078) cells to measure *in vitro* cell viability in five day studies. The IC₅₀ (µg/mL) value for **114** against SK-BR-3 was 17.2 and against KPL-4 was 68.1. The IC₅₀ value for **115** against SK-BR-3 was 12.3 and against KPL-4 was 50.7. Both **114** and **115** were effectively inactive against MCF-7, which is a HER2 non-expressing human breast adenocarcinoma cell line. Thus, conjugates **114** and **115** demonstrate targetted cell killing potency.

### Tumor growth inhibition, in vivo efficacy in high expressing HER2 transgenic explant mice.

Animals suitable for transgenic experiments can be obtained from standard commercial sources such as Taconic (Germantown, N.Y.). Many strains are suitable, but FVB female mice are preferred because of their higher susceptibility to tumor formation. FVB males were used for mating and vasectomized CD.1 studs were used to stimulate pseudopregnancy. Vasectomized mice can be obtained from any commercial supplier. Founders were bred with either FVB mice or with 129/BL6 x FVB p53 heterozygous mice. The mice with heterozygosity at p53 allele were used to potentially increase tumor formation. However, this has proven unnecessary. Therefore, some F1 tumors are of mixed strain. Founder tumors are FVB only. Six founders were obtained with some developing tumors without having litters.

Animals having tumors (allograft propagated from Fo5 mmtv transgenic mice) were treated with a single or multiple dose by IV injection of ADC. Tumor volume was assessed at various time points after injection.

Tumors arise readily in transgenic mice that express a mutationally activated form of neu, the rat homolog of HER2, but the HER2 that is overexpressed in human breast cancers is not mutated and tumor formation is much less robust in transgenic mice that overexpress nonmutated HER2 (Webster et al (1994) Semin. Cancer Biol. 5:69-76).

To improve tumor formation with nonmutated HER2, transgenic mice were produced using a HER2 cDNA plasmid in which an upstream ATG was deleted in order to prevent initiation of translation at such upstream ATG codons, which would otherwise reduce the frequency of translation initiation from the downstream authentic initiation codon of HER2 (for example, see Child et al (1999) J. Biol. Chem. 274: 24335-24341). Additionally, a chimeric intron was added to the 5' end, which should also enhance the level of expression as reported earlier (Neuberger and Williams (1988) Nucleic Acids Res. 16:6713; Buchman and Berg (1988) Mol. Cell. Biol. 8:4395; Brinster et al (1988) Proc. Natl. Acad. Sci. USA 85:836). The chimeric intron was derived from a Promega vector, Pci-neo mammalian expression vector (bp 890-1022). The cDNA 3'-end is flanked by human growth hormone exons 4 and 5, and polyadenylation sequences. Moreover, FVB mice were used because this strain is more susceptible to tumor development. The promoter from MMTV-LTR was used to ensure tissue-specific HER2 expression in the mammary gland. Animals were fed the AIN 76A diet in order to increase susceptibility to tumor formation (Rao et al (1997) Breast Cancer Res. and Treatment 45:149-158).

### Fo5 murine mammary tumor model

The Fo5 model is a transgenic mouse model in which the human HER2 gene, under transcriptional regulation of the murine mammary tumor virus promoter (MMTV-HER2), is overexpressed in mammary epithelium. The overexpression causes spontaneous development of mammary tumors that overexpress the human HER2 receptor. The mammary tumor of one of the founder animals (founder #5 [Fo5]) has been propagated in subsequent generations of FVB mice by serial transplantation of tumor fragments. Before being used for an in vivo efficacy study, the MMTV-HER2 Fo5 transgenic mammary tumor was surgically transplanted into the No. 2/3 mammary fat pad of nu/nu mice (from Charles River Laboratories) in fragments that measured approximately 2x2 mm. When tumors reached desired volumes, the tumor-bearing mice were randomized and given a single dose by IV injection of the ADC.

### Results

Figure 4 shows a plot of the in vivo mean tumor volume change over time in breast cancer-model MMTV-HER2 Fo5 mammary allograft tumors inoculated into CRL nu/nu mice after single iv dosing on day 0 with: (1) Vehicle 20mM Histidine acetate, pH 5.5, 240mM sucrose, (2) antiSteap1-MP-PEG8-Phe-Lys-PAB-PBD **107** at 10 /mg/kg, (3) antiSteap1-MP-PEG4-Phe-Lys-PAB-PBD **108** at 10 mg/kg, (4) Tr-MP-PEG8-Phe-Lys-PAB-PBD **101** at 10 mg/kg, and (5) Tr-MP-PEG4-Phe-Lys-PAB-PBD **103** at 10 mg/kg. The lines in the figure are indicated with the following symbols:
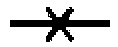 Vehicle
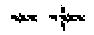 **107**
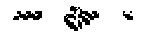 **108**
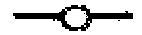 **101**
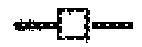 **103**

The anti-HER2 conjugates **101** and **103** showed target-specific tumor growth inhibition. From the 10 animals treated with conjugate **101,** two showed partial responses. From the 10 animals treated with conjugate **103,** three showed partial responses. Non-targeted control ADC **107** and **108** had no effect on tumor growth.

In another exemplary study, in vivo mean tumor volume change over time in breast cancer-model MMTV-HER2 Fo5 mammary allograft tumors inoculated into CRL nu/nu mice was measured after single iv dosing on day 0 with: (1) Vehicle 20mM Histidine acetate, pH 5.5, 240mM sucrose; (2) **112** gD5B60-MP-PEG8-Val-Ala-PAB-PBD at 5 mg/kg (ADC dose), 300 µg/m² (PBD drug exposure); (3) **112** gD5B60-MP-PEGB-Val-Ala-PAB-PBD at 10 mg/kg, 600 µg/m²; (4) **114** trastuzumab-MP-PEGB-Val-Ala-PAB-PBD at 5 mg/kg, 284 µg/m²; (5) **114** trastuzumab-MP-PEG8-Val-Ala-PAB-PBD at 10 mg/kg, 569 µg/m²; (6) **113** gD5B60-MP-PEGB-Val-Ala-PAB-(imp)PBD at 10 mg/kg, 807 µg/m²; and (7) **115** trastuzumab-MP-PEG8-Val-Ala-PAB-(imp)PBD at 10 mg/kg, 790 µg/m². Tumor size was measure at 0, 3, 7, and 10 days. After 10 days, animals dosed with: (1) Vehicle showed increasing tumor size and no tumor inhibition in the 10 animal group; (2) **112** showed no partial or complete responses in the 10 animal group; (3) **112** showed no partial or complete responses in the 10 animal group; (4) **114** showed nine partial responses in the 10 animal group; (5) **114** showed ten partial responses in the 10 animal group; (6) **113** showed no partial or complete responses in the 10 animal group; and (7) **115** showed ten partial responses in the 10 animal group. Thus, the antiHER2 targetted ADC **114** and **115** showed targetted tumor inhibition whereas the negative control Vehicle and non-targetted ADC **112** and **113** did not.

### LuCap35V human prostate tumor model

LuCap35V, obtained from University of Washington (Seattle, WA), is an androgen-independent variant of the LuCap35 human prostate explant tumor model (Corey E, Quinn JE, Buhler KR, et al. LuCap35: a new model of prostate cancer progression to androgen independence. The Prostate 2003;55:239-46). The tissue used to establish LuCap35 was isolated from the biopsy of inguinal lymph nodes containing metastatic prostate cancer and subsequently implanted into the flank of the mice (Corey et al. 2003).

The LuCap35V explant model was maintained by serial implantations in castrated male C.B-17 Fox Chase SCID mice for 38 passages at the University of Washington and subsequently in castrated male C.B-17 SCID-beige mice from Charles River Laboratories for continued passages at Genentech. Before being used for an in vivo efficacy study, the LuCap35V tumor pieces (approximately 20-30 mm³) were subcutaneously implanted into the right flank of the castrated male C.B-17 SCID-beige mice. Animals were castrated 2 weeks before tumor implantation to allow time for residual testosterone level to reach zero. When tumors reached desired volumes, the tumor-bearing mice were randomized and given a single dose by IV injection of the ADC.

### Results

Figure 5 shows a plot of the in vivo mean tumor volume change over time in prostate cancer-model LuCap35V xenagraft tumors in castrated male SCID beige mice after single iv dosing on day 0 with (1) Vehicle 20mM Histidine acetate, pH 5.5, 240mM sucrose (▲), (2) antiCD22-MP-PEG8-Val-Ala-PAB-(imp)PBD **110** at 5 mg/kg (●), and (3) antiSteap1-MP-PEG8-Val-Ala-PAB-(imp)PBD **109** at 5 mg/kg (■).

Figure 6 shows a plot of the in vivo mean tumor volume change over time in prostate cancer-model LuCap35V xenograft tumors in castrated male SCID beige mice after single iv dosing on day 0 with (1) Vehicle 20mM Histidine acetate, pH 5.5, 240mM sucrose (A) (2) **107** antiSteap1-MP-PEG8-Phe-Lys-PAB-PBD, 9.8 mg/kg, 60 µg/m2 (■), (3) **107** antiSteap1-MP-PEG8-Phe-Lys-PAB-PBD, 19.5 mg/kg, 120 µg/m² (●), (4) **108** antiSteap1-MP-PEG4-Phe-Lys-PAB-PBD, 3.3 mg/kg, 60 µg/m² (c), (5) **108** antiSteap1-MP-PEG4-Phe-Lys-PAB-PBD, 6.5 mg/kg, 120 µg/m² (o), (6) **105** trastuzumab-MP-PEG8-Phe-Lys-PAB-PBD 9.4 mg/kg, 120 µg/m² (◆) and (7) **106** trastuzumab-MP-PEG4-Phe-Lys-PAB-PBD, 8.6 mg/kg (ADC dose), 120 µg/m² (PBD drug exposure) (X).
In another exemplary study, in vivo mean tumor volume change over time in prostate cancer-model LuCap35V xenagraft tumors in castrated male SCID beige mice was measured after single iv dosing on day 0 with (1) Vehicle 20mM Histidine acetate, pH 5.5, 240mM sucrose (2) **112** gD5860-MP-PEG8-Val-Ala-PAB-PBD, 3 mg/kg (ADC dose), 68.3 µg/m² (PBD drug exposure), (3) **111** antiSteap1-MP-PEG8-Val-Ala-PAB-PBD, 1 mg/kg, 22.15 µg/m², (4) **111** antiSteap1-MP-PEG8-Val-Ala-PAB-PBD, 3 mg/kg, 66.4 µg/m², (5) **113** gD5B60-MP-PEG8-Val-Ala-PAB-(imp)PBD, 3 mg/kg, 70.1 µg/m², and (6) **109** antiSteap1-MP-PEG8-Val-Ala-PAB-(imp)PBD 3 mg/kg, 64.6 µg/m². Tumor size was measured every 4 days. After 27 days, animals dosed with: (1) Vehicle showed increasing tumor size and no tumor inhibition in the 8 animal group; (2) **112** showed one partial response out of the 8 animal group; (3) **111** showed four partial responses and four complete responses in the 6 animal group; (4) **111** showed five partial responses and three complete responses in the 5 animal group; (5) **113** showed no partial or complete responses in the 8 animal group; and (6) **109** showed seven partial responses and one complete response in the 7 animal group. The the antiSteap1 targetted ADC **109** and **111** showed targetted tumor inhibition whereas , the negative control Vehicle and non-targetted ADC **112** and **113** did not.

### Abbreviations

- Ac: acetyl
- Acm: acetamidomethyl
- Alloc: allyloxycarbonyl
- Boc: di-*tert*-butyl dicarbonate
- t-Bu: tert-butyl
- Bzl: benzyl, where Bzl-OMe is methoxybenzyl and Bzl-Me is methylbenzene
- Cbz or Z: benzyloxy-carbonyl, where Z-Cl and Z-Br are chloro- and bromobenzyloxy carbonyl respectively
- DMF: *N,N*-dimethylformamide
- Dnp: dinitrophenyl
- DTT: dithiothreitol
- Fmoc: 9*H*-fluoren-9-ylmethoxycarbonyl
- imp: *N*-10 imine protecting group: 3-(2-methoxyethoxy)propanoate-Val-Ala-PAB
- MC-OSu: maleimidacaproyl-O-*N-*succinimide
- Moc: methoxycarbonyl
- MP: maleimidopropanamide
- Mtr: 4-methoxy-2,3,6-trimethtylbenzenesulfonyl
- PAB: para-aminobenzyloxycarbonyl
- PEG: ethyleneoxy
- PNZ: *p*-nitrobenzyl carbamate
- Psec: 2-(phenylsulfonyl)ethoxycarbonyl
- TBDMS: tert-butyldimethylsilyl
- TBDPS: tert-butyldiphenylsilyl
- Teoc: 2-(trimethylsilyl)ethoxycarbonyl
- Tos: tosyl
- Troc: 2,2,2-trichlorethoxycarbonyl chloride
- Trt: trityl
- Xan: xanthyl

### References

The following references are incorporated by reference in their entirety:
EP 0522868
EP 0875569
EP 1295944
EP 1347046
EP 1394274
EP 1394274
EP 1439393
JP 05003790
JP 2004113151
JP 58180487
US 2001/055751
US 2002/034749
US 2002/042366
US 2002/150573
US 2002/193567
US 2003/0228319
US 2003/060612
US 2003/064397
US 2003/065143
US 2003/091580
US 2003/096961
US 2003/105292
US 2003/109676
US 2003/118592
US 2003/119121
US 2003/119122
US 2003/119125
US 2003/119126
US 2003/119128
US 2003/119129
US 2003/119130
US 2003/119131
US 2003/124140
US 2003/124579
US 2003/129192
US 2003/134790-A1
US 2003/143557
US 2003/157089
US 2003/165504
US 2003/185830
US 2003/186372
US 2003/186373
US 2003/194704
US 2003/206918
US 2003/219806
US 2003/224411
US 2003/224454
US 2003/232056
US 2003/232350
US 20030096743
US 20030130189
US 2003096743
US 2003130189
US 2004/0001827
US 2004/005320
US 2004/005538
US 2004/005563
US 2004/005598
US 2004/0101899
US 2004/018553
US 2004/022727
US 2004/044179
US 2004/044180
US 2004/101874
US 2004/197325
US 2004/249130
US 20040018194
US 20040052793
US 20040052793
US 20040121940
US 2005/271615
US 2006/116422
US 4816567
US 5362852
US 5440021
US 5583024
US 5621002
US 5644033
US 5674713
US 5700670
US 5773223
US 5792616
US 5854399
US 5869445
US 5976551
US 6011146
US 6153408
US 6214345
US 6218519
US 6268488
US 6518404
US 6534482
US 6555339
US 6602677
US 6677435
US 6759509
US 6835807
US 7223837
US 7375078
US 7521541
US 7723485
WO 00/012508
WO 00/12507
WO 00/12508
WO 01/16318
WO 01/45746
WO 02/088172
WO 03/026577
WO 03/043583
WO 04/032828
WO 2000/12130
WO 2000/14228
WO 2000/20579
WO 2000/22129
WO 2000/32752
WO 2000/36107
WO 2000/40614
WO 2000/44899
WO 2000/55351
WO 2000/75655
WO 200053216
WO 2001/00244
WO 2001/38490
WO 2001/40269
WO 2001/40309
WO 2001/41787
WO 2001/46232
WO 2001/46261
WO 2001/48204
WO 2001/53463
WO 2001/57188
WO 2001/62794
WO 2001/66689
WO 2001/72830
WO 2001/72962
WO 2001/75177
WO 2001/77172
WO 2001/88133
WO 2001/90304
WO 2001/94641
WO 2001/98351
WO 2002/02587
WO 2002/02624
WO 2002/06317
WO 2002/06339
WO 2002/101075
WO 2002/10187
WO 2002/102235
WO 2002/10382
WO 2002/12341
WO 2002/13847
WO 2002/14503
WO 2002/16413
WO 2002/16429
WO 2002/22153
WO 2002/22636
WO 2002/22660
WO 2002/22808
WO 2002/24909
WO 2002/26822
WO 2002/30268
WO 2002/38766
WO 2002/54940
WO 2002/59377
WO 2002/60317
WO 2002/61087;
WO 2002/64798
WO 2002/71928
WO 2002/72596
WO 2002/78524
WO 2002/81646
WO 2002/83866
WO 2002/86443
WO 2002/88170
WO 2002/89747
WO 2002/92836
WO 2002/94852
WO 2002/98358
WO 2002/99074
WO 2002/99122
WO 2003/000842
WO 2003/002717
WO 2003/003906
WO 2003/003984
WO 2003/004989
WO 2003/008537
WO 2003/009814
WO 2003/014294 .
WO 2003/016475
WO 2003/016494
WO 2003/018621
WO 2003/022995
WO 2003/023013
WO 2003/024392
WO 2003/025138
WO 2003/025148
WO 2003/025228
WO 2003/026493
WO 2003/029262
WO 2003/029277
WO 2003/029421
WO 2003/034984
WO 2003/035846
WO 2003/042661
WO 2003/045422
WO 2003/048202
WO 2003/054152
WO 2003/055439
WO 2003/055443
WO 2003/062401
WO 2003/062401
WO 2003/072035
WO 2003/072036
WO 2003/077836
WO 2003/081210
WO 2003/083041
WO 2003/083047
WO 2003/083074
WO 2003/087306
WO 2003/087768
WO 2003/088808
WO 2003/089624
WO 2003/089904
WO 2003/093444
WO 2003/097803
WO 2003/101283
WO 2003/101400
WO 2003/104270
WO 2003/104275
WO 2003/105758
WO 2003004529
WO 2003042661
WO 2003104399
WO 2004/000997
WO 2004/001004
WO 2004/009622
WO 2004/011611
WO 2004/015426
WO 2004/016225
WO 2004/020595
WO 2004/022709
WO 2004/022778
WO 2004/027049
WO 2004/031238
WO 2004/032828
WO 2004/032842
WO 2004/040000
WO 2004/043361
WO 2004/043963
WO 2004/044178
WO 2004/045516
WO 2004/045520
WO 2004/045553
WO 2004/046342
WO 2004/047749
WO 2004/048938
WO 2004/053079
WO 2004/063355
WO 2004/063362
WO 2004/063709
WO 2004/065577
WO 2004/074320
WO 2004000221
WO 2004020583
WO 2004042346
WO 2004065576
WO 2005/023814
WO 2005/082023
WO 2005/085251
WO 2006/111759
WO 2007/044515
WO 2007/085930
WO 2009/052249
WO 2010/091150
WO 91/02536
WO 92/07574
WO 92/17497
WO 94/10312
WO 94/28931
WO 9630514
WO 97/07198
WO 97/44452
WO 98/13059
WO 98/37193
WO 98/40403
WO 98/51805
WO 98/51824
WO 99/28468
WO 99/46284
WO 99/58658

Am. J. Hum. Genet. 49 (3):555-565 (1991)
Amiel J., et al Hum. Mol. Genet. 5, 355-357, 1996
Amir et al (2003) Angew. Chem. Int. Ed. 42:4494-4499
Amsberry, et al (1990) J. Org. Chem. 55:5887
Angew Chem. Intl. Ed. Engl. (1994) 33:183-186
Annu. Rev. Neurosci. 21:309-345 (1998)
Arai H., et al J. Biol. Chem. 268, 3463-3470, 1993
Arai H., et al Jpn. Circ. J. 56, 1303-1307, 1992
Arima, et al., J. Antibiotics, 25, 437-444 (1972)
Attie T., et al, Hum. Mol. Genet. 4, 2407-2409, 1995
Auricchio A., et al Hum. Mol. Genet. 5:351-354, 1996
Barel M., et al Mol. Immunol. 35,1025-1031, 1998
Barella et al (1995) Biochem. J. 309:773-779
Barnett T., et al Genomics 3, 59-66, 1988
Beck et al (1992) J. Mol. Biol. 228:433-441
Beck et al (1996) J. Mol. Biol. 255:1-13
Berge, et al., J. Pharm. Sci., 66, 1-19 (1977)
Biochem. Biophys. Res. Commun. (2000) 275(3):783-788
Biochem. Biophys. Res. Commun. 255 (2), 283-288 (1999)
Blood (2002) 100 (9):3068-3076
Blood 99 (8):2662-2669 (2002)
Blumberg H., et al Cell 104, 9-19, 2001
Bose, et al., Tetrahedron, 48, 751-758 (1992)
Bourgeois C., et al J. Clin. Endocrinol. Metab. 82, 3116-3123, 1997
Brinster et al (1988) Proc. Natl. Acad. Sci. USA 85:836
Buchman and Berg (1988) Mol. Cell. Biol. 8:4395
Cancer Res. 61 (15), 5857-5860 (2001)
Carl et al (1981) J- Med. Chem. 24:479-480
Carlsson et al (1978) Biochem. J. 173:723-737
Carter, P. (2006) Nature Reviews Immunology 6:343-357
Cell 109 (3):397-407 (2002)
CellTiter Glo Luminescent Cell Viability Assay, Promega Corp. Technical Bulletin TB288
Chakravarty et al (1983) J. Med. Chem. 26:638-644
Chan,J. and Watt, V.M., Oncogene 6 (6), 1087-1861 (1991)
Child et al (1999) J. Biol. Chem. 274; 24335-24341
Cho H.-S., et al Nature 421, 756-760, 2803
Ciccodicola, A., et al EMBO J. 8(7): 1987-1991 (1989)
Clackson et al (1991) Nature, 352:624-628
Clark H.F., et al Genome Res. 13, 2265-2270, 2003
Corey E, Quinn JE, Buhler KR, et al. LuCap35: a new model of prostate cancer progression to androgen independence. The Prostate 2003;55:239-46
Coussens L., et al Science (1985) 230(4730):1132-1139
Cree et al (1995) AntiCancer Drugs 6:398-404
Crouch et al (1993) J. Immunol. Meth. 160:81-88
Davis et al (2001) Proc. Natl. Acad. Sci USA 98(17):9772-9777
de Groot et al (2001) J. Org. Chem. 66:8815-8830
de Groot et al (2003) Angew. Chem. Int. Ed. 42:4490-4494
Dennis et al. (2002) "Albumin Binding As A General Strategy For Improving The Pharmacokinetics Of Proteins" J Biol Chem. 277:35035-35043
Dobner et al (1992) Eur. J. Immunol. 22:2795-2799
Dornan et al (2009) Blood 114(13):2721-2729
Doronina et al (2006) Bioconj. Chem. 17:114-124
Dubowchik et al. Bioconjugate Chemistry, 2002, 13,855-869
Dubowchik, et al. (1997) Tetrahedron Letters, 38:5257-60
Dumoutier L., et al J. Immunol. 167, 3545-3549, 2001
E. Schröder and K. Lübke, The Peptides, volume 1, pp 76-136 (1965) Academic Press Ehsani A., et al (1993) Genomics 15, 426-429
Eliel, E. and Wilen, S., "Stereochemistry of Organic Compounds", John Wiley & Sons, Inc., New York, 1994
Elshourbagy N.A., et al J. Biol. Chem. 268, 3873-3879,1993
Erickson et al (2006) Cancer Res. 66(8):1-8
Feild, J.A., et al (1999) Biochem. Biophys. Res. Commun. 258 (3):578-582
Fields, G. and Noble, R. (1990) "Solid phase peptide synthesis utilizing 9-fluoroenylmethoxycarbonyl amino acids", Int. J. Peptide Protein Res. 35:161-214
Fuchs S., et al Mol. Med. 7, 115-124, 2001
Fujisaku et al (1989) J. Biol. Chem. 264 (4):2118-2125)
Gary S.C., et al Gene 256, 139-147, 2000
Gaugitsch, H.W., et al (1992) J. Biol. Chem. 267 (16):11267-11273)
Geiser et al "Automation of solid-phase peptide synthesis" in Macromolecular
Sequencing and Synthesis, Alan R. Liss, Inc., 1988, pp. 199-218
Genome Res. 13 (10):2265-2270 (2003)
Genomics 62 (2):281-284 (1999)
Geoghegan & Stroh, (1992) Bioconjugate Chem. 3:138-146
Getz et al (1999) Anal. Biochem. Vol 273:73-80
Glynne-Jones et al (2001) Int J Cancer. Oct 15; 94(2):178-84
Gregson et al., Chem. Commun. 1999, 797-798
Gregson et al., J. Med. Chem. 2001, 44, 1161-1174
Gu Z., et al Oncogene 19, 1288-1296, 2000
Ha et al (1992) J. Immunol. 148(5):1526-1531
Haendler B., et al J. Cardiovasc. Pharmacol. 20, s1-S4, 1992
Hamann P. (2005) Expert Opin. Ther. Patents 15(9):1087-1103
Hamblett et al (2004) Clin. Cancer Res. 10:7063-7070
Handbook of Pharmaceutical Additives, 2nd Edition (eds. M. Ash and I. Ash), 2001 (Synapse Information Resources, Inc., Endicott, New York, USA)
Handbook of Pharmaceutical Excipients, 2nd edition, 1994
Hara, et al., J. Antibiotics, 41, 702-704 (1988)
Hashimoto et al (1994) Immunogenetics 40(4):287-295
Hay et al. (1999) Bioorg. Med. Chem. Lett. 9:2237
Herdwijn, P. et al., Canadian Journal of Chemistry. 1982, 60, 2903-7
Hermanson, G.T. (1996) Bioconjugate Techniques; Academic Press: New York, p 234-242
Hochlowski, et al., J. Antibiotics, 40, 145-148 (1987) -
Hofstra R.M.W., et al Eur. J. Hum. Genet. 5, 180-185, 1997
Hofstra R.M.W., et al Nat. Genet. 12, 445-447, 1996
Horie et al (2000) Genomics 67:146-152
Hubert, R.S., et al (1999) Proc. Natl. Acad. Sci. U.S.A. 96 (25):14523-14528)
Hurley and Needham-VanDevanter, Acc. Chem. Res., 19, 230-237 (1986)
Immunogenetics 54 (2):87-95 (2002)
Int. Rev. Cytol. 196:177-244 (2000)
Itoh, et al., J. Antibiotics, 41, 1281-1284 (1988)
J. Biol. Chem. 270 (37):21984-21990 (1995)
J. Biol. Chem. 276 (29):27371-27375 (2001)
J. Biol. Chem. 277 (22):19665-19672 (2002)
J. Biol. Chem. 278 (33):30813-30820 (2003)
Janeway, C., Travers, P., Walport, M., Shlomchik (2001) Immuno Biology, 5th Ed., Garland Publishing, New York
Jeffrey et al (2005) J. Med. Chem. 48:1344-1358
Jonsson et al (1989) Immunogenetics 29(6):411-413
Junutula, et al., 2008b Nature Biotech., 26(8):925-932
Kang, G-D., et al., Chem. Commun., 2003, 1680-1689
Kasahara etal (1989) Immunogenetics 30(1):66-68
King et al (2002) Tetrahedron Letters 43:1987-1990
Kingsbury et al (1984) J. Med. Chem. 27:1447
Kohler et al (1975) Nature 256:495
Kohn, in Antibiotics III. Springer-Verlag, New York, pp. 3-11 (1975).
Konishi, et al., J. Antibiotics, 37, 200-206 (1984)
Kovtun et al (2006) Cancer Res. 66(6):3214-3121
Kuhns J.J., et al J. Biol. Chem. 274, 36422-36427, 1999
Kuminoto, et al., J. Antibiotics, 33, 665-667 (1980)
Kurebayashi et al (1999) Brit. Jour. Cancer 79(5-6):707-717
Lab. Invest. 82 (11):1573-1582 (2002)
Lambent J. (2005) Current Opin. in Pharmacol. 5:543-549
Langley and Thurston, J. Org. Chem., 52, 91-97 (1987)
Larhammar et al (1985) J. Biol. Chem. 260(26):14111-14119
Law et al (2006) Cancer Res. 66(4):2328-2337
Le et al (1997) FEBS Lett. 418(1-2):195-199
Leber, et al., J. Am. Chem. Soc., 110, 2992-2993 (1988)
Leimgruber, et al., J. Am. Chem. Soc., 87, 5791-5793 (1965)
Leimgruber, et al., J. Am. Chem. Soc., 87, 5793-5795 (1965)
Levenson et al (1997) Cancer Res. 57(15):3071-3078
Liang et al (2000) Cancer Res. 60:4907-12
Manfré, F. et al., J. Org. Chem. 1992, 57, 2060-2065
Marks et al (1991)J. Mol. Biol., 222:581-597
McDonagh (2006) Protein Eng. Design & Sel., 19(7): 299-307
Mendoza et al (2002) Cancer Res. 62:5485-5488
Miller et al (2003) Jour. of Immunology 170:4854-4861
Miura et al (1996) Genomics 38(3):299-304
Miura et al (1998) Blood 92:2815-2822
Moore M., et al Proc. Natl. Acad. Sci. U.S.A. 84, 9194-9198, 1987
Morrison et al (1984) Proc. Natl. Acad. Sci. USA, 81:6851-6855
Muller et al (1992) Eur. J. Immunol. 22 (6):1621-1625
Mungall A.J., et al Nature 425, 805-811, 2003
Nagase T., et al (2000) DNA Res. 7 (2):143-150)
Nakamuta M., et al Biochem. Biophys. Res. Commun. 177, 34-39, 1991
Nakayama et al (2000) Biochem. Biophys. Res. Commun. 277(1):124-127
Naruse et al (2002) Tissue Antigens 59:512-519
Nature 395 (6699):288-291 (1998)
Neuberger and Williams (1988) Nucleic Acids Res. 16:6713
Novabiochem Catalog 2006/2007
Ogawa Y., et al Biochem. Biophys. Res. Commun. 178, 248-255, 1991
Okamoto Y., et al Biol. Chem. 272, 21589-21596, 1997
Oncogene 10 (5):897-905 (1995)
Oncogene 14(11):1377-1382 (1997))
Parrish-Novak J., et al J. Biol. Chem. 277, 47517-47523, 2002
Payne, G. (2003) Cancer Cell 3:207-212
Pingault V., et al (2002) Hum. Genet. 111, 198-206
Pletnev S., et al (2003) Biochemistry 42:12617-12624
Preud'homme et al (1992) Clin. Exp. Immunol. 90(1):141-146
Proc. Natl. Acad. Sci. U.S.A. (2003) 100 (7):4126-4131
Proc. Natl. Acad. Sci. U.S.A. 93 (1):136-140 (1996)
Proc. Natl. Acad. Sci. U.S.A. 98 (17):9772-9777 (2001)
Proc. Natl. Acad. Sci. U.S.A. 99 (26):16899-16903 (2002) -
Proc.Natl. Acad. Sci. U.S.A. 96 (20):11531-11536 (1999)
Protective Groups in Organic Synthesis, Greene and Wuts, 3rd Edition, 1999, John Wiley & Sons Inc.
Puffenberger E.G., et al Cell 79, 1257-1266, 1994
Rao et al (1997) Breast Cancer Res. and Treatment 45:149-158
Reiter R.E., et al Proc. Natl. Acad. Sci. U.S.A. 95, 1735-1740, 1998
Remington's Pharmaceutical Sciences, 20th edition, pub. Lippincott, Williams & Wilkins, 2000
Rodrigues et al (1995) Chemistry Biology 2:223
Ross et al (2002) Cancer Res. 62:2546-2553
S. P. Parker, Ed., McGraw-Hill Dictionary of Chemical Terms (1984) McGraw-Hill Book Company, New York
Sakaguchi et al (1988) EMBO J. 7(11):3457-3464
Sakamoto A., Yanagisawa M., et al Biochem. Biophys. Res. Commun, 178, 656-663, 1991
Sanderson et al (2005) Clin. Cancer Res. 11:843-852
Semba K., et al Proc. Natl. Acad. Sci. U.S.A. 82, 6497-6501, 1985
Servenius et al (1987) J. Biol. Chem. 262:8759-8766
Shamis et al (2004) J. Am. Chem. Soc. 126:1726-1731
Sheikh F., et al (2004) J. Immunol. 172, 2006-2010
Shimizu, et al, J. Antibiotics, 29, 2492-2503 (1982)
Sinha S.K., et al (1993) J. Immunol. 150, 5311-5320
Storm et al (1972) J. Amer. Chem. Soc. 94:5815
Strausberg et al (2002) Proc. Natl. Acad. Sci USA 99:16899-16903
Sun et al (2002) Bioorganic & Medicinal Chemistry Letters 12:2213-2215
Sun et al (2003) Bioorganic & Medicinal Chemistry 11:1761-1768
Svensson P.J., et al Hum. Genet. 103, 145-148, 1998
Swiercz J.M., et al J. Cell Biol. 165, 869-880, 2004
Syrigos and Epenetos (1999) Anticancer Research 19:605-614
Takeuchi, et al., J. Antibiotics, 29, 93-96 (1976)
Tawaragi Y., et al Biochem. Biophys. Res. Commun. 150, 89-96, 1988
ten Dijke,P., et al Science 264 (5155):101-104 (1994)
Thompson, J.S., et al Science 293 (5537), 2108-2111 (2001) WO 2004/058309
Thurston, et al., Chem. Brit., 26, 767-772 (1990)
Thurston, et al., Chem. Rev. 1994, 433-465 (1994)
Toki et al (2002) J. Org. Chem. 67:1866-1872
Tonnelle et al (1985) EMBO J. 4(11):2839-2847
Touchman et al (2000) Genome Res. 10:165-173
Trail et al (2003) Cancer Immunol. Immunother. 52:328-337
Tsunakawa, et al., J. Antibiotics. 41, 1366-1373 (1988)
Tsutsumi M., et al Gene 228, 43-49, 1999
Uchida et al (1999) Biochem. Biophys. Res. Commun. 266:593-602
Verheij J.B., et al Am. J. Med. Genet. 108, 223-225, 2002
Von Hoegen et al (1990) J. Immunol. 144(12):4870-4877
Webster et al (1994) Semin. Cancer Biol. 5:69-76
Weis J.J., et al J. Exp. Med. 167, 1047-1066, 1988
Weis J.J., et al Proc. Natl. Acad. Sci. U.S.A. 83, 5639-5643, 1986
Wilson et al (1991) J. Exp. Med. 173:137-146
Wu et al (2005) Nature Biotech. 23(9):1137-1145
Xie et al (2006) Expert. Opin. Biol. Ther. 6(3):281-291
Xu, M.J., et al (2001) Biochem. Biophys. Res. Commun. 280 (3):768-775 WO 2004/016225
Xu, X.Z., et al Proc. Natl. Acad. Sci. U.S.A. 98 (19):10692-10697 (2001)
Yamaguchi, N., et al Biol. Chem. 269 (2), 805-808 (1994)
Yamamoto T., et al Nature 319, 230-234,1986
Yu et al (1992) J. Immunol. 148(2) 633-637

The following clauses set out various aspects of the present invention.
1. A conjugate of formula (A): 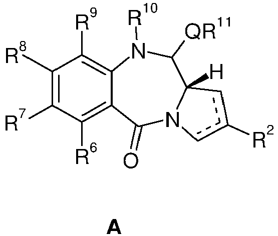 and salts and solvates thereof, wherein:
   the dotted lines indicate the optional presence of a double bond between C1 and C2 or C2 and C3;
   R² is independently selected from H, OH, =O, =CH₂, CN, R, OR, =CH-R^{D}, =C(R^{D})₂, O-SO₂-R, CO₂R and COR, and optionally further selected from halo or dihalo;
   where R^{D} is independently selected from R, CO₂R, COR, CHO, CO₂H, and halo;
   R⁶ and R⁹ are independently selected from H, R, OH, OR, SH, SR, NH₂, NHR, NRR', NO₂, Me₃Sn and halo;
   R⁷ is independently selected from H, R, OH, OR, SH, SR, NH₂, NHR, NRR', NO₂, Me₃Sn and halo;
   R⁸ is independently selected from H, R, OH, OR, SH, SR, NH₂, NHR, NRR', NO₂, Me₃Sn and halo;
   R¹⁰ is a linker connected to a cell binding agent;
   Q is independently selected from O, S and NH;
   R¹¹ is either H, or R or, where Q is O, SO₃M, where M is a metal cation;
   R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
   or any pair of adjacent groups from R⁶ to R⁹ together form a group -O-(CH₂)ₚ-O-, where p is 1 or 2,
   or the compound is a dimer with each monomer being of formula (A), or with one monomer being of formula (A) and the other being of formula (B): 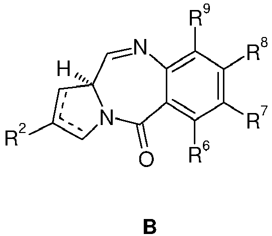
      wherein R², R⁶, R⁹, R⁷, and R⁸ are as defined according to the compounds of formula (A), and the R⁷ groups or R⁸ groups of each monomer form together a dimer bridge having the formula -X-R"-X- linking the monomers;
      wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
   and each X is O, S or N(H);
   or where the compound is a dimer with each monomer being of formula (A), the group R¹⁰ in one of the monomers is either a capping group, R^{C}, or is a linker connected to a cell binding agent.
2. The conjugate of clause 1, wherein R¹⁰ is removable from the N10 position to leave an N10-C11 imine bond.
3. The conjugate of any one of clause 1 or clause 2, wherein R¹⁰ is a group: 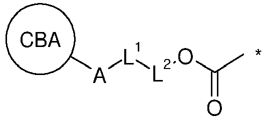 where the asterisk indicates the point of attachment to the N10 position, CBA is a cell binding agent, L¹ is a cleavable linker, A is a connecting group connecting L¹ to the cell binding agent, L² is a covalent bond or together with -OC(=O)- forms a self-immolative linker.
4. The conjugate of clause 5, wherein L¹ is enzyme cleavable.
5. The conjugate of clause 3 or clause 4, wherein L¹ comprises a contiguous sequence of amino acids.
6. The conjugate of clause 5, wherein L¹ comprises a dipeptide and the group -X₁-X₂- in dipeptide, -NH-X₁-X₂-CO-, is selected from:
   -Phe-Lys-,
   -Val-Ala-,
   -Val-Lys-,
   -Ala-Lys-,
   -Val-Cit-,
   -Phe-Cit-,
   -Leu-Cit-,
   -Ile-Cit-,
   -Phe-Arg-,
   -Trp-Cit-.
7. The conjugate according to clause 6, wherein the group -X₁-X₂- in dipeptide, -NH-X₁-X₂-CO-, is selected from:
   -Phe-Lys-,
   -Val-Ala-,
   -Val-Lys-,
   -Ala-Lys-,
   -Val-Cit-.
8. The conjugate according to clause 7, wherein the group -X₁-X₂- in dipeptide, -NH-X₁-X₂-CO-, is -Phe-Lys-, -Val-Ala- or -Val-Cit-.
9. The conjugate according to any one of clauses 6 to 8, wherein the group X₂-CO- is connected to L².
10. The conjugate according to any one of clauses 6 to 9, wherein the group NH-X₁- is connected to A.
11. The conjugate according to any one of clauses 6 to 10, wherein L² together with OC(=O) forms a self-immolative linker.
12. The conjugate according to clause 11, wherein C(=O)O and L² together form the group: 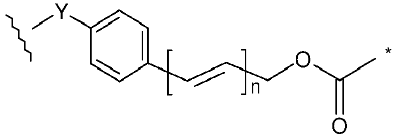 where the asterisk indicates the point of attachment to the N10 position, the wavy line indicates the point of attachment to the linker L¹, Y is NH, O, C(=O)NH or C(=O)O, and n is 0 to 3.
13. The conjugate according to clause 12, wherein Y is NH.
14. The conjugate according to clause 12 or clause 13, wherein n is 0.
15. The conjugate according to clause 3, wherein L¹ and L² together with -OC(=O)-comprise a group selected from: 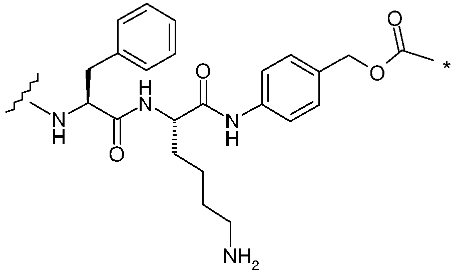 or 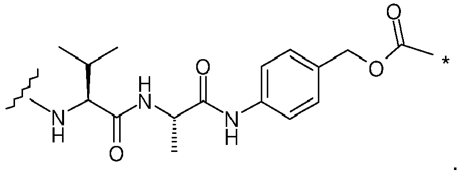 where the asterisk indicates the point of attachment to the N10 position, and the wavy line indicates the point of attachment to the remaining portion of the linker L¹ or the point of attachment to A.
16. The conjugate according to clause 15, wherein the wavy line indicates the point of attachment to A.
17. The conjugate according to any one of clauses 3 to 16, wherein A is:
   (i) 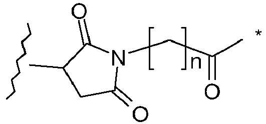 where the asterisk indicates the point of attachment to L¹, the wavy line indicates the point of attachment to the cell binding agent, and n is 0 to 6; or
   (ii) 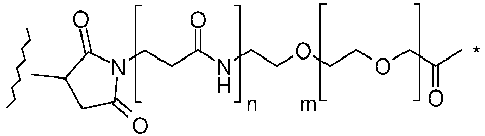 where the asterisk indicates the point of attachment to L¹, the wavy line indicates the point of attachment to the cell binding agent, n is 0 or 1, and m is 0 to 30.
18. The conjugate according to any one of clauses 3 to 17, wherein the cell binding agent is connected to A through a thioether bond formed from a cysteine thiol residue of the cell binding agent and a malemide group of A.
19. The conjugate according to any one of the preceding clauses, wherein the cell binding agent of R¹⁰ is an antibody or an active fragment thereof.
20. The conjugate according to clause 19, wherein the antibody or antibody fragment is an antibody or antibody fragment for a tumour-associated antigen.
21. The conjugate according to any one of the preceding clauses, wherein R⁹ is independently H.
22. The conjugate according to any one of the preceding clauses, wherein R⁶ is independently H.
23. The conjugate according to any one of the preceding clauses, wherein R⁷ is independently OR^{7A}, where R^{7A} is independently optionally substituted C₁₋₄ alkyl.
24. The conjugate of clause 23, wherein R^{7A} is Me.
25. The conjugate according to any one of the preceding clauses, wherein X is O.
26. The conjugate according to any one of the preceding clauses, wherein R¹¹ is H.
27. The conjugate according to any one of the preceding clauses, wherein the dotted lines indicate the optional presence of a double bond between C2 and C3.
28. The conjugate according to any one of the preceding clauses, wherein R² is independently selected from H, =O, =CH₂, R, =CH-R^{D}, and =C(R^{D})₂.
29. The conjugate according to any one of the preceding clause 28, wherein R² is independently =CH₂.
30. The conjugate according to any one of the preceding clause 28, wherein R² is independently R.
31. The conjugate according to any one of the preceding clause 30, wherein R² is independently optionally substituted C₅₋₂₀ aryl.
32. The conjugate according to any one of the preceding clauses, wherein the conjugate is a dimer, and R⁸ groups of each monomer form together the dimer bridge.
33. The conjugate according to clause 32, wherein R" is a C₃ alkylene group or a C₅ alkylene group.
34. The conjugate according to clause 32 or clause 33, wherein the conjugate is a dimer with one monomer being of formula (A) and the other being of formula (B), and the compound having the structure shown below: 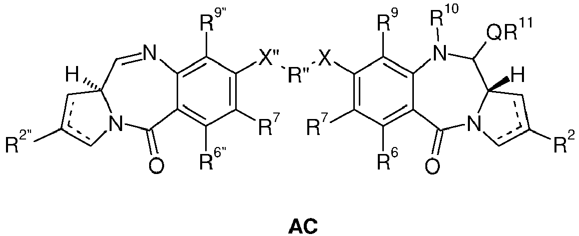 where R^{2"}, R^{6"}, R^{7"}, R^{9"}, X" and R^{11"} and are as defined according to R², R⁶, R⁷, R⁹, X, and R¹¹ respectively.
35. The conjugate according to clause 32 or clause 33, wherein the conjugate is a dimer with each monomer being of formula (A), and the compound having the structure shown below: 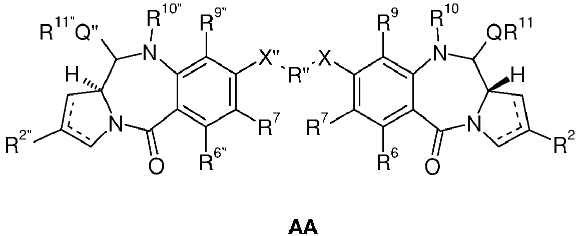 where R^{2"}, R^{6"}, R^{7"} R^{9"}, R^{10"}, X", Q" and R^{11"} and are as defined according to R², R⁶, R⁷, R⁹, R¹⁰, X, and R¹¹ respectively.
36. The conjugate according to clause 32 or clause 33, wherein the conjugate is a dimer with each monomer being of formula (A), and the compound having the structure shown below: 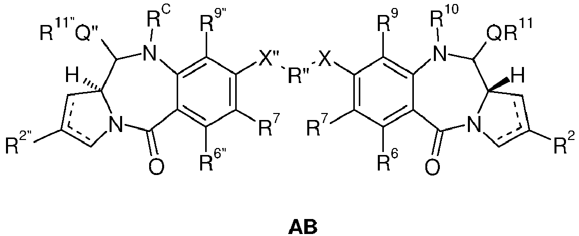 where R^{2"}, R^{6"}, R^{7"}, R^{9"}, X", Q" and R^{11"} and are as defined according to R², R⁶, R⁷, R⁹, X, and R¹¹ respectively, and R^{C} is a capping group.
37. The conjugate according to clause 36, wherein R^{C} is removable from the N10 position to leave an N10-C11 imine bond.
38. The conjugate according to clause 37, wherein R^{C} is a carbamate protecting group selected from:
   Alloc
   Fmoc
   Boc
   Troc
   Teoc
   Psec
   Cbz
   PNZ.
39. The conjugate according to clause 37, wherein R^{C} is a group: 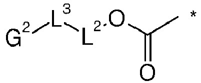 where the asterisk indicates the point of attachment to the N10 position, G² is a terminating group, L³ is a covalent bond or a cleavable linker L¹, L² is a covalent bond or together with OC(=O) forms a self-immolative linker.
40. The conjugate according to clause 39, wherein L³ is a cleavable linker L¹, and is defined in any one of clauses 4 to 9.
41. The conjugate according to clause 39 or clause 40, wherein L² together with OC(=O) forms a self-immolative linker, and the self-immolative linker is as defined in any one of clauses 12 to 14.
42. The conjugate according to any one of clauses 39 to 41, wherein G² is Ac or Moc, or is a carbamate protecting group selected from:
   Alloc
   Fmoc
   Boc
   Troc
   Teoc
   Psec
   Cbz
   PNZ.
43. The conjugate according to any one of the preceding clauses for use in therapy.
44. The conjugate according to any one of clauses 1 to 42, for use in the treatment of a proliferative disease in a subject.
45. The conjugate according to clause 44, wherein the disease is cancer.
46. The conjugate according to according to any one of the preceding clauses, having the formula:

   Ab! (L! D)ₚ

   where Ab is an antibody attached by a linker moiety (L) to the formula (A) PBD drug moiety (D), and p is an integer from 1 to about 8.
47. The conjugate of clause 46 wherein Ab is an antibody which binds to one or more tumor-associated antigens or cell-surface receptors selected from (1)-(36):
   (1) BMPR1 B (bone morphogenetic protein receptor-type IB);
   (2) E16 (LAT1, SLC7A5);
   (3) STEAP1 (six transmembrane epithelial antigen of prostate);
   (4) 0772P (CA125, MUC16);
   (5) MPF (MPF, MSLN, SMR, megakaryocyte potentiating factor, mesothelin);
   (6) Napi3b (NAPI-3B, NPTIIb, SLC34A2, solute carrier family 34 (sodium phosphate), member 2, type II sodium-dependent phosphate transporter 3b);
   (7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B);
   (8) PSCA hlg (2700050C12Rik, C530008O16Rik, RIKEN cDNA 2700050C12, RIKEN cDNA 2700050C12 gene);
   (9) ETBR (Endothelin type B receptor);
   (10) MSG783 (RNF124, hypothetical protein FLJ20315);
   (11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein);
   (12) TrpM4 (BR22450, FLJ20041, TRPM4, TRPM4B, transient receptor potential cation channel, subfamily M, member 4);
   (13) CRIPTO (CR, CR1, CRGF, CRIPTO, TDGF1, teratocarcinoma-derived growth factor);
   (14) CD21 (CR2 (Complement receptor 2) or C3DR (C3d/Epstein Barr virus receptor) or Hs 73792);
   (15) CD79b (CD79B, CD79β, IGb (immunoglobulin-associated beta), B29);
   (16) FcRH2 (IFGP4, IRTA4, SPAP1A (SH2 domain containing phosphatase anchor protein 1a), SPAP1B, SPAP1C);
   (17) HER2;
   (18) NCA;
   (19) MDP;
   (20) IL20R#;
   (21) Brevican;
   (22) EphB2R;
   (23) ASLG659;
   (24) PSCA;
   (25) GEDA;
   (26) BAFF-R (B cell -activating factor receptor, BLyS receptor 3, BR3);
   (27) CD22 (B-cell receptor CD22-B isoform);
   (28) CD79a (CD79A, CD79#, immunoglobulin-associated alpha);
   (29) CXCR5 (Burkitt's lymphoma receptor 1);
   (30) HLA-DOB (Beta subunit of MHC class II molecule (Ia antigen));
   (31) P2X5 (Purinergic receptor P2X ligand-gated ion channel 5);
   (32) CD72 (B-cell differentiation antigen CD72, Lyb-2);
   (33) LY64 (Lymphocyte antigen 64 (RP105), type I membrane protein of the leucine rich repeat (LRR) family);
   (34) FcRH1 (Fc receptor-like protein 1);
   (35) IRTA2 (Immunoglobulin superfamily receptor translocation associated 2); and
   (36) TENB2 (putative transmembrane proteoglycan).
48. The conjugate of clause 46 wherein Ab is a cysteine-engineered antibody.
49. The conjugate of either clause 46 or clause 47 wherein Ab is an antibody which binds to an ErbB receptor.
50. The conjugate of clause 49 wherein Ab is trastuzumab.
51. The conjugate of either clause 46 or clause 47 wherein Ab is an anti-HER2, an anti-Steap1, or an anti-CD22 antibody.
52. The conjugate of any one of clauses 46 to 51 wherein p is 1, 2, 3, or 4.
53. The conjugate of any one of clauses 46 to 52 comprising a mixture of the antibody-drug conjugate compounds, wherein the average drug loading per antibody in the mixture of antibody-drug conjugate compounds is about 2 to about 5.
54. The conjugate of any one of clauses 46 to 53 having a formula selected from: 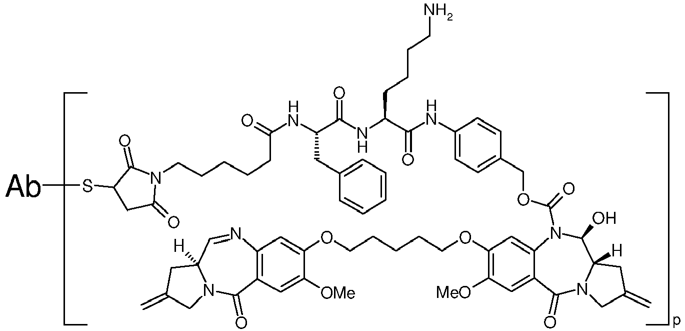 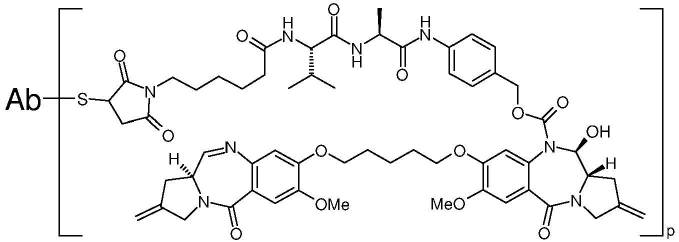 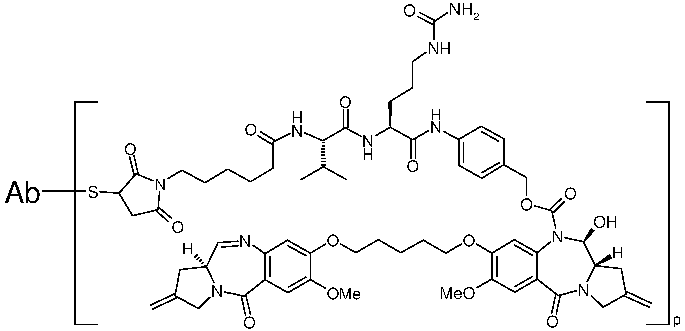 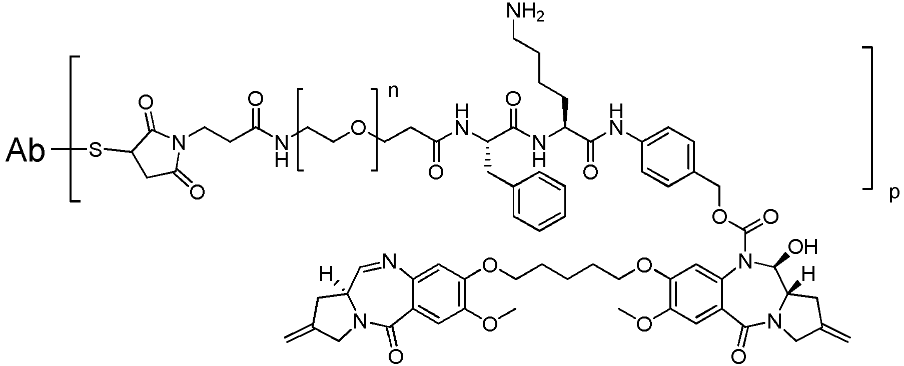 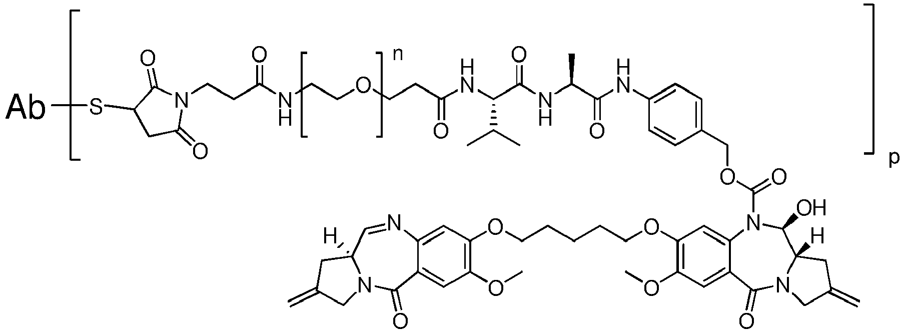 and 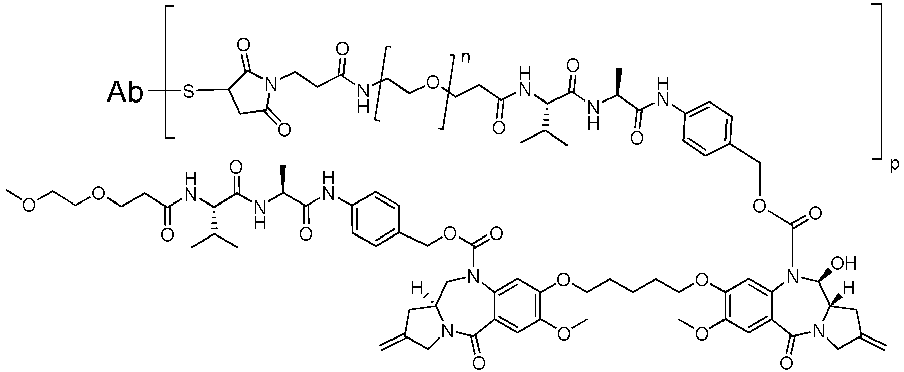 where n is an integer from 1 to 24.
55. The conjugate of clause 54, where n is an integer from 1 to 12.
56. The conjugate of clause 55, where n is 4 or 8.
57. A pharmaceutical composition comprising the conjugate of any one of clauses 1 to 56 a pharmaceutically acceptable diluent, carrier or excipient.
58. The pharmaceutical composition of clause 57 further comprising a therapeutically effective amount of a chemotherapeutic agent.
59. Use of a conjugate according to any one of clauses 1 to 56 in the preparation of a medicament for use in the treatment of a proliferative disease in a subject.
60. A method of treating cancer comprising administering to a patient the pharmaceutical composition of clause 57.
61. The method of clause 60 wherein the patient is administered a chemotherapeutic agent, in combination with the conjugate.
62. Use of a conjugate according to any one of clauses 1 to 56 to provide a compound of formula (C) at a target location: 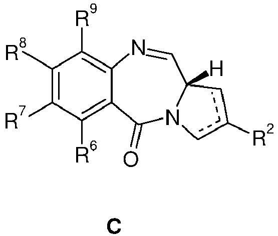 and salts and solvates thereof, wherein:
   the dotted lines indicate the optional presence of a double bond between C1 and C2 or C2 and C3;
   R² is independently selected from H, OH, =O, =CH₂, CN, R, OR, =CH-R^{D}, =C(R^{D})₂, O-SO₂-R, CO₂R and COR, and optionally further selected from halo or dihalo;
   where R^{D} is independently selected from R, CO₂R, COR, CHO, CO₂H, and halo;
   R⁶ and R⁹ are independently selected from H, R, OH, OR, SH, SR, NH₂, NHR, NRR', NO₂, Me₃Sn and halo;
   R⁷ is independently selected from H, R, OH, OR, SH, SR, NH₂, NHR, NRR', NO₂, Me₃Sn and halo;
   R⁸ is independently selected from H, R, OH, OR, SH, SR, NH₂, NHR, NRR', NO₂, Me₃Sn and halo;
   R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
   or any pair of adjacent groups from R⁶ to R⁹ together form a group -O-(CH₂)ₚ-O-, where p is 1 or 2,
   or the compound is a dimer with each monomer being of formula (C), and the R⁷ groups or R⁸ groups of each monomer form together a dimer bridge having the formula -X-R"-X- linking the monomers;
   wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
   and each X is O, S or N(H).
63. The use according to clause 62, wherein the target location is a proliferative cell population.
64. Use of a conjugate according to any one of clauses 1 to 56 to provide a compound of formula (D) at a target location: 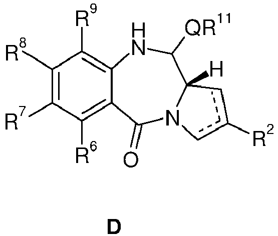 and salts and solvates thereof, wherein:
   the dotted lines indicate the optional presence of a double bond between C1 and C2 or C2 and C3;
   R² is independently selected from H, OH, =O, =CH₂, CN, R, OR, =CH-R^{D}, =C(R^{D})₂, O-SO₂-R, CO₂R and COR, and optionally further selected from halo or dihalo;
   where R^{D} is independently selected from R, CO₂R, COR, CHO, CO₂H, and halo;
   R⁶ and R⁹ are independently selected from H, R, OH, OR, SH, SR, NH₂, NHR, NRR', NO₂, Me₃Sn and halo;
   R⁷ is independently selected from H, R, OH, OR, SH, SR, NH₂, NHR, NRR', NO₂, Me₃Sn and halo;
   R⁸ is independently selected from H, R, OH, OR, SH, SR, NH₂, NHR, NRR', NO₂, Me₃Sn and halo;
   Q is independently selected from O, S and NH;
   R¹¹ is either H, or R or, where Q is O, R¹¹ is SO₃M, where M is a metal cation;
   R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
   or the compound is a dimer with each monomer being of formula (D), or with one monomer being of formula (D) and the other being of formula (C);
   and the R⁷ groups or R⁸ groups of each monomer form together a dimer bridge having the formula -X-R"-X- linking the monomers;
   wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
   and each X is O, S or N(H);
   or any pair of adjacent groups from R⁶ to R⁹ together form a group -O-(CH₂)ₚ-O-, where p is 1 or 2;
   wherein the monomer unit of formula (C) is as defined in clause 58.
65. A compound of formula (E): 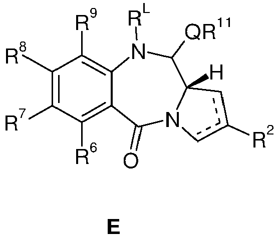 and salts and solvates thereof, wherein
   the dotted lines indicate the optional presence of a double bond between C1 and C2 or C2 and C3;
   R² is independently selected from H, OH, =O, =CH₂, CN, R, OR, =CH-R^{D}, =C(R^{D})₂, O-SO₂-R, CO₂R and COR, and optionally further selected from halo or dihalo;
   where R^{D} is independently selected from R, CO₂R, COR, CHO, CO₂H, and halo;
   R⁶ and R⁹ are independently selected from H, R, OH, OR, SH, SR, NH₂, NHR, NRR', NO₂, Me₃Sn and halo;
   R⁷ is independently selected from H, R, OH, OR, SH, SR, NH₂, NHR, NRR', NO₂, Me₃Sn and halo;
   R⁸ is independently selected from H, R, OH, OR, SH, SR, NH₂, NHR, NRR', NO₂, Me₃Sn and halo;
   R^{L} is a linker for connection to a cell binding agent;
   Q is independently selected from O, S and NH;
   R¹¹ is either H, or R or, where Q is O, R¹¹ is SO₃M, where M is a metal cation;
   R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
   or any pair of adjacent groups from R⁶ to R⁹ together form a group -O-(CH₂)ₚ-O-, where p is 1 or 2;
   or the compound is a dimer with each monomer being of formula (E), or with one monomer being of formula (E) and the other being of formula (B): 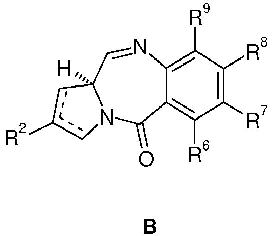
      wherein R², R⁶, R⁹, R⁷, and R⁸ are as defined according to the compounds of formula (A), and the R⁷ groups or R⁸ groups of each monomer form together a dimer bridge having the formula -X-R"-X- linking the monomers;
      wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂;
   and each X is O, S or N(H);
   and where the compound is a dimer with each monomer being of formula (E), the group R^{L} in one of the monomers is either a capping group, R^{C}, or is a linker for connection to a cell binding agent.
66. The compound of clause 65 having the structure: 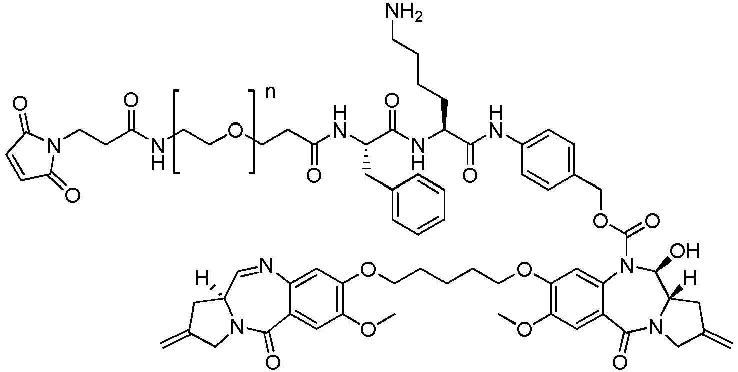 where n is an integer from 1 to 24.
67. The compound of clause 66, where n is an integer from 1 to 12.
68. The compound of clause 67 where n is 4 or 8.
69. The compound of clause 65 having the structure: 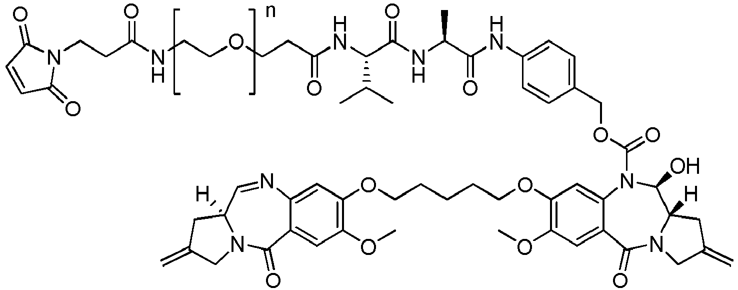 where n is an integer from 1 to 24.
70. The compound of clause 69 where n is an integer from 1 to 12.
71. The compound of clause 70 where n is 4 or 8.
72. The compound of clause 65 having the structure: 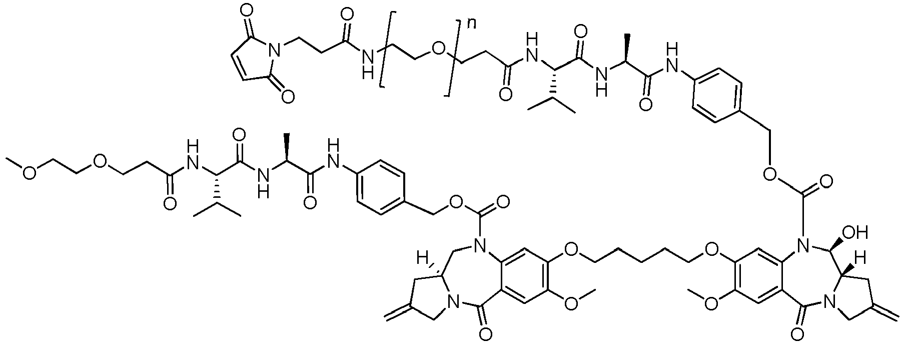 where n is an integer from 1 to 24.
73. The compound of clause 72 where n is an integer from 1 to 12.
74. The compound of clause 73 where n is 4 or 8.
75. The compound of clause 65, which is selected from:
   (i) 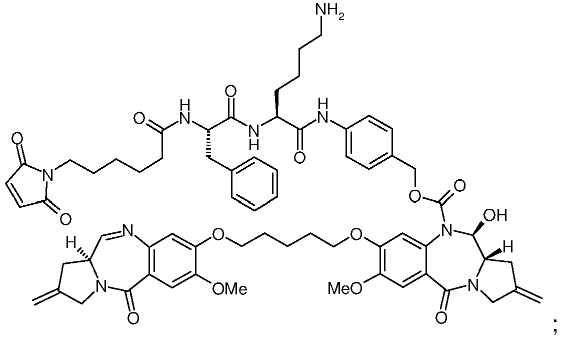
   (ii) 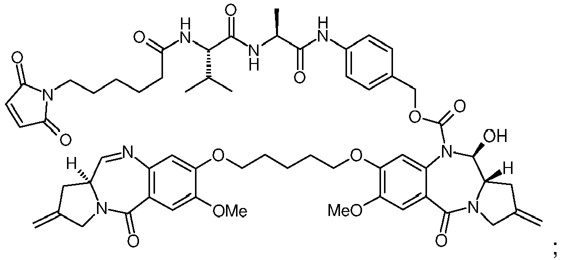 and
   (iii) 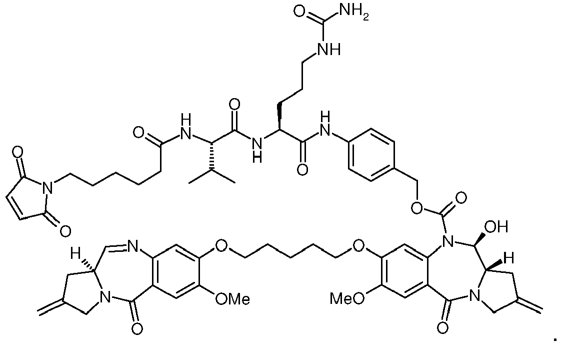
76. A method of preparing a conjugate according to any one of clauses 1 to 56, the method comprising the step of reacting a cell binding agent with compound (E) as defined in any one of clauses 65 to 75.
77. An article of manufacture comprising a pharmaceutical composition of clause 57; a container; and a package insert or label indicating that the pharmaceutical composition can be used to treat cancer.

## Claims

1. A conjugate of Formula (AC): 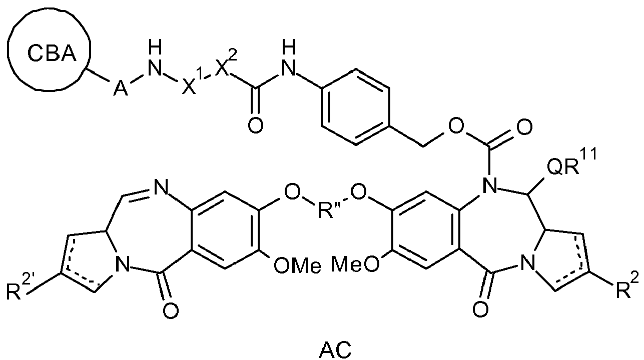 and salts and solvates thereof, wherein:
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
CBA is a cell binding agent selected from an antibody and a fragment of an antibody that contains at least one binding site;
-X₁-X₂- are selected from:
-Phe-Lys-,
-Val-Ala-,
-Val-Lys-,
-Ala-Lys-,
-Val-Cit-,
-Phe-Cit-,
-Leu-Cit-,
-Ile-Cit-,
-Phe-Arg-,
-Trp-Cit-;
A is a connecting group,
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
R" is a C₃₋₁₂ alkylene group; and
wherein R^{2"} and R^{7"} as defined according to R² and R⁷ respectively.

2. The conjugate according to claim 1, wherein -X₁-X₂- is selected from -Phe-Lys-, -Val-Ala- or -Val-Cit-.

3. The conjugate according to claim 2, wherein -X₁-X₂- is -Phe-Lys or -Val-Ala-.

4. The conjugate according to any one of claims 1 to 3, wherein A is:
(i) 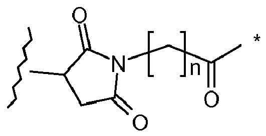 where the asterisk indicates the point of attachment to L¹, the wavy line indicates the point of attachment to the cell binding agent, and n is 0 to 6; or
(ii) 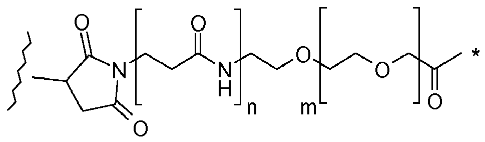 where the asterisk indicates the point of attachment to L¹, the wavy line indicates the point of attachment to the cell binding agent, n is 0 or 1, and m is 0 to 30.

5. The conjugate according to claim 4, wherein A is: 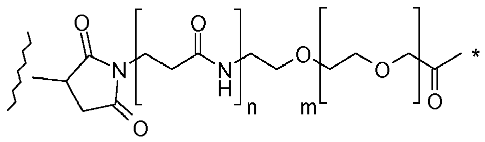 n is 1, and m is 4 or 8.

6. The conjugate according to any one of the preceding claims, wherein Q is O.

7. The conjugate according to any one of the preceding claims, wherein R¹¹ is H.

8. The conjugate according to any one of the preceding claims, wherein the dotted lines indicate the optional presence of a double bond between C2 and C3.

9. The conjugate according to any one of the preceding claims, wherein R² is independently selected from H, =O, =CH₂, R, =CH-R^{D}, and =C(R^{D})₂.

10. The conjugate according to claim 9, wherein R² is independently =CH₂.

11. The conjugate according to claim 9, wherein R² is independently R.

12. The conjugate according to any one of claims 1 to 11, wherein R" is a C₃ alkylene group or a C₅ alkylene group.

13. The conjugate according to any one of claims 1 to 12, wherein the antibody or antibody fragment is an antibody or antibody fragment for a tumour-associated antigen.

14. The conjugate according to any one of the preceding clauses for use in therapy.

15. The conjugate according to any one of claims 1 to 13, for use in the treatment of a proliferative disease in a subject, wherein the disease is cancer.
